# EUROPEAN PATENT SPECIFICATION

(11) **EP 3 185 885 B1**
(45) Date of publication and mention of the grant of the patent: **19.08.2020**
(21) Application number: 15836989.2
(22) Date of filing: 26.08.2015
(51) Int. Cl.: C07K 16/28, G01N 33/564, A61K 38/04, A61K 38/00, A61K 38/17, A61K 39/00, C07K 14/705, A61K 39/39, G01N 33/68

(54) **POLYPEPTIDES AND USES THEREOF AS A DRUG FOR TREATMENT OF AUTOIMMUNE DISORDERS**
POLYPEPTIDE UND VERWENDUNGEN DAVON ALS ARZNEIMITTEL ZUR BEHANDLUNG VON AUTOIMMUNERKRANKUNGEN
POLYPEPTIDES ET LEURS UTILISATIONS EN TANT QUE MÉDICAMENT POUR LE TRAITEMENT DE TROUBLES AUTO-IMMUNS

(30) Priority: 26.08.2014 US 201462042192 P; 14.04.2015 US 201562147000 P
(43) Date of publication of application: 05.07.2017
(73) Proprietor: Compugen Ltd., 5885849 Holon (IL)
(72) Inventor: HECHT, Iris, 69353 Tel-Aviv (IL); NERIA, Eyal, 69358 Tel Aviv (IL); ROTMAN, Galit, 46412 Herzliya (IL); MILLER, Stephen D., Chicago, Illinois 60611 (US); PODOJIL, Joseph R, Chicago, Illinois 60611 (US)
(74) Representative: Fuchs Patentanwälte Partnerschaft mbB
(86) International application number: PCT/IL2015/050854
(87) International publication number: WO 2016/030888

(56) References cited:
- WO-A1-2013/001517
- WO-A2-2009/032845
- WO-A2-2012/001647
- ROHIT AGGARWAL ET AL: "Anti-citrullinated peptide antibody assays and their role in the diagnosis of rheumatoid arthritis : ACPA Assays in RA", ARTHRITIS & RHEUMATISM, vol. 61, no. 11, 29 October 2009 (2009-10-29), pages 1472-1483, XP055452592, US ISSN: 0004-3591, DOI: 10.1002/art.24827
- ANONYMOUS: 'Compugen Announces Positive therapeutic Effect of CGEN-15001 in Animal Model of Rheumatoid Arthritis' 14 December 2010, XP002677794 Retrieved from the Internet: <URL:http:// www.cgen.com/media-center/press-releases/-1 92> [retrieved on 2015-09-12]

## Description

### FIELD OF THE INVENTION

The present invention, in at least some aspects, relates to a novel protein, and its variants, fragments and fusion proteins thereof, and methods of use thereof for immunotherapy, and drug development.

### BACKGROUND OF THE INVENTION

Induction of immune tolerance has long been considered the "holy grail" for autoimmune disease therapy. The immune system has the reciprocal tasks to protect the host against invading pathogens, but simultaneously to prevent damage resulting from unwanted reactions to self antigens. The latter part is known as immune tolerance and performed by a complex set of interactive and complementary pathways, which regulate immune responses. T cells have the ability to react to a variety of antigens, both self and nonself. Therefore, there are many mechanisms that exist naturally to eliminate potentially self-reactive responses - this is known as natural tolerance. The main mechanism for eliminating potential autoreactive T cells occurs in the thymus and is known as central tolerance. Some potentially autoreactive T cells escape central tolerance and, therefore, peripheral tolerance mechanisms also exist. Despite these mechanisms, some self-reactive T cells may 'escape' and be present in the repertoire; it is believed that their activation may lead to autoimmune disease.

Studies on therapeutic tolerance have attempted to induce and amplify potent physiological mechanisms of tolerance in order to eliminate or neutralize self-reactive T cells and prevent or treat autoimmune diseases. One way to induce tolerance is by manipulation of the interaction between costimulatory ligands and receptors on antigen presenting cells (APCs) and lymphocytes.

CTLA-4 is the most extensively studied costimulatory molecule which down-regulates immune responses. The attributes of immunosuppressive qualities and capacity to induce tolerance have made its recognition as a potential immuno-therapeutic agent for autoimmune mediated inflammatory disorders. Abatacept (commercial name: Orencia) is a fusion protein composed of the ECD (extracellular domain) of CTLA-4 fused to the Fc fragment of hIgG1. Abatacept is believed to induce costimulation blockade, which has been approved for treating patients with rheumatoid arthritis, by effectively interfering with the inflammatory cascade.

Induction of disease control with the current therapies, followed by progressive withdrawal in parallel with re-establishing immune tolerance, may be an attractive approach in the future of autoimmune therapies. Furthermore, due to their immune specificity, in the absence of global immunosuppression, such therapies should be safe for chronic use.

Multiple sclerosis (MS) is a chronic, inflammatory, demyelinating disorder of the central nervous system (CNS), which involves autoimmune responses to myelin antigens. It is characterized by lesions within the CNS and demyelination is a key feature of these lesions. Autoreactive T cells are thought to initiate an autoimmune response directed against components of CNS myelin. The main targets of the autoimmune reactions are thought to be myelin basic protein (MBP), proteolipid protein (PLP) and myelin oligodendrocyte glycoprotein (MOG). Experimental autoimmune encephalomyelitis (EAE), an animal model of MS induced by immunization with myelin components in adjuvant, shows comparable neuronal pathology. Wihout wishing to be limited by a single hypothesis, studies in EAE have provided convincing evidence that T cells specific for self-antigens mediate pathology in these diseases.

T helper type 1 (Th1) cells are induced by IL-12 and produce IFN-γ, while T helper type 2 (Th2) cells secrete IL-4, IL-5 and IL-13. Th1 cells can mediate proinflammatory or cell-mediated immune responses, whereas Th2 cells mainly promote certain types of humoral immunity. Some immune related diseases, such as autoimmune reactions, inflammation, chronic infection and sepsis, are characterized by a dysregulation of the pro-versus anti-inflammatory tendencies of the immune system, as well as an imbanlance in the Th1 versus Th2 cytokine balance. During inflammation, induction of a shift in the balance from Th1 to Th2 protects the organism from systemic 'overshooting' with Th1/proinflammatory cytokines, by reducing the inflammatory tendencies of the immune system. Immunomodulatory therapies that are associated with a Th1 to Th2 immune shift have protective effects in Th1-mediated autoimmune diseases, such as multiple sclerosis and rheumatoid arthritis. For example, Laquinimod, which has demonstrated efficacy in animal models of several autoimmune diseases including MS, shows immunomodulatory effects through Th1/Th2 shift, and does not lead to immunosuppression. Glatiramer acetate (Copaxone) also induces Th1/Th2 shift with decreased secretion of proinflammatory cytokines, and increased secretion of antiinflammatory cytokines. Furtheremore, glatiramer acetate -specific Th2 cells are able to migrate across the blood-brain barrier and cause in situ bystander suppression of autoaggressive Th1 T cells.

FoxP3+ Tregulatory cells (Tregs) are primarily generated in the thymus but also at peripheral sites or induced in cell culture in the presence of TGFβ. Several mechanisms underly the regulatory activity of Tregs, including inhbition of the expansion of differentiated effector T cells, prevention of T cell priming by acting on antigen presenting cells, such as DCs (especially inducible Tregs, iTres), and inhibition of effector T cell trafficking to the target organ (especially thymic Tregs usually designated as tTregs or nTregs). IL-10 plays an important role in the suppressive function of Tregs and/or Bregs by imparing the capacity of DCs to present antigen through increasing MARCH 1 and inhibiting CD83 expression and thus controlling the expression of peptide-MHC class II complexes on DC.

Certain immune cells and immune cell signal transduction pathways are promising targets for new agents for treating immune disorders. For example Th1, Th17, Th2 and regulatory T cells (Tregs), regulatory B cells and antigen presenting cells such as monocytes, macrophages and dendridic cells play important roles in modulating autoimmunity and inflammation. Mounting evidence from numerous studies shows the importance of these immune cells in disorders such as rheumatoid arthritis, inflammatory bowel disease, multiple sclerosis, psoriasis, lupus erythematosus, type 1 diabetes and uveitis. Most existing therapies target only one pathway at a time.

### BRIEF SUMMARY OF THE INVENTION

The background art fails to provide therapies that target multiple cells and pathways involved in autoimmunity, transplant rejection and inflammation, such as Th1, Th17, Th22, Th2, Tregs, Bregs, or other cells that secrete, or influence other cells that secrete, inflammatory molecules such as cytokines, metalloproteases, chemokines and other molecules, and which particularly induce tolerance through the IL-10 and/ or TGFβ pathways.

The present disclosure is of new uses and methods of treatment for immune related diseases by administering the C1ORF32-ECD protein to a subject in need of treatment thereof to induce immune tolerance, preferably through the IL-10 and/ or TGFβ pathways. In the context of immune related diseases, "immune tolerance" refers to reducing, ameliorating or blocking the immune related disease, while leaving intact the disease-fighting abilities of the immune system.

According to the disclosure, there is provided a use of an isolated polypeptide comprising a soluble C1ORF32 polypeptide or fragment or variant thereof, for treatment of an immune related disease, by increasing Treg and/or Breg cell population, and/or percentage, and/or activity.

The term *"immune related disease (or disorder or condition)"* as used herein should be understood to encompass any disease disorder or condition selected from the group including but not limited to autoimmune diseases, inflammatory disorders and immune disorders associated with graft transplantation rejection, such as acute and chronic rejection of organ transplantation, allogenic stem cell transplantation, autologous stem cell transplantation, bone marrow tranplantation, and graft versus host disease.

The term *"autoimmune disease"* as used herein should be understood to encompass any autoimmune disease and chronic inflammatory conditions. According to at least some embodiments of the invention, the autoimmune diseases should be understood to encompass any disease disorder or condition selected from the group including but not limited to multiple sclerosis, including relapsing-remiting multiple sclerosis, primary progressive multiple sclerosis, and secondary progressive multiple sclerosis; multiple sclerosis, psoriasis; rheumatoid arthritis; psoriatic arthritis, systemic lupus erythematosus (SLE); discoid lupus erythematosus, inflammatory bowel disease, ulcerative colitis; Crohn's disease; benign lymphocytic angiitis, thrombocytopenic purpura, idiopathic thrombocytopenia, idiopathic autoimmune hemolytic anemia, pure red cell aplasia, Sjögren's syndrome, rheumatic disease, connective tissue disease, inflammatory rheumatism, degenerative rheumatism, extra-articular rheumatism, juvenile rheumatoid arthritis, arthritis uratica, muscular rheumatism, chronic polyarthritis, cryoglobulinemic vasculitis, ANCA-associated vasculitis, antiphospholipid syndrome, myasthenia gravis, autoimmune hemolytic anaemia, Guillain-Barré syndrome, chronic immune polyneuropathy, autoimmune thyroiditis, insulin dependent diabetes mellitus, type I diabetes, latent autoimmune diabetes of the adult (LADA), type 2 diabetes with an autoimmune component, Addison's disease, membranous glomerulonephropathy, Goodpasture's disease, autoimmune gastritis, autoimmune atrophic gastritis, pernicious anaemia, pemphigus, pemphigus vulgaris, cirrhosis, primary biliary cirrhosis, dermatomyositis, polymyositis, fibromyositis, myogelosis, celiac disease, immunoglobulin A nephropathy, Henoch-Schonlein purpura, Evans syndrome, dermatitis, atopic dermatitis, psoriasis, psoriasis arthropathica, Graves' disease, Graves' ophthalmopathy, scleroderma, systemic scleroderma, progressive systemic scleroderma, asthma, allergy, primary biliary cirrhosis, Hashimoto's thyroiditis, primary myxedema, sympathetic ophthalmia, autoimmune uveitis, hepatitis, chronic action hepatitis, collagen diseases, ankylosing spondylitis, periarthritis humeroscapularis, panarteritis nodosa, chondrocalcinosis, Wegener's granulomatosis, microscopic polyangiitis, chronic urticaria, bullous skin disorders, pemphigoid, bullous pemphigoid, cicatricial pemphigoid, vitiligo, atopic eczema, eczema, chronic urticaria, autoimmune urticaria, normocomplementemic urticarial vasculitis, hypocomplementemic urticarial vasculitis, autoimmune lymphoproliferative syndrome, Devic's disease, sarcoidosis, pernicious anemia, childhood autoimmune hemolytic anemia, idiopathic autoimmune hemolytic anemia, refractory or chronic autoimmune cytopenias, prevention of development of autoimmune anti-factor viii antibodies in acquired hemophilia a, cold agglutinin disease, neuromyelitis optica, stiff person syndrome, gingivitis, periodontitis, pancreatitis, myocarditis, vasculitis, gastritis, gout, gouty arthritis, and inflammatory skin disorders, selected from the group consisting of psoriasis, atopic dermatitis, eczema, rosacea, urticaria, and acne, normocomplementemic urticarial vasculitis, pericarditis, idiopathic pericarditis, myositis, anti-synthetase syndrome, scleritis, macrophage activation syndrome, Behçet's Syndrome, PAPA syndrome, Blau's syndrome, gout, adult and juvenile Still's disease, cryropyrinopathy, Muckle-Wells syndrome, familial cold-induced auto-inflammatory syndrome, neonatal onset multisystemic inflammatory disease, familial Mediterranean fever, chronic infantile neurologic cutaneous and articular syndrome, a rheumatic disease, polymyalgia rheumatica, mixed connective tissue disease, inflammatory rheumatism, degenerative rheumatism, extra-articular rheumatism, juvenile arthritis, juvenile rheumatoid arthritis, systemic juvenile idiopathic arthritis, arthritis uratica, muscular rheumatism, chronic polyarthritis, reactive arthritis, Reiter's syndrome, rheumatic fever, relapsing polychondritis, Raynaud's phenomenon, vasculitis, cryoglobulinemic vasculitis, temporal arteritis, giant cell arteritis, Takayasu arteritis, chronic inflammatory demyelinating polyneuropathy, Crest syndrome, chronic fatigue and immune dysfunction syndrome (CFIDS), autoimmune inner ear disease, hyper IgD syndrome, Schnitzler's syndrome, autoimmune retinopathy, age-related macular degeneration, atherosclerosis, chronic prostatitis, alopecia, alopecia areata, alopecia universalis, alopecia totalis, utoimmune thrombocytopenic purpura, idiopathic thrombocytopenic purpura, pure red cell aplasia, and TNF receptor-associated periodic syndrome (TRAPS).

Optionally and preferably, the autoimmune disease includes but is not limited to any of the types and subtypes of any of multiple sclerosis, rheumatoid arthritis, type I diabetes, psoriasis, systemic lupus erythematosus, inflammatory bowel disease, uveitis, or Sjogren's syndrome.

As used herein, "multiple sclerosis" comprises one or more of multiple sclerosis, benign multiple sclerosis, relapsing remitting multiple sclerosis, secondary progressive multiple sclerosis, primary progressive multiple sclerosis, progressive relapsing multiple sclerosis, chronic progressive multiple sclerosis, transitional/progressive multiple sclerosis, rapidly worsening multiple sclerosis, clinically-definite multiple sclerosis, malignant multiple sclerosis, also known as Marburg's Variant, and acute multiple sclerosis. Optionally, "conditions relating to multiple sclerosis" include, e.g., Devic's disease, also known as Neuromyelitis Optica; acute disseminated encephalomyelitis, acute demyelinating optic neuritis, demyelinative transverse myelitis, Miller-Fisher syndrome, encephalomyelradiculoneuropathy, acute demyelinative polyneuropathy, tumefactive multiple sclerosis and Balo's concentric sclerosis.

As used herein, "rheumatoid arthritis" comprises one or more of rheumatoid arthritis, gout and pseudo-gout, juvenile idiopathic arthritis, juvenile rheumatoid arthritis, Still's disease, ankylosing spondylitis, rheumatoid vasculitis. Optionally, conditions relating to rheumatoid arthritis include, e.g., osteoarthritis, sarcoidosis, Henoch-Schonlein purpura, psoriatic arthritis, reactive arthritis, spondyloarthropathy, septic arthritis, haemochromatosis, hepatitis, vasculitis, Wegener's granulomatosis, Lyme disease, familial mediterranean fever, hyperimmunoglobulinemia D with recurrent fever, TNF receptor associated periodic syndrome, and enteropathic arthritis associated with inflammatory bowel disease.

As used herein, "Uveitis" comprises one or more of uveitis, anterior uveitis (or iridocyclitis), intermediate uveitis (pars planitis), posterior uveitis (or chorioretinitis) and the panuveitic form.

As used herein, "inflammatory bowel disease" comprises one or more of inflammatory bowel disease, Crohn's disease, ulcerative colitis (UC), collagenous colitis, lymphocytic colitis, ischaemic colitis, diversion colitis, Behçet's disease, indeterminate colitis.

As used herein, "psoriasis" comprises one or more of psoriasis, nonpustular psoriasis including psoriasis vulgaris and psoriatic erythroderma (erythrodermic psoriasis), pustular psoriasis including generalized pustular psoriasis (pustular psoriasis of von Zumbusch), pustulosis palmaris et plantaris (persistent palmoplantar pustulosis, pustular psoriasis of the Barber type, pustular psoriasis of the extremities), annular pustular psoriasis, acrodermatitis continua, impetigo herpetiformis. Optionally, conditions relating to psoriasis include, e.g., drug-induced psoriasis, inverse psoriasis, napkin psoriasis, seborrheic-like psoriasis, guttate psoriasis, nail psoriasis, psoriatic arthritis.

As used herein, "type 1 diabetes" comprises one or more of type 1 diabetes, insulin-dependent diabetes mellitus, idiopathic diabetes, juvenile type 1 diabetes, maturity onset diabetes of the young, latent autoimmune diabetes in adults, gestational diabetes. Conditions relating to type 1 diabetes include, neuropathy including polyneuropathy, mononeuropathy, peripheral neuropathy and autonomicneuropathy; eye complications: glaucoma, cataracts, and/or retinopathy.

As used herein, "Sjogren's syndrome" comprises one or more of Sjogren's syndrome, primary Sjogren's syndrome and secondary Sjogren's syndrome, as well as conditions relating to Sjogren's syndrome including connective tissue disease, such as rheumatoid arthritis, systemic lupus erythematosus, or scleroderma. Other complications include pneumonia, pulmonary fibrosis, interstitial nephritis, inflammation of the tissue around the kidney's filters, glomerulonephritis, renal tubular acidosis, carpal tunnel syndrome, peripheral neuropathy, cranial neuropathy, primary biliary cirrhosis (PBC), cirrhosis, inflammation in the esophagus, stomach, pancreas, and liver (including hepatitis), polymyositis, Raynaud's phenomenon, vasculitis, autoimmune thyroid problems, lymphoma.

As used herein, "systemic lupus erythematosus", comprises one or more of systemic lupus erythematosus, discoid lupus, lupus arthritis, lupus pneumonitis, lupus nephritis. Conditions relating to systemic lupus erythematosus include osteoarticular tuberculosis, antiphospholipid antibody syndrome, inflammation of various parts of the heart, such as pericarditis, myocarditis, and endocarditis, lung and pleura inflammation, pleuritis, pleural effusion, chronic diffuse interstitial lung disease, pulmonary hypertension, pulmonary emboli, pulmonary hemorrhage, and shrinking lung syndrome, lupus headache, Guillain-Barré syndrome, aseptic meningitis, demyelinating syndrome, mononeuropathy, mononeuritis multiplex, myasthenia gravis, myelopathy, cranial neuropathy, polyneuropathy, vasculitis.

As used herein the term "inflammatory disorders" and/or "inflammation", used interchangeably, includes inflammatory abnormalities characterized by disregulated immune response to harmful stimuli, such as pathogens, damaged cells, or irritants. Inflammatory disorders underlie a vast variety of human diseases. Non-immune diseases with etiological origins in inflammatory processes include cancer, atherosclerosis, and ischaemic heart disease. Examples of disorders associated with inflammation include: chronic prostatitis, glomerulonephritis, hypersensitivities, pelvic inflammatory disease, reperfusion injury, sarcoidosis, vasculitis, interstitial cystitis, normocomplementemic urticarial vasculitis, pericarditis, myositis, anti-synthetase syndrome, scleritis, macrophage activation syndrome, Bechet's syndrome, PAPA syndrome, Blau's syndrome, gout, adult and juvenile Still's disease, cryropyrinopathy, Muckle-Wells syndrome, familial cold-induced auto-inflammatory syndrome, neonatal onset multisystemic inflammatory disease, familial mediterranean fever, chronic infantile neurologic, cutaneous and articular syndrome, systemic juvenile idiopathic arthritis, hyper IgD syndrome, Schnitzler's syndrome, TNF receptor-associated periodic syndrome (TRAPS), gingivitis, periodontitis, hepatitis, cirrhosis, pancreatitis, myocarditis, vasculitis, gastritis, gout, gouty arthritis, and inflammatory skin disorders, selected from the group consisting of psoriasis, atopic dermatitis, eczema, rosacea, urticaria, and acne.

According to the present disclosure, there are provided C1ORF32-ECD fusion polypeptides having a first fusion partner comprising all or a part of a C1ORF32 soluble polypeptide, such as the ECD of C1ORF32, or a polypeptide comprising all or part of the extracellular domain of ILDR2-WT (SEQ ID NO:1), H19011_1_P8 (SEQ ID NO:2), H19011_{_}1_P8_V1 (SEQ ID NO:3), H19011_1_P9 (SEQ ID NO:4) or H19011_1_P9_V1 (SEQ ID NO:5), or a sequence homologous thereto, and a second fusion partner composed of a heterologous sequence (respectively non-C1ORF32), fused together directly or indirectly via a peptide linker sequence or a chemical linker.

According to the disclosure, the isolated polypeptide is at least 80, 90, 95, 96, 97, 98 or 99% (or any integral value in between) homologous to a polypeptide comprising all or part of the extracellular domain of ILDR2-WT (SEQ ID NO:1), H19011_1_P8 (SEQ ID NO:2), H19011_1_P8_V1 (SEQ ID NO:3), H19011_1_P9 (SEQ ID NO:4) or H19011_1_P9_V1 (SEQ ID NO:5). According to the disclosure, the isolated polypeptide at least 80, 90, 95, 96, 97, 98 or 99% (or any integral value in between) homologous to a polypeptide comprising all or part of the extracellular domain of ILDR2-WT (SEQ ID NO: 1), H19011_1_P8 (SEQ ID NO:2), H19011_1_P8_V1 (SEQ ID NO:3), H19011__1_P9 (SEQ ID NO:4) or H19011_1_P9_V1 (SEQ ID NO:5) has at least one of the SNP variations. The C1ORF32 polypeptide may be of any species of origin. In further embodiments, the C1ORF32 polypeptide is of murine, non-human primate or human origin.

Without wishing to be limited by a single hypothesis, according to at least some embodiments the C1ORF32 fusion protein inhibits the inflammatory activity of Th1, Th17, Th22, or other cells that secrete, or cause other cells to secrete, inflammatory molecules, including, but not limited to, TNF-alpha, IFN-gamma, IL-17, IL-23, IL-22, IL-21, GM-CSF, CCL3, CCL5 and MMPs. Again without wishing to be limited by a single hypothesis, according to at least some embodiments the C1ORF32 fusion protein can also increase the suppressive capacity of Tregs or Bregs or the immunomodulatory activity of Th2 cells. The C1ORF32 fusion protein can also increase the production of anti-inflammatory molecules such as the cytokine IL-10.

According to at least some embodiments, the C1ORF32 fusion protein modulates the IL-10 and/or TGFβ pathway. As shown herein, the C1ORF32 fusion protein upregulates the IL-10 pathway by upregulating IL-10 secretion and also maintains the TGFβ pathway.

According to at least some embodiments, the C1ORF32 fusion protein induces long term immune tolerance. By "long term" it is meant tolerance which lasts any time period between at least 72 hours to 6 months after cessation of treatment, or even greater than 6 months after cessation of treatment; and/or efficacy at a reduced dosing frequency, including but not limited to a dosing frequency of one dose per any time period from every 72 hours to every 6 months.

According to at least some embodiments, the C1ORF32 fusion protein induces tolerance, and preferably long term tolerance as defined above, to graft tissue with at least one mismatched antigen to the recipient subject. Non-limiting examples of such graft tissue include organs and bone marrow. Preferably, the fusion protein induces graft survival and increase in nTregs and/or iTregs, indicating donor specific tolerance induction (Aaron et al. Journal of Immunology, 2010, 185: 3326-3336). Also preferably, such induction of immune tolerance occurs through the IL-10 pathway and/or the TGF-beta pathway. According to at least some embodiments, the C1ORF32 fusion protein also causes an increase in Bregs or otherwise modulates Breg activity levels. Optionally such a mechanism also occurs for autoimmune disease treatment.

According to at least some embodiments, the C1ORF32 fusion protein may optionally include the full extracellular domain (ECD) of C1ORF32, or a fragment, or a homolog thereof. In one embodiment, the C1ORF32 polypeptide is a soluble fragment of full-length C1ORF32 ECD. Such fragments optionally include those that retain the ability to bind to their natural receptors and incorporate some, or all, of the extracellular domain of the C1ORF32 polypeptide, and lack some or all of the intracellular and/or transmembrane domains. In one embodiment, C1ORF32 polypeptide fragments include the entire extracellular domain of the C1ORF32 polypeptide. In other embodiments, the soluble fragments of C1ORF32 polypeptides are fragments of the extracellular domain that retain C1ORF32 biological activity.

C1ORF32 polypeptide extracellular domains can include 1, 2, 3, 4, 5 or more contiguous amino acids from the transmembrane domain, and/or 1, 2, 3, 4, 5 or more contiguous amino acids from the signal sequence. Alternatively, the extracellular domain can have 1, 2, 3, 4, 5, or more contiguous amino acids removed from the C-terminus; N-terminus, or both. Biologically active variants and/or homologs of C1ORF32 polypeptides and fragments thereof may also be used.

### Fragments of C1ORF32 polypeptides

As used herein the term "soluble C1ORF32" or "soluble C1ORF32 proteins/molecules" refers to fragments of C1ORF32 that include some or all of the IgV and/or ECD domain of the C1ORF32 polypeptide, and lack some or all of the intracellular and/or transmembrane domains, wherein said fragments retain a biological activity of inhibition of T cell activation.

The soluble C1ORF32 molecules used in the methods of the invention may or may not include a signal (leader) peptide sequence.

Particular sequences of interest, according to at least some embodiments of the present invention, include but are not limited to SEQ ID NOs: 6-38.

In particular, the fragments of the extracellular domain of C1ORF32 can include any sequence corresponding to any portion of or comprising the IgV domain of the extracellular domain of C1ORF32, having any sequence corresponding to residues of Human_ILDR2_WT_P8 (SEQ ID NO:2) starting from position 21 and ending at any position between 164 and 170 or corresponding to residues of Human_ILDR2_WT_P8_mut (SEQ ID NO:3) starting from position 21 and ending at any position between 164 and 170, or corresponding to residues of Human_ILDR2_Variant_P9 (SEQ ID NO:4) starting from position 21 and ending at any position between 164 and 170, or corresponding to residues of Human_ILDR2_Variant_P9 (SEQ ID NO:5) starting from position 21 and ending at any position between 164 and 170. The base sequences as given above are with the signal peptide.

The C1ORF32 proteins contain an immunoglobulin domain within the extracellular domain, the IgV domain (or V domain), which is related to the variable domain of antibodies. The IgV domain may be responsible for receptor binding, by analogy to the other B7 family members. The Ig domain of the extracellular domain includes one disulfide bond formed between intradomain cystein residues, as is typical for this fold and may be important for structure-function. In SEQ ID NO: 2 these cysteines are located at residues 42 and 145.

In one embodiment, the first fusion partner is a soluble fragment of C1ORF32. Without wishing to be limited by a single hypothesis, it is believed that useful fragments are those that retain the ability to bind to their natural receptor or receptors and/or retain the ability to inhibit T cell activation. A C1ORF32 polypeptide that is a fragment of full-length C1ORF32 typically has at least 20 percent, 30 percent, 40 percent, 50 percent, 60 percent, 70 percent, 80 percent, 90 percent, 95 percent, 98 percent, 99 percent, 100 percent, or even more than 100 percent of the ability to bind its natural receptor(s) and/or of the ability to inhibit T cell activation as compared to full-length C1ORF32. Soluble C1ORF32 polypeptide fragments are fragments of C1ORF32 polypeptides that may be shed, secreted or otherwise extracted from the producing cells. In other embodiments, the soluble fragments of C1ORF32 polypeptides include fragments of the C1ORF32 extracellular domain that retain C1ORF32 biological activity, such as fragments that retain the ability to bind to their natural receptor or receptors and/or retain the ability to inhibit T cell activation. The extracellular domain can include 1, 2, 3, 4, or 5 contiguous amino acids from the transmembrane domain, and/or 1, 2, 3, 4, or 5 contiguous amino acids from the signal sequence. Alternatively, the extracellular domain can have 1, 2, 3, 4, 5 or more amino acids removed from the C-terminus, N-terminus, or both.
In some embodiments the extracellular domain is only the IgV domain as set forth in SEQ ID NO: 9, or fragments or variants thereof, or the region between the conserved cysteines of the IgV domain which are located at residues 42 and 145 of the full-length protein SEQ ID NO:2, corresponding to the sequence set forth in SEQ ID NO: 38:

According to the present disclosure, there is provided a pharmaceutical composition comprising an isolated soluble C1ORF32 polypeptide, fragment, variant, or homolog or fusion protein or conjugate containing same, and a pharmaceutically acceptable diluent or carrier, adapted for treatment of immune related disorder.

In one embodiment, the C1ORF32 polypeptide may optionally be fused to one or more domains of an Ig heavy chain constant region, preferably having an amino acid sequence corresponding to the hinge, CH2 and CH3 regions of a human immunoglobulin Cγ1, Cy2, Cy3 or Cy4 chains or to the hinge, CH2 and CH3 regions of a murine immunoglobulin Cy2a chain.

The fusion proteins may optionally be dimerized or multimerized to form homodimers, heterodimers, homomultimers or heteromultimers. Dimerization/multimerization partners can be arranged either in parallel or antiparallel orientations. Optionally the fusion protein has the sequence set forth in any one of SEQ ID NOs: 42, 43, 62, 64, 66-88.

According to the present disclosure, there is provided a pharmaceutical composition comprising an isolated soluble C1ORF32 polypeptide, or fragment or variant or homolog thereof, or fusion protein containing same, capable of inhibiting T cell activation, and a pharmaceutically acceptable diluent or carrier. Optionally, the pharmaceutical composition comprises the soluble C1ORF32 polypeptide comprising the extracellular domain of ILDR2-WT (SEQ ID NO:1), H19011_1_P8 (SEQ ID NO:2), H19011_1_P8_V1 (SEQ ID NO:3), H19011_1_P9 (SEQ ID NO:4) or H19011_1_P9 _V1 (SEQ ID NO:5) or fragment thereof, and a pharmaceutically acceptable diluent or carrier. According to the present disclosure, there is provided a pharmaceutical composition comprising an isolated soluble C1ORF32 polypeptide, or fragment or variant or homolog or fusion protein containing same, and a pharmaceutically acceptable diluent or carrier, adapted for treatment of immune related disorder by any one or more of the following: inhibiting or reducing differentiation of Th1, Th17, Th22, and/or other cells that secrete, or cause other cells to secrete, inflammatory molecules; inhibiting or reducing activity of Th1, Th17, Th22, and/or other cells that secrete, or cause other cells to secrete, inflammatory molecules; inhibiting or reducing the Th1 and/or Th17 pathways; inhibiting or reducing the Th1 and/or Th17 pathways while promoting Th2 pathways/ activity/ differentiation; inhibiting or reducing inflammatory molecule production and/or secretion by Th1, Th17, Th22, and/or other cells that secrete, or cause other cells to secrete, inflammatory molecules; inhibiting or reducing proliferation of Th1, Th17, Th22, and/or other cells that secrete, or cause other cells to secrete, inflammatory molecules; interacting with Tregs or Bregs; enhancing Treg or Breg differentiation, enhancing Treg or Breg activity; enhancing IL-10 secretion by Tregs or Bregs; increasing the number of Tregs or Bregs; increasing the suppressive capacity of Tregs or Bregs; interacting with Th2 cells; enhancing Th2 activity, enhancing the immunomodulatory capacity of Th2 cells, increasing the number of Th2 cells, enhancing production of IL-4, IL-5 or IL-10 by Th2 cells; or combinations thereof; limiting the antigen presentation capability of DCs and/or other myeloid cells.

In one embodiment but without wishing to be limited by a single hypothesis, C1ORF32 polypeptides or fusion proteins or pharmaceutical composition containing same, enhance T reg or Breg differentiation, enhance the suppressive activity of Tregs or Bregs on the immune system. Tregs and Bregs can suppress differentiation, proliferation, activity, and/or cytokine production and/or secretion by Th1, Th17, Th22, and/or other cells that secrete, or cause other cells to secrete, inflammatory molecules. In one embodiment the C1ORF32 polypeptides or fusion proteins or pharmaceutical composition containing same, enhance the suppressive activity of Tregs or Bregs on effector T cells to inhibit or reduce effector functions. In one embodiment the C1ORF32 polypeptides or fusion proteins or pharmaceutical composition containing same, enhance the suppressive activity of Tregs or Bregs on naive T cells to inhibit or reduce naive T cells from differentiating into Th1, Th17, Th22 cells and thereby reduce the number of Th1, Th17, Th22, and/or other cells that secrete, or cause other cells to secrete, inflammatory molecules, including, but not limited to, TNF-alpha, IFN-gamma, IL-17, IL-23, IL-22, IL-21, GM-CSF, CCL3, CCL5 and MMPs in a subject.

In one embodiment but without wishing to be limited by a single hypothesis, C1ORF32 polypeptides or fusion proteins or pharmaceutical composition containing same, limits antigen presentation capability of myeloid cells including but not limited to dendritic cells.

In one embodiment, C1ORF32 polypeptides or fusion proteins or pharmaceutical composition containing same, enhance the activity of Th2 immune responses or to increase the number or percentage of Th2 cells. Th2 cells can modulate the differentiation, proliferation, activity, and/or cytokine production and/or secretion by Th1, Th17, Th22, and/or other cells that secrete, or cause other cells to secrete, inflammatory molecules, resulting in inhibition of Th1 and/or Th17 responses, and in immune modulation via a Th1/Th2 shift. In one embodiment the C1ORF32 polypeptides or fusion proteins or pharmaceutical composition containing same, enhance the immunomodulatory activity of Th2 on naive T cells to inhibit or reduce naive T cells from differentiating into Th1, Th17, Th22 cells and thereby reduce the number of Th1, Th17, Th22, and/or other cells that secrete, or cause other cells to secrete, inflammatory molecules, including, but not limited to, TNF-alpha, IFN-gamma, IL-17, IL-23, IL-22, IL-21, GM-CSF, CCL3, CCL5 and MMPs in a subject. In one embodiment the C1ORF32 polypeptides or fusion proteins or pharmaceutical composition containing same, promote or enhance production of IL-4, IL-5 or IL-10 by Th2 cells.

C1ORF32 polypeptides, fragments, variants, homologs, fusion proteins and/or conjugates thereof can be administered in combination with one or more additional therapeutic agents, including, but not limited to, antibodies against other lymphocyte surface markers (e.g., CD40, alpha-4 integrin) or against cytokines, other fusion proteins, e.g. CTLA4-Ig (Orencia®, belatacept), TNFR-Ig (Enbrel®), TNF- alpha blockers such as Remicade, Cimzia and Humira, CD73-Ig, cyclophosphamide (CTX) (i.e. Endoxan®, Cytoxan®, Neosar®, Procytox®, Revimmune™), methotrexate (MTX) (i.e. Rheumatrex®, Trexall®), belimumab (i.e. Benlysta®), Tysabri or other immunosuppressive drugs, antiproliferatives, cytotoxic agents, or other compounds that may assist in immunosuppression.

According to the present disclosure, there is provided use of a combination of a C1ORF32 soluble polypeptide, as recited herein, and a known therapeutic agent effective for treating immune related disorder.

According to the present disclosure, there is provided a method for treating immune related disorder, comprising administering to a subject in need thereof a pharmaceutical composition comprising: a soluble molecule having the extracellular domain of C1ORF32 polypeptide, or a fragment or a variant or a homolog thereof; or a fusion protein or a conjugate thereof; or polypeptide, comprising amino acid residues depicted in any of SEQ D NOs:6-41), or a fragment, or a variant, or a homolog thereof.

According to the present disclosure, there is provided a method for treating immune related disorder, wherein the treatment does not cause a global immunosuppression of the immune system in the subject, comprising administering to a subject in need thereof a pharmaceutical composition comprising: a soluble molecule having the extracellular domain of C1ORF32 polypeptide, fragment, variant, homolog, fusion protein or conjugate thereof; or polypeptide, comprising amino acid residues depicted in any of SEQ ID NOs 6-41, or a fragment or a variant or a homolog thereof; optionally provided as a pharmaceutical composition thereof.

According to the present disclosure, there is provided an isolated soluble C1ORF32 polypeptide, fragment, variant, or homolog thereof; optionally as a fusion protein or conjugate, wherein said polypeptide or said fusion protein or conjugate is used for anti-immune related condition immunotherapy for an immune related condition as described herein, optionally provided as a pharmaceutical composition. Optionally treating comprises one or more of curing, managing, reversing, attenuating, alleviating, minimizing, suppressing, managing, or halting the deleterious effects of the above-described diseases.

### Treatment as prevention of disease and/or symptom onset

According to at least some embodiments, treating also includes at least reducing the rate of onset of symptoms and/or etiology of the disease, for example optionally as determined by measurement of one or more diagnostic markers.

Recently such treatment was established by testing of CTLA4-Ig (abatacept), showing that such treatment could reduce the onset of cessation or reduction of insulin production in subjects recently diagnosed with adult onset (Type II) diabetes (Orban et al., Lancet. 2011 Jul 30;378(9789):412-9. doi: 10.1016/S0140-6736(11)60886-6. Epub 2011 Jun 28. Co-stimulation modulation with abatacept in patients with recent-onset type 1 diabetes: a randomised, double-blind, placebo-controlled trial). Another study, by Koura et al (Biol Blood Marrow Transplant. 2013 Nov;19(11):1638-49. doi: 10.1016/j.bbmt.2013.09.003. Epub 2013 Sep 15. In vivo T cell costimulation blockade with abatacept for acute graft-versus-host disease prevention: a first-in-disease trial), showed that graft-vs-host-disease (GVHD) could be prevented through administration of abatacept. Similar detailed studies are planned for rheumatoid arthritis and type I diabetes, to determine whether abatacept can prevent full onset of these diseases in subjects without symptoms of the disease, or with only minor initial symptoms.

With regard to the soluble proteins as described herein, including the fusion proteins as described herein, and without wishing to be limited by a single hypothesis, it is possible that for each disease described herein, prevention or delay of full onset or even symptomatic presentation of these diseases in subjects without symptoms of the disease, or with only minor initial symptoms would be possible by detecting the disease in the subject before full onset or symptomatic presentation, and then administering the soluble proteins (including the fusion proteins) as described herein to the subject according to a suitable dosing regimen.

Optionally, managing comprises reducing the severity of the disease, reducing the frequency of episodes of the disease, reducing the duration of such episodes, or reducing the severity of such episodes or a combination thereof.

Individuals at risk of developing a disease can be identified based on various approaches either before disease development or at very early stages in which disease markers can be identified (i.e. ACPA in rheumatoid arthritis patients, high blood glucose levels in pre-diabetic individuals etc.). The identification of individuals at risk as well as diagnosis of early disease can rely on various approaches including genomics, proteomics, metabolomics, lipidomics, glycomics, secretomics, and serologic approaches. Family history can also provide information either in combination with one of the previously described approaches or as a standalone approach. Furthermore, over the past decade microbiome composition is becoming recognized as an important factor in health and disease. The advent of new technologies for interrogating complex microbial communities and in the analysis of microbiome and metagenome will provide another approach for identification of individuals at risk of developing a disease.

Non-limiting examples of such biomarkers and diagnostic methods for specific autoimmune diseases are described below.

For example, various specific biomarkers have been proposed for rheumatoid arthritis, to detect the initiation of the initial disease symptoms before onset of overt (frank) disease symptoms, as described for example in the Orencia clinical trial proposal ("Arthritis Prevention In the Pre-clinical Phase of Rheumatoid Arthritis with Abatacept", DOI 10.1186/ISRCTN46017566). These biomarkers include a positive test for serum rheumatoid factor (RF) and antibodies to citrullinated protein antigens (ACPA); or alternatively being RF negative, but with high levels of serum ACPA (defined as being equal to 3 x upper limit of normal [ULN] for the assay). In addition, joint pain caused by inflammatory tissue processes is considered as a biomarker.

Treatment would optionally prevent or at least slow development of rheumatoid arthritis symptoms, including but not limited to one or more of inflammation, fatigue, joint pain, joint tenderness, joint swelling, joint redness, joint warmth, joint stiffness, loss of joint range of motion, affecting more than one joint (polyarthritis), limping or joint deformity, or a combination thereof.

Other specific biomarkers have been proposed for psoriasis, to detect the initiation of the initial disease symptoms before onset of overt (frank) disease symptoms, as described for example in the following references: Tsoi et al., Nat Genet. 2012 Dec; 44(12): 1341-1348. Identification of fifteen new psoriasis susceptibility loci highlights the role of innate immunity; published online 2012 Nov 11. doi: 10.1038/ng.2467; and Nair et al., Nature Genetics 41, 199 - 204 (2009) Genome-wide scan reveals association of psoriasis with IL-23 and NF-κB pathways, published online: 25 January 2009 doi:10.1038/ng.311. These biomarkers include genetic markers as described in these references.

Treatment would optionally prevent or at least slow development of overt symptoms of psoriasis, including one or more of red patches of skin covered with silvery scales; small scaling spots; dry, cracked skin that may bleed; itching, burning or soreness; thickened, pitted or ridged nails; or swollen and stiff joints; or a combination thereof.

### BRIEF DESCRIPTION OF THE FIGURES

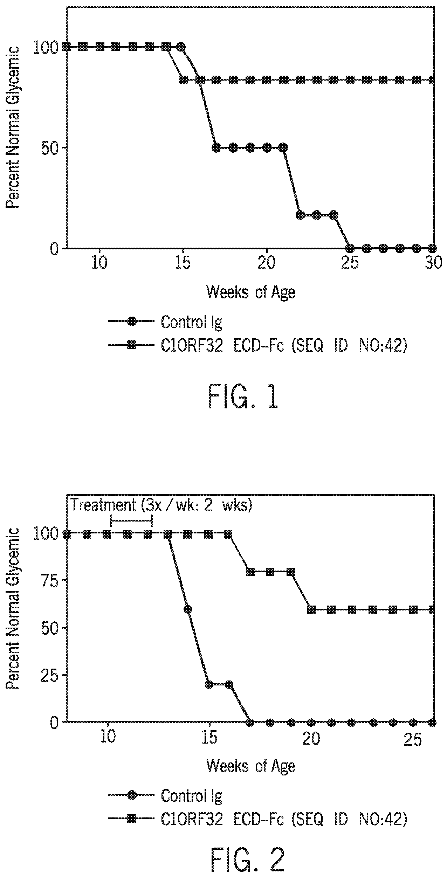

Figure 1. NOD mice were treated starting from 10wks of age with C1OFECD-Fc (SEQ ID NO:42) or control Ig (100ug/dose, at 3 times per week for 2 wks). Blood glucose levels were monitored weekly from wk 8 until 30 wks of age. Presented are percent normal glycemic mice; n=14-15.
Figure 2. NOD mice were treated with C1OFECD-Fc (SEQ ID NO:42) or Control Ig as described in legend of Figure 1. Blood glucose levels were monitored weekly from wk 8 until 26 wks of age. Presented are percent normal glycemic mice; n=6.

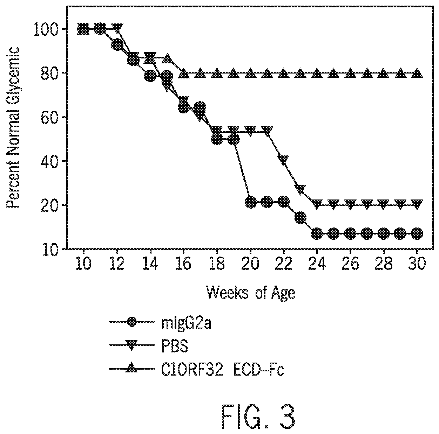

Figure 3. NOD mice were treated with C1OF ECD-Fc (SEQ ID NO:42) or control Ig as described in legend of Figure 1. Blood glucose levels were monitored weekly from wk 8 until 30 wks of age. Presented are percent normal glycemic mice; n=14-15.

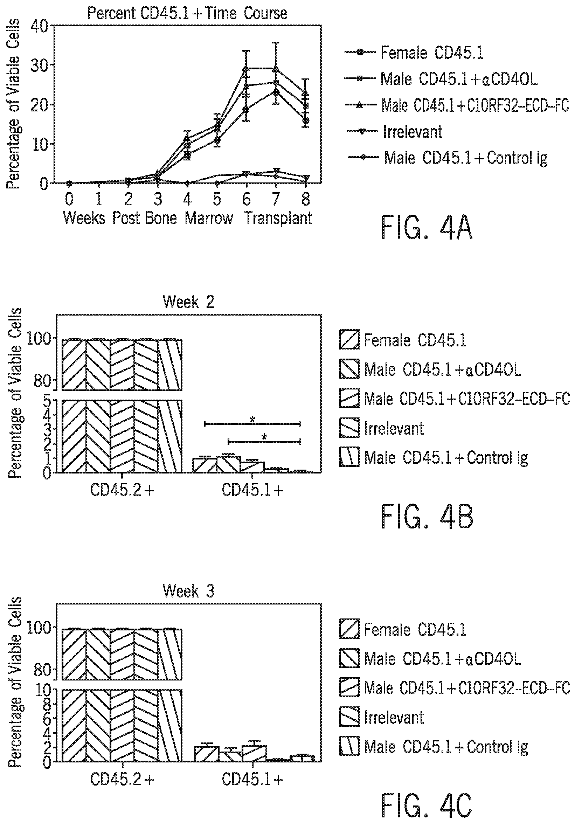

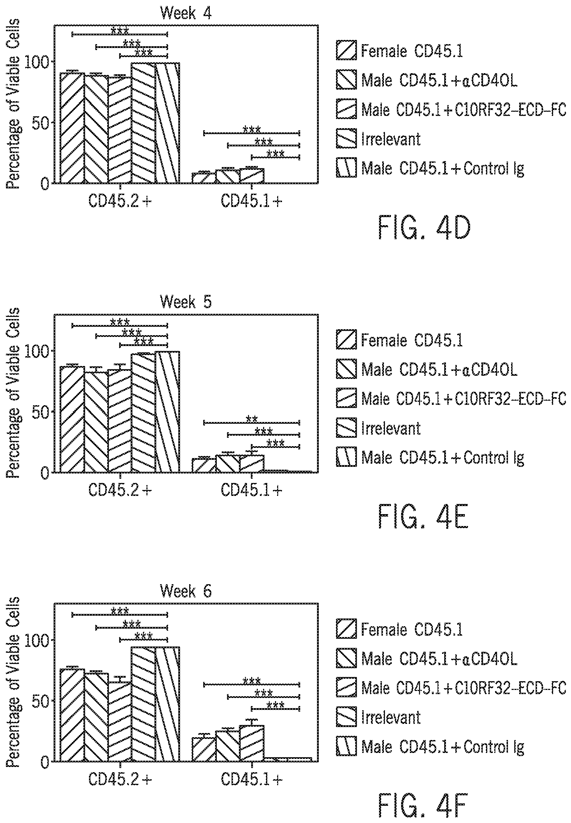

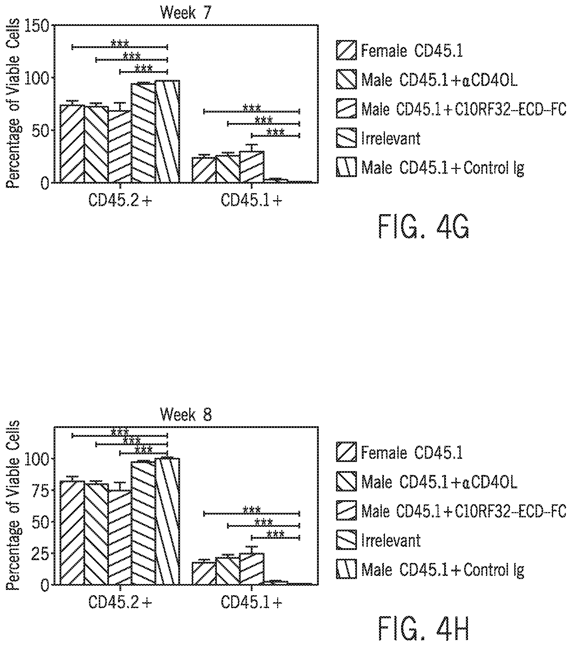

Figure 4 shows weekly percentage of viable CD45.1+ and CD45.2+ cells present in the blood following bone marrow transplantation. Sub-lethally irradiated female CD45.2 mice were transplanted with either female or male CD45.1 bone marrow (BM) cells. Recipient mice transplanted with male BM cells were treated i.p with either C1ORF ECD-Fc, anti CD40L or Control Ig at 300ug/dose, starting one week before BM transplantation. Treatment was given 3 times per week for 5 weeks. Mice were bled once a week on the indicated weeks post bone marrow cell transfer. The cells were gated on the total live cells to assess the percentage of CD45.1+ and CD45.2+ cells. The statistical analysis carried out on the data is one-way ANOVA followed by Dunnett's post test as compared to mice receiving male CD45.1+ bone marrow cells treated with Control Ig, p-values *<0.05, **<0.01 ,***<0.001, and #<0.0001

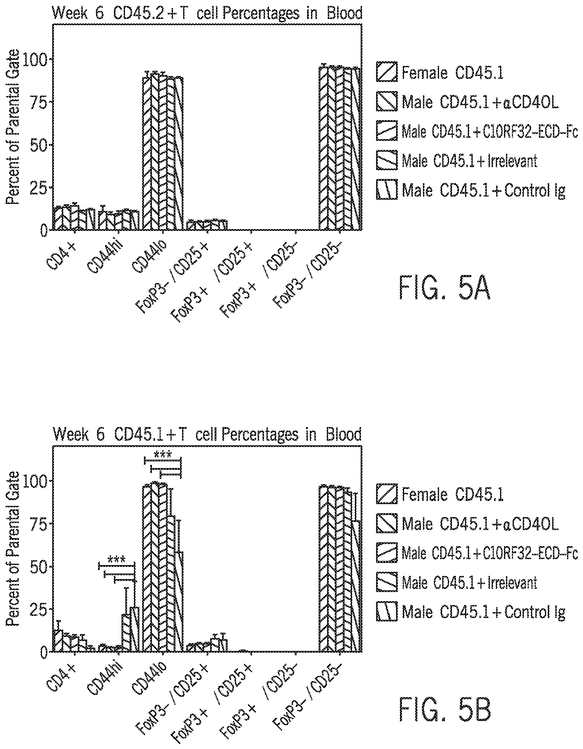

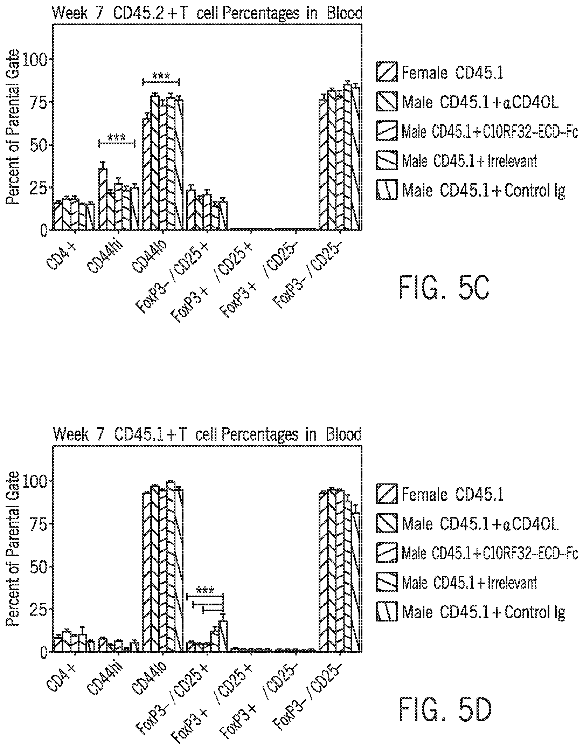

Figure 5 shows the effect of treatment on donor and recipient T cells' phenotype in the blood of recipient mice on weeks 6 and 7 post bone marrow cell transplantat, CD45.2+ (A, C) and CD45.1+ (B, D) cells were analyzed for percentage of effector/memory CD4+ T cells (CD4+/CD44hi), activated CD4+ T cells (CD4+/CD25+/FoxP3-), resting CD4+ T cells (CD4+/CD44lo), or Treg cells (CD4+/CD25+/FoxP3+) using FACS. The data is presented as the percentage of the parental gate and determined as follows: the percentage of CD4+ cells is the percentage of CD3/CD4+ T cells from the CD45.1+ and CD45.2+ gates. For the remainder of the populations, effector/memory CD4+ T cells (CD4+/CD44hi), activated CD4+ T cells (CD4+/CD25+/FoxP3-), resting CD4+ T cells (CD4+/CD44lo), or Treg cells (CD4+/CD25+/FoxP3+), the percentage presented is out of the total CD45.1+/CD4+ or CD45.2+/CD4+ cells. The statistical analysis carried out on the data is one-way ANOVA followed by Dunnett's post test as compared to the mice receiving male CD45.1+ bone marrow cells plus Control Ig treatment, p-values *<0.05, **<0.01 ,***<0.001, and #<0.0001.

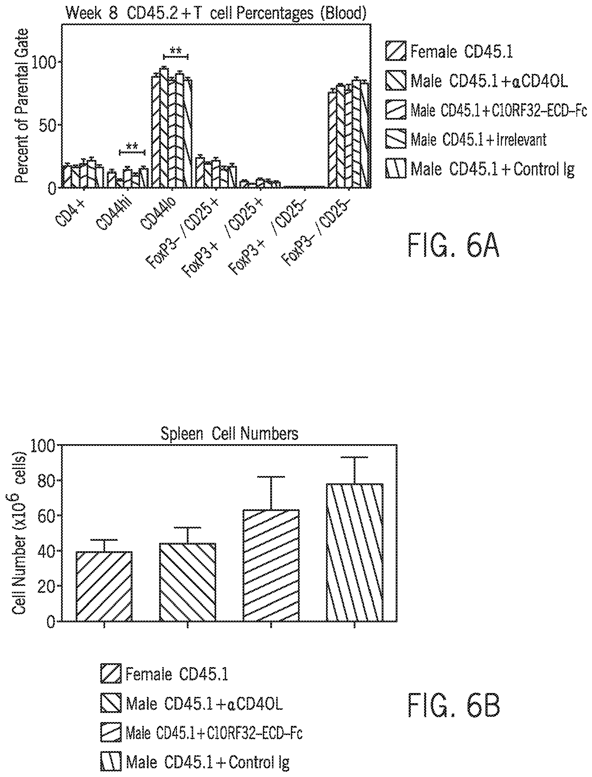

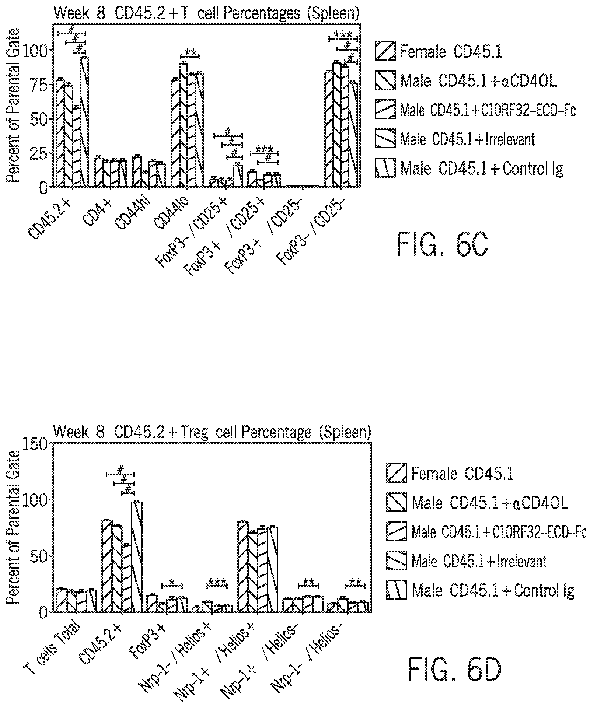

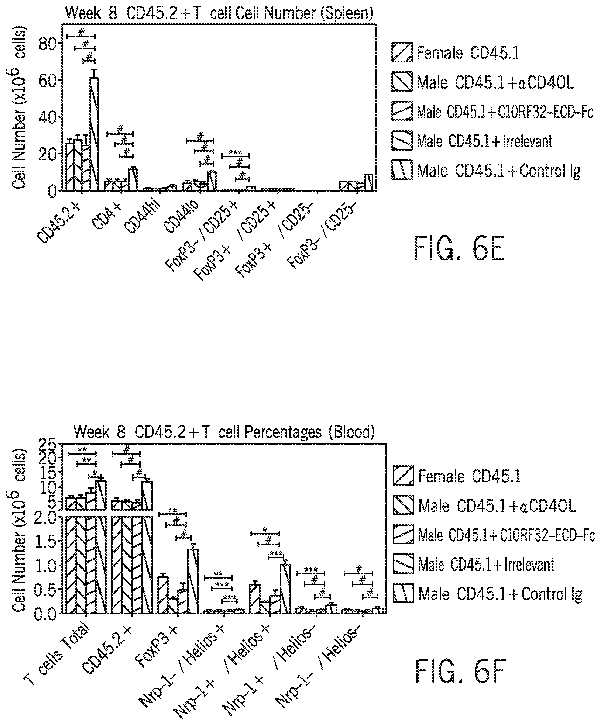

Figure 6 shows the effect of treatment on recipient's (CD45.2+) T cell subtypes and activation state in the blood and spleen on week 8 post transplantation. At 8 weeks post transplantation a blood sample was analyzed for CD45.2 T cells subpopulations presented as percentage of effector/memory CD4+ T cells (CD44hi), activated CD4+ T cells (CD25+/FoxP3-), resting CD4+ T cells (CD44lo), or Treg cells (CD25+/FoxP3+) using FACS analysis (A). Spleens were evaluated for total cell counts (B), for T cell subpopulations (C, E) as described in A, and for Treg subpopulations (D, F) using FoxP3, Helios and Nrp-1 FACS analysis. Data is presented as cell numbers or as cell percent out of CD45.2+CD4+ T cells. The data is presented as the percentage of the parental gate and determined as follows: the percentage of CD45.2+ cells is the percentage of total cells, CD4+ T cell percentage is out of CD45.2+ gate. For the remainder of the populations, percentage of effector/memory CD4+ T cells (CD44hi), activated CD4+ T cells (CD25+/FoxP3-), resting CD4+ T cells (CD44lo), or Treg cells (CD25+/FoxP3+) is out of the CD45.2+/CD4+ cells. Data was analyzed by one-way ANOVA followed by Dunnett's post test as compared to mice receiving male CD45.1+ bone marrow cells plus Control Ig treatment, p-values *<0.05, **<0.01 ,***<0.001, and #<0.0001.

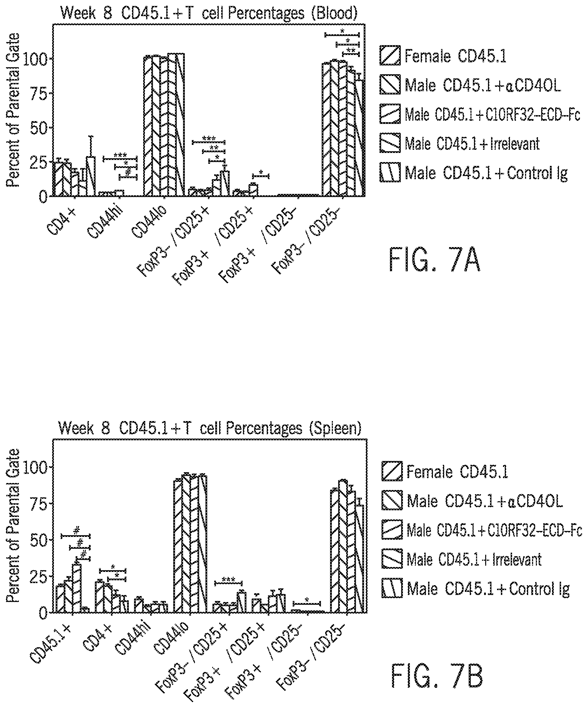

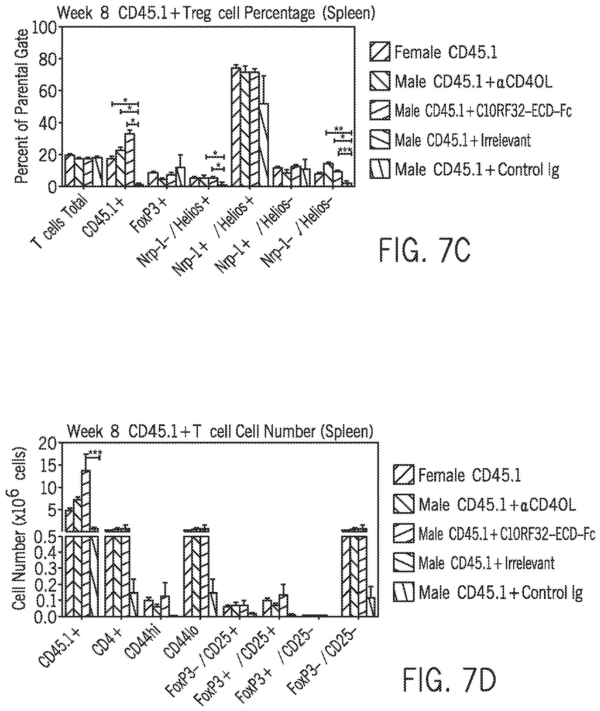

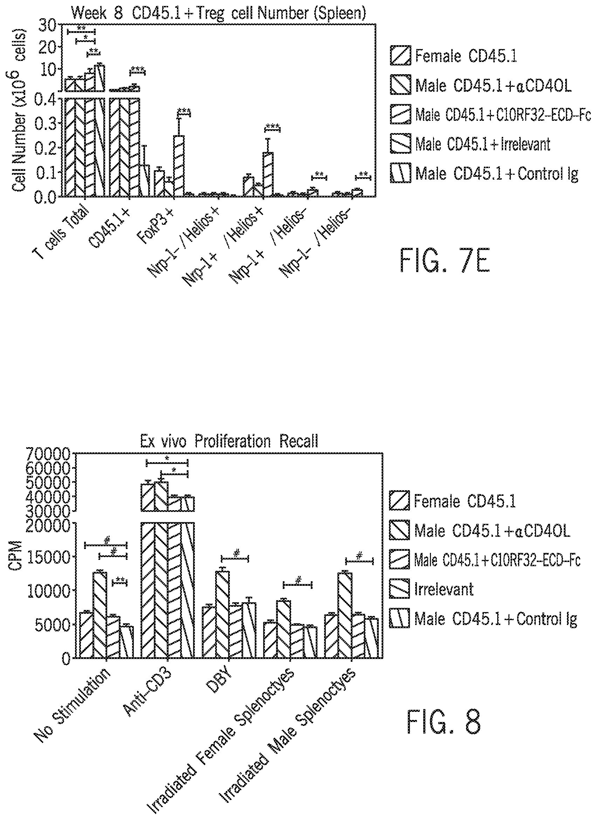

Figure 7 shows the effect of treatment on donor's (CD45.1+) T cell subtypes and activation state in the blood and spleen on week 8 post transplantation. At 8 weeks post transplantation a blood sample was analyzed for CD45.1+ T cells subpopulations presented as percentage of effector/memory CD4+ T cells (CD44hi), activated CD4+ T cells (CD25+/FoxP3-), resting CD4+ T cells (CD44lo), or Treg cells (CD25+/FoxP3+) using FACS (A). Spleen cells were evaluated for T cell subpopulations (B, D) as described in A, and for Treg subpopulations (C, E) using FoxP3, Helios and Nrp-1 FACS analysis. Data is presented as cell numbers and as cell percent. Cell percentage was determined as follows: CD45.1+ represents the percentage of total cells CD4+ T cell percentage is out of CD45.1+ gate, and the other sub- populations, i.e., effector/memory CD4+ T cells (CD44hi), activated CD4+ T cells (CD25+/FoxP3-), resting CD4+ T cells (CD44lo), or Treg cells (CD25+/FoxP3+) are determined out of the CD45.1+/CD4+ cells as the parental gate. Data was analyzed by one-way ANOVA followed by Dunnett's post test as compared to mice receiving male CD45.1+ bone marrow cells plus Control Ig treatment, p-values *<0.05, **<0.01 ,***<0.001, and #<0.0001.
Figure 8 shows the effect of C1ORF ECD-Fc on proliferation in ex vivo recall responses. On week 8 post bone marrow cell transplantion, total splenocytes (5x10⁵ cells/well) were cultured in the presence of medium alone (no stimulation), anti-CD3 (1ug/ml), DBY peptide (10ug/ml), irradiated male splenocytes (5x10⁵ cells/well), or irradiated female splenocytes (5x10⁵ cells/well). The individual splenocyte samples were cultured separately in triplicate wells. For T cell proliferative response, the cultures were pulsed with 1uCi of tritiated thymidine at 24 hours and harvested at 72 hours post culture initiation. The statistical analysis carried out on the data is one-way ANOVA followed by Dunnett's post test as compared to the mice receiving male CD45.1+ bone marrow cells plus Control Ig treatment, p-values *<0.05, **<0.01 ,***<0.001, and #<0.0001.

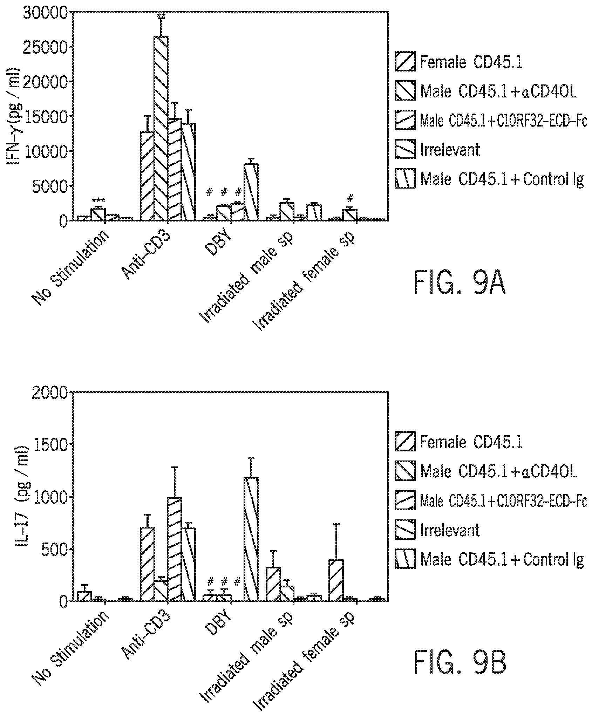

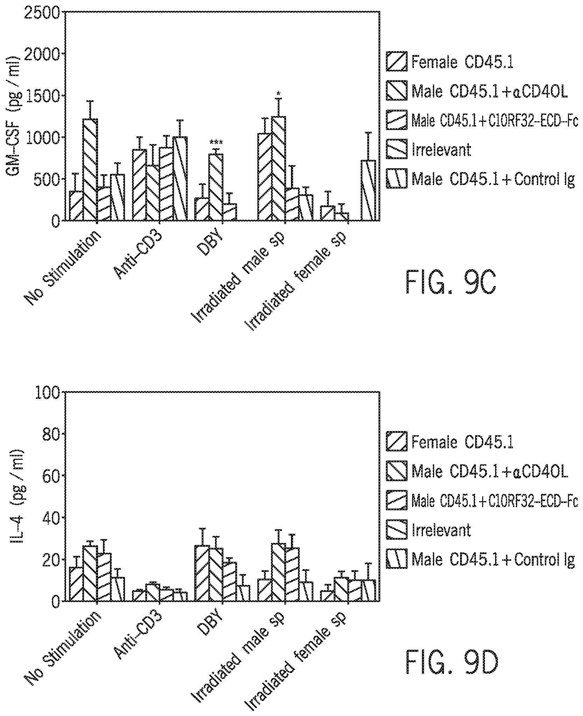

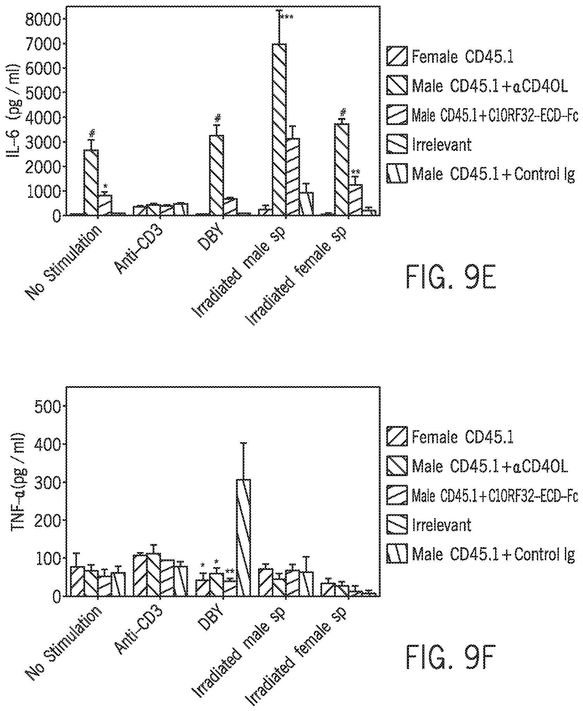

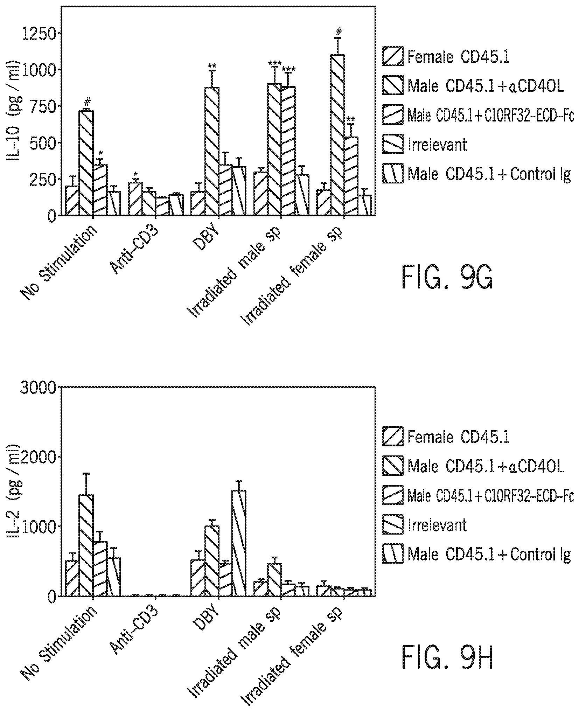

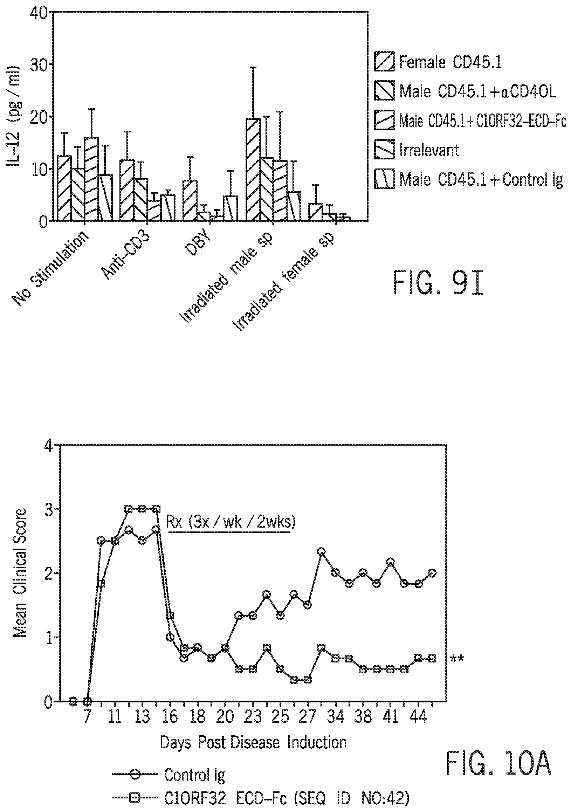

Figure 9 shows the effect of C1ORF ECD-Fc on cytokine secretion in ex vivo recall responses. On week 8 post bone marrow cell transplant, total splenocytes (5x10⁵ cells/well) were cultured in the presence of medium alone (no stimulation), anti-CD3 (1ug/ml), DBY peptide (10ug/ml), irradiated male splenocytes (5x10⁵ cells/well), or irradiated female splenocytes (5x10⁵ cells/well). The individual splenocyte samples were cultured separately in triplicate wells. For the cytokine cultures, the supernatants were collected at 72 hours post culture initiation and the level of secreted cytokine was determined via LiquiChip. The statistical analysis carried out on the data is one-way ANOVA followed by Dunnett's post test as compared to the mice receiving male CD45.1+ bone marrow cells plus Control Ig treatment, p-values *<0.05, **<0.01 ,***<0.001, and #<0.0001.

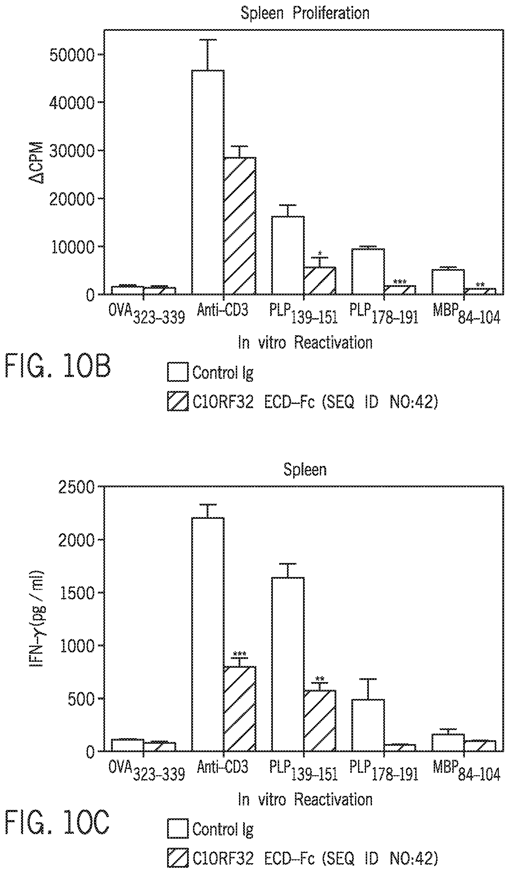

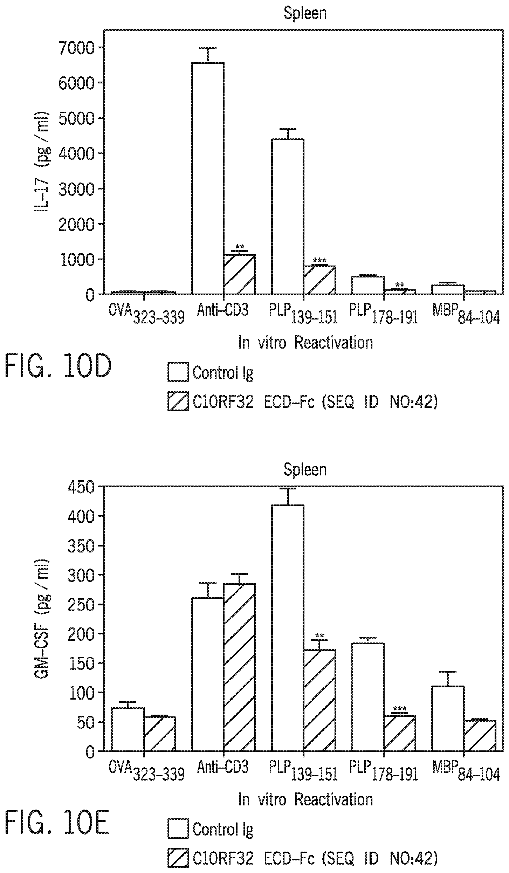

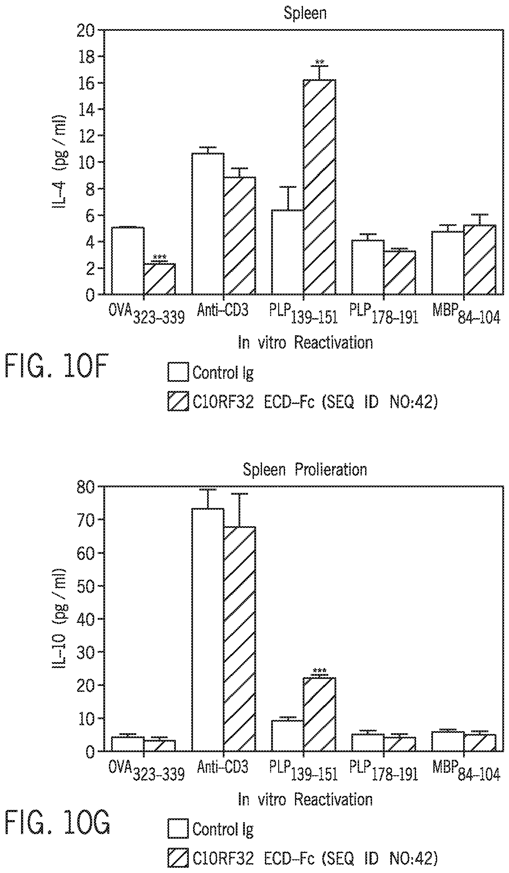

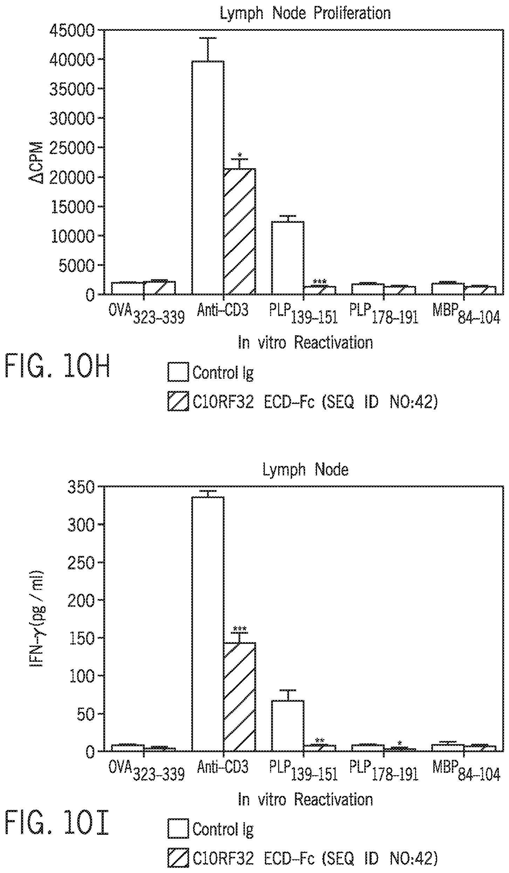

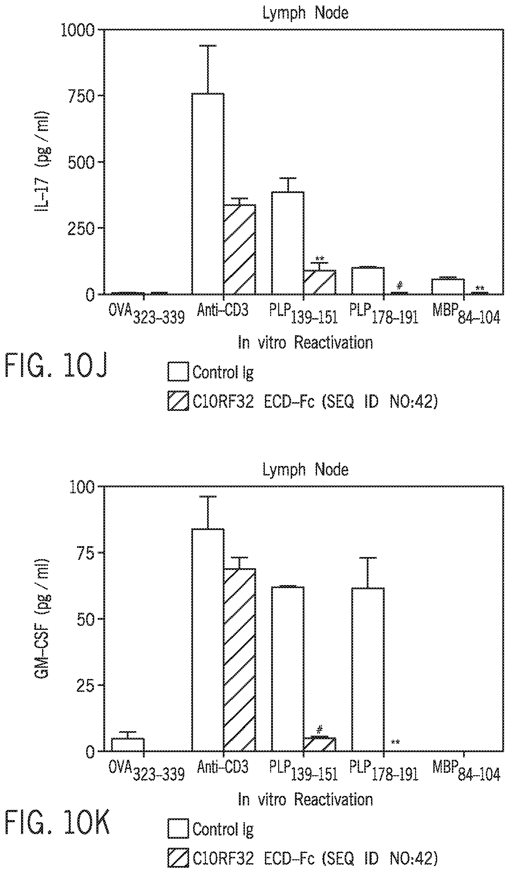

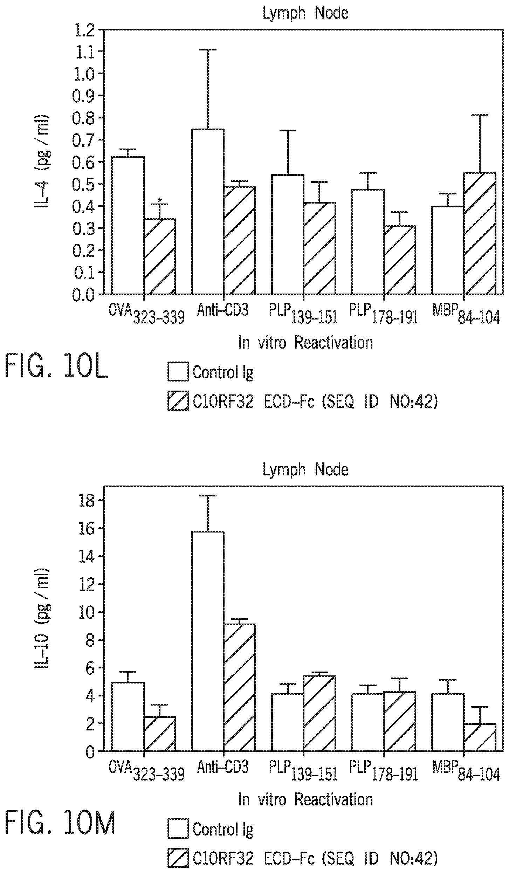

Figure 10. C1OFECD-Fc (SEQ ID NO:42) inhibits clinical signs in Adoptive Transfer EAE and induces aTh1/Th17 to Th2 shift, as manifested in recall responses
   Twenty SJL/J mice were primed with PLP₁₃₉₋₁₅₁/CFA, draining lymph nodes were collected on Day +8, reactivated ex vivo with PLP₁₃₉₋₁₅₁ (20µg/ml), and on Day 3 of culture cells 3x10⁶ blast cells were transferred i.v. into recipient SJL/J mice (n=10/group). At onset of disease remission (Day +17 post disease induction) mice received three doses (100µg/dose) per week for two weeks of Control Ig or C1ORF ECD-Fc (SEQ ID NO:42). Mice were followed for disease severity (Mean Clinical Score; A).
   On Day +45 after cell transfer spleens and cervical lymph nodes were collected. Total splenocytes and total cervical lymph node cells were used for recall responses, i.e. reactivated ex vivo in the presence of anti-CD3, OVA₃₂₃₋₃₃₉, PLP₁₃₉₋₁₅₁, PLP₁₇₈₋₁₉₁, or MBP₈₄₋₁₀₄. Duplicate cultures were established. One set of cultures were pulsed with 1µCi tritiated-thymidine at 24 hours and harvested at 72 hours (B and H). The supernatants of the second set of plates were harvested at 72 hours to evaluate the levels of IFN-γ (C and I), IL-17 (D and J), GM-CSF (E and K), IL-4 (F and L), and IL-10 (G and M). The averages of triplicate wells ±SEMs are shown.
   Statistically significant difference between control and C1ORF ECD-Fc (SEQ ID NO:42) treated groups are indicated (*,**,***[indicate p < 0.05, 0.01, 0.001 respectively).

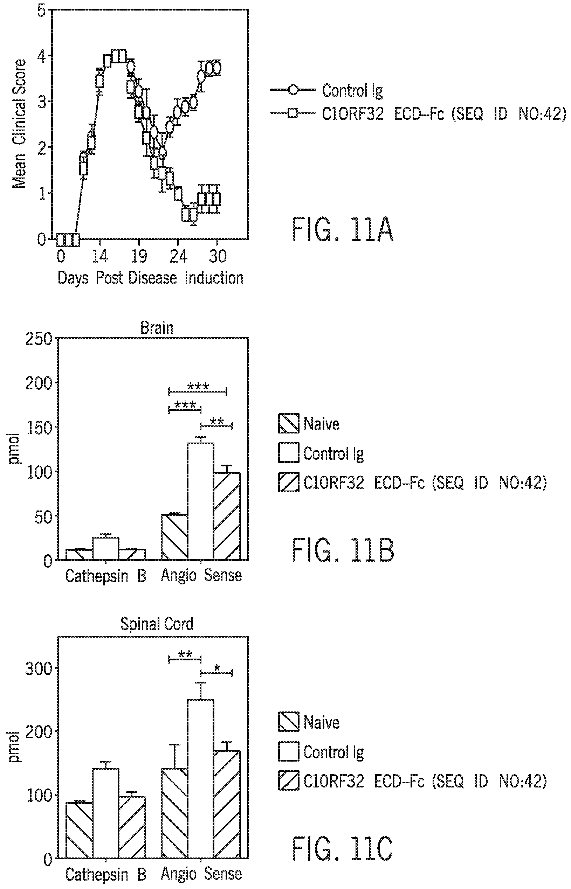

Figure 11. C1ORF ECD-Fc (SEQ ID NO:42) inhibition of clinical signs in Adoptive Transfer EAE is accompanied by reduced damage and inflammation in the CNS.
   Twenty SJL/J mice were primed with PLP₁₃₉₋₁₅₁/CFA, draining lymph nodes were collected on Day +8, reactivated ex vivo with PLP₁₃₉₋₁₅₁, and on Day +3 of culture 3x10⁶ blast cell were transferred i.v. into recipient SJL/J mice (n=9/group). At onset of disease remission (Day +19 post disease induction) mice received three doses (100µg/dose) per week for two weeks of Control Ig or C1ORF ECD-Fc (SEQ ID NO:42). Mice were followed for disease severity (Mean Clinical Score, A).
   On Day +30 post cell transfer four representative mice from both Control Ig and C1ORF ECD-Fc (SEQ ID NO:42) treatment groups were intravenously injected with AngioSense®750 or with Cat B® 680 FAST imaging agents, 24 hours prior to imaging. At the time of imaging mice were anesthetized by administration of sodium pentobarbital (50 mg/kg) and then imaged using the FMT 2500 fluorescence molecular tomography in vivo imaging system. The data is presented as pmol of CathepsinB or AngioSense present within the brain (B) or spinal cord (C).
   Statistically significant differences between control and C1ORF ECD-Fc (SEQ ID NO:42) treated groups are indicated (*,**,*** indicate p < 0.05, 0.01, 0.001 respectively).

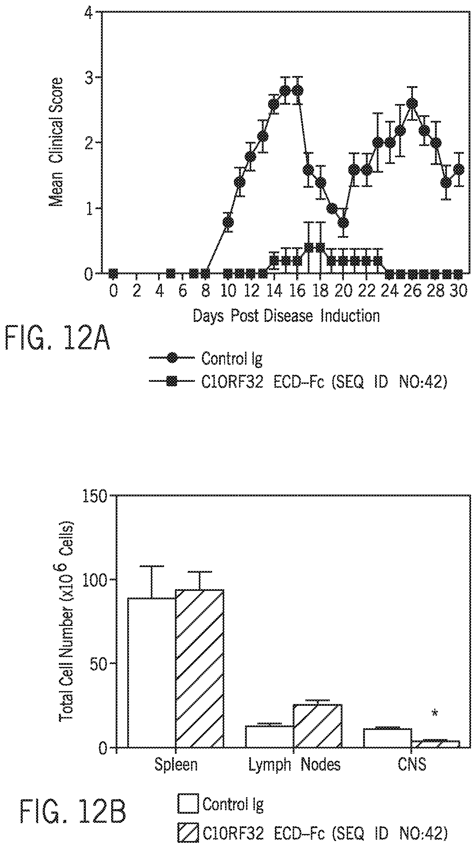

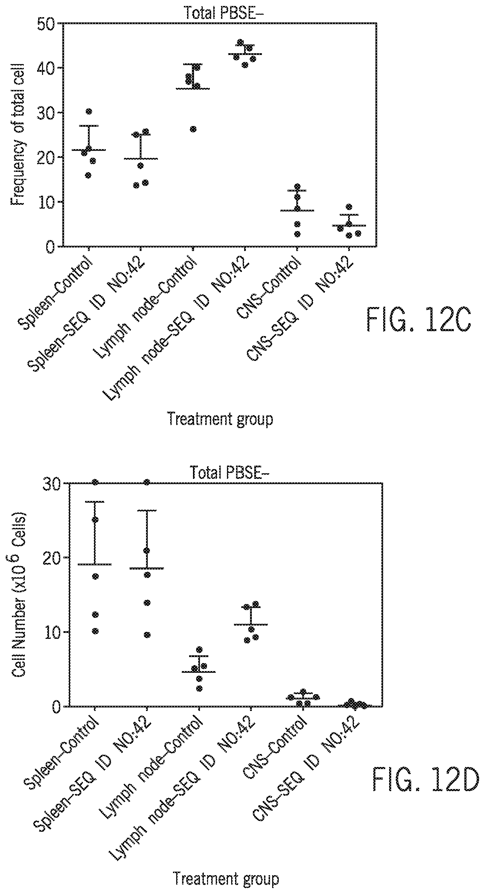

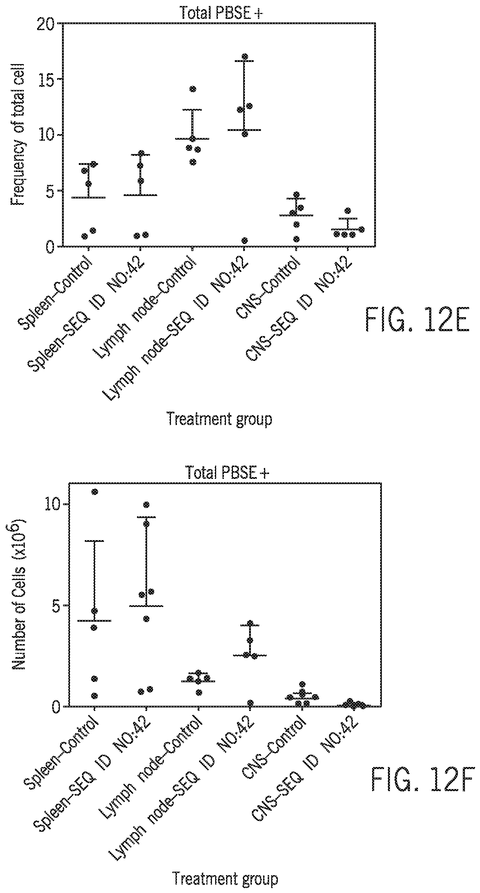

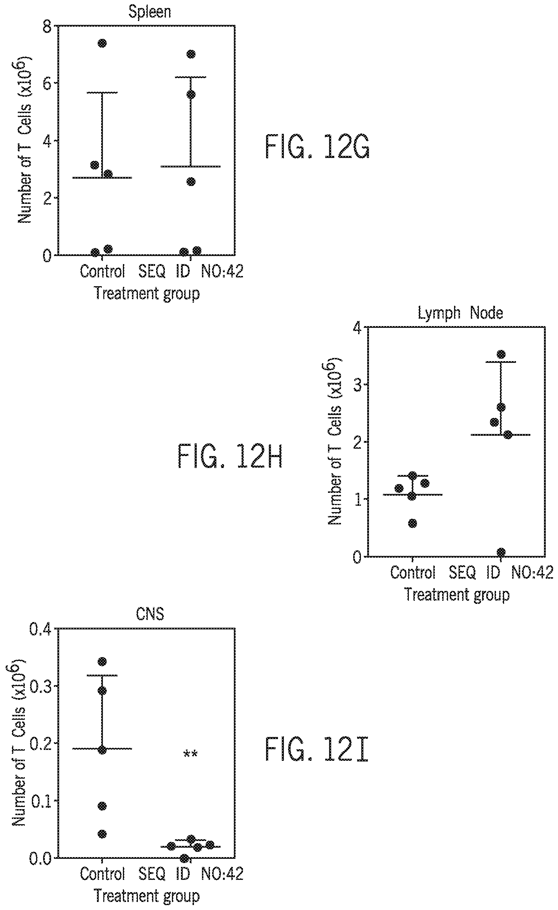

Figure 12. C1ORF ECD-Fc (SEQ ID NO:42) treatment beginning at time of cell transfer decreases PLP₁₃₉₋₁₅₁ autoreactive cell infiltration into the CNS
   SJL/J mice were primed with PLP₁₃₉₋₁₅₁/CFA, draining lymph nodes were collected on Day +8, reactivated ex vivo with PLP₁₃₉₋₁₅₁, and on Day 3 of culture, cells were labeled with PBSE and 5x10⁶ cells were transferred i.v. into recipient SJL/J mice (n=15/group). Beginning on the day of cell transfer, mice received three doses (100µg/dose) per week for two weeks of Control Ig or C1ORF ECD-Fc (SEQ ID NO:42). Mice were followed for disease severity (Mean clinical score; A).
   On Day +10 spleens, cervical lymph nodes, and CNS were collected and the number of total cells was enumerated (B). The following subpopulations were analyzed: frequency and numbers of PBSE- (C and D) and PBSE⁺ (E and F) and total number of T cells in the spleen (G), lymph nodes (H), and CNS (I).
   Statistically significant differences between control and C1ORF ECD-Fc (SEQ ID NO:42)treated groups are indicated (*,**,***, indicate p < 0.05, 0.01, 0.001 respectively).

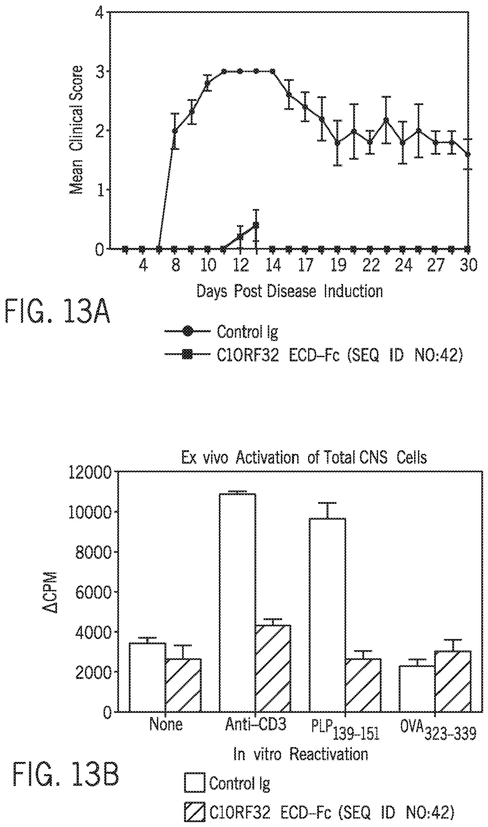

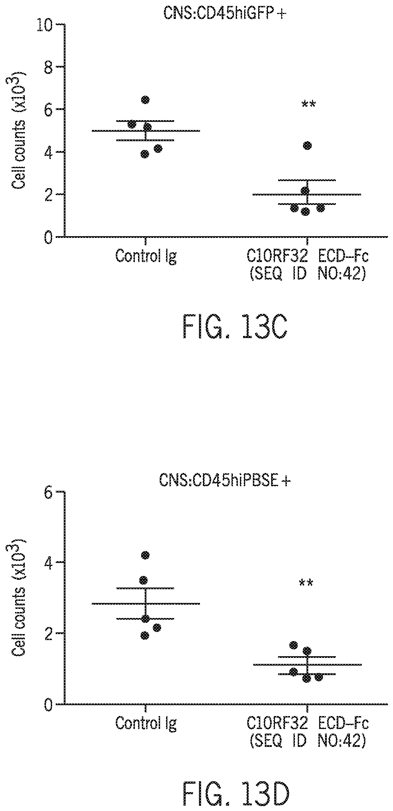

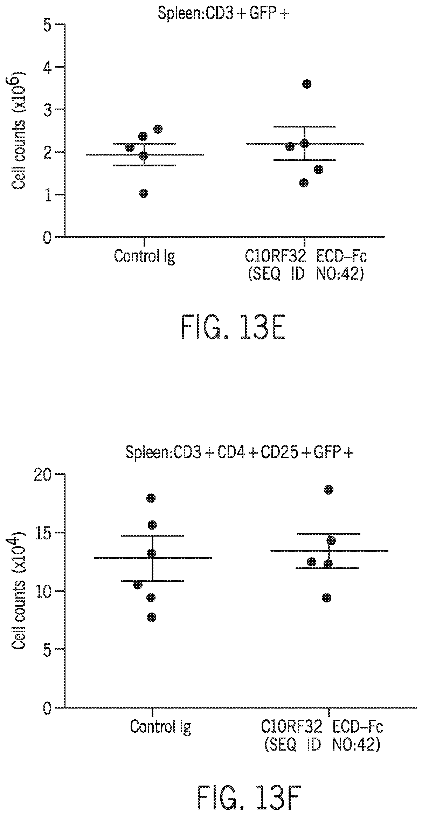

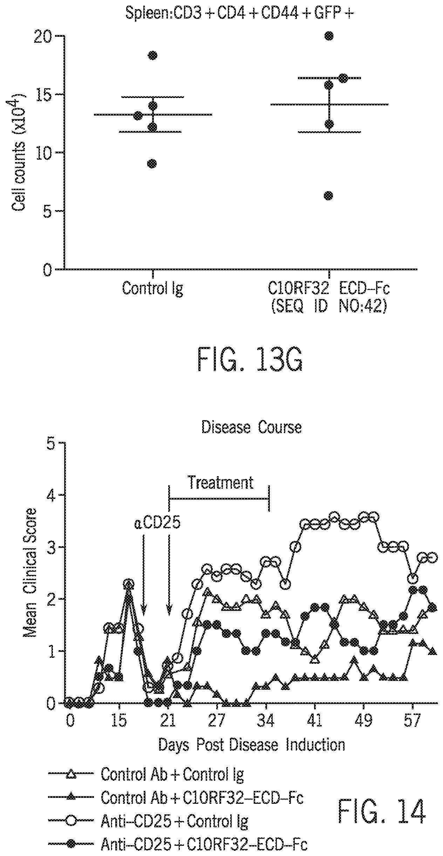

Figure 13. C1ORF ECD-Fc (SEQ ID NO:42) treatment beginning at time of cell transfer decreases PLP₁₃₉₋₁₅₁ Sensitized T cell infiltration into the CNS.
   SJL/J-Actin/GFP mice were primed with PLP₁₃₉₋₁₅₁/CFA, draining lymph nodes were collected on Day +8 reactivated ex vivo with PLP₁₃₉₋₁₅₁, on Day 3 of culture cells were labeled with PBSE, and 5x10⁶ blast cell were i.v. transferred in to recipient SJL/J mice (n=10/group). Beginning on the day of cell transfer, mice received three doses (100µg/dose) per week for two weeks of Control Ig or C1ORF ECD-Fc (SEQ ID NO:42) and the mice were followed for disease severity (A). On Day +10 post cell transfer CNS, spleens, and cervical lymph nodes were collected. CNS cells were analyzed for recall responses (B) and the number of CD45hi/GFP+ and CD45hi/PBSE+ cells was evaluated (C and D). Spleen and lymph node were analyzed for total T cells CD3+ GFP+ (E and, H), activated T cells CD3+/CD25+GFP+ (F and I) or CD3+/CD4+/CD44+/GFP+ (G and J). Statistically significant difference between control and C1ORF ECD-Fc (SEQ ID NO:42) treated groups are indicated (** indicate p < 0.01).
Figure 14 shows the efficacy of C1ORF ECD-Fc (SEQ ID NO:42)in the R-EAE model upon early Treg inactivation with anti-CD25

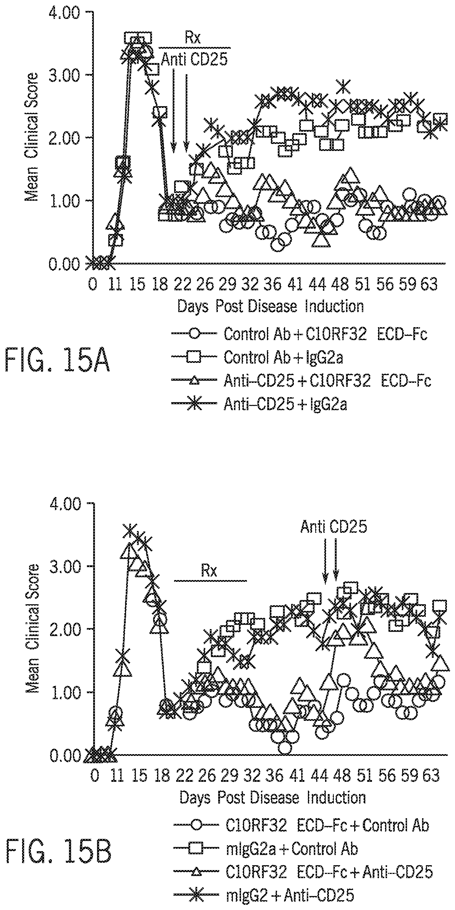

Figure 15 shows the efficacy of C1ORF ECD-Fc (SEQ ID NO:42) in the R-EAE model upon late or early Treg inactivation with anti-CD25. SJL/J mice were primed with PLP139-151/CFA and treated with C1ORF ECD-Fc (SEQ ID NO:42) or mIgG2a control from day 22, 3 x/wk x 2wks. Tregs were inactivated with 2 treatments of anti CD25 or Control mAb (0.5 mg/dose) given either immediately prior to C1ORF ECD-Fc (SEQ ID NO:42) treatment, on days 20 and 22 (A) or 2 wks after completion of C1ORF ECD-Fc (SEQ ID NO:42) treatment, on days 46 and 48 (B). Mice were followed for clinical score as described under Materials and Methods.

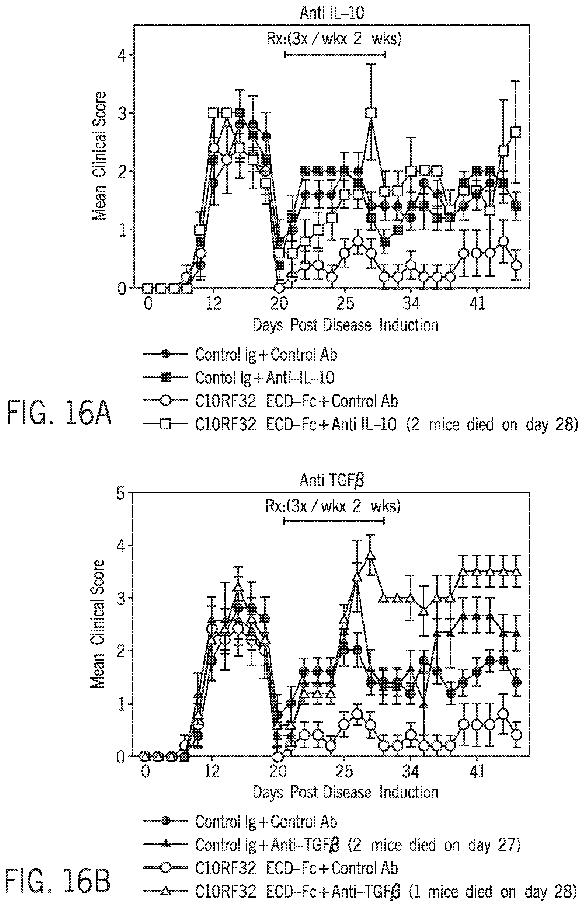

Figure 16 shows the efficacy of C1ORF ECD-Fc (SEQ ID NO:42) in the R-EAE model following concomitant administration of blocking anti-IL-10 or anti-TGF-β Abs. SJL mice were primed with PLP139-151/CFA per the standard protocol. At disease remission the mice were split into treatment groups (n=5). Mice were treated with Control Ig (mIgG2a) or C1ORF ECD-Fc (SEQ ID NO:42) (100ug/dose, each) via an i.p. injection. This was followed with a second i.p. injection of either anti-IL-10 (A), anti-TGF-β (B) or control Ab (rat IgG1) at 100ug/dose, each. All treatments were given 3x/wk for 2 wks, from day +20 and until day +31 post disease induction. Mice were followed for clinical score as described under Materials and Methods.

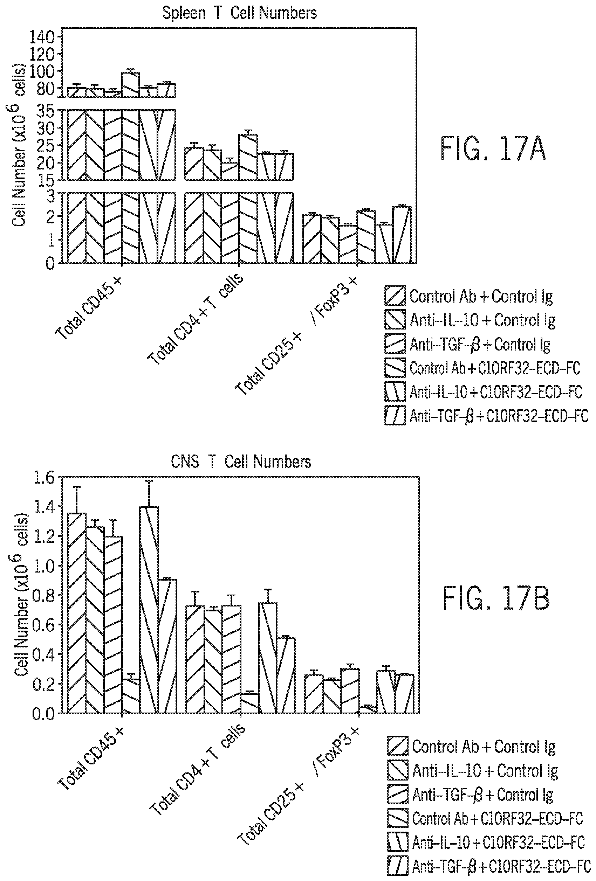

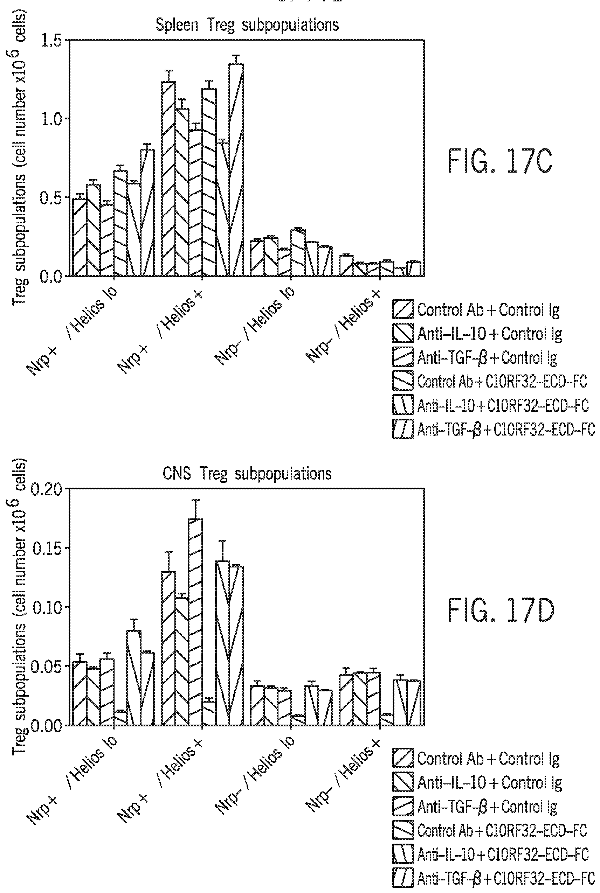

Figure 17 shows the effect of C1ORF ECD-Fc (SEQ ID NO:42) treatment on T cell and Treg counts in the spleen and CNS. Spleens and CNS were collected at the end of the study and subjected to FACS analysis for evaluation of CD45+ cells, CD4+ T cells, CD25+FoxP3+ Treg cells (A and B). Treg subpopulations were further analyzed by evaluating Nrp and Helios expression (C and D).

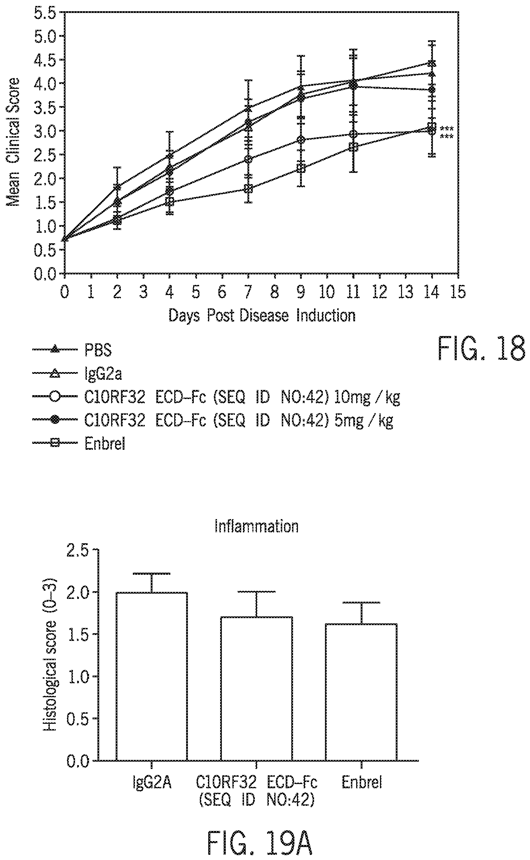

Figure 18. Effect of i.p. treatment with C1ORF ECD-Fc (SEQ ID NO:42) three times per week for two weeks on macroscopic scores of established CIA. Graph shows mean +/- SEM of n=10 mice/group. Disease course data was analyzed using one-way ANOVA with repeated measures, with a Bonferroni's post-test of selected pairs comparing all treatment groups to the mIgG2a treated group. *** indicates p value <0.001.

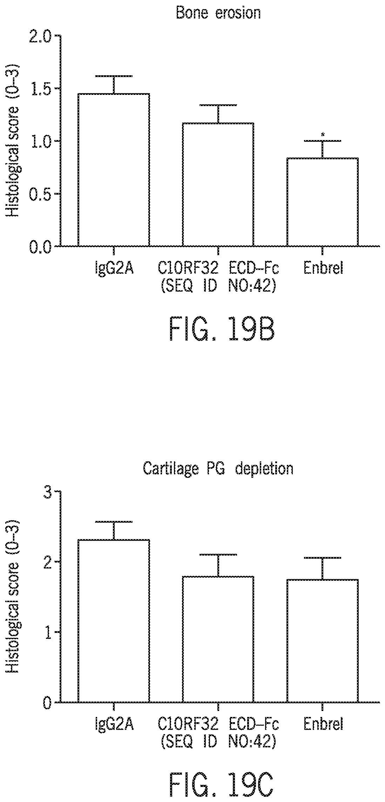

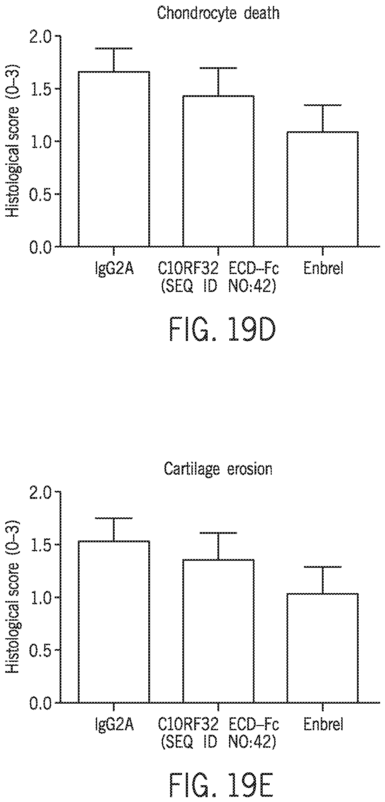

Figure 19. Effect of i.p. treatment with C1ORF ECD-Fc (SEQ ID NO:42) (10 mg/kg) or Enbrel (5mg/kg) three times per week for two weeks on histological joint pathology. Ankle joints were scored for inflammation, cartilage proteoglycan depletion, chondrocyte death, cartilage erosion, and bone erosion, on an arbitrary scale of 0-3. Graph shows mean + SEM of n=20 ankle joints/group. Tested by one-way ANOVA and Dunnett's multiple comparison test, *P < 0.05, vs. IgG2A control.

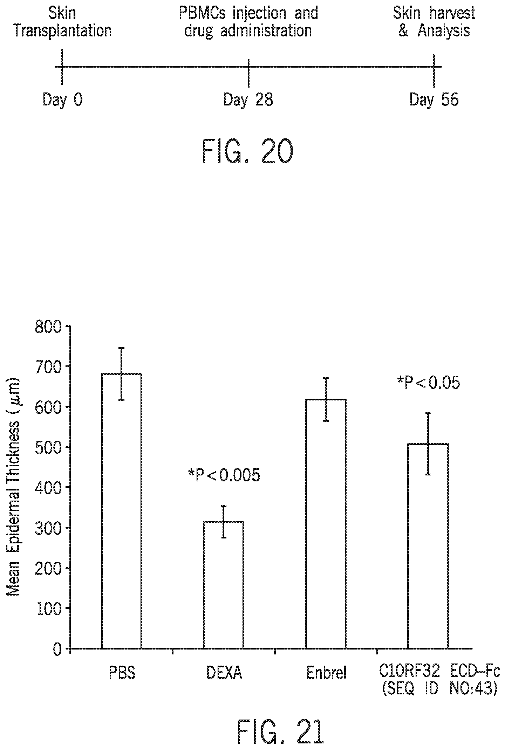

Figure 20. Study progression for psoriasis model on a time axis
Figure 21. Epidermal thickness measurements (µm) of xenografted skin. A significant reduction in epidermal thickness was observed in both the dexamethasone (p<0.005) and C1ORF ECD-Fc (SEQ ID NO:43) (p<0.05) treatment groups as compared to the vehicle treated group. Statistical analysis was performed using a one-tailed t-Test. Bar values represent the mean ±STDEV of 9-10 beige-SCID mice. Each mouse received a normal human skin graft in addition to an injection of PBMCs from a psoriatic patient followed by drug treatment, as described in Methods.

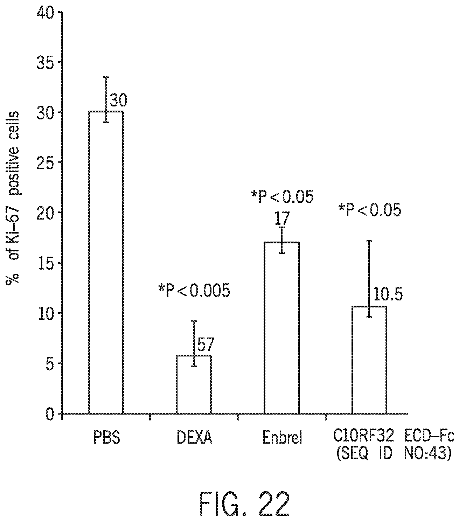

Figure 22. Percent of Ki-67 positive cells in the xenografted skin. Representative grafts were allocated for IHC analysis. Each analysis was performed on the relative number of affected and healthy grafts. A significant reduction of Ki positive cells was observed in all treatment groups as compared to the vehicle treated group, with most pronounced reduction observed in the dexamethasone and C1ORF ECD-Fc (SEQ ID NO:43) treatment groups. Statistical analysis was performed using a one-tailed t-Test. Bars values represent the mean ±STDEV of 3 beige-SCID mice. Each mouse received a normal human skin graft in addition to an injection of psoriatic patient PBMC cells followed by drug treatment, as described in Methods.

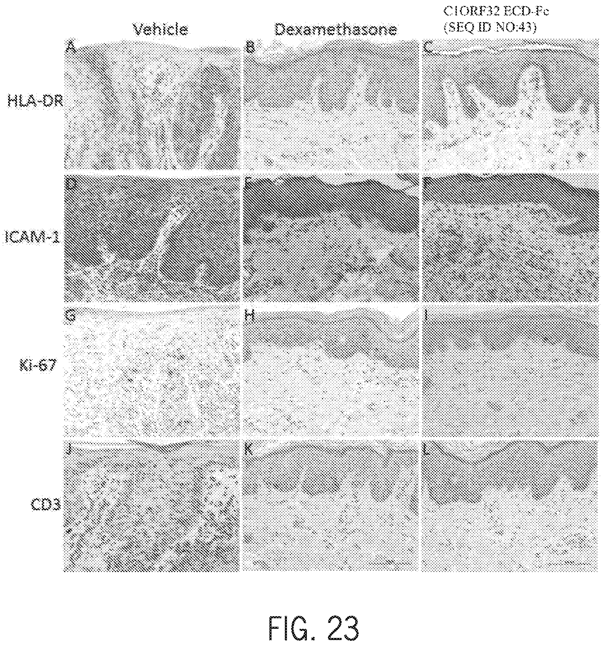

Figure 23. Immunohistochemical analysis of the grafted human skin (A-C) Expression of HLA-DR by grafts injected with enriched PBMCs and treated with PBS (A), dexamethasone (B) and C1ORF ECD-Fc (SEQ ID NO:43) (C) demonstrating high expression of HLA-DR in the epidermis and upper dermis of the vehicle treated group, compared to the dexamethasone and C1ORF ECD-Fc (SEQ ID NO:43) treated groups. (D-F) Expression of ICAM-1 by grafts injected with enriched PBMCs and treated with PBS (D), dexamethasone (E) and C1ORF ECD-Fc (SEQ ID NO:43) (F) demonstrating high expression of ICAM-1 in the epidermis and upper dermis of the vehicle treated group compared to the dexamethasone and C1ORF ECD-Fc (SEQ ID NO:43) treated groups. (G-I) Expression of Ki-67 by grafts injected with enriched PBMCs and treated with PBS (G), dexamethasone (H) and C1ORF ECD-Fc (SEQ ID NO:43) (I) demonstrating high proliferation rate of basal epidermal cells in the PBS treated group, compared with that of the dexamethasone and C1ORF ECD-Fc (SEQ ID NO:43) treated groups. (J-L) CD3 positive T cells highly infiltrate the epidermis and the dermis in the PBS treated group (J) in comparison to the low levels seen in the dexamethasone (K) and C1ORF ECD-Fc (SEQ ID NO:43) (L) treated groups.

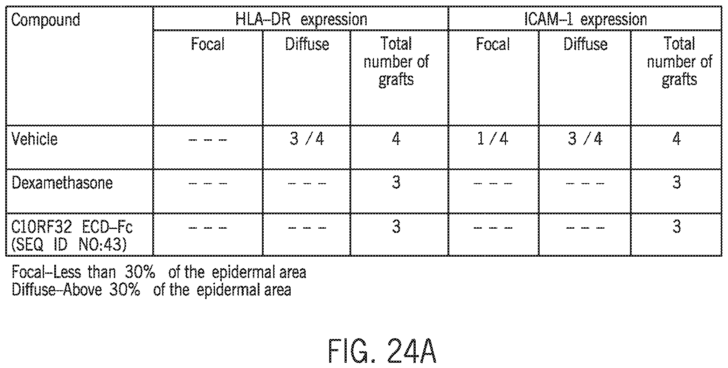

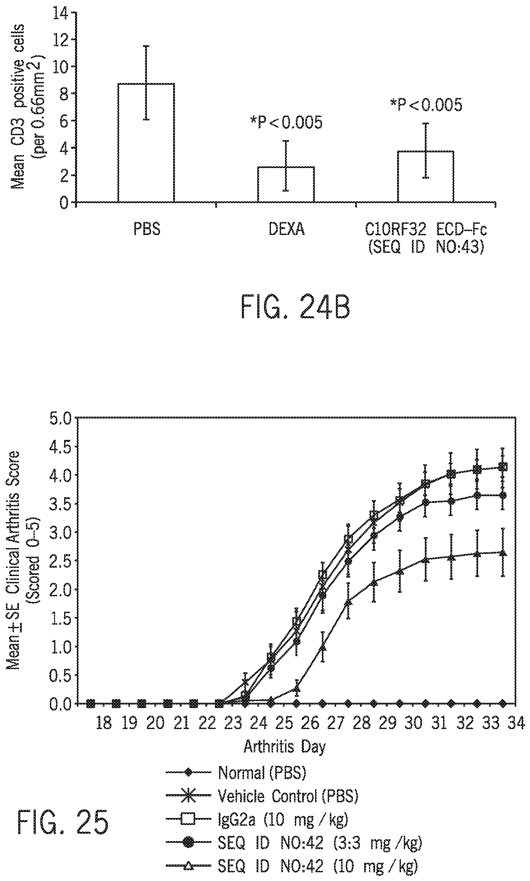

Figure 24. Expression of HLA-DR, ICAM-1 and CD3 (A) Expression of HLA-DR and ICAM-1 by grafts injected with enriched PBMCs and treated with PBS, dexamethasone and C1ORF ECD-Fc (SEQ ID NO:42) , demonstrating diffused expression of HLA-DR and ICAM-1 in the epidermis of the vehicle treated group compared to the dexamethasone and C1ORF ECD-Fc (SEQ ID NO:42) treated groups. Evaluation was performed blindly under light microscope with the following criteria: Focal - Less than 30% of the epidermal area. Diffuse - Above 30% of the epidermal area (B) A graph representing the mean number of CD3 positive cells demonstrating a significant reduction of CD3 positive cells in the dexamethasone and C1ORF ECD-Fc (SEQ ID NO:42) treatment groups. *P values compared to the PBS treated group.
Figure 25 shows the effect of semi established treatment with C1ORF32-ECD-mFc (SEQ ID NO:42), PBS or control Ig in the collagen induced arthritis (CIA) model of Rheumatoid Arthritis.

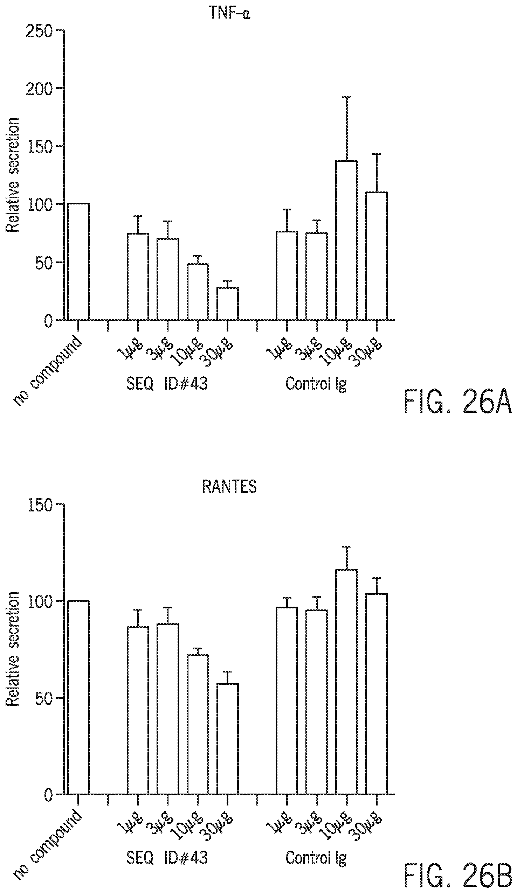

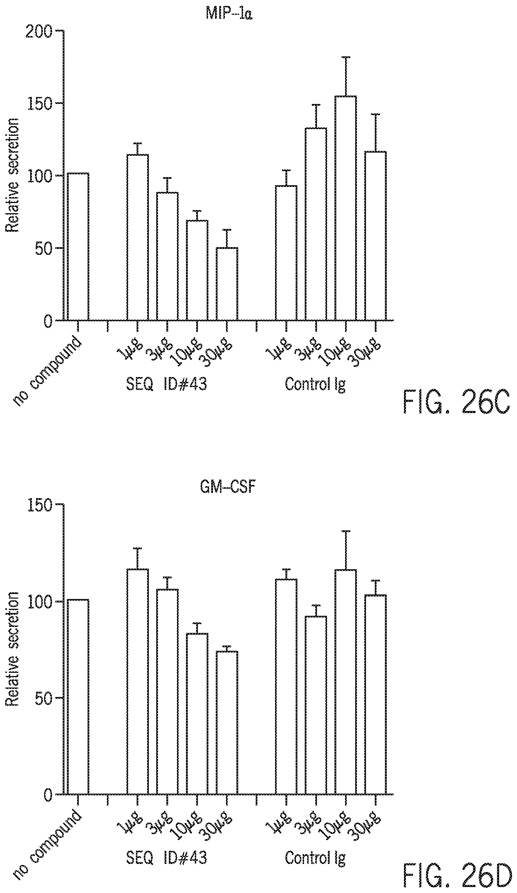

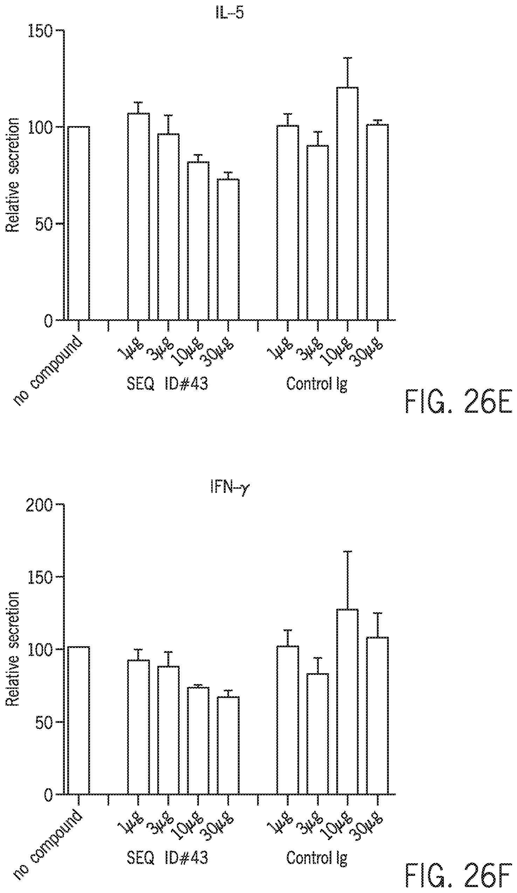

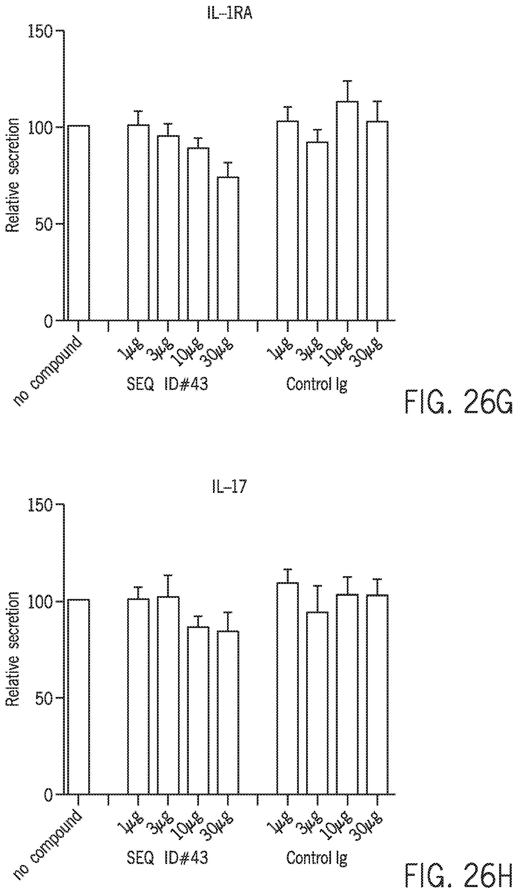

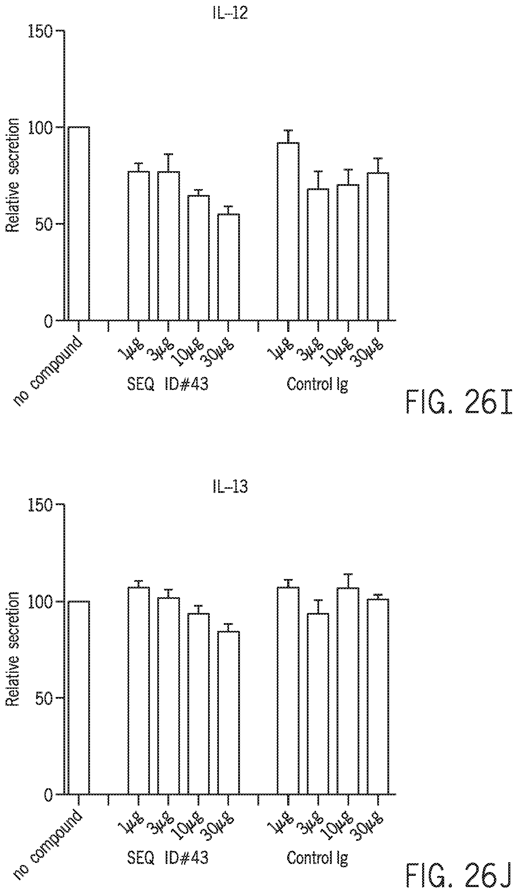

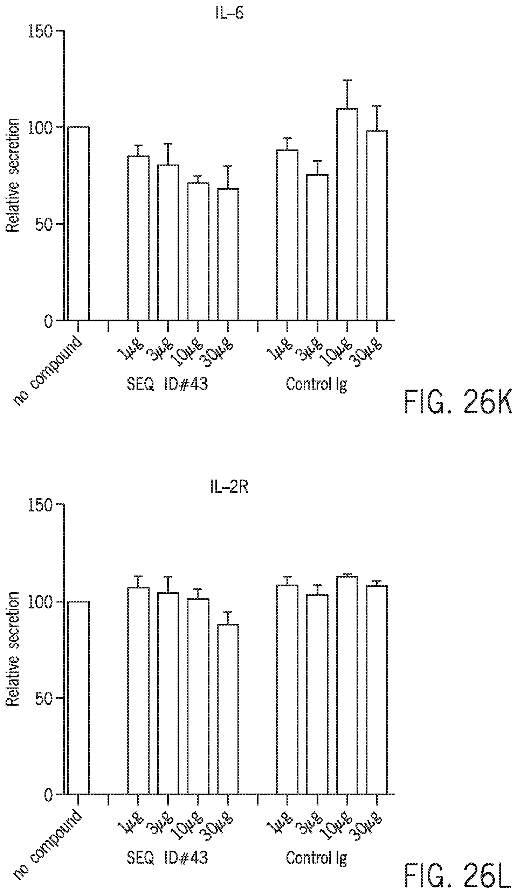

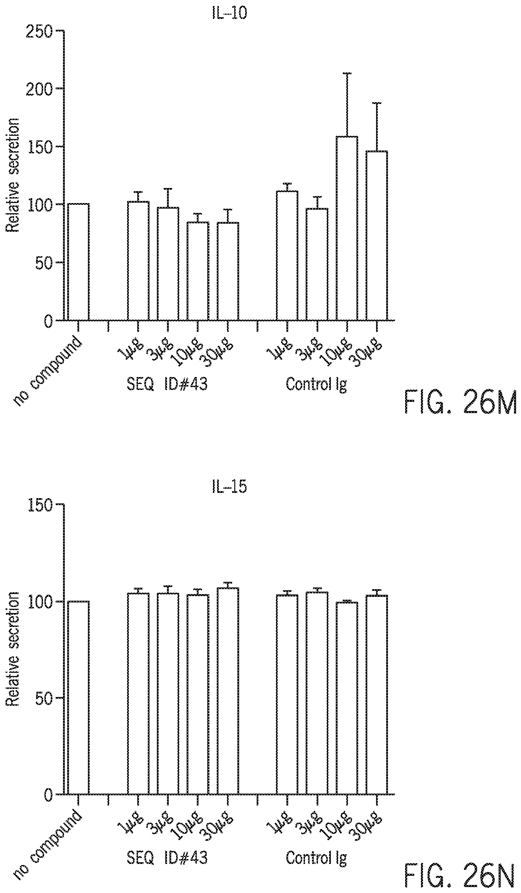

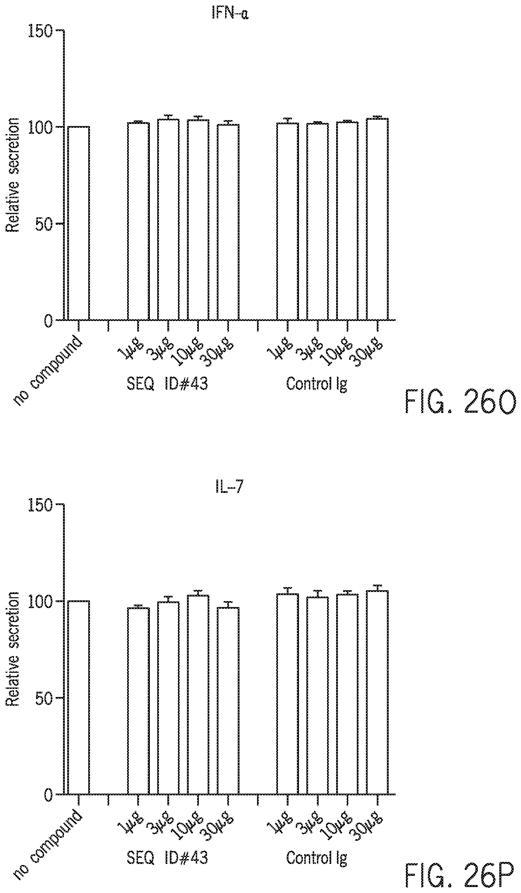

Figure 26 shows the effect of SEQ ID NO: 43 vs. control Ig on cytokine secretion by TcK:macrophages co-culture of healthy volunteers. Normalized data relative to 'no compound' for 4 donors is presented.

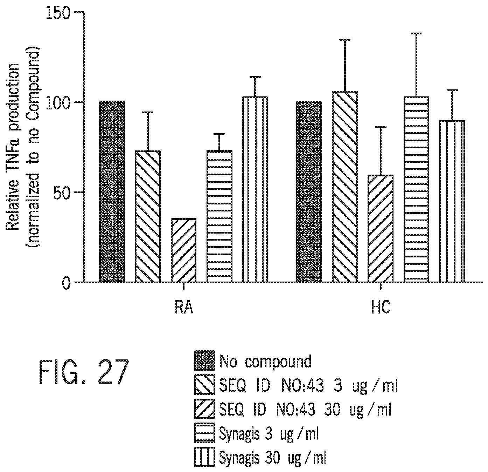

Figure 27 shows the effect of SEQ ID #43 on TNFa secretion by TcK: macrophages co-cultures from health controls (HC) and RA patients. Normalized data of 2 HCs and 2 RA patients is presented.

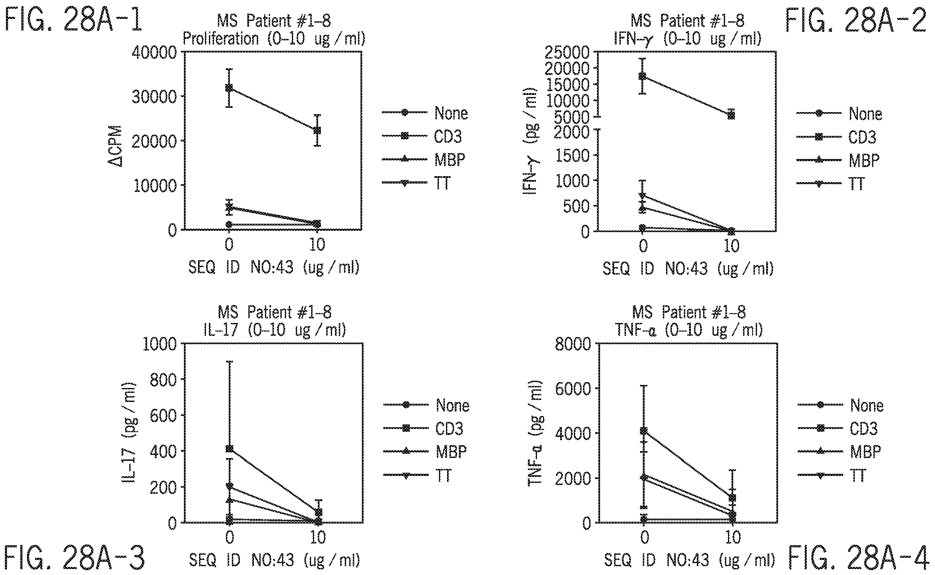

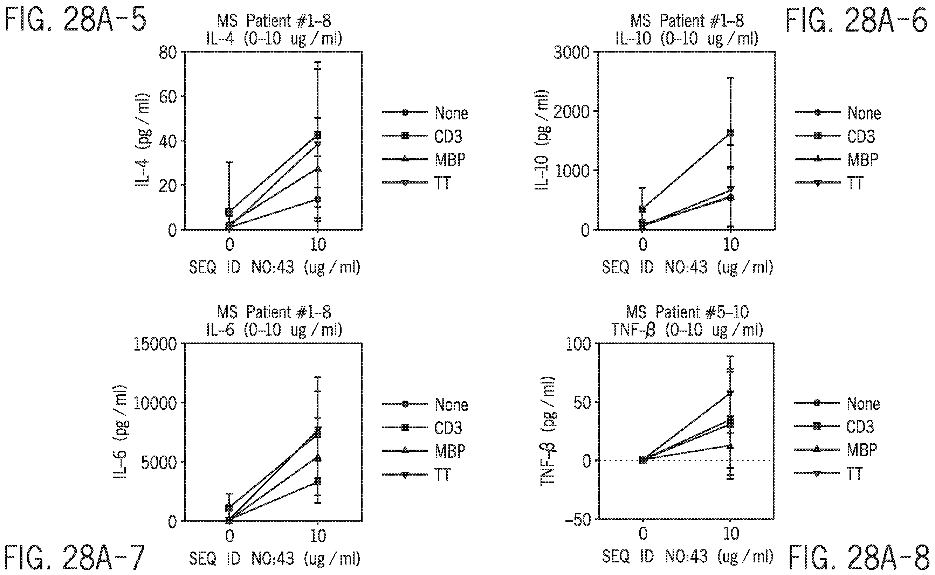

Figures 28A-28H show the effect of C1ORF32 ECD-Fc (SEQ ID NO: 43) on proliferation and on cytokine secretion following activation MS patients PBMCs with anti CD3, MBP₈₅₋₉₆, TT₈₃₀₋₈₄₃ or without activation. A. Data summary showing averages of the effect C1ORF32 ECD-Fc (SEQ ID NO: 43) at 10ug/ml vs. Control Ig (indicated as 0 ug/ml C1ORF32 ECD-Fc (SEQ ID NO: 43) on cell proliferation and cytokines secretion. Average data of five to eight donors tested is presented; B-I. Individual data showing the effect of C1ORF32 ECD-Fc (SEQ ID NO: 43) at the indicated concentrations vs. Control Ig (indicated as 0 ug/ml C1ORF32 ECD-Fc (SEQ ID NO: 43) on cell proliferation (B), IFNg (C), IL-17 (D), TNFa (E), IL-4 (F), IL-10 (G), IL-6 (H) and TGFβ (I).

### DETAILED DESCRIPTION OF THE INVENTION

The present disclosure relates to any one of the proteins referred to as C1ORF32, fragments, variants and homologs thereof and fusion proteins and conjugates containing same, and pharmaceutical compositions comprising same, and nucleic acid sequences encoding same, and the use thereof as a therapeutic agent for treatment of immune related disorder as described herein, including without limitation use of the ECD (extracellular domain) of a C1ORF32 protein, fragments and/or variants and/or homologs thereof (alone or as part of a fusion protein or conjugate).

Surprisingly, the inventors found that C1ORF ECD-Fc (SEQ ID NOs:42 and 43) had significant and profound effects on various models of human disease, performing at least as well as the current gold standard of care. As noted previously, there are two fusion proteins, a "human-human fusion protein" comprising an amino acid sequence of human C1ORF32 ECD fused to human immunoglobulin Fc (SEQ ID NO:43) and a "human-mouse fusion protein" comprising an amino acid sequence of human C1ORF32 ECD fused to mouse immunoglobulin Fc (SEQ ID NO:42).

For example, as shown in Example 1 (Figures 1-3), NOD mice (non-obese diabetic mice, a model of type I diabetes) benefitted significantly from treatment with C1ORF ECD-Fc (SEQ ID NO:42). Treatment began after the NOD mice had developed peri-insulitis and β-cell loss, such that the autoimmune disease had clearly become entrenched. Treatment of the root disease is very difficult at this point, because the autoimmune disease process is well under way, with a cascade of different pathological effects. Surprisingly, treatment with C1ORF ECD-Fc (SEQ ID NO:42) halted the disease in its tracks and prevented nearly all of the mice (77%) from developing full blown autoimmune diabetes. Moreover, the effect of treatment with C1ROF ECD-Fc (SEQ ID NO:42) was durable ans lasted long after cessation of treatment. These results also examplify the beneficial effect of "pre-disease" treatment (i.e. treatment of individuals that are prone to develop a disease based on acceptable biomarkers but which don't manifest disease symptoms) with C1ORF ECD-Fc.

Similarly, as shown in Example 5 (Figures 10-13), R-EAE mice (Relapsing Remitting Experimental Autoimmune Encephalomyelitis mice, a model of multiple sclerosis) benefitted significantly from treatment with C1ORF ECD-Fc (SEQ ID NO:42). When given during disease remission, C1ORF ECD-Fc (SEQ ID NO:42) was able to prevent disease relapse. As in the case of the NOD mice, even though the autoimmune disease process was well entrenched, with a cascade of active pathological effects - even though the disease was temporarily in remission, the underlying pathology was still active - C1ORF ECD-Fc (SEQ ID NO:42) treatment surprisingly stopped disease progression and allowed the mice to remain in a state of remission. Overall, C1ORF ECD-Fc (SEQ ID NO:42) treatment reduced inflammatory cytokines and reduced the inflammatory disease process of multiple sclerosis. These effects are representative of Th1/Th17 to Th2 shift - a true change in the underlying biological activity of the body and not a mere reduction in symptoms. C1ORF ECD-Fc (SEQ ID NO:42) treatment also reduced BBB (blood brain barrier) damage and vascular leakage. Again the effect of treatment was durable, and lasted long after cessation of treatment. Thus, C1ORF ECD-Fc (SEQ ID NO:42) treatment can actually be said to attack the root cause of the disease of multiple sclerosis, shifting the immune system in the body to a healthier balance and possibly restoration of immune tolerance to self-antigens.

Induction of immune tolerance by C1ORF ECD-Fc is further supported by the data presented in Examples 1-3 showing prevention of T1D development in NOD mice following a short course treatment with C1OFR ECD-Fc (SEQ ID NO:42) NOD mice spontaneously develop T1D around the age of 15-30 weeks. Induction of immune tolerance is further supported by example 4 which exemplify abolishment of transplant rejection upon treatment with by C1OFR ECD-Fc (SEQ ID NO:42). In this example, the abolishment of transplant rejection was accompanied by an increase in Tregs supporting a profound biological effect of Tregs in immune tolerance induction by C1ORF ECD-Fc.

As shown in Example 10 (Figures 18-19), in a mouse model of rheumatoid arthritis (known as the CIA, or collagen induced arthritis, mouse model), C1ORF ECD-Fc (SEQ ID NO:42) performed at least as well as the current gold standard of care, Enbrel. The group treated with C1ORF ECD-Fc (SEQ ID NO:42) shows delayed onset of disease symptoms and a reduced severity before onset of clinical symmptomes of rheumatoid arthritis supports "pre-disease" treatment of individulas at risk of developing a disease prior to manifestation of disease symptoms as also described for Example 1 above.

The beneficial effect of C1ORF ECD-Fc in treatment of autoimmune dieaseases, particularly rheumatoid arthritis was further supported with data obtained with blood cells taken from human rheumatoid arthritis patients, in which C1ORF ECD-Fc (SEQ ID NO:43) showed a strong beneficial effect similarly to that observed in the related animal models of these diseases (Examples 14-15). Thus, C1ORF ECD-Fc (SEQ ID NO:42 or SEQ ID NO:43 ) has been shown to provide significant therapeutic benefit in rheumatoid arthritis. Furthermore, in these examples individuals whos blood cells respond in a favorable manner to ex-vivo treatment with C1ORF ECD-Fc (SEQ ID NO:43) (i.e. decrease in Th1, Th17 and othe pro-inlalammatory cytokines such as for example GM-CSF and incresease in Th2 cytokines, IL-10 and TGFbeta) could be identified as individuls with high chances of benefitting from treatment with C1ORF ECD-Fc. Thus, such ex-vivo test for cytokine responses can serve for identifying responsive patients.

Similarly significantly strong effects were seen with the treatment of a humanized psoriatic mouse model with C1ORF ECD-Fc (SEQ ID NO:43) in Example 12 (Figures 20-24). Treatment was able to eliminate disease symptoms in about a third of the cases and to significantly reduce symptoms in many others. The efficacy was particularly interesting in that treatment employed the "human-human fusion protein" in a model involving human tissue, further supporting clinical relevance. In this model the disease is driven by human immune cells that are taken from psoriasis patients and further enriched ex-vivo to generate aggressive attach of the healthy human skin transplanted to these mice prior to transfer of these autoreactive immune cells.

Furthermore, C1ORF ECD-Fc (SEQ ID NO:43) was at least as effective as Enbrel in reducing psoriatic disease pathology and symptoms, as well as in terms of the percent of skin grafts which were free of psoriatic disease symptoms. C1ORF ECD-Fc (SEQ ID NO:43) also showed similar strength to dexamethasone in terms of reducing psoriatic disease pathology and symptoms. In addition, C1ORF32 ECD-Fc (SEQ ID NO: 43) and dexamethasone significantly reduced epidermal thickness compared with the vehicle-treated group, while no significant reduction in epidermal thickness was observed with Enbrel.

Example 16 shows an immunomodulatory activity of C1ORF32 ECD-Fc on peripheral blood cells from patients with active (relapsing/remitting) multiple sclerosis, indicating that the fusion protein is able to reduce or stop the immune processes leading to disease symptoms. This data support the therapeutic potential of C1ORF32 ECD-Fc in treating autoimmune diseases and in particular multiple sclerosis. The demonstrated induction of TGF-beta and IL-10 further support the effect of C1ORF32 ECD-Fc on regulatory immune functions including Tregs.

Furthermore such ex-vivo evaluation of the profile of cytokines secreted by patients' peripheral blood cells in resposne to C1ORF32 ECD-Fc could be used to identify patients that will potentially benefit from treatment with C1ORF32 ECD-Fc.

In order that the present invention may be more readily understood, certain terms are first defined. Additional definitions are set forth throughout the detailed description.

As used herein, if a plurality of serial integral values is given, then the series is assumed to include all integral values in between each integral value.

As used herein the term "isolated" refers to a compound of interest (for example a polynucleotide or a polypeptide) that is in an environment different from that in which the compound naturally occurs e.g. separated from its natural milieu such as by concentrating a peptide to a concentration at which it is not found in nature. "Isolated" includes compounds that are within samples that are substantially enriched for the compound of interest and/or in which the compound of interest is partially or substantially purified.

An "immune cell" refers to any cell from the hemopoietic origin including but not limited to T cells, B cells, monocytes, dendritic cells, and macrophages.

As used herein, the term "polypeptide" refers to a chain of amino acids of any length, regardless of modification (e.g., phosphorylation or glycosylation).

As used herein, a "costimulatory polypeptide" or "costimulatory molecule" is a polypeptide that, upon interaction with a cell-surface molecule on T cells, modulates T cell responses.

As used herein, a "costimulatory signaling" is the signaling activity resulting from the interaction between costimulatory polypeptides on antigen presenting cells and their receptors on T cells during antigen-specific T cell responses. Without wishing to be limited by a single hypothesis, the antigen-specific T cell response is believed to be mediated by two signals: 1) engagement of the T cell Receptor (TCR) with antigenic peptide presented in the context of MHC (signal 1), and 2) a second antigen-independent signal delivered by contact between different costimulatory receptor/ligand pairs (signal 2). Without wishing to be limited by a single hypothesis, this "second signal" is critical in determining the type of T cell response (activation vs inhibition) as well as the strength and duration of that response, and is regulated by both positive and negative signals from costimulatory molecules, such as the B7 family of proteins.

As used herein, the term "B7" polypeptide means a member of the B7 family of proteins that costimulate T cells including, but not limited to B7-1, B7-2, B7-DC, B7-H5, B7-H1, B7-H2, B7-H3, B7-H4, B7-H6, B7-S3 and biologically active fragments and/or variants thereof. Representative biologically active fragments include the extracellular domain or fragments of the extracellular domain that costimulate T cells.

As used herein, "inflammatory molecules" refers to molecules that induce inflammatory responses (directly or indirectly) including, but not limited to, cytokines and metalloproteases such as including, but not limited to, TNF-alpha, , IFN-gamma, IL-17, IL-23, IL-22, IL-21, GM-CSF, CCL3, CCL5 and MMPs.

As used herein, a "vector" is a replicon, such as a plasmid, phage, or cosmid, into which another DNA segment may be inserted so as to bring about the replication of the inserted segment. The vectors described herein can be expression vectors. As used herein, an "expression vector" is a vector that includes one or more expression control sequences

As used herein, an "expression control sequence" is a DNA sequence that controls and regulates the transcription and/or translation of another DNA sequence.

"Operably linked" refers to an arrangement of elements wherein the components so described are configured so as to perform their usual or intended function. Thus, two different polypeptides operably linked together retain their respective biological functions while physically linked together.

As used herein, "valency" refers to the number of binding sites available per molecule.

As used herein, a "variant" polypeptide contains at least one amino acid sequence alteration as compared to the amino acid sequence of the corresponding wild-type polypeptide.

As used herein, "conservative" amino acid substitutions are substitutions wherein the substituted amino acid has similar structural or chemical properties. As used herein, the term "host cell" refers to prokaryotic and eukaryotic cells into which a recombinant vector can be introduced.

As used herein, "transformed" and "transfected" encompass the introduction of a nucleic acid (e.g. a vector) into a cell by a number of techniques known in the art.

As used herein, the terms "immunologic", "immunological" or "immune" response is the development of a beneficial humoral (antibody mediated) and/or a cellular (mediated by antigen-specific T cells or their secretion products) response directed against a peptide in a recipient patient. Such a response can be an active response induced by administration of immunogen or a passive response induced by administration of antibody or primed T-cells. Without wishing to be limited by a single hypothesis, a cellular immune response is elicited by the presentation of polypeptide epitopes in association with Class I or Class II MHC molecules to activate antigen-specific CD4+ T helper cells and/or CD8+ cytotoxic T cells. The response may also involve activation of monocytes, macrophages, NK cells, basophils, dendritic cells, astrocytes, microglia cells, eosinophils, activation or recruitment of neutrophils or other components of innate immunity. The presence of a cell-mediated immunological response can be determined by proliferation assays (CD4+ T cells) or CTL (cytotoxic T lymphocyte) assays. The relative contributions of humoral and cellular responses to the protective or therapeutic effect of an immunogen can be distinguished by separately isolating antibodies and T-cells from an immunized syngeneic animal and measuring protective or therapeutic effect in a second subject.

An "immunogenic agent" or "immunogen" is capable of inducing an immunological response against itself on administration to a mammal, optionally in conjunction with an adjuvant.

As used herein, the term "C1ORF32" refers to the protein as set forth in any one of SEQ ID NOs: 1-5, variants and fragments thereof, plus any soluble ectodomain (ECD) of C1ORF32, and also to C1ORF32-ECD fusion polypeptides having a first fusion partner comprising all or a part of a C1ORF32 soluble polypeptide and a second fusion partner composed of a heterologous sequence (respectively non-C1ORF32), fused together directly or indirectly via a peptide linker sequence or a chemical linker, which can have a therapeutic effect on an immune related disorder. As used herein, the terms C1ORF32 fragments and/or C1ORF32 variants and/or C1ORF32 homologs refer to portions of C1ORF32 comprising amino acid sequence having a biological activity of inhibition of T cell activation.

As used herein the term "soluble C1ORF32" or "soluble ectodomain (ECD)" or "ectodomain" or "soluble C1ORF32 proteins/molecules" refers to fragments of C1ORF32 that include some or all of the extracellular domain of the C1ORF32 polypeptide, and lack some or all of the intracellular and/or transmembrane domains, wherein said fragments retain a biological activity of inhibition of T cell activation. In one embodiment, soluble C1ORF32 polypeptide fragments include the entire extracellular domain of the C1ORF32 polypeptide. In other embodiments, the soluble fragments of C1ORF32 polypeptides include fragments of the extracellular domain.

As used herein, the term "soluble C1ORF32" or "soluble ectodomain (ECD)" or "ectodomain" or "soluble C1ORF32 proteins/molecules" further means non-cell-surface-bound (i.e. circulating) C1ORF32 molecules or any portion of a C1ORF32 molecule including, but not limited to: C1ORF32 polypeptides, fragments or fusion proteins thereof fusion proteins, wherein the extracellular domain of C1ORF32 is fused to an immunoglobulin (Ig) moiety rendering the fusion molecule soluble, or fragments and derivatives thereof, proteins with the extracellular domain of C1ORF32 fused or joined with a portion of a biologically active or chemically active protein such as the papillomavirus E7 gene product, melanoma-associated antigen p97 or HIV env protein, or fragments and derivatives thereof; hybrid (chimeric) fusion proteins such as C1ORF32 polypeptides, fragments or fusion proteins thereof, or fragments and derivatives thereof. "Soluble C1ORF32 proteins/molecules" also include C1ORF32 molecules with the transmembrane domain removed to render the protein soluble, or fragments and derivatives thereof; and soluble C1ORF32 mutant molecules. The soluble C1ORF32 molecules used in the methods of the invention may or may not include a signal (leader) peptide sequence.

The term the "soluble ectodomain (ECD)" or "ectodomain" or "soluble" form of C1ORF32 refers also to the nucleic acid sequences encoding the corresponding proteins of C1ORF32 "soluble ectodomain (ECD)" or "ectodomain" or "soluble C1ORF32 proteins/molecules").

The C1ORF32 extracellular domain can contain one or more amino acids from the signal peptide or the putative transmembrane domain of C1ORF32. During secretion, the number of amino acids of the signal peptide that are cleaved can vary depending on the expression system and the host. Additionally or alternatively, fragments of C1ORF32 extracellular domain missing one or more amino acids from the C-terminus or the N-terminus that retain the ability to bind to the C1ORF32 receptor can be used as a fusion partner for the disclosed fusion proteins.

### Variants of C1ORF32 polypeptides

Useful variants of such C1ORF32 polypeptides include those that increase biological activity, as indicated by any of the assays described herein, or that increase half life or stability of the protein. Soluble C1ORF32 polypeptides and C1ORF32 fragments, or fusions thereof having C1ORF32 activity, can be engineered to increase biological activity. In a further embodiment, the C1ORF32 polypeptide or fusion protein has been modified with at least one amino acid substitution, deletion, or insertion that increases the binding of the molecule to an immune cell, for example a T cell, and transmits an inhibitory signal into the T cell.

Other optional variants are those C1ORF32 polypetpides that are engineered to selectively bind to one type of T cell versus other immune cells. For example, the C1ORF32 polypeptide can be engineered to bind optionally to Tregs, Th0, Th1, Th17, Th2 or Th22 cells. Preferential binding refers to binding that is at least 10%, 20%, 30%, 40%, 50%, 60%, 70%, 80%, 90%, 95%, or greater for one type of cell over another type of cell.

Still other variants of C1ORF32 can be engineered to have reduced binding to immune cells relative to wildtype C1ORF32. These variants can be used in combination with variants having stronger binding properties to modulate the immune response with a moderate impact.

Also optionally, variant C1ORF32 polypeptides can be engineered to have an increased half-life relative to wildtype. These variants typically are modified to resist enzymatic degradation. Exemplary modifications include modified amino acid residues and modified peptide bonds that resist enzymatic degradation. Various modifications to achieve this are known in the art.

### PROTEIN MODIFICATIONS

### FUSION PROTEINS

According to at least some embodiments, C1ORF32 fusion polypeptides have a first fusion partner comprising all or a part of a C1ORF32 protein fused to a second polypeptide directly or via a linker peptide sequence or a chemical linker useful to connect the two proteins. The C1 ORF32 polypeptide may optionally be fused to a second polypeptide to form a fusion protein as described herein. The presence of the second polypeptide can alter the solubility, stability, affinity and/or valency of the C1ORF32 fusion polypeptide. As used herein, "valency" refers to the number of binding sites available per molecule. In one embodiment the second polypeptide is a polypeptide from a different source or different protein.

According to at least some embodiments, the C1ORF32 protein or fragment is selected for its activity for the treatment of immune related disorder as described herein.

In one embodiment, the second polypeptide contains one or more domains of an immunoglobulin heavy chain constant region, preferably having an amino acid sequence corresponding to the hinge, CH2 and CH3 regions of a human immunoglobulin Cγ1, Cy2, Cy3 or Cy4 chain or to the hinge, CH2 and CH3 regions of a murine immunoglobulin Cy2a chain. SEQ ID NOs: 45, 46 are exemplary, non-limiting sequences for the hinge, CH2 and CH3 regions of a human immunoglobulin Cγ1.

According to at least some embodiments, the fusion protein is a dimeric fusion protein. In an optional dimeric fusion protein, the dimer results from the covalent bonding of Cys residue in the hinge region of two of the Ig heavy chains that are the same Cys residues that are disulfide linked in dimerized normal Ig heavy chains. Such proteins are referred to as C1ORF32 polypeptides, fragments or fusion proteins thereof.

In one embodiment, the immunoglobulin constant domain may contain one or more amino acid insertions, deletions or substitutions that enhance binding to specific cell types, increase the bioavailablity, or increase the stability of the C1ORF32 polypeptides, fusion proteins, or fragments thereof. Suitable amino acid substitutions include conservative and non-conservative substitutions, as described above.

The fusion proteins optionally contain a domain that functions to dimerize or multimerize two or more fusion proteins. The peptide/polypeptide linker domain can either be a separate domain, or alternatively can be contained within one of the other domains (C1ORF32 polypeptide or second polypeptide) of the fusion protein. Similarly, the domain that functions to dimerize or multimerize the fusion proteins can either be a separate domain, or alternatively can be contained within one of the other domains (C1ORF32 polypeptide, second polypeptide or peptide/polypeptide linker domain) of the fusion protein. In one embodiment, the dimerization/multimerization domain and the peptide/polypeptide linker domain are the same. Further specific, illustrative and non-limiting examples of dimerization/multimerization domains and linkers are given below.

Fusion proteins disclosed herein according to at least some embodiments of the present invention are of formula I: N-R1-R2-R3-C wherein "N" represents the N-terminus of the fusion protein, "C" represents the C-terminus of the fusion protein. In the further embodiment, "R1" is a C1ORF32 polypeptide, "R2" is an optional peptide/polypeptide or chemical linker domain, and "R3" is a second polypeptide. Alternatively, R3 may be a C1ORF32 polypeptide and R1 may be a second polypeptide.

Optionally, the fusion protein comprises the C1ORF32 polypeptide fragments as described herein, fused, optionally by a linker peptide of one or more amino acids (e.g. GS) to one or more "half-life extending moieties". A "half-life extending moiety" is any moiety, for example, a polypeptide, small molecule or polymer, that, when appended to protein, extends the in vivo half-life of that protein in the body of a subject (e.g., in the plasma of the subject). For example, a half-life extending moiety is, in an embodiment of the invention, polyethylene glycol (PEG), monomethoxy PEG (mPEG) or an immunoglobulin (Ig). In an embodiment of the invention, PEG is a 5, 10, 12, 20, 30, 40 or 50 kDa moiety or larger or comprises about 12000 ethylene glycol units (PEG12000).

The fusion protein may also optionally be prepared by chemical synthetic methods and the "join" effected chemically, either during synthesis or post-synthesis. Cross-linking and other such methods may optionally be used (optionally also with the above described genetic level fusion methods), as described for example in US Patent No. 5,547,853 to Wallner et al,

According to the present invention, a fusion protein may be prepared from a protein of the invention by fusion with a portion of an immunoglobulin comprising a constant region of an immunoglobulin. More preferably, the portion of the immunoglobulin comprises a heavy chain constant region which is optionally and more preferably a human heavy chain constant region. The heavy chain constant region is most preferably an IgG heavy chain constant region, and optionally and most preferably is an Fc chain, most preferably an IgG Fc fragment that comprises the hinge, CH2 and CH3 domains. The Fc chain may optionally be a known or "wild type" Fc chain, or alternatively may be mutated or truncated. The Fc portion of the fusion protein may optionally be varied by isotype or subclass, may be a chimeric or hybrid, and/or may be modified, for example to improve effector functions, control of half-life, tissue accessibility, augment biophysical characteristics such as stability, and improve efficiency of production (and less costly). Many modifications useful in construction of disclosed fusion proteins and methods for making them are known in the art, see for example Mueller, et al, MoI. Immun., 34(6):441-452 (1997), Swann, et al., Cur. Opin. Immun., 20:493-499 (2008), and Presta, Cur. Opin. Immun. 20:460-470 (2008). In some embodiments the Fc region is the native IgG1, IgG2, or IgG4 Fc region. In some embodiments the Fc region is a hybrid, for example a chimeric consisting of IgG2/IgG4 Fc constant regions.

Modications to the Fc region include, but are not limited to, IgG4 modified to prevent binding to Fc gamma receptors and complement, IgG1 modified to improve binding to one or more Fc gamma receptors, IgG1 modified to minimize effector function (amino acid changes), IgG1 with altered/ no glycan (typically by changing expression host or substuting the Asn at position 297), and IgG1 with altered pH- dependent binding to FcRn. The Fc region may include the entire hinge region, or less than the entire hinge region.

In another embodiment, the Fc domain may contain one or more amino acid insertions, deletions or substitutions that reduce binding to the low affinity inhibitory Fc receptor CD32B (FcyRIIB) and retain wild-type levels of binding to or enhance binding to the low affinity activating Fc receptor CD16A (FcyRIIIA).

Another embodiment includes IgG2-4 hybrids and IgG4 mutants that have reduced binding to FcR (Fc receptor) which increase their half life. Representative IgG2-4 hybrids and IgG4 mutants are described in Angal, S. et al., Molecular Immunology, 30(1):105-108 (1993); Mueller, J. et al., Molecular Immunology, 34(6): 441-452 (1997); and U.S. Patent No. 6,982,323 to Wang et al. In some embodiments the IgG1 and/or IgG2 domain is deleted; for example, Angal et al. describe IgG1 and IgG2 having serine 241 replaced with a proline.

In a further embodiment, the Fc domain contains amino acid insertions, deletions or substitutions that enhance binding to CD16A. A large number of substitutions in the Fc domain of human IgG1 that increase binding to CD16A and reduce binding to CD32B are known in the art and are described in Stavenhagen, et al., Cancer Res., 57(18):8882-90 (2007). Exemplary variants of human IgG1 Fc domains with reduced binding to CD32B and/or increased binding to CD16A contain F243L, R929P, Y300L, V305I or P296L substitutions. These amino acid substitutions may be present in a human IgG1 Fc domain in any combination.

In one embodiment, the human IgG1 Fc domain variant contains a F243L, R929P and Y300L substitution. In another embodiment, the human IgG1 Fc domain variant contains a F243L, R929P, Y300L, V3O5I and P296L substitution. In another embodiment, the human IgG1 Fc domain variant contains an N297A/Q substitution, as these mutations abolishFcyR binding. Non-limiting, illustrative, exemplary types of mutations are described in US Patent Application No. 20060034852, published on February 16, 2006. The term "Fc chain" also optionally comprises any type of Fc fragment.

Several of the specific amino acid residues that are important for antibody constant region-mediated activity in the IgG subclass have been identified. Inclusion, substitution or exclusion of these specific amino acids therefore allows for inclusion or exclusion of specific immunoglobulin constant region-mediated activity. Furthermore, specific changes may result in aglycosylation for example and/or other desired changes to the Fc chain. At least some changes may optionally be made to block a function of Fc which is considered to be undesirable, such as an undesirable immune system effect, as described in greater detail below.

Non-limiting, illustrative examples of mutations to Fc which may be made to modulate the activity of the fusion protein include the following changes (given with regard to the Fc sequence nomenclature as given by Kabat, from Kabat EA et al: Sequences of Proteins of Immunological Interest. US Department of Health and Human Services, NIH, 1991): 220C - > S; 233-238 ELLGGP - > EAEGAP; 265D - > A, preferably in combination with 434N -> A; 297N - > A (for example to block N-glycosylation); 318-322 EYKCK - > AYACA; 330-331AP - > SS; or a combination thereof (see for example M. Clark, "Chemical Immunol and Antibody Engineering", pp 1-31 for a description of these mutations and their effect). The construct for the Fc chain which features the above changes optionally and preferably comprises a combination of the hinge region with the CH2 and CH3 domains.

The above mutations may optionally be implemented to enhance desired properties or alternatively to block non-desired properties. For example, aglycosylation of antibodies was shown to maintain the desired binding functionality while blocking depletion of T-cells or triggering cytokine release, which may optionally be undesired functions (see M. Clark, "Chemical Immunol and Antibody Engineering", pp 1-31). Substitution of 331proline for serine may block the ability to activate complement, which may optionally be considered an undesired function (see M. Clark, "Chemical Immunol and Antibody Engineering", pp 1-31). Changing 330 alanine to serine in combination with this change may also enhance the desired effect of blocking the ability to activate complement.

Residues 235 and 237 were shown to be involved in antibody-dependent cell-mediated cytotoxicity (ADCC), such that changing the block of residues from 233-238 as described may also block such activity if ADCC is considered to be an undesirable function.

Residue 220 is normally a cysteine for Fc from IgG1, which is the site at which the heavy chain forms a covalent linkage with the light chain. Optionally, this residue may be changed to another amino acid residue (e.g., serine), to avoid any type of covalent linkage (see M. Clark, "Chemical Immunol and Antibody Engineering", pp 1-31) or by deletion or truncation.

The above changes to residues 265 and 434 may optionally be implemented to reduce or block binding to the Fc receptor, which may optionally block undesired functionality of Fc related to its immune system functions (see "Binding site on Human IgG1 for Fc Receptors", Shields et al, Vol 276, pp 6591-6604, 2001).

The above changes are intended as illustrations only of optional changes and are not meant to be limiting in any way. Furthermore, the above explanation is provided for descriptive purposes only, without wishing to be bound by a single hypothesis.

In a further embodiment, the Fc domain is composed of hybridization of hinge, CH1, CH2 and CH3 regions, which are involved in hinge flexibility, binding to Fcgamma and/or FcRn receptors, from different Fc isotypes; wherein the different Fc isotypes optionally and preferably comprise IgD and IgG4. Such "chimeric" Fc enables to control for example, affinity to the counterpart molecule (ligand/receptor), lytic activity (ADCC or CDC) and half-life (Im et al., PLoS One. 2011;6(9) Natural form of noncytolytic flexible human Fc as a long-acting carrier of agonistic ligand, erythropoietin).

In a further embodiment, the fusion protein includes the extracellular domain of C1ORF32, or a fragment thereof fused to an Ig Fc region. Recombinant Ig-C1ORF32 polypeptides, fragments or fusion proteins thereof fusion proteins can be prepared by fusing the coding region of the extracellular domain of C1ORF32 or a fragment thereof to the Fc region of human IgG1 or mouse IgG2a, as described previously (Chapoval, et al., Methods MoI. Med, 45:247-255 (2000)).

Optionally, C1ORF32 ECD refers also to fusion protein, comprising an amino acid sequence of human C1ORF32 ECD fused to human immunoglobulin Fc (human-human fusion protein). Optionally, said fusion protein comprises the amino acid sequence of the human C1ORF32 ECD set forth in any one of SEQ ID NOs: 6-41 fused to human IgG1 Fc set forth in any one of SEQ ID NOs:45, 46, 47, 65. Optionally, the amino acid sequence of said fusion protein is set forth in any one of SEQ ID NOs: 43, 64.
Optionally, C1ORF32 ECD refers also to a fusion protein comprising an amino acid sequence of human C1ORF32 ECD fused to mouse immunoglobulin Fc (human-mouse fusion protein. Optionally, said fusion protein comprises the amino acid sequence of the human C1ORF32 ECD set forth in any one of SEQ ID NOs: 6-41 fused to mouse IgG2a_Fc set forth in any of SEQ ID NOs:43, 44. Optionally, the amino acid sequence of said fusion protein is set forth in any one of SEQ ID NOs: 42, 62.

The aforementioned exemplary fusion proteins can incorporate any combination of the variants described herein. In another embodiment the terminal lysine of the aforementioned exemplary fusion proteins is deleted.

The disclosed fusion proteins can be isolated using standard molecular biology techniques. For example, an expression vector containing a DNA sequence encoding a C1ORF32 ECD polypeptides, fragments or fusion proteins thereof fusion protein is transfected into 293 cells by calcium phosphate precipitation and cultured in serum-free DMEM. The supernatant is collected at 72 h and the fusion protein is purified by Protein G, or preferably Protein A SEPHAROSE® columns (Pharmacia, Uppsala, Sweden). Optionally, a DNA sequence encoding a C1ORF32 ECD polypeptides, fragments or fusion proteins thereof fusion protein is transfected into GPEx® retrovectors and expressed in CHO-S cells following four rounds of retrovector transduction. The protein is clarified from supernatants using protein A chromatography.

In another embodiment the second polypeptide may have a conjugation domain through which additional molecules can be bound to the C1ORF32 ECD fusion proteins. In one such embodiment, the conjugated molecule is capable of targeting the fusion protein to a particular organ or tissue; further specific, illustrative, non-limiting examples of such targeting domains and/or molecules are given below.

In another such embodiment the conjugated molecule is another immunomodulatory agent that can enhance or augment the effects of the C1ORF32 ECD fusion protein. In another embodiment the conjugated molecule is Polyethylene Glycol (PEG).

### Peptide or polypeptide linker domain

The disclosed C1ORF32 ECD fusion proteins optionally contain a peptide or polypeptide linker domain that separates the C1ORF32 ECD polypeptide from the second polypeptide. In one embodiment, the linker domain contains the hinge region of an immunoglobulin. In a further embodiment, the hinge region is derived from a human immunoglobulin. Suitable human immunoglobulins that the hinge can be derived from include IgG, IgD and IgA. In a further embodiment, the hinge region is derived from human IgG. Amino acid sequences of immunoglobulin hinge regions and other domains are well known in the art. In one embodiment, C1ORF32 ECD fusion polypeptides contain the hinge, CH2 and CH3 regions of a human immunoglobulin Cγ1 chain, optionally with the Cys at position 220 (according to full length human IgG1, position 5 in SEQ ID NO:45) replaced with a Ser (SEQ ID NO: 46) having at least 85%, 90%, 95%, 99% or 100% sequence homology to amino acid sequence set forth in SEQ ID NO:45:

The hinge can be further shortened to remove amino acids 1, 2, 3, 4, 5, or combinations thereof of any one of SEQ ID NOs: 45, 46. In one embodiment, amino acids 1-5 of any one of SEQ ID NOs: 45, 46 are deleted. Exemplary C1ORF32 ECD fusion polypeptides comprised of the hinge, CH2 and CH3 regions of a human immunoglobulin Cγ1 chain with the Cys at position 220 repalced with a Ser are set forth in SEQ ID NOs:43.

In another embodiment, C1ORF32 ECD fusion polypeptides contain the CH2 and CH3 regions of a human immunoglobulin Cγ1 chain having at least 85%, 90%, 95%, 99% or 100% sequence homology to amino acid sequence set forth in SEQ ID NO:47:

In another embodiment, the C1ORF32 ECD fusion polypeptides contain the CH2 and CH3 regions of a murine immunoglobulin Cy2a chain at least 85%, 90%, 95%, 99% or 100% sequence homology to amino acid sequence set forth in SEQ ID NO: 44: EPRGPTIKPCPPCKCP APNLLGGPSVFIFPPKIKDVLMISLSPIVTCVVVDVSEDDPDV QISWFVNNVEVHTAQTQTHREDYNSTLRVVSALPIQHQDWMSGKEFKCKVNNKD LPAPIERTISKPKGSVRAPQVYVLPPPEEEMTKKQVTLTCMVTDFMPEDIYVEWTN NGKTELNYKNTEPVLDSDGSYFMYSKLRVEKKNWVERNSYSCSVVHEGLHNHHT TKSFSRTPGK. In another embodiment, the linker domain contains a hinge region of an immunoglobulin as described above, and further includes one or more additional immunoglobulin domains.

Other suitable peptide/polypeptide linker domains include naturally occurring or non-naturally occurring peptides or polypeptides. Peptide linker sequences are at least 2 amino acids in length. Optionally the peptide or polypeptide domains are flexible peptides or polypeptides. A "flexible linker" herein refers to a peptide or polypeptide containing two or more amino acid residues joined by peptide bond(s) that provides increased rotational freedom for two polypeptides linked thereby than the two linked polypeptides would have in the absence of the flexible linker. Such rotational freedom allows two or more antigen binding sites joined by the flexible linker to each access target antigen(s) more efficiently. Exemplary flexible peptides/polypeptides include, but are not limited to, the amino acid sequences Gly-Ser (SEQ ID NO:48), Gly-Ser-Gly-Ser (SEQ ID NO:49), Ala-Ser (SEQ ID NO:50), Gly-Gly-Gly-Ser (SEQ ID NO:51), Gly4-Ser (SEQ ID NO:52), (Gly4-Ser)2 (SEQ ID NO:53), (Gly4-Ser)3 (SEQ ID NO:54) and (Gly4-Ser)4 (SEQ ID NO: 55). Additional flexible peptide/polypeptide sequences are well known in the art. Other suitable peptide linker domains include the TEV linker ENLYFQG, a linear epitope recognized by the Tobacco Etch Virus protease. Exemplary peptides/polypeptides include, but are not limited to, GSENLYFQGSG (SEQ ID NO: 56). Other suitable peptide linker domains include helix forming linkers such as Ala-(Glu-Ala-Ala-Ala-Lys)n-Ala (n= 1-5). Additional helix forming peptide/polypeptide sequences are well known in the art. Non-limiting examples of such linkers are depicted in SEQ ID NOs:57-61.

### Dimerization, multimerization and targeting domains

The fusion proteins disclosed herein optionally contain a dimerization or multimerization domain that functions to dimerize or multimerize two or more fusion proteins. The domain that functions to dimerize or multimerize the fusion proteins can either be a separate domain, or alternatively can be contained within one of the other domains (C1ORF32 ECD polypeptide, second polypeptide, or peptide/polypeptide linker domain) of the fusion protein.

Dimerization or multinierization can occur between or among two or more fusion proteins through dimerization or multimerization domains. Alternatively, dimerization or multimerization of fusion proteins can occur by chemical crosslinking. The dimers or multimers that are formed can be homodimeric/homomultimeric or heterodimeric/ heteromultimeric.The second polypeptide "partner" in the C1ORF32 ECD fusion polypeptides may be comprised of one or more other proteins, protein fragments or peptides as described herein, including but not limited to any immunoglobulin (Ig) protein or portion thereof, preferably the Fc region, or a portion of a biologically or chemically active protein such as the papillomavirus E7 gene product, melanoma-associated antigen p97), and HIV env protein (gp120). The "partner" is optionally selected to provide a soluble dimer/multimer and/or for one or more other biological activities as described herein.

A "dimerization domain" is formed by the association of at least two amino acid residues or of at least two peptides or polypeptides (which may have the same, or different, amino acid sequences). The peptides or polypeptides may interact with each other through covalent and/or non- covalent associations). Optional dimerization domains contain at least one cysteine that is capable of forming an intermolecular disulfide bond with a cysteine on the partner fusion protein. The dimerization domain can contain one or more cysteine residues such that disulfide bond(s) can form between the partner fusion proteins. In one embodiment, dimerization domains contain one, two or three to about ten cysteine residues. In a further embodiment, the dimerization domain is the hinge region of an immunoglobulin.

Additional exemplary dimerization domains can be any known in the art and include, but not limited to, coiled coils, acid patches, zinc fingers, calcium hands, a C_{H}1-C_{L} pair, an "interface" with an engineered "knob" and/or "protruberance" as described in U.S. Patent No. 5,821,333, leucine zippers (e.g., fromjun and/or fos) (U.S. Patent No. 5,932,448), and/or the yeast transcriptional activator GCN4, SH2 (src homology 2), SH3 (src Homology 3) (Vidal, et al, Biochemistry, 43, 7336- 44 ((2004)), phosphotyrosine binding (PTB) (Zhou, et al., Nature, 378:584- 592 (1995)), WW (Sudol, Prog, Biochys. MoL Bio., 65:113-132 (1996)), PDZ (Kim, et al., Nature, 378: 85-88 (1995); Komau, et al, Science, 269.1737-1740 (1995)) 14-3-3, WD40 (Hu5 et al., J Biol Chem., 273, 33489- 33494 (1998)) EH, Lim, an isoleucine zipper, a receptor dimer pair (e.g., interleukin-8 receptor (IL-8R); and integrin heterodimers such as LFA-I and GPIIIb/IIIa), or the dimerization region(s) thereof, dimeric ligand polypeptides (e.g. nerve growth factor (NGF), neurotrophin-3 (NT-3), interleukin-8 (IL-8), vascular endothelial growth factor (VEGF), VEGF-C, VEGF-D, PDGF members, and brain-derived neurotrophic factor (BDNF) (Arakawa, et al., J Biol. Chem., 269(45): 27833-27839 (1994) and Radziejewski, et al., Biochem., 32(48): 1350 (1993)) and can also be variants of these domains in which the affinity is altered. The polypeptide pairs can be identified by methods known in the art, including yeast two hybrid screens. Yeast two hybrid screens are described in U.S. Pat. Nos. 5,283,173 and 6,562,576. Affinities between a pair of interacting domains can be determined using methods known in the art, including as described in Katahira, et at, J. Biol Chem, 277, 9242-9246 (2002)). Alternatively, a library of peptide sequences can be screened for heterodimerization, for example, using the methods described in WO 01/00814. Useful methods for protein-protein interactions are also described in U.S. Patent No. 6,790,624.

A "multimerization domain" is a domain that causes three or more peptides or polypeptides to interact with each other through covalent and/or non-covalent association(s). Suitable multimerization domains include, but are not limited to, coiled-coil domains. A coiled-coil is a peptide sequence with a contiguous pattern of mainly hydrophobic residues spaced 3 and 4 residues apart, usually in a sequence of seven amino acids (heptad repeat) or eleven amino acids (undecad repeat), which assembles (folds) to form a multimeric bundle of helices. Coiled-coils with sequences including some irregular distribution of the 3 and 4 residues spacing are also contemplated. Hydrophobic residues are in particular the hydrophobic amino acids Val, Ile, Leu, Met, Tyr, Phe and Trp. "Mainly hydrophobic" means that at least 50% of the residues must be selected from the mentioned hydrophobic amino acids.

The coiled coil domain may be derived from laminin. In the extracellular space, the heterotrimeric coiled coil protein laminin plays an important role in the formation of basement membranes. Apparently, the multifunctional oligomeric structure is required for laminin function. Coiled coil domains may also be derived from the thrombospondins in which three (TSP-I and TSP-2) or five (TSP-3, TSP-4 and TSP-5) chains are connected, or from COMP (COMPcc) (Guo, et at., EMBO J, 1998, 17: 5265-5272) which folds into a parallel five-stranded coiled coil (Malashkevich, et al., Science, 274: 761-765 (1996)). Additional non limiting examples of coiled-coil domains derived from other proteins, and other domains that mediate polypeptide multimerization are known in the art such as the vasodialator-stimulated phosphoprotein (VASP) domain, matrilin-1 (CMP), viral fusion peptides, soluble NSF (N-ethylmaleimide-sensitive factor) Attachment Protein receptor (SNARE) complexes, leucine-rich repeats, certain tRNA synthetases, are suitable for use in the disclosed fusion proteins.

In another embodiment, C1ORF32 ECD polypeptides, fusion proteins, or fragments thereof can be induced to form multimers by binding to a second multivalent polypeptide, such as an antibody. Antibodies suitable for use to multimerize C1ORF32 ECD polypeptides, fusion proteins, or fragments thereof include, but are not limited to, IgM antibodies and cross-linked, multivalent IgG, IgA, IgD, or IgE complexes.

Dimerization or multimerization can occur between or among two or more fusion proteins through dimerization or multimerization domains, including those described above. Alternatively, dimerization or multimerization of fusion proteins can occur by chemical crosslinking. Fusion protein dimers can be homodimers or heterodimers. Fusion protein multimers can be homomultimers or heteromultimers. Fusion protein dimers as disclosed herein are of formula II: N-R1-R2-R3-C N-R4-R5-R6-C or, alternatively, are of formula III: N-R1-R2-R3-C C-R4-R5-R6-N wherein the fusion proteins of the dimer provided by formula II are defined as being in a parallel orientation and the fusion proteins of the dimer provided by formula III are defined as being in an antiparallel orientation. Parallel and antiparallel dimers are also referred to as cis and trans dimers, respectively. "N" and "C" represent the N- and C-termini of the fusion protein, respectively. The fusion protein constituents "R1", "R2" and "R3" are as defined above with respect to formula I. With respect to both formula II and formula III, "R4" is a C1ORF32 ECD polypeptide or a second polypeptide, "R5" is an optional peptide/polypeptide linker domain, and "R6" is a C1ORF32 ECD polypeptide or a second polypeptide, wherein "R6" is a C1ORF32 ECD polypeptide when "R4" is a second polypeptide, and "R6"' is a second polypeptide when "R4" is a C1ORF32 ECD polypeptide. In one embodiment, "R1" is a C1ORF32 ECD polypeptide, "R4" is also a C1ORF32 ECD polypeptide, and "R3" and "R6" are both second polypeptides.

Fusion protein dimers of formula II are defined as homodimers when "R1" = "R4", "R2" = "R5" and "R3" = "R6". Similarly, fusion protein dimers of formula III are defined as homodimers when "R1" = "R6", "R2" = "R5" and "R3" = "R4". Fusion protein dimers are defined as heterodimers when these conditions are not met for any reason. For example, heterodimers may contain domain orientations that meet these conditions (i.e., for a dimer according to formula II, "R1" and "R4" are both C1ORF32 ECD polypeptides, "R2" and "R5" are both peptide/polypeptide linker domains and "R3" and "R6" are both second polypeptides), however the species of one or more of these domains is not identical. For example, although "R3" and "R6" may both be C1ORF32 ECD polypeptides, one polypeptide may contain a wild-type C1ORF32 ECD amino acid sequence while the other polypeptide may be a variant C1ORF32 ECD polypeptide. An exemplary variant C1ORF32 ECD polypeptide is C1ORF32 ECD, polypeptide that has been modified to have increased or decreased binding to a target cell, increased activity on immune cells, increased or decreased half life or stability. Dimers of fusion proteins that contain either a CHI or CL region of an immunoglobulin as part of the polypeptide linker domain preferably form heterodimers wherein one fusion protein of the dimer contains a CHI region and the other fusion protein of the dimer contains a CL region.

Fusion proteins can also be used to form multimers. As with dimers, multimers may be parallel multimers, in which all fusion proteins of the multimer are aligned in the same orientation with respect to their N- and C- termini. Multimers may be antiparallel multimers, in which the fusion proteins of the multimer are alternatively aligned in opposite orientations with respect to their N- and C-termini. Multimers (parallel or antiparallel) can be either homomultimers or heteromultimers. The fusion protein is optionally produced in dimeric form; more preferably, the fusion is performed at the genetic level as described below, by joining polynucleotide sequences corresponding to the two (or more) proteins, portions of proteins and/or peptides, such that a joined or fused protein is produced by a cell according to the joined polynucleotide sequence. A description of preparation for such fusion proteins is described with regard to US Patent No. 5,851,795 to Linsley et al,

### Targeting Domains

The C1ORF32 ECD polypeptides and fusion proteins can contain a targeting domain to target the molecule to specific sites in the body. Optional targeting domains target the molecule to areas of inflammation. Exemplary targeting domains are antibodies, or antigen binding fragments thereof that are specific for inflamed tissue or to a proinflammatory cytokine including but not limited to IL17, IL-4, IL-6, IL-12, IL-21, IL-22, and IL-23. In the case of neurological disorders such as Multiple Sclerosis, the targeting domain may target the molecule to the CNS or may bind to VCAM-I on the vascular epithelium. Additional targeting domains can be peptide aptamers specific for a proinflammatory molecule. In other embodiments, the C1ORF32 ECD fusion protein can include a binding partner specific for a polypeptide displayed on the surface of an immune cell, for example a T cell. In still other embodiments, the targeting domain specifically targets activated immune cells. Optional immune cells that are targeted include Th0, Th1, Th 17, Th2 and Th22 T cells, other cells that secrete, or cause other cells to secrete inflammatory molecules including, but not limited to, TNF-alpha, IFN-gamma, IL-17, IL-23, IL-22, IL-21, GM-CSF, CCL3, CCL5 and MMPs, and Tregs. For example, a targeting domain for Tregs may bind specifically to CD25. The above changes are intended as illustrations only of optional changes and are not meant to be limiting in any way. Furthermore, the above explanation is provided for descriptive purposes only, without wishing to be bound by a single hypothesis.

### ADDITION OF GROUPS

If a protein according to the present invention is a linear molecule, it is possible to place various functional groups at various points on the linear molecule which are susceptible to or suitable for chemical modification. Functional groups can be added to the termini of linear forms of the protein according to at least some embodiments of the invention. In some embodiments, the functional groups improve the activity of the protein with regard to one or more characteristics, including but not limited to, improvement in stability, penetration (through cellular membranes and/or tissue barriers), tissue localization, efficacy, decreased clearance, decreased toxicity, improved selectivity, improved resistance to expulsion by cellular pumps, and the like. For convenience sake and without wishing to be limiting, the free N-terminus of one of the sequences contained in the compositions according to at least some embodiments of the invention will be termed as the N-terminus of the composition, and the free C-terminal of the sequence will be considered as the C-terminus of the composition. Either the C-terminus or the N-terminus of the sequences, or both, can be linked to a carboxylic acid functional groups or an amine functional group, respectively.

Non-limiting examples of suitable functional groups are described in Green and Wuts, "Protecting Groups in Organic Synthesis", John Wiley and Sons, Chapters 5 and 7, 1991, Preferred protecting groups are those that facilitate transport of the active ingredient attached thereto into a cell, for example, by reducing the hydrophilicity and increasing the lipophilicity of the active ingredient, these being an example for "a moiety for transport across cellular membranes".

These moieties can optionally and preferably be cleaved in vivo, either by hydrolysis or enzymatically, inside the cell. (Ditter et al., J. Pharm. Sci. 57:783 (1968); Ditter et al., J. Pharm. Sci. 57:828 (1968); Ditter et al., J. Pharm. Sci. 58:557 (1969); King et al., Biochemistry 26:2294 (1987); Lindberg et al., Drug Metabolism and Disposition 17:311 (1989); and Tunek et al., Biochem. Pharm. 37:3867 (1988), Anderson et al., Arch. Biochem. Biophys. 239:538 (1985) and Singhal et al., FASEB J. 1:220 (1987)). Hydroxyl protecting groups include esters, carbonates and carbamate protecting groups. Amine protecting groups include alkoxy and aryloxy carbonyl groups, as described above for N-terminal protecting groups. Carboxylic acid protecting groups include aliphatic, benzylic and aryl esters, as described above for C-terminal protecting groups. In one embodiment, the carboxylic acid group in the side chain of one or more glutamic acid or aspartic acid residue in a composition of the present invention is protected, preferably with a methyl, ethyl, benzyl or substituted benzyl ester, more preferably as a benzyl ester.

Non-limiting, illustrative examples of N-terminal protecting groups include acyl groups (-CO-R1) and alkoxy carbonyl or aryloxy carbonyl groups (-CO-O-R1), wherein R1 is an aliphatic, substituted aliphatic, benzyl, substituted benzyl, aromatic or a substituted aromatic group. Specific examples of acyl groups include but are not limited to acetyl, (ethyl)-CO-, n-propyl-CO-, iso-propyl-CO-, n-butyl-CO-, sec-butyl-CO-, t-butyl-CO-, hexyl, lauroyl, palmitoyl, myristoyl, stearyl, oleoyl phenyl-CO-, substituted phenyl-CO-, benzyl-CO- and (substituted benzyl)-CO-. Examples of alkoxy carbonyl and aryloxy carbonyl groups include CH3-O-CO-, (ethyl)-O-CO-, n-propyl-O-CO-, iso-propyl-O-CO-, n-butyl-O-CO-, sec-butyl-O-CO-, t-butyl-O-CO-, phenyl-O- CO-, substituted phenyl-O-CO- and benzyl-O-CO-, (substituted benzyl)- O-CO-, Adamantan, naphtalen, myristoleyl, toluen, biphenyl, cinnamoyl, nitrobenzoy, toluoyl, furoyl, benzoyl, cyclohexane, norbornane, or Z-caproic. In order to facilitate the N-acylation, one to four glycine residues can be present in the N-terminus of the molecule.

The carboxyl group at the C-terminus of the compound can be protected, for example, by a group including but not limited to an amide (i.e., the hydroxyl group at the C-terminus is replaced with -NH ₂, -NHR₂ and -NR₂R₃) or ester (i.e. the hydroxyl group at the C-terminus is replaced with -OR₂). R₂ and R₃ are optionally independently an aliphatic, substituted aliphatic, benzyl, substituted benzyl, aryl or a substituted aryl group. In addition, taken together with the nitrogen atom, R₂ and R₃ can optionally form a C4 to C8 heterocyclic ring with from about 0-2 additional heteroatoms such as nitrogen, oxygen or sulfur. Non-limiting suitable examples of suitable heterocyclic rings include piperidinyl, pyrrolidinyl, morpholino, thiomorpholino or piperazinyl. Examples of C-terminal protecting groups include but are not limited to -NH₂, -NHCH₃, -N(CH₃)₂, -NH(ethyl), -N(ethyl)₂, -N(methyl) (ethyl), -NH(benzyl), -N(C1-C4 alkyl)(benzyl), -NH(phenyl), -N(C1-C4 alkyl) (phenyl), -OCH₃, -O-(ethyl), -O-(n-propyl), -O-(n-butyl), -O-(iso-propyl), -O-(sec- butyl), -O-(t-butyl), -O-be nzyl and -O-phenyl.

### SUBSTITUTION BY PEPTIDOMIMETIC MOIETIES

A "peptidomimetic organic moiety" can optionally be substituted for amino acid residues in the composition of this invention both as conservative and as non-conservative substitutions. These moieties are also termed "non-natural amino acids" and may optionally replace amino acid residues, amino acids or act as spacer groups within the peptides in lieu of deleted amino acids. The peptidomimetic organic moieties optionally and preferably have steric, electronic or configurational properties similar to the replaced amino acid and such peptidomimetics are used to replace amino acids in the essential positions, and are considered conservative substitutions. However such similarities are not necessarily required. According to preferred embodiments of the present invention, one or more peptidomimetics are selected such that the composition at least substantially retains its physiological activity as compared to the native protein according to the present invention.

Peptidomimetics may optionally be used to inhibit degradation of the peptides by enzymatic or other degradative processes. The peptidomimetics can optionally and preferably be produced by organic synthetic techniques. Non-limiting examples of suitable peptidomimetics include D amino acids of the corresponding L amino acids, tetrazol (Zabrocki et al., J. Am. Chem. Soc. 110:5875-5880 (1988)); isosteres of amide bonds (Jones et al., Tetrahedron Lett. 29: 3853-3856 (1988)); LL-3-amino-2-propenidone-6-carboxylic acid (LL-Acp) (Kemp et al., J. Org. Chem. 50:5834-5838 (1985)). Similar analogs are shown in Kemp et al., Tetrahedron Lett. 29:5081-5082 (1988) as well as Kemp et al., Tetrahedron Lett. 29:5057-5060 (1988), Kemp et al., Tetrahedron Lett. 29:4935-4938 (1988) and Kemp et al., J. Org. Chem. 54:109-115 (1987). Other suitable but exemplary peptidomimetics are shown in Nagai and Sato, Tetrahedron Lett. 26:647-650 (1985); Di Maio et al., J. Chem. Soc. Perkin Trans., 1687 (1985); Kahn et al., Tetrahedron Lett. 30:2317 (1989); Olson et al., J. Am. Chem. Soc. 112:323-333 (1990); Garvey et al., J. Org. Chem. 56:436 (1990). Further suitable exemplary peptidomimetics include hydroxy- 1,2,3,4-tetrahydroisoquinoline- 3-carboxylate (Miyake et al., J. Takeda Res. Labs 43:53-76 (1989)); 1,2,3,4-tetrahydro- isoquinoline-3-carboxylate (Kazmierski et al., J. Am. Chem. Soc. 133:2275-2283 (1991)); histidine isoquinolone carboxylic acid (HIC) (Zechel et al., Int. J. Pep. Protein Res. 43 (1991)); (2S, 3S)-methyl-phenylalanine, (2S, 3R)-methyl-phenylalanine, (2R, 3S)-methyl- phenylalanine and (2R, 3R)-methyl-phenylalanine (Kazmierski and Hruby, Tetrahedron Lett. (1991)).

Exemplary, illustrative but non-limiting non-natural amino acids include beta-amino acids (beta3 and beta2), homo-amino acids, cyclic amino acids, aromatic amino acids, Pro and Pyr derivatives, 3-substituted Alanine derivatives, Glycine derivatives, ring-substituted Phe and Tyr Derivatives, linear core amino acids or diamino acids. They are available from a variety of suppliers, such as Sigma-Aldrich (USA) for example.

### PROTEIN CHEMICAL MODIFICATIONS

In the present invention any part of a protein according to at least some embodiments of the invention may optionally be chemically modified, i.e. changed by addition of functional groups. For example the side amino acid residues appearing in the native sequence may optionally be modified, although as described below alternatively other parts of the protein may optionally be modified, in addition to or in place of the side amino acid residues. The modification may optionally be performed during synthesis of the molecule if a chemical synthetic process is followed, for example by adding a chemically modified amino acid. However, chemical modification of an amino acid when it is already present in the molecule ("in situ" modification) is also possible.

The amino acid of any of the sequence regions of the molecule can optionally be modified according to any one of the following exemplary types of modification (in the peptide conceptually viewed as "chemically modified"). Non-limiting exemplary types of modification include carboxymethylation, acylation, phosphorylation, glycosylation or fatty acylation. Ether bonds can optionally be used to join the serine or threonine hydroxyl to the hydroxyl of a sugar. Amide bonds can optionally be used to join the glutamate or aspartate carboxyl groups to an amino group on a sugar (Garg and Jeanloz, Advances in Carbohydrate Chemistry and Biochemistry, Vol. 43, Academic Press (1985); Kunz, Ang. Chem. Int. Ed. English 26:294-308 (1987)). Acetal and ketal bonds can also optionally be formed between amino acids and carbohydrates. Fatty acid acyl derivatives can optionally be made, for example, by acylation of a free amino group (e.g., lysine) (Toth et al., Peptides: Chemistry, Structure and Biology, Rivier and Marshal, eds., ESCOM Publ., Leiden, 1078-1079 (1990)).

As used herein the term "chemical modification", when referring to a protein or peptide according to the present invention, refers to a protein or peptide where at least one of its amino acid residues is modified either by natural processes, such as processing or other post-translational modifications, or by chemical modification techniques which are well known in the art. Examples of the numerous known modifications typically include, but are not limited to: acetylation, acylation, amidation, ADP-ribosylation, glycosylation, GPI anchor formation, covalent attachment of a lipid or lipid derivative, methylation, myristylation, pegylation, prenylation, phosphorylation, ubiquitination, or any similar process.

Other types of modifications optionally include the addition of a cycloalkane moiety to a biological molecule, such as a protein, as described in PCT Application No. WO 2006/050262, These moieties are designed for use with biomolecules and may optionally be used to impart various properties to proteins.

Furthermore, optionally any point on a protein may be modified. For example, pegylation of a glycosylation moiety on a protein may optionally be performed, as described in PCT Application No. WO 2006/050247, One or more polyethylene glycol (PEG) groups may optionally be added to O-linked and/or N-linked glycosylation. The PEG group may optionally be branched or linear. Optionally any type of water-soluble polymer may be attached to a glycosylation site on a protein through a glycosyl linker.

### ALTERED GLYCOSYLATION

Proteins according to at least some embodiments of the invention may be modified to have an altered glycosylation pattern (i.e., altered from the original or native glycosylation pattern). As used herein, "altered" means having one or more carbohydrate moieties deleted, and/or having at least one glycosylation site added to the original protein.

Glycosylation of proteins is typically either N-linked or O-linked. N-linked refers to the attachment of the carbohydrate moiety to the side chain of an asparagine residue. The tripeptide sequences, asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may also be used.

Addition of glycosylation sites to proteins according to at least some embodiments of the invention is conveniently accomplished by altering the amino acid sequence of the protein such that it contains one or more of the above-described tripeptide sequences (for N-linked glycosylation sites). The alteration may also be made by the addition of, or substitution by, one or more serine or threonine residues in the sequence of the original protein (for O-linked glycosylation sites). The protein's amino acid sequence may also be altered by introducing changes at the DNA level.

Another means of increasing the number of carbohydrate moieties on proteins is by chemical or enzymatic coupling of glycosides to the amino acid residues of the protein. Depending on the coupling mode used, the sugars may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330, and in Aplin and Wriston, CRC Crit. Rev. Biochem., 22: 259-306 (1981).

Removal of any carbohydrate moieties present on proteins according to at least some embodiments of the invention may be accomplished chemically or enzymatically. Chemical deglycosylation requires exposure of the protein to trifluoromethanesulfonic acid, or an equivalent compound. This treatment results in the cleavage of most or all sugars except the linking sugar (N-acetylglucosamine or N-acetylgalactosamine), leaving the amino acid sequence intact.

Chemical deglycosylation is described by Hakimuddin et al., Arch. Biochem. Biophys., 259: 52 (1987); and Edge et al., Anal. Biochem., 118: 131 (1981). Enzymatic cleavage of carbohydrate moieties on proteins can be achieved by the use of a variety of endo-and exo-glycosidases as described by Thotakura et al., Meth. Enzymol., 138: 350 (1987).

The terms "individual", "host", "subject", and "patient" are used interchangeably herein, and refer any human or nonhuman animal. The term "nonhuman animal" includes all vertebrates, e.g., mammals and non-mammals, such as nonhuman primates, sheep, dogs, cats, horses, cows chickens, amphibians, reptiles, etc.

Various aspects of the invention are described in further detail in the following subsections.

### PEPTIDES

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, e.g., by the addition of carbohydrate residues to form glycoproteins. The terms "polypeptide," "peptide" and "protein" include glycoproteins, as well as non-glycoproteins.

Polypeptide products can be biochemically synthesized such as by employing standard solid phase techniques. Such methods include exclusive solid phase synthesis, partial solid phase synthesis methods, fragment condensation, classical solution synthesis. These methods are preferably used when the peptide is relatively short (i.e., 10 kDa) and/or when it cannot be produced by recombinant techniques (i.e., not encoded by a nucleic acid sequence) and therefore involves different chemistry.

Solid phase polypeptide synthesis procedures are well known in the art and further described by John Morrow Stewart and Janis Dillaha Young, Solid Phase Peptide Syntheses (2nd Ed., Pierce Chemical Company, 1984).

Synthetic polypeptides can be purified by preparative high performance liquid chromatography [Creighton T. (1983) Proteins, structures and molecular principles. WH Freeman and Co. N.Y.] and the composition of which can be confirmed via amino acid sequencing.

In cases where large amounts of a polypeptide are desired, it can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

It will be appreciated that peptides identified according to the teachings of the present invention may be degradation products, synthetic peptides or recombinant peptides as well as peptidomimetics, typically, synthetic peptides and peptoids and semipeptoids which are peptide analogs, which may have, for example, modifications rendering the peptides more stable while in a body or more capable of penetrating into cells. Such modifications include, but are not limited to N terminus modification, C terminus modification, peptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press Further details in this respect are provided hereinunder.

Peptide bonds (-CO-NH-) within the peptide may be substituted, for example, by N-methylated bonds (-N(CH3)-CO-), ester bonds (-C(R)H-C-O-O-C(R)-N-), ketomethylen bonds (-CO-CH2-), α-aza bonds (-NH-N(R)-CO-), wherein R is any alkyl, e.g., methyl, carba bonds (-CH2-NH-), hydroxyethylene bonds (-CH(OH)-CH2-), thioamide bonds (-CS-NH-), olefinic double bonds (-CH=CH-), retro amide bonds (-NH-CO-), peptide derivatives (-N(R)-CH2-CO-), wherein R is the "normal" side chain, naturally presented on the carbon atom.

These modifications can occur at any of the bonds along the peptide chain and even at several (2-3) at the same time.

Natural aromatic amino acids, Trp, Tyr and Phe, may be substituted by synthetic non-natural acid such as Phenylglycine, TIC, naphthylelanine (Nol), ring-methylated derivatives of Phe, halogenated derivatives of Phe or o-methyl-Tyr.

In addition to the above, the peptides of the present invention may also include one or more modified amino acids or one or more non-amino acid monomers (e.g. fatty acids, complex carbohydrates etc).

As used herein in the specification and in the claims section below the term "amino acid" or "amino acids" is understood to include the 20 naturally occurring amino acids; those amino acids often modified post-translationally in vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

Since the peptides of the present invention are preferably utilized in therapeutics which require the peptides to be in soluble form, the peptides of the present invention preferably include one or more non-natural or natural polar amino acids, including but not limited to serine and threonine which are capable of increasing peptide solubility due to their hydroxyl-containing side chain.

In cases where large amounts of the peptides of the present invention are desired, the peptides of the present invention can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

### EXPRESSION SYSTEMS

To enable cellular expression of the polynucleotides of the present invention, a nucleic acid construct may be used, which includes at least a coding region of one of the above nucleic acid sequences, and further includes at least one cis acting regulatory element. As used herein, the phrase "cis acting regulatory element" refers to a polynucleotide sequence, preferably a promoter, which binds a trans acting regulator and regulates the transcription of a coding sequence located downstream thereto.

Any suitable promoter sequence can be used by the nucleic acid construct.

Preferably, the promoter utilized by the nucleic acid construct is active in the specific cell population transformed. Examples of cell type-specific and/or tissue-specific promoters include promoters such as albumin that is liver specific [Pinkert et al., (1987) Genes Dev. 1:268-277], lymphoid specific promoters [Calame et al., (1988) Adv. Immunol. 43:235-275]; in particular promoters of T-cell receptors [Winoto et al., (1989) EMBO J. 8:729-733] and immunoglobulins; [Banerji et al. (1983) Cell 33729-740], neuron-specific promoters such as the neurofilament promoter [Byrne et al. (1989) Proc. Natl. Acad. Sci. USA 86:5473-5477], pancreas-specific promoters [Edlunch et al. (1985) Science 230:912-916] or mammary gland-specific promoters such as the milk whey promoter (U.S. Pat. No. 4,873,316 and European Application Publication No. 264,166). The nucleic acid construct of the present invention can further include an enhancer, which can be adjacent or distant to the promoter sequence and can function in up regulating the transcription therefrom.

The nucleic acid construct preferably further includes an appropriate selectable marker and/or an origin of replication. Preferably, the nucleic acid construct utilized is a shuttle vector, which can propagate both in E. coli (wherein the construct comprises an appropriate selectable marker and origin of replication) and be compatible for propagation in cells, or integration in a gene and a tissue of choice. The construct can be, for example, a plasmid, a bacmid, a phagemid, a cosmid, a phage, a virus or an artificial chromosome.

Examples of suitable constructs include, but are not limited to, pcDNA3, pcDNA3.1 (+/-), pGL3, PzeoSV2 (+/-), pDisplay, pEF/myc/cyto, pCMV/myc/cyto each of which is commercially available from Invitrogen Co. (www.invitrogen.com). Examples of retroviral vector and packaging systems are those sold by Clontech, San Diego, Calif., including Retro-X vectors pLNCX and pLXSN, which permit cloning into multiple cloning sites and the transgene is transcribed from CMV promoter. Vectors derived from Mo-MuLV are also included such as pBabe, where the transgene will be transcribed from the 5'LTR promoter.

Currently preferred in vivo nucleic acid transfer techniques include transfection with viral or non-viral constructs, such as adenovirus, lentivirus, Herpes simplex I virus, or adeno-associated virus (AAV) and lipid-based systems. Useful lipids for lipid-mediated transfer of the gene are, for example, DOTMA, DOPE, and DC-Chol [Tonkinson et al., Cancer Investigation, 14(1): 54-65 (1996)]. The most preferred constructs for use in gene therapy are viruses, most preferably adenoviruses, AAV, lentiviruses, or retroviruses. A viral construct such as a retroviral construct includes at least one transcriptional promoter/enhancer or locus-defining elements, or other elements that control gene expression by other means such as alternate splicing, nuclear RNA export, or post-translational modification of messenger. Such vector constructs also include a packaging signal, long terminal repeats (LTRs) or portions thereof, and positive and negative strand primer binding sites appropriate to the virus used, unless it is already present in the viral construct. In addition, such a construct typically includes a signal sequence for secretion of the peptide from a host cell in which it is placed. Preferably the signal sequence for this purpose is a mammalian signal sequence or the signal sequence of the polypeptides of the present invention. Optionally, the construct may also include a signal that directs polyadenylation, as well as one or more restriction sites and a translation termination sequence. By way of example, such constructs will typically include a 5' LTR, a tRNA binding site, a packaging signal, an origin of second-strand DNA synthesis, and a 3' LTR or a portion thereof. Other vectors can be used that are non-viral, such as cationic lipids, polylysine, and dendrimers.

### PROTEIN CHEMICAL MODIFICATIONS

In the present invention any part of a protein of the invention may optionally be chemically modified, i.e. changed by addition of functional groups. For example the side amino acid residues appearing in the native sequence may optionally be modified, although as described below alternatively other parts of the protein may optionally be modified, in addition to or in place of the side amino acid residues. The modification may optionally be performed during synthesis of the molecule if a chemical synthetic process is followed, for example by adding a chemically modified amino acid. However, chemical modification of an amino acid when it is already present in the molecule ("in situ" modification) is also possible.

The amino acid of any of the sequence regions of the molecule can optionally be modified according to any one of the following exemplary types of modification (in the peptide conceptually viewed as "chemically modified"). Non-limiting exemplary types of modification include carboxymethylation, acylation, phosphorylation, glycosylation or fatty acylation. Ether bonds can optionally be used to join the serine or threonine hydroxyl to the hydroxyl of a sugar. Amide bonds can optionally be used to join the glutamate or aspartate carboxyl groups to an amino group on a sugar (Garg and Jeanloz, Advances in Carbohydrate Chemistry and Biochemistry, Vol. 43, Academic Press (1985); Kunz, Ang. Chem. Int. Ed. English 26:294-308 (1987)). Acetal and ketal bonds can also optionally be formed between amino acids and carbohydrates. Fatty acid acyl derivatives can optionally be made, for example, by acylation of a free amino group (e.g., lysine) (Toth et al., Peptides: Chemistry, Structure and Biology, Rivier and Marshal, eds., ESCOM Publ., Leiden, 1078-1079 (1990)).

As used herein the term "chemical modification", when referring to a protein or peptide according to the present invention, refers to a protein or peptide where at least one of its amino acid residues is modified either by natural processes, such as processing or other post-translational modifications, or by chemical modification techniques which are well known in the art. Examples of the numerous known modifications typically include, but are not limited to: acetylation, acylation, amidation, ADP-ribosylation, glycosylation, GPI anchor formation, covalent attachment of a lipid or lipid derivative, methylation, myristylation, pegylation, prenylation, phosphorylation, ubiquitination, or any similar process.

Other types of modifications optionally include the addition of a cycloalkane moiety to a biological molecule, such as a protein, as described in PCT Application No. WO 2006/050262, These moieties are designed for use with biomolecules and may optionally be used to impart various properties to proteins.

Furthermore, optionally any point on a protein may be modified. For example, pegylation of a glycosylation moiety on a protein may optionally be performed, as described in PCT Application No. WO 2006/050247, One or more polyethylene glycol (PEG) groups may optionally be added to O-linked and/or N-linked glycosylation. The PEG group may optionally be branched or linear. Optionally any type of water-soluble polymer may be attached to a glycosylation site on a protein through a glycosyl linker.

### ALTERED GLYCOSYLATION PROTEIN MODIFICATION

Proteins of the invention may be modified to have an altered glycosylation pattern (i.e., altered from the original or native glycosylation pattern). As used herein, "altered" means having one or more carbohydrate moieties deleted, and/or having at least one glycosylation site added to the original protein.

Glycosylation of proteins is typically either N-linked or O-linked. N-linked refers to the attachment of the carbohydrate moiety to the side chain of an asparagine residue. The tripeptide sequences, asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may also be used.

Addition of glycosylation sites to proteins of the invention is conveniently accomplished by altering the amino acid sequence of the protein such that it contains one or more of the above-described tripeptide sequences (for N-linked glycosylation sites). The alteration may also be made by the addition of, or substitution by, one or more serine or threonine residues in the sequence of the original protein (for O-linked glycosylation sites). The protein's amino acid sequence may also be altered by introducing changes at the DNA level.

Another means of increasing the number of carbohydrate moieties on proteins is by chemical or enzymatic coupling of glycosides to the amino acid residues of the protein. Depending on the coupling mode used, the sugars may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330, and in Aplin and Wriston, CRC Crit. Rev. Biochem., 22: 259-306 (1981).

Removal of any carbohydrate moieties present on proteins of the invention may be accomplished chemically, enzymatically or by introducing changes at the DNA level. Chemical deglycosylation requires exposure of the protein to trifluoromethanesulfonic acid, or an equivalent compound. This treatment results in the cleavage of most or all sugars except the linking sugar (N-acetylglucosamine or N-acetylgalactosamine), leaving the amino acid sequence intact.

Chemical deglycosylation is described by Hakimuddin et al., Arch. Biochem. Biophys., 259: 52 (1987); and Edge et al., Anal. Biochem., 118: 131 (1981). Enzymatic cleavage of carbohydrate moieties on proteins can be achieved by the use of a variety of endo- and exo-glycosidases as described by Thotakura et al., Meth. Enzymol., 138: 350 (1987).

### METHODS OF TREATMENT

As mentioned hereinabove the C1ORF32 proteins and polypeptides of the present invention as fusion proteins, preferably of the ectodomain or secreted forms of C1ORF32 proteins, can be used to treat any immune related disorder as described herein.

As used herein the term "treating" refers to preventing, delaying the onset of, curing, reversing, attenuating, alleviating, minimizing, suppressing or halting the deleterious effects of the above-described diseases, disorders or conditions. It also includes managing the disease as described above. By "manage" it is meant reducing the severity of the disease, reducing the frequency of episodes of the disease, reducing the duration of such episodes, reducing the severity of such episodes and the like.

Treating, according to the present invention, can be effected by specifically upregulating the amount and/or the expression of at least one of the polypeptides of the present invention in the subject.

Optionally, upregulation may be effected by administering to the subject at least one of the polypeptides of the present invention (e.g., recombinant or synthetic) or an active portion thereof, as described herein. The polypeptide or peptide may optionally be administered in as part of a pharmaceutical composition, described in more detail below.

It will be appreciated that treatment of the above-described diseases according to at least some embodiments of the present invention may be combined with other treatment methods known in the art useful for treating immune related condition (i.e., combination therapy), as described herein.

The treatment of above-described diseases according to at least some embodiments of the present invention is combined with identification of individuals at risk for developing an autoimmune disease or condition and treatment according to the present invention initiated prior to full manifestation of disease symptoms.

Optionally the moiety is selected from the group consisting of immunosuppressants such as corticosteroids, cyclosporin, cyclophosphamide, prednisone, azathioprine, methotrexate, rapamycin, tacrolimus, leflunomide or an analog thereof; mizoribine; mycophenolic acid; mycophenolate mofetil; 15-deoxyspergualine or an analog thereof; biological agents such as TNF-alpha blockers or antagonists, or any other biological agent targeting any inflammatory cytokine, nonsteroidal antiinflammatory drugs/Cox-2 inhibitors, hydroxychloroquine, sulphasalazopryine, gold salts, etanercept, infliximab, mycophenolate mofetil, basiliximab, atacicept, rituximab, cytoxan, interferon beta-la, interferon beta-lb, glatiramer acetate, mitoxantrone hydrochloride, anakinra and/or other biologics and/or intravenous immunoglobulin (IVIG), interferons such as IFN-beta-1a (REBIF®. AVONEX® and CINNOVEX ®) and IFN-beta-1b (BETASERON®); EXTAVIA®, BETAFERON®, ZIFERON®); glatiramer acetate (COPAXONE®), a polypeptide; natalizumab (TYSABRI®), mitoxantrone (NOVANTRONE®), a cytotoxic agent, a calcineurin inhibitor, e.g. cyclosporin A or FK506; an immunosuppressive macrolide, e.g. rapamycine or a derivative thereof; e.g. 40-O-(2-hydroxy)ethyl-rapamycin, a lymphocyte homing agent, e.g. FTY720 or an analog thereof, corticosteroids; cyclophosphamide; azathioprene; methotrexate; leflunomide or an analog thereof; mizoribine; mycophenolic acid; mycophenolate mofetil; 15-deoxyspergualine or an analog thereof; immunosuppressive monoclonal antibodies, e.g., monoclonal antibodies to leukocyte receptors, e.g., MHC, CD2, CD3, CD4, CD11a/CD18, CD7, CD25, CD27, B7, CD40, CD45, CD58, CD137, ICOS, CD150 (SLAM), OX40, 4-1BB or their ligands; or other immunomodulatory compounds, e.g. CTLA4-Ig (abatacept, ORENCIA®, belatacept), CD28-Ig, B7-H4-Ig, or other costimulatory agents, or adhesion molecule inhibitors, e.g. mAbs or low molecular weight inhibitors including LFA-1 antagonists, Selectin antagonists and VLA-4 antagonists, or another immunomodulatory agent.

Thus, treatment of rheumatoid arthritis, using the agents according to at least some embodiments of the present invention may be combined with, for example, any known therapeutic agent or method for treating rheumatoid arthritis. Non-limiting examples of such known therapeutic agents or methods for treating rheumatoid arthritis include glucocorticoids, nonsteroidal anti-inflammatory drug (NSAID) such as salicylates, or cyclooxygenase-2 inhibitors, ibuprofen and naproxen, diclofenac, indomethacin, etodolac Disease-modifying antirheumatic drugs (DMARDs)- Oral DMARDs: Auranofin (Ridaura), Azathioprine (Imuran), Cyclosporine (Sandimmune, Gengraf, Neoral, generic), D-Penicillamine (Cuprimine), Hydroxychloroquine (Plaquenil), IM gold Gold sodium thiomalate (Myochrysine) Aurothioglucose (Solganal), Leflunomide (Arava), Methotrexate (Rheumatrex), Minocycline (Minocin), Staphylococcal protein A immunoadsorption (Prosorba column), Sulfasalazine (Azulfidine). Biologic DMARDs: TNF-α blockers including Adalimumab (Humira), Etanercept (Enbrel), Infliximab (Remicade), golimumab (Simponi), certolizumab pegol (Cimzia), and other Biological DMARDs, such as Anakinra (Kineret), Rituximab (Rituxan), Tocilizumab (Actemra), CD28 inhibitor including Abatacept (Orencia) and Belatacept.

Thus, treatment of IBD, using the agents according to at least some embodiments of the present invention may be combined with, for example, any known therapeutic agent or method for treating IBD. Non-limiting examples of such known therapeutic agents or methods for treating IBD include immunosuppression to control the symptom, such as prednisone, Mesalazine (including Asacol, Pentasa, Lialda, Aspiro), azathioprine (Imuran), methotrexate, or 6-mercaptopurine, steroids, Ondansetron, TNF-α blockers (including infliximab, adalimumab golimumab, certolizumab pegol), Orencia (abatacept), ustekinumab (Stelara®), Briakinumab (ABT-874), Certolizumab pegol (Cimzia®), ITF2357 (givinostat), Natalizumab (Tysabri), Firategrast (SB-683699), Remicade (infliximab), vedolizumab (MLN0002), other drugs including GSK1605786 CCX282-B (Traficet-EN), AJM300, Stelara (ustekinumab), Semapimod (CNI-1493) tasocitinib (CP-690550), LMW Heparin MMX, Budesonide MMX, Simponi (golimumab), MultiStem®, Gardasil HPV vaccine, Epaxal Berna (virosomal hepatitis A vaccine), surgery, such as bowel resection, strictureplasty or a temporary or permanent colostomy or ileostomy; antifungal drugs such as nystatin (a broad spectrum gut antifungal) and either itraconazole (Sporanox) or fluconazole (Diflucan); alternative medicine, prebiotics and probiotics, cannabis, Helminthic therapy or ova of the Trichuris suis helminth.

Thus, treatment of psoriasis, using the agents according to at least some embodiments of the present invention may be combined with, for example, any known therapeutic agent or method for treating psoriasis. Non-limiting examples of such known therapeutics for treating psoriasis include topical agents, typically used for mild disease, phototherapy for moderate disease, and systemic agents for severe disease. Non-limiting examples of topical agents: bath solutions and moisturizers, mineral oil, and petroleum jelly; ointment and creams containing coal tar, dithranol (anthralin), corticosteroids like desoximetasone (Topicort), Betamethasone, fluocinonide, vitamin D3 analogues (for example, calcipotriol), and retinoids. Non-limiting examples of phototherapy: sunlight; wavelengths of 311-313 nm, psoralen and ultraviolet A phototherapy (PUVA). Non-limiting examples of systemic agents: Biologics, such as interleukin antagonists, TNF-α blockers including antibodies such as infliximab (Remicade), adalimumab (Humira), golimumab, certolizumab pegol, and recombinant TNF-α decoy receptor, etanercept (Enbrel); drugs that target T cells, such as efalizumab (Xannelim/Raptiva), alefacept (Ameviv), dendritic cells such Efalizumab; monoclonal antibodies (MAbs) targeting cytokines, including anti- IL-12/IL-23 (ustekinumab (brand name Stelara)) and anti-Interleukin-17; Briakinumab (ABT-874); small molecules, including but not limited to ISA247; Immunosuppressants, such as methotrexate, cyclosporine; vitamin A and retinoids (synthetic forms of vitamin A); and alternative therapy, such as changes in diet and lifestyle, fasting periods, low energy diets and vegetarian diets, diets supplemented with fish oil rich in Vitamin A and Vitamin D (such as cod liver oil), Fish oils rich in the two omega-3 fatty acids eicosapentaenoic acid (EPA) and docosahexaenoic acid (DHA) and contain Vitamin E. Ichthyotherapy, Hypnotherapy, cannabis.

The present disclosure also encompasses the use of the compositions of the invention according to at least some embodiments together with other pharmaceutical agents to treat immune system diseases. For example, MS disease may be treated with molecules of the invention in conjunction with, but not limited to, immunosuppressants such as corticosteroids, cyclosporin, prednisone, azathioprine, methotrexate, TNF-alpha blockers or antagonists, or any other biological agent targeting any inflammatory cytokine, nonsteroidal antiinflammatory drugs/Cox-2 inhibitors, hydroxychloroquine, sulphasalazopryine, gold salts, etanercept, infliximab, rapamycin, mycophenolate mofetil, azathioprine, tacrolismus, basiliximab, cytoxan, interferon beta-la, interferon beta-lb, glatiramer acetate, mitoxantrone hydrochloride, anakinra and/or other biologics. The C1ORF32 polypeptides, fragments or fusion proteins thereof can also be used in combination with one or more of the following agents to regulate an immune response: soluble gp39 (also known as CD40 ligand (CD40L), CD154, T-BAM, TRAP), soluble CD29, soluble CD40, soluble CD80 (e.g. ATCC 68627), soluble CD86, soluble CD28 (e.g. 68628), soluble CD56, soluble Thy-1, soluble CD3, soluble TCR, soluble VLA-4, soluble VCAM-1, soluble LECAM-1, soluble ELAM-1, soluble CD44, antibodies reactive with gp39 (e.g. ATCC HB-10916, ATCC HB-12055 and ATCC HB-12056), antibodies reactive with CD40 (e.g. ATCC HB-9110), antibodies reactive with B7 (e.g. ATCC HB-253, ATCC CRL-2223, ATCC CRL-2226, ATCC HB-301, ATCC HB-11341, etc), antibodies reactive with CD28 (e.g. ATCC HB-11944 or mAb 9.3), antibodies reactive with LFA-1 (e.g. ATCC HB-9579 and ATCC TIB-213), antibodies reactive with LFA-2, antibodies reactive with IL-2, antibodies reactive with IL-12, antibodies reactive with IFN-gamma, antibodies reactive with CD2, antibodies reactive with CD48, antibodies reactive with any ICAM (e.g., ICAM-1 (ATCC CRL-2252), ICAM-2 and ICAM-3), antibodies reactive with CTLA4 (e.g. ATCC HB-304), antibodies reactive with Thy-1, antibodies reactive with CD56, antibodies reactive with CD3, antibodies reactive with CD29, antibodies reactive with TCR, antibodies reactive with VLA-4, antibodies reactive with VCAM-1, antibodies reactive with LECAM-1, antibodies reactive with ELAM-1, antibodies reactive with CD44. In certain embodiments, monoclonal antibodies are preferred. In other embodiments, antibody fragments are preferred. As persons skilled in the art will readily understand, the combination can include the C1ORF32 polypeptides, fragments or fusion proteins thereof with one other immunosuppressive agent, with two other immunosuppressive agents, with three other immunosuppressive agents, etc. The determination of the optimal combination and dosages can be determined and optimized using methods well known in the art.

The C1ORF32 polypeptides, fragments or fusion proteins thereof can also be used in combination with one or more of the following agents: L104EA29YIg, CD80 monoclonal antibodies (mAbs), CD86 mAbs, gp39 mAbs, CD40 mAbs, CD28 mAbs; anti-LFAl mAbs, antibodies or other agents targeting mechanisms of the immune system such as CD52 (alemtuzumab), CD25 (daclizumab), VLA-4 (natalizumab), CD20 (rituximab), IL2R (daclizumab) and MS4A1 (ocrelizumab); novel oral immunomodulating agents have shown to prevent lymphocyte recirculation from lymphoid organs such as fingolimod (FTY720) or leading to lymphocyte depletion such as mylinax (oral cladribine) or teriflunomide; and agents that prevent immunoactivation such as panaclar (dimethyl fumarate BG-12) or laquinimod (ABR216062). Other combinations will be readily appreciated and understood by persons skilled in the art.

The soluble C1ORF32 polypeptides, fragments or fusion proteins thereof may be administered as the sole active ingredient or together with other drugs in immunomodulating regimens or other anti-inflammatory agents e.g. for the treatment or prevention of allo- or xenograft acute or chronic rejection or inflammatory or autoimmune disorders, or to induce tolerance. For example, it may be used in combination with a calcineurin inhibitor, e.g. cyclosporin A or FK506; an immunosuppressive macrolide, e.g. rapamycine or a derivative thereof; e.g. 40-O-(2-hydroxy)ethyl-rapamycin, a lymphocyte homing agent, e.g. FTY720 or an analog thereof, corticosteroids; cyclophosphamide; azathioprene; methotrexate; leflunomide or an analog thereof; mizoribine; mycophenolic acid; mycophenolate mofetil; 15-deoxyspergualine or an analog thereof; immunosuppressive monoclonal antibodies, e.g., monoclonal antibodies to leukocyte receptors, e.g., MHC, CD2, CD3, CD4, CD11a/CD18, CD7, CD25, CD 27, B7, CD40, CD45, CD58, CD 137, ICOS, CD150 (SLAM), OX40, 4-1BB or their ligands; or other immunomodulatory compounds, e.g. CTLA4/CD28-Ig, or other adhesion molecule inhibitors, e.g. mAbs or low molecular weight inhibitors including LFA-1 antagonists, Selectin antagonists and VLA-4 antagonists.

Where the C1ORF32 polypeptides, fragments or fusion proteins thereof are administered in conjunction with other immunosuppressive/immunomodulatory or anti-inflammatory therapy, e.g. as hereinabove specified, dosages of the co-administered immunosuppressant, immunomodulatory or anti-inflammatory compound will of course vary depending on the type of co-drug employed, e.g. whether it is a steroid or a cyclosporin, on the specific drug employed, on the condition being treated and so forth.

According to at least some embodiments of the present invention, additional embodiments of combination therapy are provided as described below.

An exemplary embodiment relates to a combination of the C 1 ORF32 polypeptide, preferably as a fusion protein, with other Treg promoting and/ or activating methods for treating the subject. For example, isolation and ex vivo expansion of natural Treg from a subject may optionally be performed, followed by reintroduction to the subject, as an example of a therapy which may optionally be combined with C1 ORF32 polypeptide treatment.

Another non-limiting example of such a therapy relates to in vitro induction of Treg from non-Treg (n-Tr) cells from a subject, for example by retinoic acid or by TGF-β and IL-2 treatment, followed by reinfusion into the subject.

Another non-limiting example of such a therapy relates to in vivo induction and expansion of Treg by administration of one or more of anti-CD3 antibodies (αCD3), HADC inhibitors (HADCi) and neuropetides, such as VIP; agents promoting activation and/or expansion of FOXP3+ Tregs: IL-10, TGFb, IL-2, IL-35, IL-30; or agents promoting secretion of these cytokines, retinoic acid, adenosine; or a combination thereof to the subject, preferably concurrently with administration of C1ORF32 polypeptide.

Another non-limiting example of such a therapy relates to potentiation of T cell function, for example by administration of B7-H4-Ig to the subject, preferably concurrently with administration of C1ORF32 polypeptide.

Another non-limiting example of such a therapy relates to mucosal tolerization (tolerance induction) with self-antigen, such as heat shock proteins (HSPs), or HSP-derived peptides, preferably concurrently with administration of C1ORF32 polypeptide.

Another non-limiting example of such a therapy relates to enhancing the responsiveness of effector cells to suppression and blocking pro-inflammatory cytokines.

Another non-limiting example of such a therapy relates to the administration of one or more agents that modulate antigen-presenting cell (APC) through cell-cell contact, for example through reverse signalling via Treg-CTLA-4 engagement of B7 on dendritic cells, preferably concurrently with administration of C1ORF32 polypeptide.

Another non-limiting example of such a therapy relates to the inhibition of costimulatory signals for example by administering an anti CD40L or blocking antibody for OX-40, GITR pathways (i.e. to the ligand or to the receptor) to the subject, preferably concurrently with administration of C1ORF32 polypeptide.

Another non-limiting example of such a therapy relates to the promotion of anti-stimulatory signals by administering one or more of PDL-1-Ig or anti PD1, CTLA4-Ig or soluble receptor (fusion protein and the like) to the subject, preferably concurrently with administration of C 1 ORF32 polypeptide.

Another non-limiting example of such a therapy relates to agents that induce effector cell death: The apoptotic death induced in the targets was shown to be Bcl-2 interacting mediator of cell death (BIM)-dependent. Changing the balance of effector cells and Tregs is one mechanism of enhancing tolerance, thus the combination of treatment with a polypeptide according to the present invention that induces Tregs with a treatment that will induce effector cell death would increase tolerance.

8. Treg function can be modulated by a variety of proinflammatory signals including Toll-like receptor triggering as such triggering can increase Treg number and function (Lu H., Front Immunol. 2014 Mar 3;5:83).

Another non-limiting example of such a therapy relates to the administration of one or more agents that activate signaling pathways of Cbl-b, NFATc1, c3, and TRAF6 or to inhibit AKT, PI3-K to the subject, preferably concurrently with administration of C1ORF32 polypeptide.

### Methods of Therapeutic Use

The C1ORF32 polypeptides, or fragments, or fusions thereof disclosed herein are useful as therapeutic agents. According to at least some embodiments, immune cells, preferably T cells, can be contacted in vivo or ex vivo with C 1 ORF32 fusion polypeptides to decrease or inhibit immune responses including, but not limited to inflammation. The T cells contacted with C1ORF32 fusion polypeptides can be any cell which expresses the T cell receptor, including α/β and γ/δ T cell receptors. T-cells include all cells which express CD3, including T-cell subsets which also express CD4 and CDS. T-cells include both naive and memory cells and effector cells such as CTL. T-cells also include cells such as Th1, Tc1, Th2, Tc2, Th3, Th17, Th22, Treg, and Tr1 cells. T-cells also include NKT-cells and similar unique classes of the T-cell lineage. For example the compositions can be used to modulate Th1, Th17, Th22, or other cells that secrete, or cause other cells to secrete, inflammatory molecules, including, but not limited to, TNF-alpha, IFN-gamma, IL-17, IL-23, IL-22, IL-21, GM-CSF, CCL3, CCL5 and MMPs. The compositions can also be used to increase or promote the activity of Tregs, increase the production of cytokines such as IL-10 from Tregs, increase the differentiation of Tregs, increase the number of Tregs, or increase the survival of Tregs. The compositions can also be used to increase or promote the activity of Th2 cells, increase the production of cytokines such as IL-10 or IL-4 from Th2 cells, increase the differentiation of Th2 cells, increase the number of Th2 cells, or increase the survival of Th2 cells.

In some embodiments, the disclosed C1ORF32 polypeptides, or fragments, or fusions thereof are administered in combination with a second therapeutic. Combination therapies may be useful in immune modulation. In some embodiments, C1ORF32 polypeptides, or fragments, or fusions can be used to attenuate or reverse the activity of a pro-inflammatory drug, and/or limit the adverse effects of such drugs. Other immune cells that can be treated with the disclosed C1ORF32 polypeptides, fragments or fusion thereof include T cell precursors, antigen presenting cells such as dendritic cells and monocytes or their precursors, B cells or combinations thereof. The C1ORF32 compositions can be used to modulate the production of antibodies by B cells by contacting the B cells with an effective amount of the C1ORF32 composition to inhibit or reduce antibody production by the B cell relative to a control. The C1ORF32 compositions can also modulate the production of cytokines by the B cells.

According to the present disclosure, there is provided new uses and methods of treatment for immune related diseases by administering the C1OF32-ECD protein to a subject in need of treatment thereof to induce immune tolerance, preferably through the IL-10 and/ or TGFβ pathways. In the context of immune related diseases, "immune tolerance" refers to reducing, ameliorating or blocking the immune related disease, while leaving intact the disease-fighting abilities of the immune system.

According to at least some embodiments, the C1ORF32 fusion protein modulates the IL-10 and/or TGFβ pathway. As shown herein, the C1ORF32 fusion protein upregulates the IL-10 pathway by upregulating IL-10 secretion and also maintains the TGFβ pathway.

According to at least some embodiments, the C1ORF32 fusion protein induces long term immune tolerance. By "long term" it is meant tolerance which lasts any time period between at least 72 hours to 6 months after cessation of treatment, or even greater than 6 months after cessation of treatment; and/or efficacy at a reduced dosing frequency, including but not limited to a dosing frequency of one dose per any time period from every 72 hours to every 6 months.

According to the disclosure, the C1ORF32 fusion protein induces tolerance, and preferably long term tolerance as defined above, to graft tissue with at least one mismatched antigen to the recipient subject. Non-limiting examples of such graft tissue include organs and bone marrow. Preferably, the fusion protein induces graft survival and increase in both nTregs and iTregs, indicating donor specific tolerance induction (Aaron et al. Journal of Immunology, 2010, 185: 3326-3336). Also preferably, such induction of immune tolerance occurs through the IL-10 pathway and/or the TGF-beta pathway.

### Methods of treating inflammatory responses

Surprisingly, it was found that inhibition of TGFbeta or of IL10 in the EAE model abolishes C1ORF32-ECD-Fc activity (see Example 6 herein). In addition, C1ORF32-ECD-Fc enhances TGF beta secretion (see Example 15 herein) and also IL10 (as shown previously and herein in Examples 3, 4, 15). Furthermore, it was shown herein that neutralization of Tregs in the EAE model abolishes long-term remission (tolerance) (see Example 5 herein).

According to the present disclosure, it is therefore provided a method of treatment of immune related conditions by administrating to the subject in need thereof any of the C1ORF32 soluble polypeptides, fragments or fusion proteins thereof or pharmaceutical composition comprising same, which increase TGFbeta and IL10 that are involved in the induction and function of regulatory immune cells, particularly Tregs.

According to the present disclosure, it is provided a method of treatment of immune related conditions by administrating to the subject in need thereof any of the C1ORF32 soluble polypeptides, fragments or fusion proteins thereof or pharmaceutical composition comprising same, wherein the treatment results in long-term therapeutic benefit which is associated with regulatory immune cells, particularly Tregs.

The C1ORF32 soluble polypeptides, fragments or fusion proteins thereof or pharmaceutical composition comprising same, that inhibit T cell activation, as manifested by T cell proliferation and cytokine secretion. Specifically, the proteins inhibit Th1 and Th17 responses, while promoting Th2 responses.

The C1ORF32 soluble polypeptides, fragments or fusion proteins thereof or pharmaceutical composition comprising same, are potentially used for therapy of diseases that require down-regulation of costimulatory pathways and or such that require downregulation of Th1 and/or Th17 responses.

The present disclosure provides methods for treating or alleviating one or more symptoms of inflammation. In a further embodiment, the compositions and methods disclosed are useful for treating chronic and persistent inflammation. Inflammation in general can be treated using the disclosed C1ORF32 polypeptides or fragment or fusions thereof.

An immune response including inflammation can be inhibited or reduced in a subject, preferably a human, by administering an effective amount of C1ORF32 polypeptide or fragment, or fusion thereof to inhibit or reduce the biological activity of an immune cell or to reduce the amounts of proinflammatory molecules at a site of inflammation. Exemplary proinflammatory molecules include, but are not limited to, TNF-alpha, IFN-gamma, IL-17, IL-23, IL-22, IL-21, GM-CSF, CCL3, CCL5 and MMPs. Th1 and Th17 are exemplary T cells that can be targeted for inhibition by C1ORF32 polypeptides, fusion proteins or fragments thereof to inhibit or reduce inflammation.

Without wishing to be limited by a single hypothesis for this biological mechanism or any other biological mechanism described herein, the C1ORF32 polypeptides, fragments or fusion proteins thereof are useful for treating inflammation by any or all of the following: inhibiting or reducing differentiation of Th1, Thl7, Th22, and/or other cells that secrete, or cause other cells to secrete, inflammatory molecules, including, but not limited to, TNF-alpha, IFN-gamma, IL-17, , IL-23, IL-22, IL-21, GM-CSF, CCL3, CCL5 and MMPs; inhibiting or reducing activity of Th1, Th 17, Th22, and/or other cells that secrete, or cause other cells to secrete, inflammatory molecules, including, but not limited to, TNF-alpha, IFN-gamma, IL-17, IL-23, IL-22, IL-21, GM-CSF, CCL3, CCL5 and MMPs; inhibiting or reducing the Th1 and/or Thl7 pathways; inhibiting or reducing cytokine production and/or secretion by Th1, Th17, Th22, and/or other cells that secrete, or cause other cells to secrete, inflammatory molecules, including, but not limited to, TNF-alpha, IFN-gamma, IL-17, IL-23, IL-22, IL-21, GM-CSF, CCL3, CCL5 and MMPs; inhibiting or reducing proliferation of Th1, Th17, Th22, and/or other cells that secrete, or cause other cells to secrete, inflammatory molecules, including, but not limited to, TNF-alpha, IFN-gamma, IL-17, IL-23, IL-22, IL-21, GM-CSF, CCL3, CCL5 and MMPs.

Additionally, C1ORF32 polypeptides, fragments or fusion proteins thereof can also enhance Th2 immune responses. C1ORF32 polypeptides, fragments or fusion proteins thereof can also act directly on Th2 cells to promote or enhance production of IL-4, IL-5 or IL-10, or TGFbeta or to increase the number or percentage of Th2 cells, resulting in inhibition of Th1 and/or Th17, and in immune modulation via a Th1/Th2 shift.
Additionally, C1ORF32 polypeptides, fragments or fusion proteins thereof can also enhance TGFbeta secretion or responsiveness to TGFbeta.

Additionally, C1ORF32 polypeptides, fragments or fusion proteins thereof can increase Tregs numbers or percentage.

Additionally, C1ORF32 polypeptides, fragments or fusion proteins thereof can cause Tregs to have an enhanced suppressive effect on an immune response. Tregs can suppress differentiation, proliferation, activity, and/or cytokine production and/or secretion by Th1, Th17, Th22, and/or other cells that secrete, or cause other cells to secrete, inflammatory molecules, including, but not limited to, TNF-alpha, IFN-gamma, IL-17, IL-23, IL-22, IL-21, GM-CSF, CCL3, CCL5 and MMPs. For example, C1ORF32 polypeptides, fragments or fusion proteins thereof can cause Tregs to have an enhanced suppressive effect on Th1 and/or Th17 cells to reduce the level of IFN-gamma and IL-17 produced, respectively. C1 ORF32 polypeptides, fragments or fusion proteins thereof can also act directly on Tregs to promote or enhance production of IL-10 to suppress the Th1 and/or Th17 pathway, and/or to increase the number or percentage of Tregs.

Additionally, C1ORF32 polypeptides, fragments or fusion proteins thereof can cause Th2 to have an enhanced modulatory effect on an immune response. Th2 cells can modulate differentiation, proliferation, activity, and/or cytokine production and/or secretion by Th1, Th17, Th22, and/or other cells that secrete, or cause other cells to secrete, inflammatory molecules, including, but not limited to TNF-alpha, IFN-gamma, IL-17, IL-23, IL-22, IL-21, GM-CSF, CCL3, CCL5 and MMPs. For example, C1ORF32 polypeptides, fragments or fusion proteins thereof can cause Th2 cells to have an enhanced modulatory effect on Th1 and/or Th17 cells to reduce the level of IFN-gamma and IL-17 produced, respectively. C1ORF32 polypeptides, fragments or fusion proteins thereof can also act directly on Th2 cells to promote or enhance production of IL-10 to suppress the Th1 and/or Th17 pathway, and/or to increase the number or percentage of Th2 cells.

Without wishing to be limited by a single hypothesis, it is believed that C1ORF32 polypeptides, fragments or fusion proteins thereof acts at multiple points in multiple T cell pathways. For example, C1ORF32 polypeptides, fragments or fusion proteins thereof can inhibit the differentiation of naive T cells into either Th1 or Th17 cells. Alternatively, C1ORF32 polypeptides, fragments or fusion proteins thereof can interact with Th1 cells or Th17 cells, or both to inhibit or reduce the production of proinflammatory molecules.

Additionally, C1ORF32 polypeptides, fragments or fusion proteins thereof may increase the differentiation of and/or promote Th2 responses resulting in an immunomdulatory effect on the Th1 and/or Th17 pathways to reduce the level of INF-gamma and/or IL-17 produced. C1ORF32 polypeptides, fragments or fusion proteins thereof enhances the production of IL-10 and or TGFbeta from cells such as Th2 and/or Tregs, which in turn inhibits the activity of Th1 and/or Th17 cells and promote immune regulatory activity.

Additionally, C1ORF32 polypeptides, fragments or fusion proteins thereof can affect Tregs to have an enhanced suppressive effect on Th1 and/or Th17 pathways to reduce the level of INF-gamma and/or IL-17 produced. Additionally, C1ORF32 polypeptides, fragments or fusion proteins thereof can enhance the production of IL-10 and/ or TGFbeta which inhibits the activity of Th1 and/or Th17 cells and enhance immune regulatory activity.

### Inhibition of Th1 Responses

### a. Inhibition of Th1 Development

One method for treating immune related diseases and/or inhibiting or reducing inflammation includes administering an effective amount of a C1ORF32 polypeptide, fusion protein, variants thereof, or fragments thereof to inhibit Th1 development in a subject in need thereof. Inflammation can be inhibited or reduced by blocking naive T cells from differentiating into Th1 cells by administering C1ORF32 polypeptides, fusion proteins, fragments thereof or variants thereof. In one embodiment, the C1ORF32 polypeptides or fusion protein thereof may inhibit or reduce proliferation of Th1 cells. C1ORF32 polypeptides, fragments or fusion proteins thereof may also reduce naive T cells from differentiating into Th1 cells, by blocking antigen presenting cell maturation. Alternatively, C1ORF32 polypeptides, fragments or fusion proteins thereof increase the differentiation of Th2 cells and thereby reduce the number of Th1 cells in a subject. By restricting the number of Th1 cells that can develop in the subject, the amount of proinflammatory molecules such as INF-gamma can be reduced or contained. INF-gamma stimulates the production or release of other proinflammatory molecules including TNF-alpha, and MMPs. Thus, by controlling the number of Th1 cells in a subject, the levels of these other proinflammatory molecules can be controlled, thereby reducing inflammatory responses.

### b. Inhibition of Proinflammatory molecules

The disclsoure provides a method of inhibiting or reducing inflammation in a subject by administering to the subject an effective amount of a C1ORF32 polypeptide, fusion protein thereof, or fragment thereof to inhibit or reduce production of proinflammatory molecules by Th1 cells.
Exemplary proinflammatory molecules produced by Th1 cells includes IFN-gamma. In this embodiment the C1ORF32 polypeptide, fusion protein thereof, or fragment thereof can interact directly with the Th1 cell and inhibit or reduce IFN-gamma production by the Th1 cells. In this embodiment, the amount of proinflammatory molecules is regulated rather than the population of Th1 cells.

The disclosure provides a method of inhibiting or reducing immune related disease and/or inflammation in a subject by administering to the subject an effective amount of a C 1 ORF32 polypeptide, fusion protein thereof, or fragment thereof to inhibit or reduce production of disease related proinflammatory molecules including but not limited to cytokines/ chemokines such as GM-CSF, CCL3 and CCL5.

### Inhibition of Th17 Responses

### a. Inhibition of Th17 Development

Inflammation can also be inhibited or reduced in a subject by administering an effective amount of a C1ORF32 polypeptide, fragment or fusion thereof, to inhibit or block naive T cells from developing into Th17cells. In one aspect, the C1ORF32 polypeptide or fusion protein increases the suppressive activity of Tregs on the differentiation of naive T cells into Th17 cells by an amount sufficient to reduce the number of Th17 cells in a subject. Alternatively, the C1 ORF32 polypeptide or fusion protein thereof inhibits or reduces proliferation of Th17 cells. C1ORF32 polypeptides or fusion proteins thereof may also reduce naive T cells from differentiating into Th17 cells, by blocking antigen presenting cell maturation. By reducing the population of Th17 cells in a subject, the amount of IL-17 can be reduced, as well as IL-22 and IL-21. IL-17 is a proinflammatory cytokine that causes increases in other proinflammatory molecules such as IL-1beta, TNF-alpha, and MMPs. Thus, by reducing the amount of IL- 17 these other proinflammatory molecules can be reduced, thereby reducing or inhibiting immune related disease and/or inflammation.

### b. Inhibition of IL-17 Production

Still another aspect provides a method for treating immune related diseases in a subject by administering an effective amount of C1ORF32 polypeptide, fusion protein thereof, or fragments thereof, to inhibit production of IL-17 by Th17 cells, as well as IL-22 and IL-21. In this aspect, the C1ORF32 polypeptide or fusion protein can act directly on Th17 cells, for example by binding to Th17 cells resulting in inhibition of IL-17 (or IL-22 and IL-21) production by those Th17 cells. As noted above, inhibition or reduction of IL-17 (and IL-22 or IL-21) leads to the reduction of other proinflammatory molecules, thereby reducing or inhibitng immune related disease and/or inflammation.

### Inhibiting Th1 and Thl7 Responses

The disclosed C1ORF32 polypeptides, fusion proteins, and fragments thereof can be used to inhibit both the Th1 and Th17 pathways simultaneously. Using one anti-inflammatory agent to inhibit two separate pathways provides more robust inhibition or reduction of the immune response.

### Promoting Th2 Responses, and production of IL-10 and TGFbeta.

immune related diseases can also be treated by administering C1ORF32 polypeptides, fusion proteins thereof, or fragments thereof to a subject in an amount effective to enhance Th2 responses, and the suppressive activity of IL-10 producing cells, and to increase TGFbeta secretion, activation or responsiveness and to enhance suppressive or modulatory activity on the Th1 and/or Th17 pathways. In this aspect the disclosed C1ORF32 polypeptides and fusion proteins cause an increased suppressive effect on IFN-gamma and/or IL-17 production. Another aspect provides a method for treating immune related diseases by administering an effective amount of C1ORF32 polypeptide, fusion proteins thereof, or fragments thereof to increase production of IL-10 by Th2, Tregs or other immune cells.

Increased production of IL-10 results in the decreased production of IL-17 by Th17 cells and deceased production of IFN-gamma by Th1 cells. In this embodiment, the C1ORF32 polypeptides, fusion proteins, and fragments thereof can interact directly with with immune cells to increase IL-10 production.
Still another aspect provides a method for treating immune related diseases by administering an effective amount of C1ORF32 polypeptides, fusion proteins thereof, and fragments thereof to inhibit or interfere with the Th1 pathway and Th17 pathway, and to enhance the suppressive effect on the Th1 and/or Th17 pathways by Th2 cells.
The C1ORF32 polypeptides, fusion proteins thereof and fragments thereof can also be administered to a subject in an amount effective to increase Th2 cell populations or numbers.
IL-10 production can be increased relative to a control by contacting Th2 cells, Tregs or other immune cells with an effective amount of C1ORF32 polypeptides, C1ORF32 fusion proteins, or fragments thereof having C1ORF32 activity. The increase can occur in vitro or in vivo,

### Promoting immune regulatory cells

Another aspect provides a method for treating immune related diseases by administering an effective amount of C1ORF32 polypeptide, fusion proteins thereof, or fragments thereof to increase production and/or secretion of TGFbeta by macrophages or other cell types, or increasing the secretion of serum proteinases such as plasmin that catalyze the release of active TGFbeta from a latent complex or increase the responsiveness to TGFbeta.

Increase in TGFbeta results in increase in regulatory cells including Tregs and Treg17 cells which directly inhibit effector T cell functions.

Treg17 can derive from Th17 cells in an altered differentiation program giving rise to protective and non-pathogenic cells. Treg17 cells can be induced by TGF-beta plus IL-6. Like conventional Treg, induction of regulatory Treg17 cells could play an important role in modulating and preventing certain autoimmune diseases.

Another aspect provides a method for treating immune related diseases by administering an effective amount of C1ORF32 polypeptide, fusion proteins thereof, or fragments thereof to increase immune regulatory cells including but not limited to Tregs, Treg17, and Bregs.

### Immune related diseases and disorders to Be Treated

Immune related diseases and disorders that may be treated using C1ORF32 fusion polypeptides are described herein.

C1ORF32 acts at multiple points in the inflammatory pathway as a master regulator to control the expression and/or activity of effectory cytokines such as IFN-gamma and TNF-alpha. Therefore, the C1ORF32 compositions described herein are particularly useful for treating patients that do not respond to TNF-alpha blockers such as Enbrel, Remicade, Cimzia and Humira, or where TNF-alpha blockers are not safe or effective. In a further embodiment, the C1ORF32 compositions described herein are used to treat autoimmune diseases. In addition, because of its activity as a master regulator in the inflammatory pathway, the C1ORF32 compositions disclosed are particularly useful for treating chronic and persistent inflammation.

### Combination therapy

C1ORF32 fusion polypeptides can be used alone or in combination with additional therapeutic agents. The additional therapeutic agents include, but are not limited to, immunosuppressive agents (e.g., antibodies against other lymphocyte surface markers (e.g., CD40, alpha-4 integrin) or against cytokines), other fusion proteins (e.g., CTLA-4-Ig (Orencia®), TNFR-Ig (Enbrel®)), TNF-alpha blockers such as Enbrel, Remicade, Cimzia and Humira, cyclophosphamide (CTX) (i.e. Endoxan®, Cytoxan®, Neosar®, Procytox®, Revimmune™), methotrexate (MTX) (i.e. Rheumatrex®, Trexall®), belimumab (i.e. Benlysta®), or other immunosuppressive drugs (e.g., cyclosporin A, FK506-like compounds, rapamycin compounds, or steroids), anti-proliferatives, cytotoxic agents, or other compounds that may assist in immunosuppression.

### PHARMACEUTICAL COMPOSITIONS

The present disclosure, in some aspects, features a pharmaceutical composition comprising a therapeutically effective amount of a therapeutic agent according to the present invention. According to the present invention the therapeutic agent could be any one of soluble C 1 ORF32 protein, C 1 ORF32 ectodomain, or a fragment or variant thereof, or a fusion protein. The pharmaceutical composition is further used for the treatment of autoimmunity and preferably for treating an immune related disorder as described herein. The therapeutic agents of the present invention can be provided to the subject alone, or as part of a pharmaceutical composition where they are mixed with a pharmaceutically acceptable carrier.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion). Depending on the route of administration, the active compound, i.e., soluble C1ORF32 protein, C1ORF32 ectodomain, or a fragment or variant thereof, or a fusion protein or a corresponding nucleic acid sequence encoding the pharmaceutical compounds according to at least some embodiments of the present invention may include one or more pharmaceutically acceptable salts. A "pharmaceutically acceptable salt" refers to a salt that retains the desired biological activity of the parent compound and does not impart any undesired toxicological effects (see e.g., Berge, S. M., et al. (1977) J. Pharm. Sci. 66: 1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition also may include a pharmaceutically acceptable antioxidants. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like. A pharmaceutical composition according to at least some embodiments of the present invention also may include additives such as detergents and solubilizing agents (e.g., TWEEN 20 (polysorbate-20), TWEEN 80 (polysorbate-80)) and preservatives (e.g., Thimersol, benzyl alcohol) and bulking substances (e.g., lactose, mannitol).

Examples of suitable aqueous and nonaqueous carriers that may be employed in the pharmaceutical compositions according to at least some embodiments of the present invention include water, buffered saline of various buffer content (e.g., Tris-HCl, acetate, phosphate), pH and ionic strength, ethanol, polyols (such as glycerol, propylene glycol, polyethylene glycol, and the like), and suitable mixtures thereof, vegetable oils, such as olive oil, and injectable organic esters, such as ethyl oleate.

Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions according to at least some embodiments of the present invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin. Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred per cent, this amount will range from about 0.01 per cent to about ninety-nine percent of active ingredient, preferably from about 0.1 per cent to about 70 per cent, most preferably from about 1 per cent to about 30 per cent of active ingredient in combination with a pharmaceutically acceptable carrier.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms according to at least some embodiments of the present invention are dictated by and directly dependent on (a) the unique characteristics of the active compound and the particular therapeutic effect to be achieved, and (b) the limitations inherent in the art of compounding such an active compound for the treatment of sensitivity in individuals.

A composition of the present disclosure can be administered via one or more routes of administration using one or more of a variety of methods known in the art. As will be appreciated by the skilled artisan, the route and/or mode of administration will vary depending upon the desired results. Preferred routes of administration for therapeutic agents according to at least some embodiments of the present invention include intravascular delivery (e.g. injection or infusion), intravenous, intramuscular, intradermal, intraperitoneal, subcutaneous, spinal, oral, enteral, rectal, pulmonary (e.g. inhalation), nasal, topical (including transdermal, buccal and sublingual), intravesical, intravitreal, intraperitoneal, vaginal, brain delivery (e.g. intra-cerebroventricular, intra-cerebral, and convection enhanced diffusion), CNS delivery (e.g. intrathecal, perispinal, and intra-spinal) or parenteral (including subcutaneous, intramuscular, intraperitoneal, intravenous (IV) and intradermal), transdermal (either passively or using iontophoresis or electroporation), transmucosal (e.g., sublingual administration, nasal, vaginal, rectal, or sublingual), administration or administration via an implant, or other parenteral routes of administration, for example by injection or infusion, or other delivery routes and/or forms of administration known in the art. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion or using bioerodible inserts, and can be formulated in dosage forms appropriate for each route of administration. In a specific embodiment, a protein, a therapeutic agent or a pharmaceutical composition according to at least some embodiments of the present invention can be administered intraperitoneally or intravenously.

Compositions of the present disclosure can be delivered to the lungs while inhaling and traverse across the lung epithelial lining to the blood stream when delivered either as an aerosol or spray dried particles having an aerodynamic diameter of less than about 5 microns. A wide range of mechanical devices designed for pulmonary delivery of therapeutic products can be used, including but not limited to nebulizers, metered dose inhalers, and powder inhalers, all of which are familiar to those skilled in the art. Some specific examples of commercially available devices are the Ultravent nebulizer (Mallinckrodt Inc., St. Louis, Mo.); the Acorn II nebulizer (Marquest Medical Products, Englewood, Colo.); the Ventolin metered dose inhaler (Glaxo Inc., Research Triangle Park, N.C.); and the Spinhaler powder inhaler (Fisons Corp., Bedford, Mass.). Nektar, Alkermes and Mannkind all have inhalable insulin powder preparations approved or in clinical trials where the technology could be applied to the formulations described herein.

In some in vivo approaches, the compositions disclosed herein are administered to a subject in a therapeutically effective amount. As used herein the term "effective amount" or "therapeutically effective amount" means a dosage sufficient to treat, inhibit, or alleviate one or more symptoms of the disorder being treated or to otherwise provide a desired pharmacologic and/or physiologic effect. The precise dosage will vary according to a variety of factors such as subject-dependent variables (e.g., age, immune system health, etc.), the disease, and the treatment being effected. For the polypeptide compositions disclosed herein and nucleic acids encoding the same, as further studies are conducted, information will emerge regarding appropriate dosage levels for treatment of various conditions in various patients, and the ordinary skilled worker, considering the therapeutic context, age, and general health of the recipient, will be able to ascertain proper dosing. The selected dosage depends upon the desired therapeutic effect, on the route of administration, and on the duration of the treatment desired. For polypeptide compositions, generally dosage levels of 0.0001 to 100 mg/kg of body weight daily are administered to mammals and more usually 0.001 to 20 mg/kg. For example dosages can be 0.3 mg/kg body weight, 1 mg/kg body weight, 3 mg/kg body weight, 5 mg/kg body weight or 10 mg/kg body weight or within the range of 1-10 mg/kg. An exemplary treatment regime entails administration once per week, once every two weeks, once every three weeks, once every four weeks, once a month, once every 3 months or once every three to 6 months. Generally, for intravenous injection or infusion, dosage may be lower. Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms according to at least some embodiments of the present invention are dictated by and directly dependent on (a) the unique characteristics of the active compound and the particular therapeutic effect to be achieved, and (b) the limitations inherent in the art of compounding such an active compound for the treatment of sensitivity in individuals.

Optionally the polypeptide formulation may be administered in an amount between 0.0001 to 100 mg/kg weight of the patient/day, preferably between 0.001 to 20.0 mg/kg/day, according to any suitable timing regimen. A therapeutic composition according to at least some embodiments according to at least some embodiments of the present invention can be administered, for example, three times a day, twice a day, once a day, three times weekly, twice weekly or once weekly, once every two weeks or 3, 4, 5, 6, 7 or 8 weeks. Moreover, the composition can be administered over a short or long period of time (e.g., 1 week, 1 month, 1 year, 5 years).

Alternatively, therapeutic agent can be administered as a sustained release formulation, in which case less frequent administration is required. Dosage and frequency vary depending on the half-life of the therapeutic agent in the patient. The half-life for fusion proteins may vary widely. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A "therapeutically effective dosage" of C1ORF32 soluble protein or C1ORF32 ectodomain or C1ORF32 fusion protein containing same, preferably results in a decrease in severity of disease symptoms, an increase in frequency and duration of disease symptom-free periods, an increase in lifepan, disease remission, or a prevention or reduction of impairment or disability due to the disease affliction.

One of ordinary skill in the art would be able to determine a therapeutically effective amount based on such factors as the subject's size, the severity of the subject's symptoms, and the particular composition or route of administration selected.
In certain embodiments, the polypeptide compositions are administered locally, for example by injection directly into a site to be treated. Typically, the injection causes an increased localized concentration of the polypeptide compositions which is greater than that which can be achieved by systemic administration. For example, in the case of a neurological disorder like Multiple Sclerosis, the protein may be administered locally to a site near the CNS. In another example, as in the case of an arthritic disorder like Rheumatoid Arthritis, the protein may be administered locally to the synovium in the affected joint. The polypeptide compositions can be combined with a matrix as described above to assist in creating a increased localized concentration of the polypeptide compositions by reducing the passive diffusion of the polypeptides out of the site to be treated.

Pharmaceutical compositions may be administered with medical devices known in the art. For example, in an optional embodiment, a pharmaceutical composition according to at least some embodiments of the present invention can be administered with a needles hypodermic injection device, such as the devices disclosed in U.S. Pat. Nos. 5,399,163; 5,383,851; 5,312,335; 5,064,413; 4,941,880; 4,790,824; or 4,596,556. Examples of well-known implants and modules useful in the present invention include: U.S. Pat. No. 4,487,603, which discloses an implantable micro-infusion pump for dispensing medication at a controlled rate; U.S. Pat. No. 4,486,194, which discloses a therapeutic device for administering medicaments through the skin; U.S. Pat. No. 4,447,233, which discloses a medication infusion pump for delivering medication at a precise infusion rate; U.S. Pat. No. 4,447,224, which discloses a variable flow implantable infusion apparatus for continuous drug delivery; U.S. Pat. No. 4,439,196, which discloses an osmotic drug delivery system having multi-chamber compartments; and U.S. Pat. No. 4,475,196, which discloses an osmotic drug delivery system. Many other such implants, delivery systems, and modules are known to those skilled in the art.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g., Sustained and Controlled Release Drug Delivery Systems, J. R. Robinson, ed., Marcel Dekker, Inc., New York, 1978.

Therapeutic compositions can be administered with medical devices known in the art. For example, a therapeutic composition can be administered with a needles hypodermic injection device, such as the devices disclosed in U.S. Pat. Nos. 5,399,163; 5,383,851; 5,312,335; 5,064,413; 4,941,880; 4,790,824; or 4,596,556. Examples of well-known implants and modules useful in the present invention include: U.S. Pat. No. 4,487,603, which discloses an implantable micro-infusion pump for dispensing medication at a controlled rate; U.S. Pat. No. 4,486,194, which discloses a therapeutic device for administering medicaments through the skin; U.S. Pat. No. 4,447,233, which discloses a medication infusion pump for delivering medication at a precise infusion rate; U.S. Pat. No. 4,447,224, which discloses a variable flow implantable infusion apparatus for continuous drug delivery; U.S. Pat. No. 4,439,196, which discloses an osmotic drug delivery system having multi-chamber compartments; and U.S. Pat. No. 4,475,196, which discloses an osmotic drug delivery system. Many other such implants, delivery systems, and modules are known to those skilled in the art.

In certain embodiments, C1ORF32 soluble proteins, C1ORF32 ectodomains, C1ORF32 fusion proteins, other proteins or other therapeutic agents according to at least some embodiments of the present invention can be formulated to ensure proper distribution in vivo. For example, the blood-brain barrier (BBB) excludes many highly hydrophilic compounds. To ensure that the therapeutic compounds according to at least some embodiments of the present invention cross the BBB (if desired), they can be formulated, for example, in liposomes. For methods of manufacturing liposomes, see, e.g., U.S. Pat. Nos. 4,522,811; 5,374,548; and 5,399,331. The liposomes may comprise one or more moieties which are selectively transported into specific cells or organs, thus enhance targeted drug delivery (see, e.g., V. V. Ranade (1989) J. Clin. Pharmacol. 29:685). Exemplary targeting moieties include folate or biotin (see, e.g., U.S. Pat. No. 5,416,016 to Low et al.); mannosides (Umezawa et al., (1988) Biochem. Biophys. Res. Commun. 153:1038); antibodies (P. G. Bloeman et al. (1995) FEBS Lett. 357:140; M. Owais et al. (1995) Antimicrob. Agents Chemother. 39:180); surfactant protein A receptor (Briscoe et al. (1995) Am. J Physiol. 1233:134); p120 (Schreier et al. (1994) J. Biol. Chem. 269:9090); see also K. Keinanen; M. L. Laukkanen (1994) FEBS Lett. 346:123; J. J. Killion; I. J. Fidler (1994) Immunomethods 4:273.

### Formulations for parenteral administration

In a further aspect, compositions disclosed herein, including those containing peptides and polypeptides, are administered in an aqueous solution, by parenteral injection. The formulation may also be in the form of a suspension or emulsion. In general, pharmaceutical compositions are provided including effective amounts of a peptide or polypeptide, and optionally include pharmaceutically acceptable diluents, preservatives, solubilizers, emulsifiers, adjuvants and/or carriers. Such compositions optionally include one or more for the following: diluents, sterile water, buffered saline of various buffer content (e.g., Tris-HC1, acetate, phosphate), pH and ionic strength; and additives such as detergents and solubilizing agents (e.g., TWEEN 20 (polysorbate-20), TWEEN 80 (polysorbate-80)), antioxidants (e.g., water soluble antioxidants such as ascorbic acid, sodium metabisulfite, cysteine hydrochloride, sodium bisulfate, sodium metabisulfite, sodium sulfite; oil-soluble antioxidants, such as ascorbyl palmitate, butylated hydroxyanisole (BHA), butylated hydroxytoluene (BHT), lecithin, propyl gallate, alpha-tocopherol; and metal chelating agents, such as citric acid, ethylenediamine tetraacetic acid (EDTA), sorbitol, tartaric acid, phosphoric acid), and preservatives (e.g., Thimersol, benzyl alcohol) and bulking substances (e.g., lactose, mannitol). Examples of non-aqueous solvents or vehicles are ethanol, propylene glycol, polyethylene glycol, vegetable oils, such as olive oil and corn oil, gelatin, and injectable organic esters such as ethyl oleate. The formulations may be freeze dried (lyophilized) or vacuum dried and redissolved/resuspended immediately before use. The formulation may be sterilized by, for example, filtration through a bacteria retaining filter, by incorporating sterilizing agents into the compositions, by irradiating the compositions, or by heating thecompositions.

### Formulations for topical administration

C 1 ORF32 polypeptides, fragments, fusion polypeptides, nucleic acids, and vectors disclosed herein can be applied topically. Topical administration does not work well for most peptide formulations, although it can be effective especially if applied to the lungs, nasal, oral (sublingual, buccal), vaginal, or rectal mucosa.

Compositions can be delivered to the lungs while inhaling and traverse across the lung epithelial lining to the blood stream when delivered either as an aerosol or spray dried particles having an aerodynamic diameter of less than about 5 microns.

A wide range of mechanical devices designed for pulmonary delivery of therapeutic products can be used, including but not limited to nebulizers, metered dose inhalers, and powder inhalers, all of which are familiar to those skilled in the art. Some specific examples of commercially available devices are the Ultravent nebulizer (Mallinckrodt Inc., St. Louis, Mo.); the Acorn II nebulizer (Marquest Medical Products, Englewood, Colo.); the Ventolin metered dose inhaler (Glaxo Inc., Research Triangle Park, N.C.); and the Spinhaler powder inhaler (Fisons Corp., Bedford, Mass.). Nektar, Alkermes and Mannkind all have inhalable insulin powder preparations approved or in clinical trials where the technology could be applied to the formulations described herein.

Formulations for administration to the mucosa will typically be spray dried drug particles, which may be incorporated into a tablet, gel, capsule, suspension or emulsion. Standard pharmaceutical excipients are available from any formulator. Oral formulations may be in the form of chewing gum, gel strips, tablets or lozenges.

Transdermal formulations may also be prepared. These will typically be ointments, lotions, sprays, or patches, all of which can be prepared using standard technology. Transdermal formulations will require the inclusion of penetration enhancers.

### Controlled delivery polymeric matrices

C1ORF32 polypeptides, fragments, fusion polypeptides, nucleic acids, and vectors disclosed herein may also be administered in controlled release formulations. Controlled release polymeric devices can be made for long term release systemically following implantation of a polymeric device (rod, cylinder, film, disk) or injection (microparticles). The matrix can be in the form of microparticles such as microspheres, where peptides are dispersed within a solid polymeric matrix or microcapsules, where the core is of a different material than the polymeric shell, and the peptide is dispersed or suspended in the core, which may be liquid or solid in nature. Unless specifically defined herein, microparticles, microspheres, and microcapsules are used interchangeably. Alternatively, the polymer may be cast as a thin slab or film, ranging from nanometers to four centimeters, a powder produced by grinding or other standard techniques, or even a gel such as a hydrogel.

Either non-biodegradable or biodegradable matrices can be used for delivery of polypeptides or nucleic acids encoding the polypeptides, although biodegradable matrices are preferred. These may be natural or synthetic polymers, although synthetic polymers are preferred due to the better characterization of degradation and release profiles. The polymer is selected based on the period over which release is desired. In some cases linear release may be most useful, although in others a pulse release or "bulk release" may provide more effective results. The polymer may be in the form of a hydrogel (typically in absorbing up to about 90% by weight of water), and can optionally be crosslinked with multivalent ions or polymers.

The matrices can be formed by solvent evaporation, spray drying, solvent extraction and other methods known to those skilled in the art. Bioerodible microspheres can be prepared using any of the methods developed for making microspheres for drug delivery, for example, as described by Mathiowitz and Langer, J. Controlled Release, 5:13-22 (1987); Mathiowitz, et al., Reactive Polymers, 6:275-283 (1987); and Mathiowitz, et al., J. Appl Polymer ScL, 35:755-774 (1988).

The devices can be formulated for local release to treat the area of implantation or injection - which will typically deliver a dosage that is much less than the dosage for treatment of an entire body - or systemic delivery. These can be implanted or injected subcutaneously, into the muscle, fat, or swallowed.

### EXAMPLES

### Example 1

### THE EFFECT OF C1ORF32-P8-V1-ECD-mFc (SEQ ID NO:42) IN MODULATION OF TYPE 1 DIABETES IN NOD MICE

The effect of C1ORF32 ECD-Fc treatment on modulation of disease incidence was tested in NOD mice, which spontaneously develop a T1D disease. In NOD mice, autoreactive CD4+ can be observed within the β-islets as early as 2 weeks of age (Yang et al., Immunity. 1996 Feb; 4(2):189-94), and by 10 weeks of age, when the treatment with C1ORF32 ECD-Fc (SEQ ID NO: 42) began in the present experiments, NOD mice develop peri-insulitis and β-cell loss (Jimeno et al., Immunology and Cell Biology. (2010) 88, 734-745).

Materials: C1ORF ECD-Fc (SEQ ID NO:42) was used in these studies, as described below. Control Ig was mouse IgG2a from BioXcell (Cat# BE0085). NOD mice were purchased from Jackson labs, and blood glucose was measured via sampling a small drop of blood from the tail vein.

Methods: Six week old NOD mice were purchased from Jackson labs. At 10 weeks of age, mice were split into groups and treated with either vehicle (PBS) Control Ig or C1ORF ECD-Fc (SEQ ID NO:42) as detailed below. Treatment was given via IP injections 3x/wk/2wks. Blood glucose levels were monitored weekly from 8 weeks of age until the mice reached 30 or 30 weeks of age as detailed below. Mice were considered diabetic upon having two consecutive blood glucose readings of>250 mg/dL. Diabetic mice were sacrificed on the day of the second high glucose level reading. Blood glucose levels were obtained from the tail vein, and the level of blood glucose measured via use of a OneTouch. UltraSmart. Blood Glocuse Monitoring System (OneTouch; Johnson & Johnson; New Brunswick, NJ) in a blinded manner. This study was carried out 3 times; For study 3 proteins were provided coded.

### STUDY I:

### Treatment groups (n=5 mice/ group):

1. Control Ig (mouse IgG2a), 100ug/dose
2. C1ORF ECD-Fc (SEQ ID NO:42), 100ug/dose.

### Results for Study 1:

As expected, NOD mice in the Control Ig treated group began to develop T1D at 13 wks of age, and all 5 mice in this control group developed the disease by the age of 17 weeks. In contrast, C1ORF ECD-Fc (SEQ ID NO:42) treatment resulted in inhibition of T1D development: 3 out of 5 mice did not develop T1D, while 2 mice developed the disease considerably later than the control mice - at 17 and 20 wks of age (Figure 1A).

### STUDY II:

### TREATMENT GROUPS (N=6 MICE/ GROUP):

1. CONTROL IG (MOUSE IgG2a) (100UG/DOSE)
2. C1ORF ECD-Fc (SEQ ID NO:42) (100UG/DOSE)

### Results for Study 2:

All 6 Control Ig treated mice developed T1D by the age of 25 weeks. In contrast, C1ORF ECD-Fc (SEQ ID NO:42) treatment inhibited the development of T1D, and only 1 out of 6 mice developed the disease (Figure 1B).

### STUDY III:

### Treatment groups (n=14-15):

1. Control Ig (mouse IgG2a; BioXcell) (100ug/dose)
2. C1ORF ECD-Fc (SEQ ID NO:42) (100ug/dose)
3. PBS.

All compounds for this study were provided coded.

### Results for Study 3

Only 3 out of 15 mice treated with C1ORF ECD-Fc (SEQ ID NO:42) developed T1D while 13 out of 14 Control Ig treated and 12 out of 15 PBS treated NOD mice developed T1D by the age of 24 weeks. In comparing the various treatment groups statistically via the use of a One-Way ANOVA and a Tukey's Multiple Comparison Post Test, the analyses revealed that the C1ORF ECD-Fc (SEQ ID NO:42) treatment had a significantly decreased incidence of T1D (p<0.001) in comparison to the Control Ig, and PBS treatment groups. Additionally, there was no significant difference in T1D incidence between the Control Ig and the PBS treatment groups. Results are shown in Figure 1C.

### Summary

The effect of C1ORF ECD-Fc (SEQ ID NO:42) treatment on modulation of disease incidence was tested in NOD mice, which spontaneously develop a T1D disease. The data presented herein show that C1ORF ECD-Fc (SEQ ID NO:42) treatment starting before onset of diabetes, significantly decreases the incidence of disease. The combined data from all 3 studies show that C1ORF ECD-Fc (SEQ ID NO:42) treatment inhibited T1D development, as the disease was observed in only 6 out of 26 (23%) of C1ORF ECD-Fc (SEQ ID NO:42) treated NOD mice. In contrast, 24 out of 25 (96%) Control Ig treated NOD mice developed T1D in the same experiments. As early as 2 weeks of age in NOD mice, CD4+ can be observed within the β-islets (1) and by 10 weeks of age, when the treatment schedule began in the present experiments, NOD mice develop peri-insulitis and β-cell loss (2). These published findings suggest that the early stages of the CD4+ cell autoimmune response to antigens present within β-islets of the pancreas are already ongoing at the time of C1ORF ECD-Fc (SEQ ID NO:42) treatment. The highly significant decrease in disease incidence suggests that further analyses are warranted, and that C1ORF ECD-Fc (SEQ ID NO:42) holds promise as a potential therapy for T1D.

### References:

1. Yang, Y., Charlton, B., Shimada, A., Del Canto, R. and Fathman, C.G. Monoclonal T cells identified in early NOD islet infiltrates. Immunity. 1996 Feb;4(2):189-94.
2. Jimeno, R., Gomariz, R.P., Gutiérrez-Cañas, I., Martinez, C., Juarranz, Y., and Leceta, J. New insights into the role of VIP on the ratio of T-cell subsets during the development of autoimmune diabetes. Immunology and Cell Biology. (2010) 88, 734-745.

### EXAMPLE 2

### THE EFFECT OF ANY ONE OF C1ORF32 PROTEIN FRAGMENTS AND/OR FUSION PROTEINS THEREOF IN MODULATION OF TYPE 1 DIABETES IN NOD MICE, CD28-KO NOD, AND B7-2-KO NOD

The effect of any one of C 1 ORF32 protein fragments and/or fusion proteins thereof is studied in a widely used mouse model of type 1 diabetes: nonobese diabetic (NOD) mice which develop spontaneous In NOD mice, spontaneous insulitis, the hallmark pathologic lesion, evolves through several characteristic stages that begin with peri-insulitis and end with invading and destructive insulitis and overt diabetes. Peri-insulitis is first observed at 3-4 wk of age, invading insulitis at 8-10 wk, and destructive insulitis appears just before the onset of clinical diabetes, with the earliest cases at 10-12 wk. At 20 wk of age, 70-80% of female NOD mice become diabetic (Ansari et al 2003 J. Exp. Med. 198: 63-69).

The study is performed in NOD mice. The efficacy of C1ORF ECD-Fc (SEQ ID NO:42) in T1D model using two KO NOD mice: CD-28-KO NOD mice and B7-1/B7-2 double KO NOD mice, -which develop accelerated diabetes (Lenschow et al 1996 Immunity 5: 285-293; Salomon et al 2000 Immunity 12: 431-440), are also carried out.

Study I: NOD mice are treated with any one of C1ORF32 protein fragments and/or fusion proteins thereof early and late phases during the evolution of diabetes, before or after disease onset, to examine the effects of these compounds on disease pathogenesis and to demonstrate that such treatment reduces disease onset and ameliorates pathogenesis. To study the effect on insulitis, blood glucose levels are measured 3 times/ week, for up to 25 weeks (Ansari et al 2003 J. Exp. Med. 198: 63-69).

Mechanism of disease modification and mode of action is studied by experimental evaluation of individual immune cell types: pancreas, pancreatic LNs and spleen are harvested to obtain Tregs, Th subtypes and CD8 T cells, DCs and B cells. Effect on cytokines secretion from cells isolated from pancreas, pancreatic LN and spleen is analysed, focused on IFNg, IL-17, IL-4, IL-10 and TGFb. Upon effect of the tested compounds, the mechanism of disease modification is studied by examination of individual immune cell types (including Tregs, Th subtypes and CD8 T cells, DCs and B cells); cytokines (IFNg, IL-17, IL-4, IL-10 and TGFb) and histology. Histologycal analysis of the pancreas is carried out to compare the onset of insulitis, and the lymphocyte infiltration.

Study II- The effect of any one of C1 ORF32 protein fragments and/or fusion proteins thereof in modulation of Type 1 Diabetes in Adoptive transfer model

To further investigate the mode of action of the Ig fusion proteins, an adoptive transfer model of diabetes is used. T cells from diabetic or prediabetic NOD donors are transfered to NOD SCID recipient mice. These mice are monitored for development of diabetes. The urine glucose and blood glucose, and assess histology of the pancreas, and T cell responses are monitored as described in the previous example.

Study III- Diabetes is also induced by the transfer of activated CD4+CD62L+CD25- BDC2.5 T cells (transgenic for TCR recognizing islet specific peptide 1040-p31 activated by incubation with 1040-p31) to NOD recipients. Mice are treated with any one of C1ORF32 protein fragments and/or fusion proteins thereof, control mIgG2a or positive control. Treatments begin 1 day following transfer. Mice are followed for glucose levels 10-28 days post transfer (Bour-Jordan et al., J Clin Invest. 2004;114(7):979-87).

Seven days post treatment pancreas, spleen, pancreatic LN and peripheral lymph node cells are extracted and examined for different immune cell populations. In addition, recall responses are measured by testing ex-vivo proliferation and cytokine secretion in response to p31 peptide.

C 1 ORF32 protein fragments and/or fusion proteins thereof prevent or reduce disease onset or the severity thereof in the above studies.

### EXAMPLE 3-Immune tolerance and Tregs induction by C1ORF ECD-Fc (SEQ ID NO:42) in the HY bone marrow transplantation model

A transplantation model utilizing male bone marrow (BM) cells from C57BL/6 mice transplanted into female mice of the same starin was used to study whether C1ORF32 ECD-Fc induces immune tolerance. In this model BM cells of the male mouse are rejected by the female recipients due to mismatch in the minor histocompatibility antigen encoded by the Y chromosome (Hya). To allow identification of the male cells in the female mice blood, male mice expressing CD45.1 isotype were selected while the female mice carry CD45.2 isotype. This model provides a tool for testing the effect of potential drugs on induction of tolerance towards the grafted cells.

In this model, C1ORF32 ECD-Fc (SEQ ID NO:42) treatment started one week before bone marrow transplantation and continued for five weeks. Female recipient mice were followed for percentage of CD45.1+ and CD45.2+ cells present within the blood.

### Experimental Procedure

Female C57BL/6 recipient mice (CD45.2+) were sub-lethally irradiated with 200 rads/cGy 24 hours before transplantation of either female C57B1/6 (CD45.1+) or male C57B1/6 (CD45.1+) 5x10⁶ bone marrow cells. Recipient mice were allocated into treatment groups as detailed below (n=6 per group). Treatments were given at 300ug/dose i.p, 3x/wk starting one week prior to transplantation and continued for 4 weeks following transplantation, giving a total of 15 treatments during this 5 weeks period. Anti-CD40L was used as a positive control, as it has previously been shown to have efficacy in this model (unpublished data).

### Treatment groups:

1. Female CD45.1 into female CD45.2
2. Male CD45.1 into female CD45.2 + anti-CD40L (300ug/dose) (Clone MR-1; BioXCell, Cat#BE0017 -1)
3. Male CD45.1 into female CD45.2 + C1ORF ECD-Fc (SEQ ID NO:42) (300ug/dose)2
4. Male CD45.1 into female CD45.2 + Control Ig (mouse IgG2a) (300ug/dose)

Beginning at 2 weeks following bone marrow transplantation, blood samples from the tail were collected from each mouse once a week for 4 weeks. Approximately 2-3 drops of blood were collected per mouse into 200ul of PBS + EDTA (50mM), samples were spun down, and RBCs lysed with ammonium chloride. The cells were washed 3x with PBS, stained with the viability dye Aqua Dead Cell Stain (Invitrogen; Cat#L34957), blocked with Fc Receptor block (anti-mouse CD16/32; eBioscience; Cat#14-0161-86), and stained with anti-mouse CD45.1 APC-Cy7 (eBiosciences; Cat#47-0453-80) and anti-mouse CD45.2 FITC (eBioscience; Cat#11-0454-82). The analysis for chimerism was determined by flow cytometric analysis (on total live cells) for the percentage of CD45.1+ versus CD45.2+ cells present in the blood of female recipient mice.

During weeks 6, 7, and 8 the blood samples were also stained to determine the percentage of CD4+ Teff cells and CD4+ Treg cells present within the blood using the cellular markers CD44, CD25, and FoxP3. Details of the Abs used in the flow panels for weeks 6, 7, and 8 are listed below.

These same populations of cells were analyzed at week 8 within the spleen. In addition, the proliferative and secreted cytokine responses of total splenocytes in *ex vivo* recall cultures were tested in the presence of medium alone, anti-CD3 (1ug/ml), the HY antigen DBY peptide (NAGFNSNRANSSRSS; Genemed Synthesis; San Antonio, TX) (10ug/ml), irradiated male splenocytes (1:1 ratio), or irradiated female splenocytes (1:1 ratio).

### Flow cytometry panels for Weeks 6-8 blood (Panel 1) and Week 8 spleen samples (Panel 2):

### Flow Panel 1

Viability Dye (LIVE/DEAD® Fixable Aqua Dead Cell Stain Kit *for 405 nm excitation; Invitrogen; Cat# L34957)
anti-CD45.2 FITC (eBioscience; Cat#11-0454-82)
anti-CD45.1 APC-Cy7 (eBiosciences; Cat#47-0453-80)
anti-CD3 PerCP (BD Bioscience; Cat#553067)
anti-CD4 eFluor 450 (eBiosciences; Cat#48-0042-82)
anti-FoxP3 PE-Cy7 (eBiosciences; Cat#25-5773-82)
anti-CD25 APC (eBiosciences; Cat#17-0251-82)
anti-CD44 PE (eBiosciences; Cat#12-0441-82)

### Flow Panel 2

Viability Dye
anti-CD45.2 FITC (eBioscience; Cat#11-0454-82)
anti-CD45.1 APC-Cy7 (eBiosciences; Cat#47-0453-80)
anti-CD3 PerCP (BD Bioscience; Cat#553067)
anti-CD4 eFluor 450 (eBiosciences; Cat#48-0042-82)
anti-FoxP3 PE-Cy7 (eBiosciences; Cat#25-5773-82)
anti-Nrp-1 APC (R&D Systems; Cat#FAB566A)
anti-Helios PE (eBiosciences; Cat#12-9883-42)

### Results

The data for the weekly percentage of CD45.1+ and CD45.2+ cells present within the blood are shown in Figure 4. The data show that as early as 2 weeks post bone marrow cell transplant of either female CD45.1+ bone marrow or male CD45.1+ bone marrow in combination with either anti-CD40L or C1ORF ECD-Fc (SEQ ID NO:42) treatment, a detectible population of transferred cells is present. By week 4 post bone marrow cell transplantation, a highly significant number of male CD45.1+ cells were present in the blood of recipient females following bone marrow transplant from CD45.1+ female bone marrow cells, or from male CD45.1+ bone marrow cells in the anti-CD40L or C1ORF ECD-Fc treatment groups. Furthermore, the presence of these cells was maintained until the final time point of 8 weeks post bone marrow transplantation. The decrease in viable cell counts observed on week 8 is probably due to the rejection of cells based on the differences in CD45.1 vs. CD45.2 alleles. In the Control Ig treatment groups no substantial number of CD45.1+ cells were present at any time point in the blood of recipient females following bone marrow transplant from male CD45.1 bone marrow cells, pointing to a rejection of these grafts.

The percentage of CD45.2+ cells in the blood of the female recipients is roughly in inverse correlation to that of grafted CD45.1+ cells, as would be expected.

During weeks 6 and 7 post bone marrow cell transplantation, the CD45.1+ and CD45.2+ cells in the blood of recipient mice were further analyzed to determine if C1ORF ECD-Fc (SEQ ID NO:42) treatment altered the percentage of effector/memory CD4+ T cells (CD4+/CD44hi), activated CD4+ T cells (CD4+/CD25+/FoxP3-), resting CD4+ T cells (CD4+/CD441o), or Treg cells (CD4+/CD25+/FoxP3+).

The data presented in Figure 5B show that by week 6 post bone marrow cell transplant, C1ORF ECD-Fc (SEQ ID NO:42) treatment resulted in decrease in effector/memory CD4+ T cells (CD45.1+/CD4+/CD44hi) within the blood compared to mice treated with Control Ig. This low effector/memory CD4+ T cell percentage was comparable to that observed in mice receiving female CD45.1+ bone marrow cells or mice receiving male CD45.1+ bone marrow cells plus the positive control, anti-CD40L. Conversely, mice receiving male CD45.1+ bone marrow cells and treated with C1ORF ECD-Fc had a significantly higher percentage of resting CD4+ T cells (CD45.1+/CD4+/CD441o), as compared to mice receiving male CD45.1+ bone marrow cells plus Control Ig treatment. This finding is again comparable to that obtained with mice receiving female CD45.1+ bone marrow cells or male CD45.1+ bone marrow cells and treated with anti-CD40L.

No differences were observed in the recipients' CD45.2+ T cell populations among the different treatment groups by week 6 post bone marrow cell transplant (Figure 5A).

By week 7, mice receiving male CD45.1+ bone marrow cells plus C1ORF ECD-Fc (SEQ ID NO:42) treatment had significantly lower percentage of activated donor CD4+ T cells (CD45.1+/CD4+/CD25+/FoxP3-) as compared to mice treated with Control Ig (Figure 5D). This finding is comparable to that observed for mice that received female CD45.1+ bone marrow cells, or male CD45.1+ bone marrow cells plus anti-CD40L treatment.

A similar analysis as that shown in Figure 5 for CD45.1+ and CD45.2+ CD4+ T cell subtypes in the blood of recipient mice was carried out also at the end of the experiment, *i.e.,* on week 8 post bone marrow cell transplant. In addition, spleen cells were evaluated for total cell counts, and for CD4+ T cells and Treg subtypes. Figure 6 shows the results of the FACS analysis of recipient's endogenous CD45.2+ cell populations in the blood and spleen, while Figure 7 shows the corresponding results on the donor's engrafted CD45.1+ cell populations.

No differences were observed in the recipients In addition, spleen cells were evaluated for ice treated with C1ORF ECD-Fc (SEQ ID NO:42) as compared to mice treated with Control Ig by week 8 post bone marrow cell transplant (Figure 6A), similarly to the results obtained at weeks 6 or 7 (Figure 5A and 5C, respectively). In the spleen of recipient female mice there was a trend towards an increased number of total cells in mice transplanted with male CD45.1+ bone marrow cells and treated with either C1ORF ECD-Fc (SEQ ID NO:42) or Control Ig, as compare to mice that received female CD45.1+ bone marrow cells, however, these differences were not statistically significant (Figure 6B).

In contrast to the blood, significant differences within the various CD4+ T cell populations of the recipient's endogenous cells (CD45.2+) were found in the spleen of C1ORF ECD-Fc (SEQ ID NO:42) treated mice compared to those treated with Control Ig. Mice transplanted with male CD45.1+ bone marrow cells and treated with C1ORF ECD-Fc, or with other treatments that lead to successful engraftment (i.e. anti-CD40L or mice transplanted with female CD45.1 cells) had lower percentage and number of CD45.2+, reflective of the increase in CD45.1+ cells, and CD4+ T cells within the spleen compared to mice that were treated with Control Ig and rejected the graft (Figures 6C & 6E). As expected, the percentage and number of the CD45.1+/CD4+ T cells present within the spleen of mice following treatments that lead to successful engraftment, such as C1ORF ECD-Fc or anti-CD40L, were increased (Figures 7C & 7E).

The data focused on the donor'he data focused on (CD45.1+) show a significant increase in effector/memory CD4+ T cells (CD44hi) and Tregs (FoxP3+CD25+), as well as a decrease in activated CD4+ T cells (FoxP3-CD25+) in the blood of female recipient mice receiving male donor CD45.1+ bone marrow cells and C1ORF ECD-Fc treatment, as compared to the mice with Control Ig treatment (Figure 7A). These effects were similar to those observed for mice with anti-CD40L treatment or for mice receiving female CD45.1+ cells. Coactivated CD4+ T cells (FoxP3-CD25+) were also observed in the spleen of female recipient mice following C1ORF ECD-Fc treatment, as compared to Control Ig (Figure 7C).

In addition, C1ORF ECD-Fc (SEQ ID NO:42) treatment resulted in a striking increase in the number of donor-derived (CD45.1+) CD4+/CD25+/FoxP3+ Treg cells, as well as Treg subpopulations: Nrp-1+/Helios+, Nrp-1+/Helios-, and Nrp-1-/Helios- (Figure 7F). As with most other endogenous-derived (CD45.2+) T cell subtypes in the spleen (Figure 6E), the number of Tregs and Treg subtypes were reduced following C1ORF ECD-Fc and the other treatments that lead to successful engraftment, compared to the Ig control group which lead to graft rejection (Figure 6F).

Ex vivo reactivation (recall responses) of splenocytes on week 8 was carried out with transplant-related stimuli (HY Ag DBY peptide or irradiated male splenocytes) or transplant-unrelated stimuli (anti-CD3 or irradiated female splenocytes). Compared to cells cultured in medium alone (i.e. without any added stimuli), only the addition of anti-CD3 lead to a substantial increase in cell proliferation (Figure 8). All other stimuli, whether transplant related or unrelated, had no substantial effect on splenocytes proliferation, hindering our ability to make any conclusions from this assay.

Parallel splenocytes reactivation cultures were also evaluated for cytokine secretion. The data, presented in Figure 9, show increased secretion of IFN splenocytes) or transplant-unrelated stimuli (anti-CD3 or irradiated female splenocytes). Compared to cells from mice treated with C1ORF ECD-Fc (SEQ ID NO:42) in comparison to the control Ig treated group, similarly to splenocytes from mice receiving female CD45.2+ bone marrow cells, or mice receiving male CD45.1+ bone marrow cells plus anti-CD40L treatment, i.e. treatments that lead to successful engraftment. An inhibitory trend in IFNg, IL-17, IL-2, IL-12 and GM-CSF secretion was also observed in C1ORF ECD-Fc cultures stimulated with male irradiated splenocytes. The level of IL-10 and IL-4 secreted within the cultures of splenoctyes from mice treated with C1ORF ECD-Fc (SEQ ID NO:42) was significantly increased in response to male or female splenocytes treated.

### Summary

The data from this study show that C1ORF ECD-Fc treatment with a 5 week dosing schedule, beginning one week before bone marrow cell transplant, is effective in preventing graft rejection and induces a tolerogenic environment towards the Hya minor mismatch antigen. This is demonstrated by the presence of donor CD45.1+ chimerism within the blood of CD45.2+ recipient female mice, similar to that obtained following transplantation of female CD45.1+ bone marrow cells or treatment with anti-CD40L, which was used as a positive control.

The increase in the number of donor derived Treg cells present within the spleens of C1ORF ECD-Fc treated mice suggests that tolerance induction by C1ORF ECD-Fc is potentially mediated by its effect on Treg cells. This finding is in agreement with previous in vitro data showing that C1ORF ECD-Fc treatment appears to increase the number of iTreg cells. In the present study C1ORF ECD-Fc treatment resulted in increased number of Treg subtypes, such as Helios+/Nrp-1+ and Helios-/Nrp-1- (which are proposed to represent nTregs and iTregs, respectively) (Thornton et al., J. Immunol. 184: 3433-3441; Yadav et al., J Exp Med. 2012;209:1713-1722) J Exp Med. 2012 Sep 24; 209(10):1713-22 J Exp Med. 2012 Sep 24; 209(10):1713-22.

Functionally, the ex vivo recall data show that splenoctyes isolated from mice receiving C1ORF ECD-Fc treatment produce significantly lower levels of inflammatory cytokines (IFN-gamma, IL-17, GM-CSF, IL-12, IL-2 and TNF-alpha) upon activation with male irradiated splenocytes or DBY peptide compared to Ig control treated mice and an increased level of IL-10 and IL-4 upon activation with irradiated male splenocytes. The latter is supportive of previous similar findings with both splenoctyes and draining lymph node ex vivo recall cultures in the PLP139-151/CFA primed SJL mouse model.

### EXAMPLE 4:

### EFFICACY AND MODE OF ACTION OF ANY ONE OF THE C1ORF32 PROTEIN FRAGMENTS AND/OR FUSION PROTEINS THEREOF IN MOUSE ADOPTIVE TRANSFER R-EAE MODEL OF MULTIPLE SCLEROSIS

The therapeutic effect of C1ORF32 ECD-Fc (SEQ ID NO:42) in treatment of autoimmune diseases was tested in a mouse model of multiple sclerosis; Relapsing Remitting Experimental Autoimmune Encephalomyelitis (R-EAE):

### Materials and Methods

### Animals

Female SJL/J mice ∼six week old were purchased from Harlan, and allowed to acclimate at the animal care facility for 1 week before use. SJL-Actin/GFP mice were bred in-house at Northwestern Univeristy and primed at 6-12 weeks of age.

### Induction of adoptive transfer R-EAE

Female SJL/J mice were primed with 50µg of PLP139-151/CFA according to the standard laboratory protocol on day 0. On day 8 post disease induction, the inguinal lymph nodes were collected and the total lymph node cells were re-activated in culture in the presence of PLP139-151 for 3 days. At the end of culture, cells were collected and transferred into naive recipient SJL/J mice.

### CFA/PLP Emulsion preparation

a. PLP139-151 peptide stock (Genemed Sythesis Inc) was diluted in PBS (1mg/ml) in an omni-mixer bucket, followed by addition of an equal volume of CFA.
b. The bucket was mixed in an omnimixer on high speed (setting 4-5) for 5 minutes.
c. The whole emulsion was scooped into 10ml snap-cap tube, quick spined in a centrifuge (up to 1200 rpm then stopped) and loaded into glass Hamilton syringes. All air bubbles were removed and attached to a 25G needle.

Priming donor SJL/J mice, PLP139-151 reactivation cultures, and blast cell transfer
a. Mice were shaved (about a 1in.x1in, square on the back between the hind flanks) and injected with CFA/ PLP139-151 emulsion at 100ul per mouse s.c., divided between three spots on the back and flanks. Peptide and final dose given per mouse: 50µg of PLP139-151 peptide on day 0.
b. On day 8 post priming with PLP139-151/CFA, the draining inguinal lymph nodes were collected, and single cell suspensions were prepared. Total lymph node cells were cultured at a density of 8x10⁶ cells per ml in the HL-1 medium plus PLP139-151 peptide (20µg/ml) in T75 flasks.
c. After 3 days of culture, the PLP139-151 reactivated cells were collected and counted using an hemacytometer. The number of total viable cells and the number of blast cells was determined. The percentage of blast cells expected following this protocol is 20-30% of the total viable cells.
d. Unless otherwise specified, 3x10⁶ cells were transferred to each recipient mouse by intravenous injection.
e. Mice were split into treatment groups as detailed for each specific experiment.
f. Mice were scored on the indicated days, on a 0-5 disease score scale per standard laboratory protocol: 0, no abnormality; 1, limp tail; 2, limp tail and hind limb weakness; 3, hind limb paralysis; 4, hind limb paralysis and forelimb weakness; and 5, moribund.

### Ex vivo recall responses by total splenocytes and lymph node cells from treated mice:

On day 45 (unless otherwise indicated) following cell transfer, spleen and cervical lymph nodes or CNS from 3 representative mice of each group were collected, and the total cells were activated ex vivo at 0.5x10⁶ cells per well with either OVA323-339, PLP139-151, PLP178-191, MBP84-104 (20ug/ml), or anti-CD3 (lug/ml). Two sets of cultures were set up side-by-side. One set was pulsed with 1uCi of tritiated thymidine at 24 hours and harvested at 72 hours to determine cell proliferation. In the second set of cultures, supernatants were collected at 72 hours and tested for peptide-specific cytokine production as determined by LiquiChip.

### Evaluation of BBB breakdown and inflammation by fluorescence molecular tomography

Commercially available near infrared (NIR) imaging agents were used to perform tomographic imaging (PerkinElmer Inc., Boston, MA). AngioSense®750 was used as a vascular imaging agent, and Cat B® 680 FAST was used to detect regions of increased lysosomal cathepsin B activity. For the in vivo imaging, mice were injected intravenously with 4 nmol of NIR fluorescent agent 24 hrs prior to imaging, and imaged following the last treatment with Control Ig or C1ORF32 ECD-Fc (SEQ ID NO: 42) (Day +30 post-PLP139-151 blast cell transfer). At the time of imaging, mice were anesthetized by administration of sodium pentobarbital (50 mg/kg) and then imaged using the FMT 2500 fluorescence molecular tomography in vivo imaging system (PerkinElmer Inc., Boston, MA). For whole body imaging, the anesthetized mice were placed in the supine position, centrally in the imaging cassette to capture the whole body (excluding the head) of the animal within the imaging scan field of the imaging system. For imaging the head, mice were placed in the prone position in the imaging cassette for a separate scan. After positioning the mouse, the imaging cassette was adjusted to the proper depth to gently restrain the mouse and then inserted into the heated docking system (regulated at 37°C) in the FMT imaging chamber. A FR/NIR laser diode transilluminated (i.e. passed light through the body of the animal to be collected on the opposite side) the animal body, with signal detection occurring via a thermoelectrically cooled CCD camera placed on the opposite side of the imaged animal. Appropriate optical filters allowed the collection of both fluorescence and excitation datasets, and the multiple source-detector fluorescence projections were normalized to the paired collection of laser excitation data. The entire image acquisition sequence took approximately 8-10 min (torso) and 2-3 min (head) per mouse. After image acquisition was complete, mice were perfused with phosphate buffered saline and the CNS was removed to acquire ex vivo images of the tissue to corroborate findings from 3D images. The collected fluorescence data was reconstructed by FMT 2500 system software (TrueQuant, PerkinElmer Inc., Boston, MA) which compensates for the effects of tissue heterogeneity on light scattering, allowing for the quantification of fluorescence signal within multiple organ regions. Three-dimensional regions of interest (ROI) were drawn to encompass lung, heart, liver, stomach, kidney, intestine, and bladder tissue regions. For visualization and analysis purposes, FMT 2500 system software provided 3D images and tomographic slices. The total amount of fluorescence (in pmoles) in different organ sites was automatically calculated relative to internal standards generated with known concentrations of appropriate FR/NIR dyes. All data was normalized to fluorescence detected in CFA/saline-immunized healthy control mice.

### Proteins tested:

C1ORF32 ECD-Fc (SEQ ID NO:42) mIgG2a as Ig Control (BioXcell Cat # BE0085).

### Statistical Analysis:

Disease course data was analyzed by one-way ANOVA with repeated measures. Recall responses data were analyzed using student's T test.

C1ORF32 ECD-Fc (SEQ ID NO:42) treatment starting at onset of disease remission results in decrease of disease severity, Th1/Th17 to Th2 shift, and reduced BBB vascular leakage

Two independent studies were performed to evaluate the effect of C1ORF32 ECD-Fc (SEQ ID NO:42) treatment starting at the time of disease remission in the adoptive transfer RR-EAE model (Figures 10 and 11 respectively). Adoptive transfer of PLP139-151-sensitized cells (see Table 1 for cell count data) resulted in relapsing remitting EAE disease as shown in the control Ig treatment groups (Figures 10A and 11A). C1ORF32 ECD-Fc (SEQ ID NO:42) treatment with 100ug/mouse, i.p. 3x/wk for 2 wks, starting from onset of disease remission resulted in a robust inhibition of clinical signs of the disease (Figures 10A and 11A).

C1ORF32 ECD-Fc (SEQ ID NO:42) treatment from onset of disease remission also inhibited recall responses of splenocytes and lymph node cells on day 45, as manifested in a decreased level of cellular proliferation (Figures 10B and 10H). In addition, C1ORF32 ECD-Fc (SEQ ID NO:42) treatment resulted in inhibition of proinflammatory cytokines secretion manifested as inhibition of IFN-gamma (Figures 10C and 10I) and IL-17 (Figures 10D and 10J) secretion upon activation with anti-CD3, PLP139-151, and PLP178-191 as well as inhibition of GM-CSF secretion in response to activation with PLP139-151 and PLP178-191 (Figure 10E and 10K). Furthermore, C1ORF32 ECD-Fc (SEQ ID NO:42) treatment resulted in increased splenocyte secretion of IL-4 and IL-10 in response to PLP139-151 (Figures 10F and 10G). These results are in line with the Th1/Th17 to Th2 shift observed in recall responses following C1ORF32 ECD-Fc (SEQ ID NO:42) treatment in the active EAE model. The weaker recall response to the MBP84-104 epitope was probably due to the fact that this epitope is exposed only at later stages of the disease. OVA323-339 was used as negative control for irrelevant activation in this study and did not induce proliferation or cytokine secretion, as expected.

The effects of C1ORF32 ECD-Fc (SEQ ID NO:42) treatment from onset of remission on vascular leakage in the CNS, as a marker for BBB damage, and on the level of inflammation by monocyte/ macrophage activation, were studied in vivo using AngioSense720 and Cat B 680 FAST, respectively, as NIR imaging agents. Four mice were selected from each treatment group and analyzed as described in Materials and Methods. The disease scores in this subgroup of mice were 4, 4, 4, and 4 in the Control Ig treatment group, and 0, 2, 0, and 2 in the C1ORF32 ECD-Fc (SEQ ID NO:42) treatment group. The results, presented on Figures 11B and 11C, show that treatment with C1ORF32 ECD-Fc (SEQ ID NO:42) from onset of disease remission results in decreased vascular leakage in the brain and spinal cord, as manifested in significantly reduced levels of AngioSense720. Similarly, decreased monocyte/macrophage activity in the brain and spinal cord was detected, as determined by reduced lysosomal CathepsinB activity; however this effect did not reach statistical significance.

**Table 1. Cell counts of reactivation cultures**

| **Study** # | **1b** | **1b**.**1** |
|---|---|---|
| Total viable cells | 312x10⁶ | 298.2x10⁶ |
| Blast cells | 84x10⁶ | 76.9x10⁶ |
| Percent of blast cells | 26.9%. | 25.8%. |

C1ORF32 ECD-Fc (SEQ ID NO:42) treatment beginning at the time of cell transfer decreases the ability of the transferred cells to induce disease, and decreases the number of transferred cell infiltrates within the CNS.

PLP139-151 sensitized cells were generated as described in Materials and Methods (cell counts in the reactivation culture are presented in Table 2) and labeled with PBSE (4uM) before transfer to naive mice. In study "Cgen data" (Figure 12), disease was induced in recipient mice (n=10 per group) by i.v. transfer of 5x10⁶ blast cells to each recipient mouse. A relatively large number of transferred cells were used in order to allow detection of the transferred cells by flow cytometry analysis at the end of the study. Mice were split into Control Ig and C1ORF32 ECD-Fc (SEQ ID NO:42) treatment groups, receiving 100ug/dose intraperitoneal injection three times per week for 2 weeks. On Day +14 after cell transfer, 5 representative mice from each group were selected for analysis of trafficking of transferred cells into the CNS. Spleens, cervical lymph nodes, and CNS were collected, and the number of PBSE- or PBSE+/CD45+/CD3+/CD4+ T cells was analyzed by flow cytometric analysis. C1ORF32 ECD-Fc (SEQ ID NO:42) treatment from the time of cell transfer resulted in complete abolishment of disease induction (Figure 12A) and in a decrease of the number of transferred CD4+ T cells within the CNS (Figure 12I).

An additional experiment (designated "MYC48 FACS", Figure 13) was performed using an alternative staining follow-up approach. SJL-Actin/GFP mice were primed with PLP139-151/CFA, and sensitized blast cells were generated as described in Materials and Methods (cell counts in the reactivation culture are presented in Table 2). On Day +3 of culture, PLP139-151 sensitized cells were collected and labeled with PBSE (4uM). Disease was induced in the recipient mice (n=10 per group), by i.v. transfer of 5x10⁶ blast cells/mouse. Beginning at the time of cell transfer, mice were treated with Control Ig or C1ORF32 ECD-Fc (SEQ ID NO:42) (100ug/dose i.p. three times per week for 2 weeks). C1ORF32 ECD-Fc (SEQ ID NO:42) treatment abolished disease development (Figure 13A) as well as day 10 recall responses of CNS cells to PLP139-151 and anti CD3 (Figure 13B). The inhibition of PLP139-151 induced responses indicate inhibition of the activity of the autoreactive T cells used for disease induction while the inhibition recall responses to anti-CD3 are probably due to inhibition of resident T cells responses. To evaluate C1ORF32 ECD-Fc (SEQ ID NO:42) effect on immune cell trafficking to the CNS, spleens, cervical lymph nodes, and CNS cells collected form 5 mice on day +10 post cell transfer, and the number of transferred CD45hi PBSE+or GFP+cells was analyzed by flow cytometry. A decrease in the number of GFP+ and PBSE+ transferred cells (i.e. autoreactive cells) was observed in the CNS of C1ORF32 ECD-Fc (SEQ ID NO:42) treated mice (Figure 13C and D). No change in the number of total (CD3+GFP+) or activated (CD3+CD4+CD25+GFP+ or CD3+CD4+CD44+GFP+) transferred T cells was observed in the spleen and cervical lymph nodes (Figure 13E-J). In the CNS these sub populations of activated cells were too low to evaluate, and thus only total, CD45hi, cell trafficking is presented. The low number of autoreactive transferred T cells in the CNS could be due to the inhibition of their trafficking to the CNS, but could also stem from an intrinsically low GFP signal of the cells that was obtained in this study. These results further support the data obtained in the previous experiment, presented in Figure 12, suggesting that C1ORF32 ECD-Fc (SEQ ID NO:42) reduces autoreactive T cells infiltration into the CNS.

**Table 2. Cell counts of reactivation cultures**

| **Study name** | **Transfer Experiment (Cgen Data)** | **MYC48 FACS** |
|---|---|---|
| Total viable cells | 583.5x10⁶ | 295.3x10⁶ |
| Blast cells | 124.3x10⁶ | 63.2x10⁶ |
| Percent of blast cells | 21.3%. | 21.4%. |

### Summary

Treatment of SJL/J mice either at the time of PLP13 9-151-specific reactivated cells transfer or at onset of disease remission resulted in inhibition of disease induction and in a reduction of BBB leakage, as well as CNS inflammation and damage in C1ORF32 ECD-Fc (SEQ ID NO:42) treated mice.

In addition, using the adoptive transfer model described herein, inhibition of recall responses to inducing and spread epitopes and Th1/Th17 to Th2 shift were observed following treatment with C1ORF32 ECD-Fc (SEQ ID NO:42). These effects are similar to the immune effects observed in C1 ORF32 ECD-Fc (SEQ ID NO:42) treated mice in the active RR-EAE studies.

When GFP and/ or PBSE labeled cells were used for transfer, a reduction of autoreactive cell infiltration into the CNS was detected in the C1ORF32 ECD-Fc (SEQ ID NO:42) treated groups. Reduced CNS infiltration of autoreactive cells might underlie the lower cell numbers that were observed in the active RR-EAE studies, as assessed either by flow cytometry analysis or by immunohistochemical analysis. No reduction was observed in the number of total transferred cells or of T cells in the lymph nodes or in the spleen of C1ORF32 ECD-Fc (SEQ ID NO:42) treated mice. This observation suggests that no general cell depletion is induced by C1ORF32 ECD-Fc (SEQ ID NO:42) treatment.

Altogether the data presented herein support previous observations and further establish the mode of action underlying the beneficial effects of C1ORF32 ECD-Fc (SEQ ID NO:42) in RR-EAE models.

### Example 5 - Effect of Treg inactivation on C1ORF32 ECD-Fc (SEQ ID NO:42) efficacy in the EAE model

Depletion and/or inactivation of CD4+CD25+FoxP3+ Tregs using anti-CD25 mAb treatment is an accepted strategy to characterize Treg function in vivo.

### 21.1.1 Study design

In two studies, EAE was induced in SJL mice via PLP139-151/CFA priming as described under Methods, below. The mice were divided into 4 and 8 groups in Study I and Study II, respectively groups (n=10/treatment group). In both studies, groups 1-4 the mice received two injections separated by one day, of either Control Ab or anti-CD25 (clone 7D4 at 500 µg/injection i.p. (BioXCell Cat# BE0013; West Lebanon, NH)) starting at the onset of disease remission (Day +19 and 21 in Study I or Day +20 and 22 in Study II), followed by treatment with either mIgG2a Control Ig or C1ORF32 ECD-Fc (100ug/dose; 3x/wk; 2wks) beginning on the day of the second treatment with anti CD25 (Day 21 in study I and day 22 in study II).

To study the importance of Treg function for maintenance of tolerance by C1ORF32 ECD-Fc a second set of mice was set in study II, groups 5-8, in which mice were treated with Control Ig or C1ORF32 ECD-Fc (SEQ ID NO:42) first in a similar regimen as groups 1-4 (beginning on Day +22), and then two weeks following the last C1ORF32 ECD-Fc (SEQ ID NO:42) treatment, on days 46 and 48, mice received either Control Ab or anti-CD25 (500 µg/injection) to inactivate the Treg cells. The mice were followed for the level of disease severity.
C1ORF32 ECD-Fc (SEQ ID NO:42) was diluted in PBS pH 6.0, 0.01% Tween 80, anti-CD25 and control Abs were diluted in PBS.

### Study Itreatment groups (n=10mice per group)

1. Control Ab (Day +19 and 21) plus Control Ig (100ug/dose; 3x/wk; 2wks; beginning on Day +21)
2. Anti-CD25 Ab (Day +19 and 21) plus Control Ig (100ug/dose; 3x/wk; 2wks; beginning on Day +21)
3. Control Ab (Day +19 and 21) plus C1ORF ECD-Fc (SEQ ID NO:42) (100ug/dose; 3x/wk; 2wks; beginning on Day +21)
4. Anti-CD25 Ab (Day +19 and 21) plus C1ORF ECD-Fc (SEQ ID NO:42) (100ug/dose; 3x/wk; 2wks; beginning on Day +21)

### Study II treatment groups (n=10 mice per group)

1. Control Ab (Day +20 and 22) plus Control Ig (100ug/dose; 3x/wk; 2wks; beginning on Day +22)
2. Anti-CD25 Ab (Day +20 and 22) plus Control Ig (100ug/dose; 3x/wk; 2wks; beginning on Day +22)
3. Control Ab (Day +20 and 22) plus C1ORF32 ECD-Fc (SEQ ID NO:42) (100ug/dose; 3x/wk; 2wks; beginning on Day +22)
4. Anti-CD25 Ab (Day +20 and 22) plus C1ORF32 ECD-Fc (SEQ ID NO:42) (100ug/dose; 3x/wk; 2wks; beginning on Day +22)
5. Control Ig (100ug/dose; 3x/wk; 2wks; beginning on Day +22) plus Control Ab (Day +46 and 48)
6. Control Ig (100ug/dose; 3x/wk; 2wks; beginning on Day +22) plus Anti-CD25 Ab (Day +46 and 48)
7. C1ORF32 ECD-Fc (SEQ ID NO:42) (100ug/dose; 3x/wk; 2wks; beginning on Day +22) plus Control Ab (Day +46 and 48)
8. C1ORF32 ECD-Fc (SEQ ID NO:42) (100ug/dose; 3x/wk; 2wks; beginning on Day +22) plus Anti-CD25 Ab (Day +46 and 48)

### 21.1.2 Methods

### 21.1.2.1 Stock of CFA:

Five 10ml ampules of Incomplete Freunds Adjuvent (IFA; Difco cat #263910) were combined with two, 100µg ampules of M.tuberculosis H37RA (Difco cat#231141) in a glass 100ml bottle. The final concentration of the Complete Freund's Adjuvant was 4mg/ml. Stored at 4°C.

### 21.1.2.2 CFA & PLP Emulsion preparation:

In omni-mixer bucket, peptide stock was diluted in PBS to desired concentration (e.g. 1mg/ml for PLP139-151) and then equal volume of CFA was added. Before removal of the needed volume from the Complete Freund's Adjuvant it was mixed thoroughly to ensure that the M. tuberculosis is evenly mixed (since M. tuberculosis falls out of solution). Bucket was attached to omnimixer, lowered into ice-filled dish until flush with counter, and mixed on high speed (setting 4-5) for 5 minutes. All the emulsion was scooped into 10ml snap-cap tube. Quickspin in centrifuge (up to 1200 rpm then stopped) and loaded into glass Hamilton syringes. Removed all air bubbles and attach 25g needle.

### 21.1.2.3 Animal Procedures and Treatments:

Naive ∼six week old SJL/J mice were purchased from Harlan, and allowed to acclimate to the animal care facility for 1 week. On day of disease induction, day 0, mice were shaved (about a 1in.x 1in, square on the back between the hind flanks) and injected s.c. with 100µl of CFA & PLP emulsion per mouse, divided between three spots on back and flanks. PLP139-151 peptide final dose given per mouse was 50µg.

SJL mice were primed with 50µg PLP139-151/CFA to induce R-EAE, and followed for disease through the acute phase of disease and into disease remission. At disease remission mice were divided into 4 treatment groups. On Days +19 and 21, mice received either Control Ab or anti-CD25 treatment (500µg/dose). Beginning on Day +21 mice received Control Ig or C1ORF32 ECD-Fc (SEQ ID NO:42) treatment (100µg/dose; 3 doses/wk; 2wks). Clinical results are expressed as the mean clinical score.

Mice were followed for disease and scored on a 0-5 scale per standard lab protocol as follows: 0, no abnormality; 1, limp tail; 2, limp tail and hind limb weakness; 3, hind limb paralysis; 4, hind limb paralysis and forelimb weakness; and 5, moribund.

### 21.1.3 Results

### Study I

In the absence of anti-CD25 treatment a relapsing EAE disease was observed which was abrogated by treatment with C1ORF ECD-Fc (SEQ ID NO:42) (Figure 14, groups Control Ab + Control Ig vs. Control Ab + C1ORF32 ECD-Fc). Transient inactivation of Tregs in the EAE model by administration of anti-CD25 at early stage of the disease, right after the acute phase resulted in disease exacerbation, as expected. Treatment with C1ORF32 ECD-Fc (SEQ ID NO:42) starting after Anti CD25 administration resulted significant decrease in disease severity (Figure 14, groups Anti CD25 + control Ig vs. Anti-CD25 + C1ORF32 ECD-Fc).

### Study II

In a second study transient inactivation of Tregs in the EAE model by administration of anti-CD25 (antibody) at early stage of the disease, right after the acute phase, did not affect the therapeutic efficacy of C1ORF32 ECD-Fc (SEQ ID NO:42) which completely inhibited diseases symptoms (Figure 15A). However anti-CD25 treatment given at a later stage, 2 weeks after completion of treatment with C1ORF32 ECD-Fc (SEQ ID NO:42), resulted in immediate relapse which was alleviated as the effect of anti-CD25 dropped off (Figure 15B). Anti CD25 treatment impairs Treg function. Its administration results in disease exacerbation, hence the relapse observed right after treatment with anti-CD25. However, the effect of anti CD25 is transient and and decreases significantly after several days, allowing Treg cells to gain back their regulatory function. The group that was treated with C1ORF32 ECD-Fc had their disease alleviated after the transient effect of anti-CD25 ended, while the control Ig treated group continued to suffer significantly from the disease, indicating the longevity of the effect of C1ORF32 ECD-Fc treatment.

This alleviation of disease is probably related to iTregs induced by C1ORF32 ECD-Fc (SEQ ID NO:42) (as previously shown in vitro and in vivo) which are important for the durable effect of C1ORF32 ECD-Fc in R-EAE and are important players in restoration of immune tolerance by C1ORF32 ECD-Fc (SEQ ID NO:42) treatment. The efficacy induced by C1ORF32 ECD-Fc treatment following Treg inactivation at early stage of the disease, right after the acute phase, indicates that at early stages of the disease other mechanisms, probably inhibition of Th1 and Th17 responses, underly the therapeutic effect of C1ORF32 ECD-Fc.

### Example 6: Efficacy of C1ORF32 ECD-Fc (SEQ ID NO:42) in the EAE model upon IL-10 or TGFb blockade

### Study design

SJL/J mice were purchased from Harlan and primed with PLP139-151/CFA per the standard protocol, as described in 21.1.2 Methods, above). At disease remission the mice were split into 6 different treatment groups (n=5) as follows:
Treatment Groups (n=5 mice per group):
1. mIgG2a (control for C1ORF32 ECD-Fc (SEQ ID NO:42)) followed by rIgG1 (control for anti-IL-10)
2. mIgG2a followed by anti-IL-10
3. mIgG2a followed by anti-TGF-β
4. C1ORF32 ECD-Fc (SEQ ID NO:42) followed by rIgG1
5. C1ORF32 ECD-Fc (SEQ ID NO:42) followed by anti-IL-10
6. C1ORF32 ECD-Fc (SEQ ID NO:42) followed by anti-TGF-β

Mice were treated with Control Ig (mouse IgG2a) or C1ORF32 ECD-Fc (SEQ ID NO:42) at 100ug/dose, each via an i.p. injection. This was followed with a second i.p. injection on the same day of either anti-IL-10 (rat IgG1), rat IgG1 (control Ab), or anti-TGFβ (mouse IgG1) at 100ug/dose, each. All treatments were given 3x/wk for 2 wks, beginning on day +20 (onset of disease remission) and until day +31 post disease induction. All mice were followed for disease scores until day 44 post disease induction. On day 44, mice were sacrificed and analyzed for total T cell and Treg cell numbers in spleen and CNS.

C1ORF ECD-Fc (SEQ ID NO:42) was diluted in PBS pH 6.0, 0.01% Tween 80, anti-CD25 and control Abs were diluted in PBS.

### Materials and Methods

### Animal Procedures and Treatments:

EAE was induced in SJL mice as described above.

At onset of disease remission (day +20 post disease induction) mice were allocated into 6 treatment groups (described above) and treated with either C1ORF32 ECD-Fc (SEQ ID NO:42) or control Ig (mIgG2a), and followed on the same day by treatment with anti-IL-10, anti-TGF-β, or rat IgG1 isotype control. All treatments were given at 100ug/dose in 100µl of PBS; via i.p. injection, at 3x/week for 2 weeks from onset of remission.

Mice were followed for disease and scored on a 0-5 scale per standard lab protocol as follows: 0, no abnormality; 1, limp tail; 2, limp tail and hind limb weakness; 3, hind limb paralysis; 4, hind limb paralysis and forelimb weakness; and 5, moribund. Dead mice were scored with a score of 5 on the day of death only, and were not further scored on the next days.

On day 44 post disease induction, the rest of the mice were sacrificed and spleens and CNS were taken for cell counts and cell population analysis as described below.

### Reagents

- Mouse IgG2a; BioXCell, Clone C1.18.4, Cat# BE0085
- Rat IgG1; BioXCell, Clone HRPN, Cat# BE0088
- C1ORF32 ECD-Fc (SEQ ID NO:42)
- Anti-IL-10; BioXCell, Clone JESS-2A5, Cat#BE0049
- Anti-TGF-β; BioXCell, Clone 1D11.16.8, Cat#BE0057

### Tissue collection:

1. Mice were deeply anesthetized using Nembutal (500ug via i.p. injection), and the mice were perfused with 30ml of sterile 1x PBS.
2. The spleens and CNS were collected.
3. Each tissue was kept separate for each individual mouse, and the tissues were processed individually.
4. The spleens were processed via tissue disruption by mashing the tissues through 100µm nitex filter. The filters were washed with 10ml of HBSS + 5% FCS.
5. For the spleens, the red blood cells (RBCs) were lysed via ammonium chloride treatment for 4 min at 37deg C. The cells were pelleted, washed 3 times with HBSS + 5% FCS, any cellular debris was removed via the use of 40µm nitex filter, and counted on a hemocytomter via trypan blue exclusion.
6. The CNS tissue was processed as follows. The CNS tissue was chopped coarsely with scissors, and incubated with 1ml per CNS of Accutase (Millipore; Cat# SCR005) for 30 min at 37degC.
7. After the 30min incubation 5ml of HBSS+5% FCS was added to each sample, and the sample was transferred to a 40um nytex mesh cell strainer on top of a 50ml conical tube, and push CNS through mesh by scraping mesh with the blunt end of a 1cc-syringe plunger.
8. The cell strainer was washed twice with 10ml of HBSS+5% FCS.
9. The isolated cells were pelleted by centrifugation at 300xg for 10min.
10. The pellet was resuspended in 3ml/CNS 70% Percoll and transfer to 15ml conical tube.
11. An equal volume of 30% Percoll was layered on top.
12. Tubes were spun at room temp centrifuge at 1600 rpm (600g) for 30 minutes with no brake.
13. The myelin layer on top was removed by aspiration, and the cells at the 70/30 interface were collected.
14. The collected CNS cells were washed 2x with HBSS+5% FCS, and counted.

### Flow Cytometry:

### Flow cytometry control tubes and experimental sample tubes

1. Unstained control
2. Single stain controls
3. FMO controls
4. Experimental stains (CD45, CD3, CD4, CD25, FoxP3, Nrp-1, and Helios)

### Cells preparation for flow cytometry:

Harvest, wash and count cells, and resuspend at 10 x 106 cells/mL
1. Plate 50-100uL (0.5-1 x 10⁶ cells) in each well, wash cells three times with IX PBS, and resuspend with 100uL of LIVE/DEAD® Fixable Aqua Dead Cell Stain Kit *for 405 nm excitation (Invitrogen; Cat# L34957) for 20 minutes on ice.
2. Wash cells three times in 1xPBS+5% FCS.
3. Resuspend cells in 100ul of mouse Fc Block (anti-CD16/32; eBioscience; Cat# 14-0161-86) diluted 1:100 in 1xPBS+5% FCS. Cells were incubated at 4degC for 20 min.
4. The cells were washed three times in 1xPBS+5% FCS, and then resuspended in the cell surface staining cocktail of antibodies listed below in a final volume of 100ul. The cells were incubated for 30min. For the FMO controls, one of the specific antibodies was removed per FMO control.
   a. Anti-CD45 APC-Cy7 (eBioscience; Clone 30-F11; Cat#47-0451)
   b. Anti-CD3 PE-Cy7 (eBioscience; Clone 145-2C11; Cat#25-0031)
   c. Anti-CD4 Pacific Blue (eFluor 450) (eBioscience; Clone GK1.5; Cat#48-0041)
   d. Anti-CD25 Fitc (Alexa Fluor 488) (eBioscience; Clone 7D4; Cat#53-0252)
   e. Anti-Nrp-1 APC (eBioscience; Clone 3DS304M; Cat#17-3041)
5. The cells were washed three times in 1xPBS, and resuspend 200uL in freshly made Fix/Perm solution (eBioscience; Cat# 00-5523-00) and incubate overnight (or 30min) at 4degC.
6. The cells were washed three times in 1x Perm. Buffer, and resuspended in the intracellular staining cocktail of antibodies listed below in a final volume of 100ul of Perm. buffer.
   a. Anti-FoxP3 PercP-Cy5.5 (eBioscience; Clone FJK-16s; Cat#45-5773)
   b. Anti-Helios PE (eBioscience; Clone 22F6; Cat#12-9883)
7. Following a 30min incubation at 4degC, the cells were washed three times with Perm buffer, and two times with 1xPBS+5% FCS.
8. The cells were resuspended in 400ul of 1xPBS+5% FCS, and analyzed by flow.
9. Gating scheme for flow: Singlets (FSC-A vs. FSC-H) -> Cells (SSC-A vs. FSC-A) -> Live (VID stain negative cells) -> CD45hi -> CD3/CD4+ -> CD25/FoxP3+ -> Helios/Nrp-1

### Results

Treatment of mice primed with PLP/CFA with C10RF32 ECD-Fc (SEQ ID NO:42) (followed by rIgG1) resulted in pronounced inhibition of disease progression as compared to mice treated with control Abs only (mIgG2a followed by rIgG1). Neutralization of IL-10 (in the presence of control mIgG2a) did not significantly affect disease severity compared to control Abs only (Figure 16A). Neutralization of TGFβ (in the presence of control mIgG2a) led to exacerbation of disease severity (Figure 16B), manifested by increased average clinical score. These findings suggest that the treatment with either anti-IL-10 or anti-TGFβ neutralized the effector function of IL-10 and TGFβ in vivo. Importantly, concomitant administration of anti-IL-10 or anti-TGFβ following treatment with C10RF32 ECD-Fc (SEQ ID NO:42), abrogated the beneficial effect of C1ORF32 ECD-Fc (SEQ ID NO:42) (Figures 16A and 16B).

In both groups treated with anti-TGFβ, either alone or in combination with C10RF32 ECD-Fc (SEQ ID NO:42) and in the group treated with anti-IL-10 in combination with C10RF32 ECD-Fc (SEQ ID NO:42), mice were found dead on days 27-28 of the study. Specifically, in the group treated with mIgG2a and anti-TGFβ, 2 mice were found dead on day 27; 1 dead mouse was found in the group treated with C10RF32 ECD-Fc (SEQ ID NO:42) and anti-TGFβ on day 28, and 2 dead mice were found on day 28 in the group treated with C10RF32 ECD-Fc (SEQ ID NO:42) in combination with anti-IL-10. Death of these mice is manifested as a 'spike' in the score graph of the respective group on the day of death (Figures 16A and 16B). These deaths are probably due to the more severe disease resulting from inactivation of Tregs which play a major role in this disease model.

Figures 17A and 17B show that C10RF32 ECD-Fc (SEQ ID NO:42) treatment resulted in pronounced decrease of leukocytes and T cell counts in the CNS (CD45+, CD4+, and CD25+/FoxP3+), which was accompanied by an increase in T cell counts in the spleen (mainly CD4+). These observations suggest inhibition of autoreactive T cell migration to the CNS, and does not support generalized T cell depletion. These effects of C10RF32 ECD-Fc (SEQ ID NO:42) were not observed following concomitant neutralization of IL-10 or TGF-β. Importantly, the reversal of the C10RF32 ECD-Fc (SEQ ID NO:42)-induced decrease in the number of infiltrating cells within the CNS suggests that both IL-10 and TGF-β are required for C10RF32 ECD-Fc (SEQ ID NO:42) biological function in vivo with regard to the level of disease severity.

In addition, analysis of the Treg population with Helios and Nrp markers shows a trend of increase in the Nrp+/HeliosLo subpopulation in the spleen following treatment with C10RF32 ECD-Fc (SEQ ID NO:42) alone or concomitantly with anti-TGFβ. An increase in the NRP+/Helios+ subpopulation was observed when C10RF32 ECD-Fc (SEQ ID NO:42) and anti-TGFβ were administered concomitantly. C10RF32 ECD-Fc (SEQ ID NO:42) treatment alone also resulted in upregulation of Nrp-/HeliosLo (Figure 17C). These trends are not evident following concomitant treatment with anti-IL-10. In the CNS, analysis of Treg subpopulations is not informative due to the strong reduction in Treg cell numbers in the CNS following treatment with C10RF32 ECD-Fc (SEQ ID NO:42) (Figures 17B and 17D).

Breg analysis could be performed in a similar manner as described above for Treg: at week 8 post transplantation, blood samples and spleens are analyzed for B cell populations including Breg cells by FACS analysis for CD19, CD24, CD38 and intracellular IL-10. The data shows a generally similar effect as for Treg as described above.

### Summary of the Above Examples

The data presented herein show loss of C1ORF32 ECD-Fc (SEQ ID NO:42) therapeutic effect in the EAE model upon neutralization of TGF-beta or IL-10, and abolishment of C1ORF32 ECD-Fc (SEQ ID NO:42)-induced reduction of CD45+ cells, T cells and Treg cell counts in the CNS. This finding suggests that the therapeutic effect of C1ORF32 ECD-Fc (SEQ ID NO:42) in this model is dependent on TGF-beta and IL-10. Since TGF-beta and IL-10 are important factors in the induction, differentiation and function of Treg cells, this dependency supports an important role for Treg cells in the therapeutic effect of C1ORF32 ECD-Fc (SEQ ID NO:42). In addition, looking at subpopulations of Treg cells in the spleen, a trend towards increase in Nrp+HeliosLo and Nrp-HeliosLo (based on the literature, the latter may be considered as iTreg cells) was observed. Following treatment with C10RF32 ECD-Fc (SEQ ID NO:42) concomitantly with anti-TGF-beta a trend was observed towards an increase in Nrp+Helios+ (based on the literature, these may be considered as nTreg cells). These trends were not observed upon IL-10 neutralization.

Overall, these results demonstrate that C10RF32 ECD-Fc induces long term efficacy in the EAE and inhibits graft rejection in a model of BM transplantation both of which are accompanied by elevation in Treg counts. Furthermore, the long term efficacy of C10RF32 ECD-Fc requires functional Treg presence. As C10RF32 ECD-Fc induces IL-10 secretion, which is a major mediator of Treg function, and as its efficacy in the EAE model seem to depend on IL-10 or on maintenance of iTreg stability by TGFβ, the above described results indicate that the tolerogenic effects of C1ORF32 ECD-Fc are mediated at least in part by IL-10 and/ or TGFβ.

### Example 7 - Evaluation of induction of tolerance by C1ORF32 ECD-Fc in the PLP139-151-induced EAE model by re-challenge of mice with the EAE with either PLP139-151 or OVA323

### Induction of adoptive transfer PLP139-151-R-EAE:

a. Donor mice are shaved (about a 1in.1in, square on the back between the hind flanks) and injected with CFA/ PLP139-151 emulsion at 100µl per mouse s.c., divided between three spots on the back and flanks. Peptide and final dose given per mouse: 50µg of PLP139-151 peptide on day 0.
b. On day 8 post priming with PLP 139-151/CFA, the draining inguinal lymph nodes are collected, and single cell suspensions are prepared. Total lymph node cells are cultured at a density of 8x10⁶ cells per ml in the HL-1 medium plus PLP 139-151 peptide (20µg/ml) in T75 flasks.
c. After 3 days of culture, the PLP139-151 reactivated cells are collected and counted using an hemacytometer. The number fo total viable cells and the number of blast cells is determined. The percentage of blast cells expected following this protocol is 20-30% of the total viable cells.
d. 3x10⁶ cells are transferred to each recipient mouse by intravenous injection.
e. Mice are treated with Control Ig (mIgG2a) or C1ORF32 ECD-Fc (3x/wk; 2wks) beginning at the time of cell transfer. The Control Ig or C1ORF32 ECD-Fc treated mice are then primed with PLP139-151/CFA, or OVA323/CFA at 14 days post the final treatment.
f. Mice are scored on a 0-5 disease score scale per standard laboratory protocol: 0, no abnormality; 1, limp tail; 2, limp tail and hind limb weakness; 3, hind limb paralysis; 4, hind limb paralysis and forelimb weakness; and 5, moribund.

If there appears to be a long-lived protection in the specific ability of PLP139-151/CFA priming to induce disease in C1ORF32 ECD-Fc treated mice, indicative of specific tolerance induction, follow up experiments are performed as follows:
I. Similar rechallenge experiments are carried out with other myelin (spread) epitopes (i.e. PLP178-191/CFA and MOG92-106/CFA) in addition to the inducing epitope PLP139-151/CFA
II. Rechallenge with the inducing PLP139-151/CFA at a longer time point (30 or 60 days) after completion of the treatment with C1ORF32 ECD-Fc or control Ig
III. Examining the phenotype of the CD4+ T cell effector and CD4+ T cell regulatory populations via flow cytometric analysis and ex vivo recall responses and suppression assays.

It is possible that due to CFA breaking tolerance during the re-priming, in the first experiment tolerance won't be observed with C1ORF32 ECD-Fc to the inducing PLP139-151/CFA. In such case, the re-challenge is carried out by a 2nd adoptive transfer of lymph node cells taken from SJL mice that have been primed with PLP139-151/CFA, PLP178-191/CFA, or MOG92-106/CFA.

### Example 8: Effect of C1ORF32 ECD-Fc on alteration of Ag-specific Tregs in vitro and in vivo, and on Treg suppressive activity.

### Ag-specific Tregs, in vitro

Naive PLP139-151-specific T cells from 5B6 mice are activated with PLP-pulsed APCs in iTreg promoting conditions and tested for in vitro suppressive activity and ability to inhibit induction and progression of PLP139-151-induced vs. MOG92-106-induced EAE.
- Naive PLP139-151-specific T cells from 5B6 mice (Tg for TCR specific for PLP139-151) will be isolated via automax sort (CD4-negative sort plus and CD25 positive isolation, followed by CD62L-positive sort)
- The naive cells are activated in the presence of irradiated APCs (at 1:1 ratio; 5x10⁵ T cells per well) and OVA323-339 (20ug/ml)
- C10RF32 ECD-Fc at 1, 3 or 10 ug/ml or Control Ig (mIgG2a) at 10ug/ml will be added to the wells. Control Ig are used to complete the amount of tested protein in each well to a total of 10ug/ml
- iTregs are induced by IL-2 (100U/ml) and TGF-beta (10ng/ml), in the absence or presence of retinoic acid (100nM)
- On day 4 of culture, cells are harvested and stained for viability, and for CD4, CD25, and FoxP3 expression as markers of iTregs
- Effector T cells are induced by ex-vivo activation of CD4+ T cells with anti-CD3 and anti-CD28 in the presence of irradiated APCs.
- Suppression activity is tested in vitro by culture of FoxP3+ Treg cells with effector T cells at different ratios of Tregs vs. effector T cells, and T cell proliferation is measured by H³-Thymidine incorporation.
- Suppression activity is tested in vivo by transfer of the FoxP3+ T cells form C10RF32 ECD-Fc treated cultures described above to mice that were induced to develop EAE with PLP139-151 vs. MOG 92-106. Cell transfer is done at time of disease induction or at onset of disease remission to test the effect of C1ORF32 ECD-Fc-induced Tregs on disease induction and progression respectively.

### Ag-specific Tregs, in vivo

Naive CD4+ T cells are transferred from 5B6 mice (Tg for TCR specific for PLP139-151) into PLP178-191/CFA primed SJL mice at the peak of the acute phase of disease. The recipient mice with EAE are treated from onset of remission with Control Ig or C10RF32 ECD-Fc, and the number, location (CNS or spleen), and phenotype of the transferred 5B6 T cells are determined via flow cytometry. Analysis is carried out after 1 week of treatment and after the full 2 weeks of treatment.

This experiment can also be completed with 5B6-FoxP3/GFP cells such that the 5B6-FoxP3/GFP Treg cells can be sorted purified, and placed into an in vitro (ex vivo) suppression assay.

### General Tregs, in vitro

Naive CD4+ T cells from FoxP3-GFP mice are activated in the presence of iTreg cell promoting conditions (100U/ml IL-2, 10ng/ml TGF-beta, in the absence or presence of 100nM retinoic acid) in the presence of either Control Ig (mIgG2a) C10RF32 ECD-Fc. Following culture, FoxP3/GFP+ cells are sort purified, and the function of the iTreg cells is assessed in an in vitro suppression assay by culture of FoxP3+ Treg cells with effector T cells at different ratios of Tregs vs. effector T cells. The effector T cells are induced by ex-vivo activation of CD4+ T cells with anti-CD3 and anti-CD28 in the presence of irradiated APCs. T cell proliferation is measured by H3-Thymidine incorporation.

If the C10RF32 ECD-Fc treated iTreg cells do appear to have increased suppressor function, it will be determined whether this suppressor function is cell contact mediated or via secreted factors. For this a combination of trans-well Treg cell suppression assays and conditioned culture medium transfer in the absence or presence of blocking Abs is utilized.

### General Tregs, in vivo

R-EAE is induced in FoxP3-GFP mice on SJL background PLP139-151/CFA. Mice are treated with C1ORF32 ECD-Fc or mIgG2a (100ug/dose, 3times/ week x 2 weeks) and are followed for disease severity. (A) Unseparated total CD4+ T cells and (B) FACS-purified CD4+CD25+Foxp3/GFP+ Treg cells are isolated right after the last treatment and are tested in a 'suppression assay' by culturing each of the above T cell populations (A or B) with effector T cells at different ratios of Tregs vs. effector T cells (as described in 23.1.1 above). T cell proliferation is measured by H3-Thymidine incorporation and cytokine production will be evaluated using ELISA.

In addition the ratio of Treg to Teff cells in the spleen and CNS is also evaluated.

### EXAMPLE 9:

### EFFICACY OF ANY OF THE C1ORF32 PROTEIN FRAGMENTS AND/OR FUSION PROTEINS THEREOF IN MOUSE CIA MODELS OF RHEUMATOID ARTHRITIS

In brief, this study indicates that C1ORF32 ECD-Fc (SEQ ID NO:42) at 10 mg/kg has a therapeutic effect during experimental arthritis, which is at least similar to, or even greater than that of Enbrel. For certain measurements of rheumatoid arthritis markers in this mouse model, C1ORF32 ECD-Fc (SEQ ID NO:42) had an even greater effect than Enbrel. Indeed in this model, Enbrel appeared far weaker than in previous mouse model studies, perhaps due to the extreme severity of this model. By contrast, C1ORF32 ECD-Fc (SEQ ID NO:42) was able to ameliorate the disease even in this severe model of the disease. However, it should be noted that in three studies, C1ORF32 ECD-Fc (SEQ ID NO:42) showed excellent results in two studies and weak results in one study.

### Study protocol

Bovine type II collagen (CII) (RUNMC in house production, batch 03-04-08) at a concentration of 2 mg/ml in acetic acid 0.05M was emulsified in equal volumes of Freund's complete adjuvant (2 mg/ml of Mycobacterium tuberculosis strain H37Ra; Difco, Detroit, MI).

On day 0, 10-12 week old male DBA-1 mice (Janvier Elevage, France) (n=95) were immunized i.d. at the base of the tail with 100 µg of bovine CII. On day 21, mice received an intraperitoneal booster injection of 50 µg of CII dissolved in phosphate buffered saline (PBS), and the onset of arthritis occurred a few days after this booster.

Mice were considered to have arthritis when significant changes of redness and/or swelling were noted in the digits or in other parts of the paws. Joint inflammation in each paw was scored visually, using a scale of 0-2 per paw with a maximal score of 8 per animal: 0 = non inflamed, 1 = mild inflammation, 1.5 = marked inflammation, and 2 = severe inflammation. Scoring was performed three times a week.

Treatment was started in established CIA early after onset of disease (i.e. therapeutic regimen), when the macroscopic scores per animal were between 0.25 and 1.25 (on a scale of 0-8). The i.p. injections were given three times per week for two weeks, after which the study was terminated and mice were terminally bled to collect serum. Thereafter, the knee and ankle joints were collected and stored in formalin. Histology was scored for inflammation and destruction on an arbitrary scale of 0-3, for five different parameters:
- Joint inflammation
- Cartilage proteoglycan (PG) depletion
- Chondrocyte death
- Cartilage erosion
- Bone erosion

### Treatment groups, 10 mice/group:

1. Negative control: PBS
2. Negative control mIgG2A (Bioxcell, BE0085): 10mg/kg
3. C1ORF32 ECD-Fc (SEQ ID NO:42): 10 mg/kg
4. C10RF32 ECD-Fc (SEQ ID NO:42): 5 mg/kg
5. Positive control: Enbrel (Wyeth, F01421) 5 mg/kg

### Outcome parameters

- Macroscopic scores throughout the study (14 days of treatment)
- Histological analysis of the ankle joints at day 14 after start of treatment

### Histological analysis

Joints were fixed for at least 4 days in 4% formaldehyde, decalcified in 5% formic acid, and subsequently dehydrated and embedded in paraffin. Standard frontal sections of 7 µm were mounted on SuperFrost slides (Menzel-Gläser, Braunschweig, Germany). Haematoxylin and eosin (H&E) staining was performed to study joint inflammation. The severity of inflammation in the joints was scored on a scale of 0-3 (0 = no cells, 1 = mild cellularity, 2 = moderate cellularity, and 3 = maximal cellularity). To study proteoglycan (PG) depletion from the cartilage matrix, sections were stained with safranin O (SO) followed by counterstaining with fast green. Depletion of PG was determined using an arbitrary scale of 0-3, ranging from normal, fully stained cartilage to destained cartilage, fully depleted of PGs. Chondrocyte death was scored on a scale of 0-3 ranging from no loss of chondrocyte nuclei to complete empty cartilage surface. The degree of cartilage surface erosion was scored on a scale of 0- 3, ranging from no damage to complete loss of articular cartilage. Bone destruction was graded separately on a scale of 0-3, ranging from no damage to the complete loss of bone structure. Histopathological changes in the joint were scored on three semiserial sections of the joint spaced 70 µm apart. Scoring was performed in a blindfolded manner.

Statistical analysis: Disease course data was analyzed using one-way ANOVA with repeated measures, with a Bonferroni's post-test of selected pairs to determine statistical differences between C10RF32 ECD-Fc (SEQ ID NO:42) and Enbrel treated mice, each as compared to Control Ig (IgG2a) treated group.

### Results

### Effect of treatment with C1ORF32 ECD-Fc (SEQ ID NO:42) on macroscopic scores of CIA

Collagen-induced arthritis in mice treated from disease onset with the negative control PBS or mIgG2A isotype control antibodies showed clear progression of the macroscopic scores of inflammation (Figure 18). High dose of C1ORF32 ECD-Fc (SEQ ID NO:42) (10 mg/kg) significantly reduced the clinical scores of arthritis (p<0.001) similarly to the effect of TNF blocking with Enbrel that was used as positive control treatment (Figure 18). Importantly, the effect of Enbrel in this study was not as potent in reducing the arthritis scores as expected from previous studies, suggesting that a more severe disease was obtained in this CIA experiment. C1ORF32 ECD-Fc (SEQ ID NO:42) at 5 mg/kg did not demonstrate any effect in this experiment.

### Effect of treatment with high dose C1ORF32 ECD-Fc (SEQ ID NO:42) on histological scores

To potentially obtain a better differentiation in the effects of high dose C10RF32 ECD-Fc (SEQ ID NO:42), ankle joints were processed for full histological analysis. As depicted in Figure 19, C1ORF32 ECD-Fc (SEQ ID NO:42) treatment during collagen-induced arthritis resulted in a trend of reduced arthritis pathology after two weeks of therapy, but these effects did not reach statistical significance. Enbrel treatment resulted in significant reduction of bone erosions but in the other histological parameters of inflammation and cartilage damage, only a trend for protection was observed. These results are in agreement with the weaker effect of Enbrel on macroscopic scores (Figure 18), compared to previous studies with similar treatment protocols, further supporting a conclusion that a more severe disease was obtained in this CIA experiment (Figure 19).

### Summary

The treatment with C10RF32 ECD-Fc (SEQ ID NO:42) during collagen-induced arthritis, at the high dose of 10 mg/kg, significantly reduced the macroscopic scores and showed a trend of reduction of the histological scores for inflammation and destruction, although the latter parameters did not reach statistical significance. The treatment with the clinically applied TNF inhibitor Enbrel was not as potent on macroscopic scores as expected, probably due to the fact that this CIA experiment was more severe than previous studies. No therapeutic effect was observed with the low dose C10RF32 ECD-Fc (SEQ ID NO:42) (5 mg/kg).

Overall, this study indicates that C10RF32 ECD-Fc (SEQ ID NO:42) at 10 mg/kg has a therapeutic effect during experimental arthritis, which is at least similar to, or even greater than that of Enbrel. Options to consider are (1) an earlier start of treatment, since prophylactic/semi-therapeutic approaches are often less challenging for treatment than the therapeutic regimen, (2) a longer follow-up, since the effects of C1ORF32 ECD-Fc (SEQ ID NO:42) were most obvious in the second week of treatment during this study, (3) use higher dose of C10RF32 ECD-Fc (SEQ ID NO:42). Although further preclinical research in animal models of arthritis are required, these data suggest that inhibition of costimulation by C10RF32 ECD-Fc (SEQ ID NO:42) might be a good option to reduce joint pathology in patients suffering from rheumatoid arthritis.

### Example 10 - Evaluation of the effect of C1ORF32 ECD-Fc on iTreg differentiation in T cell: DCs co-culture

### In vitro differentiation of iTregs:

iTregs are induced from naive T cells 96-well flat bottom tissue culture plates (Sigma, Cat. # Z707910) are coated with anti-CD3 mAb (2ug/mL) and C1ORF ECD-Fc (H:M) or control Ig control (MOPC-173, Biolegend) at 10ug/ml. Naive CD4⁺CD25⁻ T cells are plated at 0. 5x10⁵/well in the presence of soluble anti-CD28 (1ug/ml), TGF-β (Cat# 7666-MB; R&D systems) and IL-2 (Cat# 202-IL; R&D systems). On Day 5 post stimulation the percentage of CD4⁺CD25⁺FoxP3⁺ cells is assessed by flow cytometry.

### Isolation of splenic DCs

Mouse spleens are fragmented and digested for 30 min at 37°C in complete RPMI 1640 in the presence of Liberase and DNase. After digestion, RBCs are lysed with ACK-lysing buffer. DCs are isolated by labeling cells with CD11c magnetic beads and MACS (>95% purity). DCs are pulsed with 1 mM PCC88-104 or 10 mM HEL48-62 peptides for 37°C for an hour, washed two times with complete media, and then cultured alone or cocultured with Foxp3+ iTregs from Control Ig or C1ORF ECD-Fc cultures described above or with Teff cells at a ratio of 1:1 for 18 h. The cocultures are treated with 4 mM EDTA to separate DC-iTreg conjugates. Cells are first gated on singlets and then sorted for CD4 CD11c+MHC-II+DC population (Chattopadhyay and Shevach, J Immunol. 2013; 191(12):5875-84).

### EXAMPLE 11:

### THE EFFECT OF ANY ONE OF C1ORF32 PROTEIN FRAGMENTS AND/OR FUSION PROTEINS THEREOF IN MOUSE MODEL OF PSORIASIS

The purpose of this study was to evaluate the effectiveness of C1ORF32 ECD-Fc (SEQ ID NO: 43) on the histological parameters of psoriasis in a humanized model of psoriasis. As previously noted, SEQ ID NO:43 is a fusion protein, comprising an amino acid sequence of human C1ORF32 ECD fused to human immunoglobulin Fc (human-human fusion protein). The previous studies related to human C1ORF32 ECD fused to mouse immunoglobulin Fc (human-mouse fusion protein SEQ ID NO:42).

In this model, psoriasis is induced in xenotransplants of healthy human skin transplanted onto beige severe combined immunodeficiency (SCID) mice followed by intradermal (i.d.) injection of IL-2 enriched Peripheral Blood Mononuclear Cells (PBMCs) isolated from blood of psoriasis patients (Nickoloff, 1995; Wrone-Smith, 1996 Gilhar et al., 2002, 2006, 2011). The activity of these compounds was compared to that of Enbrel, a TNF inhibitor which is an approved biological drug for treatment of psoriasis. As a control for model robustness, we used dexamethasone, a steroid with suppressive effects on inflammatory cytokines and chemokines such as TNF-alpha and interferon-gamma which cause severe psoriasis. All treatments were administered intradermally, except for dexamethasone which was given topically.

### METHODS

Patient Demographics: All experiments with human materials were conducted after receiving written informed consent from the participants according to a protocol reviewed and approved by the RAMBAM Medical Center Institutional Review Board in accordance with the Declaration of Helsinki Principles. All experiments using laboratory mice were approved by the Baruch Rappaport Faculty of Medicine, Technion-Israel Institute of Technology, institutional committee on animal use. Ten psoriatic patients were included in this study (8 men and 2 women), mean age 46 years, ranged from 26 to 67 years. All patients had classic plaque psoriasis and none of them had been treated with drugs. Normal skin from 1 healthy volunteer was obtained for grafting.

Study Protocol: A) Healthy human skin pieces with a width of 0.4 mm and surface area of 1.5 x 1.5 cm were provided from residual skin of routine plastic surgery procedures from the Plastic Surgery Department of the Rambam Medical Center, Israel. In addition, 20 mL blood samples were taken from psoriatic patients. B) Seventy (70) beige-severe combined immunodeficient mice (SCID) (weight ∼20- 25 g) were included in this study. Normal healthy human donor skin was transplanted onto the beige-SCID mice as previously described (Nickoloff et al., 1995; Wrone-Smith, 1996; Gilhar et al., 2002, 2006, 2011; Kalish et al., 2009) and the mice were divided into 7 groups. C) Peripheral Blood Mononuclear Cells (PBMCs) isolated from psoriatic patients' blood were cultured in the presence of IL-2 [Prospec, 100 U/mL of RPMI 1640 media, 10% human AB serum (Sigma, St.Louis, MO), 1% glutamine, 1% antibiotics (media components; Biological Industries, Kibbutz Beit Haemeck, Israel)] for 14 days, as previously described (Gilhar et al, 2002). D) Four weeks following engraftment, each mouse was injected intra-dermally (i.d.) into the grafted skin lesion, with 1 X 10⁷ activated allogeneic enriched PBMCs from psoriatic patients (Figure 20). Cells from different psoriasis patients were equally distributed between treatment groups so that each patient is represented in each treatment group. E) On the day of PBMCs injection into the skin grafts, mice were divided randomly into treatment groups (n=9-10 mice per group) and administration of all drugs started (Figure 20). All tested compounds including the vehicle were given intradermally (intralesional), three times a week, except for dexamethasone which was given topically for 14 consecutive days (Table 3).

**Table 3 - summary of treatment protocol**

| **Group** | **Compound/batch number** | **Compound name** | **Route** | **Frequency** | **Dose per administration** | **Injection volume** |
|---|---|---|---|---|---|---|
| **1** (Blinded) | Vehicle - phosphate-buffered saline (PBS) pH6 (10mM Na/K Phosphate, 140mM NaCl) - batch #153 | | I.D. | 3X/week | XX | 180 ul/mouse |
| **2** | Dexamethasone 50mg/ml | | Topical | 5X/week | 2 mg | 40 ul/mouse |
| **3** | Enbrel | | I.D. | 3X/week | 90 µg | 180 ul/mouse |
| **4** (Blinded) | | **C1ORF32 ECD-Fc (SEQ ID NO**: **43)** | I.D. | 3X/week | 100 µg | 180 ul/mouse |

F) Twenty eight days after starting the injections and treatment, skin was harvested (Figure 20). Grafts were analyzed for the following psoriatic parameters by histology: epidermal thickening (acanthosis), suprapapillary epidermal thinning (papillomatosis), hyperkeratosis, parakeratosis, orthokeratosis, agranulosis - hypogranulosis, appearance of neutrophils in the upper spinous layer or in the corneal cell layer - Munro microabscess, regular elongation of rete ridges, angiogenesis, edema and dilated tortuous blood vessels in the dermal papilla and mononuclear cell infiltrate in the papillary dermis. Epidermal thickness was measured as detailed below, and HLA-DR, ICAM-1, Ki-67 and immune infiltrates (anti-CD3 stain) were evaluated by immunohistochemistry (IHC).

### Determination of Epidermal Thickness:

The entire skin graft was excised and placed in 10% formal saline overnight. Then, the specimens were place in 70% ethanol and were embedded according to the standard H&E protocol. Histological assessment of the skin grafts was performed by light microscopy and evaluated by two blinded observers [the second observer evaluated random slides (about 25% of all slides) to compare the data obtained by the first observer. Rarely, the results were not comparable, and in these cases the first observer re-evaluated the slides]. Epidermal thickness was determined with an ocular micrometer at a minimum of 50 points along the epidermis selected to represent points of maximal and minimal thickness.
IHC
Representative grafts were allocated for IHC analysis. Each analysis was performed on equal samples of affected and healthy grafts. The following markers were evaluated by IHC:
- HLA-DR: MHC class II highly expressed in keratinocytes from psoriatic patients
- ICAM-1: an intercellular adhesion molecule, highly expressed in the epidermis and upper dermis of psoriatic patients
- Ki-67: a marker of cell proliferation which is highly expressed in psoriatic skin lesions and correlates with the clinical severity of the disease
- CD3: a marker for T cells infiltration

Mouse anti-human HLA-DR (abcam, cat# ab20181), rabbit anti-human ICAM-1 (AbD Serotec, AHP2183), mouse anti-human CD3 (Dako), and mouse anti-human Ki-67 (Life Technologies, cat# ZY-180192Z) were used on deparaffinized and peroxidase blocked slides. Sections were treated with citrate buffer, pH6 for HLA-DR and ICAM-1, and EDTA buffer, pH8 for CD3 and Ki-67, in the microwave oven for 20 minutes, cooled for 30 minutes at room temperature, and blocked for nonspecific binding. All washes were performed with phosphate-buffered saline. All antibodies were applied overnight at 40C. After washing, slides were incubated with appropriate secondary antibodies (biotinylated horse anti-mouse IgG (Vector Labs, Burlingame, CA), and horseradish peroxidase-conjugated donkey anti-rabbit IgG), followed by a wash and incubation with streptavidin-horseradish peroxidase (Jackson ImmunoResearch, West Grove, PA) for all the primary mouse antibodies. The markers were revealed by treating sections with 3-amino-9-ethylcarbazole. Samples were examined under light microscopy. Ki-67 index was determined by counting Ki-67 positive cells in the basal layer of the epidermis out of the total number of basal cells. Mean CD3 positive cells was calculated with an ocular micrometer at a minimum of 10 points with an area of 0.66mm2 each, along the upper dermis.

Statistical Analysis: Data are presented as the mean ± standard deviation (STDEV). The study parameters of each group were compared to those of the PBS control group using one-tailed distribution T-Test. Statistical significance was set at p<0.05.

### RESULTS

### Evaluation of the Human Skin Grafts

Vehicle and Positive Controls Treatment of grafts with vehicle, starting at the time of psoriasis induction, resulted in psoriasis/psoriasiform features as listed in study protocol, section F. Within the vehicle treated group, most grafts (8/10) showed psoriatic or psoriasiform features, including epidermal thickening, massive epidermal proliferation as was observed by Ki-67 immunostaining, high expression of HLA-DR, ICAM-1 and massive infiltration of CD3 positive T cells around and within the epidermis (Table 4 and 5, Figures 21-24). In this group, 2/10 mice showed no psoriasiform features.
Conversely, 7/10 grafts treated with dexamethasone showed lack of psoriasiform features and 1 additional graft showed partial psoriasiform features with tendency toward healthy skin features, while 2 grafts in this group showed psoriasiform features (Table 4). Specifically, the 7/10 dexamethasone-treated grafts displayed normal skin including a low epidermal thickness, low proliferation index, low HLA-DR and ICAM-1 expression and low CD3 positive T cells infiltrate (Tables 4 and 5, Figures 21-24). Moderate lymphocytic infiltration was detected in only 1/10 (partial psoriasiform features) of grafts treated with dexamethasone (Table 4).

Treatment with Enbrel demonstrated lack of psoriasiform features in 3/9 (33%) grafts (Table 4). These three Enbrel-treated grafts showed features of normal skin including a low epidermal thickness (Table 5, Figure 21). The remaining 6/9 graft in this group displayed psoriasiform features. In addition, a trend of decrease in proliferation index (% Ki-67 positive cells) was observed in this group but there was no substantial effect on epidermal thickness (Figure 22). Overall, it should be noted that there is some similarity between the results of the present study and the clinical efficacy of Enbrel. In this study, healthy skin was observed in 33% grafts, which can be compared to the 22% of patients achieving PASI 90 (PASI score is a tool used to measure the severity and extent of psoriasis, PASI 90 indicates a 90% reduction in PASI scores) observed in clinical trials with Enbrel.

Mice treated with C10RF32 ECD-Fc (SEQ ID NO: 43) demonstrated lack of psoriasiform features in 5/9 grafts (55.5%), including features of normal skin, low epidermal thickness, low proliferation index, low HLA-DR and ICAM-1 expression and low CD3 positive T cells infiltrate (Table 4 and 5, Figures 21-24). 4/9 grafts displayed histological features of psoriasiform. There was a trend towards decrease in proliferation index, as observed using Ki-67 staining, but without effect on epidermal thickness (Figures 21 and 22). Additional IHC stains were not carried out for this group.

**Table 4 Number of affected grafts according to the histological evaluation**

| **Compound** | **Psoriasiform Skin** | **Partial Psoriasiform Features** | **Partial Psoriasiform With Tendency Towards Healthy Healthy skin** | **Healthy Skin** | **Total No. of Healthy Grafts (%)** |
|---|---|---|---|---|---|
| **C1ORF32 ECD-Fc (SEQ ID NO: 43)** | **4/9** | **--** | -- | **5/9** | **55.5%** |
| **Vehicle** | **8/10** | -- | -- | **2/10** | **20%** |
| **Dexa** | **2/10** | -- | **1/10** | **7/10** | **70%** |
| **Enbrel** | **6/9** | -- | -- | **3/9** | **33%** |

**Table 5 Epidermal thickness measurements (µm) of grafts**

| **Source of graft (Patient)** | C1ORF32 ECD-FC (SEQ ID NO:43) | **PBS** | **Dexa** | **Enbrel** |
|---|---|---|---|---|
| A | 904 | **823** | **359** | |
| **B** | **397** | **599** | **596** | **658** |
| **C** | **603** | **557** | **183** | **770** |
| **D** | **441** | **881** | **377** | **679** |
| **E** | **335** | **734** | **391** | **678** |
| **F** | | **745** | **204** | **683** |
| **G₁** | **598** | **336** | **266** | **830** |
| **H** | **253** | **780** | **262** | **496** |
| **P** | | **962** | **232** | **492** |
| **K** | **268** | **407** | **289** | **296** |
| **G₂** | **775** | | | |
| **Mean** | **508** | **682** | **315** | **620** |
| **STDEV** | **227** | **203** | **121** | **164** |
| **P-value** | **P<0.05** | | **P<0.005** | **N.S** |

In this study, C1ORF ECD-Fc (SEQ ID NO:43) was tested for its efficacy in the humanized psoriatic SCID mouse model upon prophylactic, intralesional administration of 100 µg per graft, given from the day of PBMCs injection, 3 times a week. Dexamethasone and Enbrel, drugs which are in clinical use for the treatment of psoriatic patients, were used as positive controls.

C10RF32 ECD-Fc (SEQ ID NO: 43) treatment reduced the occurrence of psoriasiform features in 55% of the grafts. This reduction was also manifested in the immunohistochemical analysis showing decreased expression of HLA-DR (highly expressed in keratinocytes from psoriatic patients), ICAM-1 (an intercellular adhesion molecule, highly expressed in the epidermis and upper dermis of psoriatic patients) and Ki-67 (a marker of cell proliferation which is highly expressed in psoriasis and correlates with the clinical severity of the disease), together with decreased infiltration of T cells (as demonstrated by lower level of CD3- expressing cells).

Furthermore, C10RF32 ECD-Fc (SEQ ID NO: 43) and dexamethasone significantly reduced epidermal thickness compared with the vehicle-treated group, while no significant reduction in epidermal thickness was observed with Enbrel. In terms of other clinical effects, there is some similarity between the results of the present study and the clinical efficacy of Enbrel. Forty nine percent of psoriasis patients achieved 75% improvement in the psoriasis area-and-severity index (PASI 75) at week 12, while 22% demonstrated PASI 90 (considered almost clear) response (Leonardi et al., 2003). In our study, healthy skin features were observed in 33% grafts, which is comparable to the 22% of PASI 90. PASI score is a tool used to measure the severity and extent of psoriasis. The IHC parameters analyzed in selected grafts are in good correlation with the histological scoring. This demonstrates that this model captures multiple features of psoriatic skin and treatment effects are observed on all of these parameters. The data obtained in this study suggest a potential beneficial effect of C1ORF32 ECD-Fc (SEQ ID NO: 43) in psoriasis.

### REFERENCES

Batista MD, Ho EL, Kuebler PJ, Milush JM, Lanier LL, Kallas EG, York VA, Chang D, Liao W, Unemori P, Leslie KS, Maurer T, Nixon DF Exp Dermatol. 2013;22:64-6.
Bowcock AM, Krueger JG. Getting under the skin: the immunogenetics of psoriasis. Nat Rev Immunol. 2005;5(9):699-711.
Creamer JD, Barker JN. Vascular proliferation and angiogenic factors in psoriasis. Clin Exp Dermatol. 1995;20(1):6-9
Nestle, F.O., D.H. Kaplan, and J. Barker.. Psoriasis. New England Journal of Medicine. 2009; 361: 496-509.
Gilhar A, Ullmann Y, Kerner H, et al. Psoriasis is mediated by a cutaneous defect triggered by activated immunocytes: induction of psoriasis by cells with natural killer receptors. J Invest Dermatol. 2002;119(2):384-91. Gilhar A, Bergman R, Assay B, Ullmann Y, Etzioni A The beneficial effect of blocking Kv1.3 in the psoriasiform SCID mouse model. J Invest Dermatol. 2011; 131, 118-24
Gilhar A, Yaniv R, Assy B, Serafimovich S, Ullmann Y, Kalish RS (2006) Fas pulls the trigger on psoriasis. Am J Pathol. 2006; 168:170-5.
Griffiths CE. The immunological basis of psoriasis. J Eur Acad Dermatol Venereol. 2003 ;17 Suppl 2:1-5. Review.
Kalish RS, Simon M, Harrington R, Gottlieb AB, Gilhar A. Skin equivalent and natural killer cells: a new model for psoriasis and GVHD. J Invest Dermatol. 2009 ;129:773-6.
Leonardi CL, Powers JL, Matheson RT, Goffe BS, Zitnik R, Wang A, Gottlieb AB. Etanercept as monotherapy in patients with psoriasis. N Engl J Med. 2003;349:2014-22.
Martin DA, Towne JE, Kricorian G, Klekotka P, Gudjonsson JE, Krueger JG, Russell CB. The emerging role of IL-17 in the pathogenesis of psoriasis: preclinical and clinical findings. J Invest Dermatol. 2013;133:17-26.
Mudigonda, P., T. Mudigonda, A. N. Feneran, H. S. Alamdari, L. Sandoval, and S. R. Feldman.. Interleukin-23 and interleukin-17: importance in pathogenesis and therapy of psoriasis. Dermatol. 2012; Online J. 18: 1.
Nestle FO, Kaplan DH, Barker J. Psoriasis. N Engl J Med. 2009;361:496-509. Nickoloff BJ, Kunkel SL, Burdick M, et al. Severe combined immunodeficiency mouse and human psoriatic skin chimeras. Validation of a new animal model. Am J Pathol. 1995;146(3):580-8.
Nograles KE, Krueger JG. Anti-cytokine therapies for psoriasis. Exp Cell Res. 2011;317:1293-300.
Pan, H.F., X.P. Li, S.G. Zheng, et al.. Emerging role of interleukin-22 in autoimmune diseases. Cytokine & Growth Factor Reviews. 2012; 24: 51-57.
Wrone-Smith T, Nickoloff BJ. Dermal injection of immunocytes induces psoriasis. J Clin Invest. 1996;98(8):1878-87.

### Summary

C10RF32 ECD-Fc (SEQ ID NO: 43) was tested in the humanized skin SCID mouse model of psoriasis along with dexamethasone and Enbrel as positive controls, both of which are treatment options for severe plaque-type psoriasis. Skin grafts from mice treated with vehicle alone displayed histological features of psoriasiform in 8/10 mice. Notably, C10RF32 ECD-Fc (SEQ ID NO: 43) treatment reduced the occurrence of psoriasiform to only 4/9 mice. Skin grafts from mice treated with dexamethasone reduced the occurrence of psoriasiform to only 2/10 grafts, while treatment with Enbrel resulted in 6/9 grafts displaying psoriasiform features. Furthermore, a significant reduction in epidermal thickness was observed in grafts treated with C10RF32 ECD-Fc (SEQ ID NO: 43) and dexamethasone (P < 0.05, P<0.005, respectively) compared with the vehicle-treated group. None of the other treatment groups including Enbrel significantly affected epidermal thickness. These data suggest a potential beneficial effect of C1ORF32 ECD-Fc (SEQ ID NO: 43) in the treatment of psoriasis. However, the relatively low number of mice included in each group does not permit a definitive conclusion and additional studies are needed to substantiate these results.

### EXAMPLE 12 - ISLET TRANSPLANTATION

In order to demonstrate general efficacy for preventing transplantation rejection with a subject having at least one antigen mismatch, this experiment determines the effects of C1ORF ECD-Fc on the ability to modulate islet transplant rejection across a fully allogeneic MHC barrier. 500 BALB/c pancreatic islets are transplanted under the kidney capsule of recipient C57BL/6 mice rendered diabetic 7 days previously by treatment with streptozotocin. Recipient mice are treated three times per week with control Ig or C1ORF ECD-Fc and monitored for blood glucose levels as a measure of graft acceptance/rejection. Tolerance with ECDI-fixed donor splenocytes is used as the positive control for successful modulation islet graft rejection.

### EXAMPLE 13 - CIA Treatment with C1ORF32-P8-V1-ECD-MFC

This Example shows the effect of early stage treatment with C1ORF32-P8-V1-ECD-MFC (SEQ ID NO:42) on disease development in collagen induced arthritis (CIA) model of rheumatoid arthritis.

The aim of this study was to estimate the effect of C1ORF32-P8-V1-ECD- mFc (SEQ ID NO:42) on disease development in a type II Collagen induced arthritis (CIA) mouse model upon administration at early stage of disease; i.e., when specific and predictive markers for RA development are present in the serum, but before onset of clinical symptoms.

In the CIA model, arthritis is induced by immunization with type II collagen in CFA on day 1 which is followed by boosting with type II collagen in CFA on day 21. Antibodies against both CII and cyclic citrullinated peptide (CCPs) appear early after immunization, before joint swelling is observed. Kuhn et la., (J Clin Invest. 2006 Apr 3; 116(4): 961-973) demonstrated that autoantibodies against CII and CCP begin to develop around day 7 after the first immunization and steadily increase while clinical evidence of arthritis are evident 25 days after the initial immunization with CII in CFA.

To evaluate the effect of early stage treatment with C1ORF32-P8-V1-ECD- mFc (SEQ ID NO:42) on disease development treatment started on day 18 and was given IP injection, Q2D to mice from day 18 until day 32.

### Method

Male DBA/1 OlaHsd mice weighing 16-25 grams (mean 22 g) on day 18 of the study were obtained from Harlan, Inc., Indianapolis, Indiana. Mice (10/group) were anaesthetized with Isoflurane, shaved at the base of the tail, and injected intradermally with 150 µl of Freund's Complete Adjuvant (Sigma) containing bovine type II collagen (Elastin Products, Owensville, MO) (2 mg/ml) at the base of the tail on day 0 and again on day 21. On study day 18, mice were randomized by body weight into treatment groups. Treatment was initiated after enrollment and continued every other day (Q2D at 48 h intervals) through study day 33. On study days 23-34, onset of arthritis occurred. Mice were terminated on study day 34. Clinical scores were given for each of the paws (right front, left front, right rear, left rear) on arthritis days 18-34.
Experimental groups were as follows:

| **Compound** | **N** | **Route** | **Regimen** | **Dose Level (mg/kg)** | **Dose (mg/ml)** | **Dose (ml/kg)** |
|---|---|---|---|---|---|---|
| Vehicle - Normal PBS | 4 | IP | | 0 | 0 | 0 |
| Vehicle Disease Control PBS | 10 | IP | Q2D | 0 | 0 | 10 |
| IgG2a (BioXcell) | 10 | IP | Q2D | 10 | 1.25 | 8 |
| C1ORF32-P8-V1-ECD-mFc (SEQ ID NO:42) | 10 | IP | Q2D | 3.3 | 0.4125 | 8 |
| C1ORF32-P8-V1-ECD-mFc (SEQ ID NO:42) | 10 | IP | Q2D | 10 | 1.25 | 8 |

Mice were weighed on study days 18, 20, 22, 24, 26, 28, 30, 32 and 34 (prior to necropsy). Daily clinical scores were given for each of the paws (right front, left front, right rear, left rear) on arthritis days 18-34 using the following criteria:
- 0 = normal
- 1 = 1 hind or fore paw joint affected or minimal diffuse erythema and swelling
- 2 = 2 hind or fore paw joints affected or mild diffuse erythema and swelling
- 3 = 3 hind or fore paw joints affected or moderate diffuse erythema and swelling
- 4 = 4 hind or fore paw joints affected or marked diffuse erythema and swelling
- 5 = Entire paw affected, severe diffuse erythema and severe swelling, unable to flex digits

### Results

The method for induction of CIA used in this example, which involves a boost of type II collagen in CFA on day 21 after immunization, results in a severe disease which is difficult to inhibit. In this setup, mice develop visual clinical symptoms of arthritis around day 25 post immunization. Early stage treatment (i.e., from day 18, before onset of clinical symptoms) with C1ORF32-P8-V1-ECD- mFc (SEQ ID NO:42) at 10 mg/kg Q2D until day 34 resulted in inhibition of clinical arthritis score compared to groups treated with negative controls (PBS or mIgG2a isotype control) however, this effect did not reach significance. C1 ORF32-P8-V1-ECD- mFc (SEQ ID NO:42) treatment at 10 mg/kg also had beneficial effect on body weight loss (measured as percent change from baseline) which was significantly inhibited toward normal beginning on study day 30 and continuing until study termination (data not shown). These results point to improvement in overall wellbeing of mice treated with C1ORF32-P8-V1-ECD- mFc (SEQ ID NO:42). No effect was observed upon treatment with a lower dose of 3.3mg/kg of C1ORF32-P8-V1-ECD- mFc (SEQ ID NO:42).

The beneficial effect of C1ORF32-P8-V1-ECD- mFc (SEQ ID NO:42) in this model upon treatment from day 18 when autoimmune activity is already established but clinical symptoms are not yet manifested suggest beneficial effect of a human version of this molecule for early stage treatment of autoimmune diseases, particularly RA.

Figure 25 shows the effect of semi established treatment with C1ORF32-ECD-mFc (SEQ ID NO:42), PBS or control Ig in the collagen induced arthritis (CIA) model of Rheumatoid Arthritis. Treatments were given i.p., 3 times per week from day 18 until day 34. Shown is the effect on clinical score. PBS or control Ig were used as negative controls.

### EXAMPLE 15 The Effect of C1ORF32 ECD-Fc (SEQ ID NO:43) on Human CD4+ T Cell Responses from Healthy Donors and RA Patients

### Effect of C1ORF32 ECD-Fc (SEQ ID NO:43) on T cell activation upon increasing ConA & PHA stimulation

CD4+ T cell are isolated by negative selection from PBMCs using Miltenyi CD4+ T cell Isolation Kit 130-096-533 as per manufactures instructions as follows:
1. Cells resuspended in 40 µl of MACs buffer per 10⁷ total cells.
2. 10 µl of CD4+ T cell Biotin-Antibody Cocktail added per 10⁷ total cells.
3. Mix well and incubate for 5 minutes at 40 C.
4. Add 30 µl of MACs buffer per 10⁷ cells.
5. Add 20 µl CD4+ T cell Microbead Cocktail per 10⁷ cells.
6. Mix well and incubate for 10 minutes at 40 C.
7. Proceed with CD4+ cell separation (program "Depletes") on AutoMACs Pro Machine according to operation manual. Collect negative (CD4+) fraction and add MACs Buffer to total volume of 5 ml.
8. Take 10 µl of CD4+ cells into FACS tube containing 90 µl of MACS buffer and put on side for flow cytometry to assess purity.
9. Count positive CD4+ cells.

CD4+ T cells (1.5 x 10⁵ cells/well and 5 x 10⁴) of healthy volunteers and from RA patients are activated with different concentrations of ConA (0.1, 1, 2, 5 & 10 µg/ml) or PHA (0.1, 1, 5, 10 & 20 µg/ml) for selection of sub-optimal activation conditions.

Following selection of suboptimal activation conditions, C1ORF32 ECD-Fc (SEQ ID NO:43) or control Ig are added each to the above described culture at 1, 3, 10, 30 µg/ml, 1 hour prior to cell stimulation with ConA or PHA. IL-2 (10 ng/ml) is added to cultures after 24 hours. Following 72 hour incubation, cell culture supernatants removed and stored at -20°C. Supernatants are analyzed for TNFα, IL-6, IL-10, IL-12, IL-23, IL-17 and IL-35 using ELISA as per manufacturer's instructions.

### Effect of Immunoinhibitory C1ORF32 ECD-Fc (SEQ ID NO:43) in Macrophage and T cell co-cultures or DCs and T cells co-cultures

The following experiments will be carried out with blood taken from healthy volunteers as well as from Rheumatoid Arthritis (RA) patients.

### PBMC Isolation

1. Dilute blood in dPBS 1:1 (RT).
2. Gently layer on 10 ml of the diluted blood on top of 4 ml Histopaque®. Spin at 2100 rpm for 20 minutes, RT (Medium, Histopaque and centrifuge must be equilibrated to RT).
3. Carefully transfer the opaque interface containing mononuclear cells, with a transfer pipette, into a clean tube. Add an equal amount of wash medium (dPBS) and spin at 1800 rpm for 10 minutes, RT.
4. Discard supernatant and resuspend cells in 25 ml of cold MACS buffer. Pass cells through a 70 µm mesh strainer to remove cell clumps/debris and transfer into a new tube. Count cells.
5. Spin down at 1800 rpm for 10 minutes, 4° C.

### CD14+ Positive Selection for co-culture experiments

1. Following PBMC isolation, discard the supernatant and proceed with CD14+ cell isolation.
2. Resuspend PBMC in MACS buffer (80 µl per 1x10⁷cells).
3. Proceed with CD14+ cells separation (program "Posseld") on AutoMACs Pro Machine according to operation manual using automatic labelling function with CD14⁺ Microbeads. Collect positive (CD14⁺) fraction and add 4.5 ml MACs Buffer for total 5 ml volume.
4. Take 10 µl of CD14⁺ cells into FACS tube containing 90 µl of MACS buffer and put on side for Flow cytometry to assess purity.
5. Count positive CD14⁺ cells.
6. Cells plated in 96 well plates at 5 x 10⁴ cells/well.

### Macrophage differentiation

For macrophage differentiation, cells differentiated with 50 ng/ml M-CSF for 6 days at 37°C in 5% humidified CO₂. Cells fed at day 3 with complete media plus 50 ng/ml M-CSF.

### DC differentiation

For DC differentiation, cells differentiated with 100 ng/ml GM-CSF, 20 ng/ml IL-4 and 100 ng/ml FLT3-ligand for 6 days at 37°C in 5% humidified CO2. Cells fed at day 3 with complete media plus 100 ng/ml GM-CSF, 20 ng/ml IL-4 and 100 ng/ml FLT3-ligand.

### CD4⁺ Negative selection, from Negative fraction of CD14⁺ Isolation, for co-culture experiments

1. Resuspend the cells from CD14⁺ negative fraction in 40 ul of MACs buffer per 10⁷ total cells.
2. 10 µl of CD4+ T cell Biotin-Antibody Cocktail added per 10⁷ total cells.
3. Mix well and incubate for 5 minutes at 4°C.
4. Add 30 µl of MACs buffer per 10⁷ cells.
5. Add 20 µl CD4⁺ T cell Microbead Cocktail per 10⁷ cells.
6. Mix well and incubate for 10 minutes at 4°C.
7. Proceed with CD4⁺ cell separation (program "**Depletes**") on AutoMACs Pro Machine according to operation manual. Collect negative (CD4⁺) fraction and add MACs Buffer to total volume of 5 ml.
8. Take 10 µl of CD4+ cells into FACS tube containing 90 µl of MACS buffer and put on side for flow cytometry to assess purity.
9. Count positive CD4+ cells.

### Flow Cytometry - evaluation of CD4⁺& CD14⁺ purity.

The following stains will be performed for each donor:
1. PBMC Unstained
2. CD14⁺PE/CD4⁺APC
3. PE/APC isotype control

### Protocol:

1. Add 500 µl FACS Buffer and spin cells at 400 g for 5 min at 4°C.
2. Add 100 µL to tube 1 (unstained sample).
3. Add 90 µl of FACs buffer and 10 µl of anti CD14-PE/anti CD4- APC antibody to tube 2.
4. Add 90 µl of FACs buffer and 10 µl of mIgG2a-PE/mIgG1-APC to tube 3.
5. Incubate all tubes at room temperature in dark for 15 minutes.
6. Wash cells twice with 200 µl of FACS buffer and spinning cells at 400 g for 1 min at 4°C followed by 500 µl FACS Buffer and spin cells at 400 g for 5 min at 4°C.
7. Resuspend cells in 150 µl of FACS buffer and 150 µl of 4% formaldehyde fixative to prepare for FACS acquisition.
8. FACS acquisition data analysed using FlowJo Software.

### T cell activation for TCK generation

- Purified CD4+ cells (2x10⁶ cells/ml) are activated for 6 days in complete medium containing IL-15 (100 ng/ml), IL-6 (100 ng/ml) and TNFa (25 ng/ml).

### Effect of Immunoinhibitory C1ORF32 ECD-Fc (SEQ ID NO:43) in Macrophage - T cell co-cultures

After 6 days in culture, T cells are washed 3 times in complete medium and counted TcKs are added to macrophage culture following removal of existing culture media at a ratio of 4:1 (5x10⁴ cells/well/CD4⁺ T cells plus 2 x 10⁵ cells/well CD14⁺ Macrophages). C1ORF32 ECD-Fc (SEQ ID NO:43) or Control IgG are added at 1, 3, 10, 30 µg/ml. For additional controls co-cultures are added with vehicle or with medium only (Wennink et al., 2012).

After 24hrs of culture, cytokines (IL-10, IFN-a, IL-6, IL-12, CCL5, IL-13, IL-15, IL-17, CCL3, GM-CSF, IFNγ, TNFα, IL-1RA, IL-7, IL-1B, eotaxin, IL-2 IL-4 and IL-2R) are analyzed using ELISA and/or Luminex.
The effect of C1ORF32 ECD-Fc (SEQ ID NO:43) on T cells and Macrophages activation during co-culture is also investigated. T cells are gated using CD3/CD4 staining and investigated at 15 minutes and 24h after compounds addition to the co-cultures. At 15 minutes time point, STAT3 and STAT5 phosphorylation will be analyzed. At 24h time point proliferation (CFSE assay), activation marker expression CD69 and chemokine receptors CCR6, CXCR3 (Th17/Th1), CCR4 (TH2) and CCR5 (TH1) are analyzed by FACS. Macrophages are investigated at 24 time point by co-staining for the lineage marker CD64 and for activation markers MHCII, CD86 and CD80.

### Effect of C1ORF32 ECD-Fc (SEQ ID NO:43) in DC and T cell co-cultures

Differentiated DCs (5 x 10⁴ cells/well) are co-cultured with CD4⁺ T cells (2 x 10⁵ cells/well). C1ORF32 ECD-Fc (SEQ ID NO:43) **or** Control IgG are added at 1, 3, 10, 30 µg/ml. For additional controls co-cultures are added with vehicle or with medium only.

After 24hrs of culture, cytokines (IL-10, IFN-a, IL-6, IL-12, CCL5, IL-13, IL-15, IL-17, CCL3, GM-CSF, IFNγ, TNFα, IL-1RA, IL-7, IL-1B, eotaxin, IL-2, IL-4, IL-2R and IL-35) are analyzed using ELISA and/or Luminex.

The effect of C1ORF32 ECD-Fc (SEQ ID NO:43) on T cells and DC activation during co-culture is also investigated. T cells are gated using CD3/CD4 staining and investigated at 15 minutes and 24h after compounds addition to the co-cultures. At 15 minutes time point, STAT3 and STAT5 phosphorylation will be analyzed. At 24h time point proliferation (CFSE assay), activation marker expression CD69 and chemokine receptors CCR6, CXCR3 (Th17/Th1), CCR4 (TH2) and CCR5 (TH1) are analyzed by FACS. The effect of C1ORF32 ECD-Fc (SEQ ID NO:43) on DCs activation is investigated at 24 time point.

### Effect of C1ORF32 ECD-Fc (SEQ ID NO:43) on Dendritic Cells following TLR activation

Differentiated DCs (5 x 10⁴ cells/well) are activated with TLR agonists: LPS (1 ng/ml), PAM3 (100 ng/ml), CLO97 (1 µg/ml), PolyIC (50 µg/ml), or with 1-2 % RA Synovial Fluid.

C10RF32 ECD-Fc (SEQ ID NO:43) **or** Control IgG are added prior to cell stimulation at 0.1, 1, 3, 10, 30 µg/ml either during the differentiation phase as well as at day 6 upon dendritic cell stimulation.
Cytokine secretion and cell activation are analyzed as described above.

### Results

Purified human peripheral T cells that are cultured ex-vivo in the presence of IL-15, IL-6 and TNFα (TcK) recapitulate the functional properties of synovial T cells of rheumatoid arthritis patients (Wenink et al., 2014). Furthermore, monocytes activated in the presence of GM-CSF exhibit the same characteristics as these of macrophages infiltrating the synovioum during RA.

Co-culture of TcK with autologeuos ex-vivo differentiated macrophages mimics one of the crucial pathways mediating synovial macrophage activation in RA thus serving as a translational tool for evaluating the therapeutic potential of drug candidates. Therefore the effect of C1ORF32 ECD-Fc (SEQ ID NO:43) was tested on the secretion of a range of pro-inflammatory and anti-inflammatory cytokines in such co-cultures. Two studies were carried out, one in which buffy coats from healthy volunteers was used and another study in which the responses of RA patients blood cells responses were compared to that of healthy voluntiers in which blood was used rather than buffy coats.

In co-cultures of TcK and macrophages from healthy volunteers, C10RF32 ECD-Fc (SEQ ID NO:43) inhibited the secresion of TNFa, CCL5, CCL3, GM-CSF, and IL-5 and to a lesser extent also downregulated the secretion of IFNg, IL-1RA, IL-17, IL-12, IL-13, IL-6 and IL-2R (Figure 26). No effect was observed on IL-10, IL-15, IFNa, IL-7 secretion levels in these co-cultures.

Similarly, a marked inhibition of TNFa secretion was observed in co-cultures of TcK and macrophages from RA patients' (n=2) and healthy volunteers blood (n=1) samples (Figure 27).

### Example 15: Immunomodulatory activity of C1ORF32 ECD-Fc on peripheral blood cells from patients with active multiple sclerosis

To study the potential efficacy of C1ORF32 ECD-Fc for treatment of multiple sclerosis, the effect on blood cells taken from MS patients that have had a relapsing remitting disease for years to decades was tested.

### Method:

PBMCs were taken from eight MS patients with diagnosed relapsing-remitting disease who have experienced a relapse 1-2 days prior to blood drawing.

PBMCs were plated in a flat-bottom 96-well plate at 1e6 cells/well. The cells were cultured in the presence of anti-CD3 (0.5ug/ml), MS-specific peptide MBP₈₅₋₉₆ (10ug/ml), or tetanus toxoid peptide TT₈₃₀₋₈₄₃ (10ug/ml). C10RF32 ECD-Fc (SEQ ID NO: 43) or Control Ig were added to the cuture at the indicated concentrations. Two replicate plates were set to study for the effect of C1ORF32 ECD-Fc (SEQ ID NO: 43) on proliferation and on pro-inflammatory and anti-inflammatory cytokines secretion. To test for proliferation, the cultures were pulsed on day +1 of culture with 1uCi of tritiated thymidine and then harvested 3 days later on day +4 of culture. For cytokine analysis, super natantes from a replicate cultures were collected on day +4 of culture and tested using Millipore multiplex assay.

### Results:

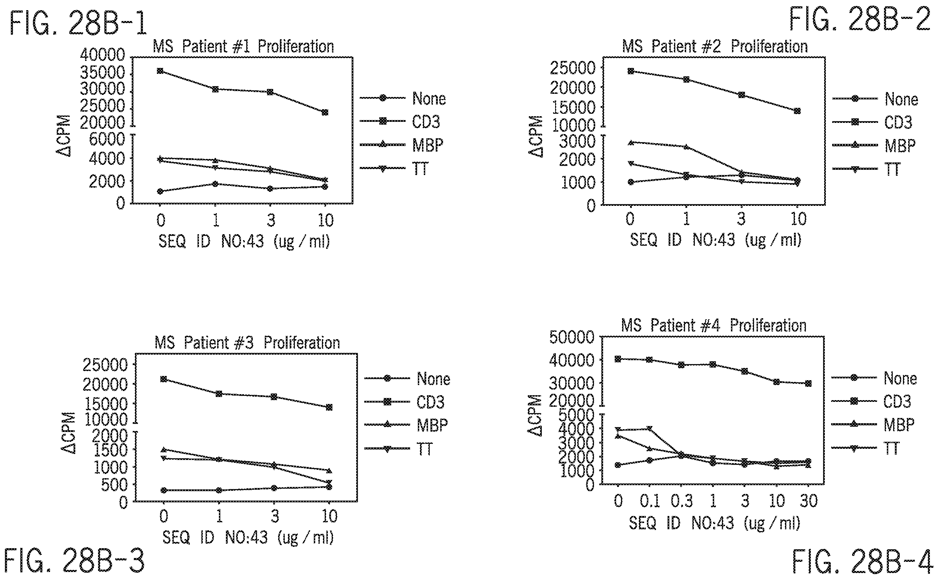

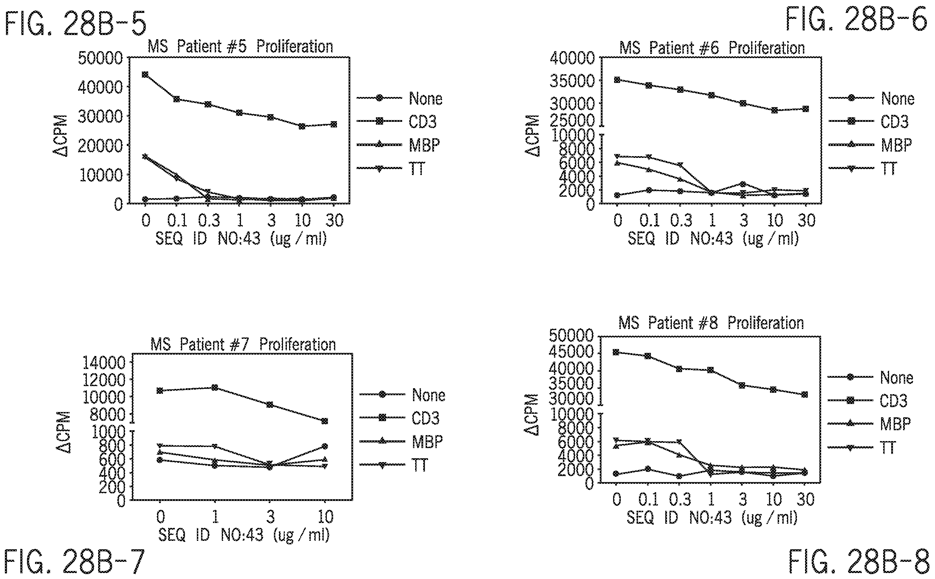

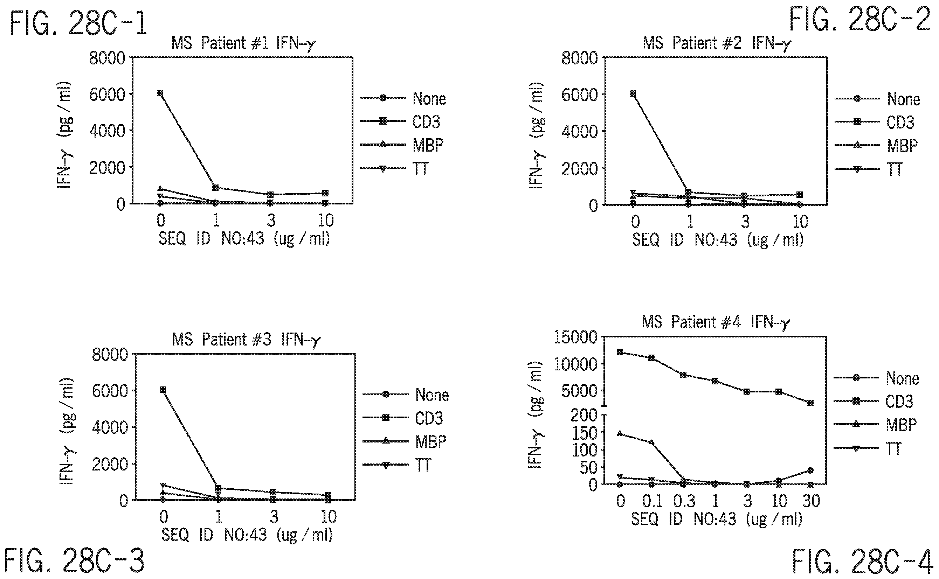

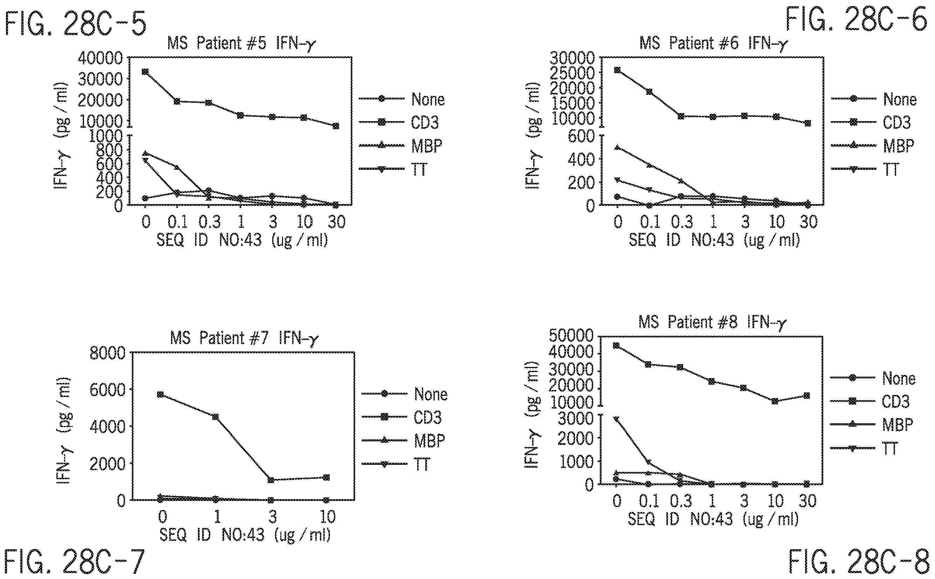

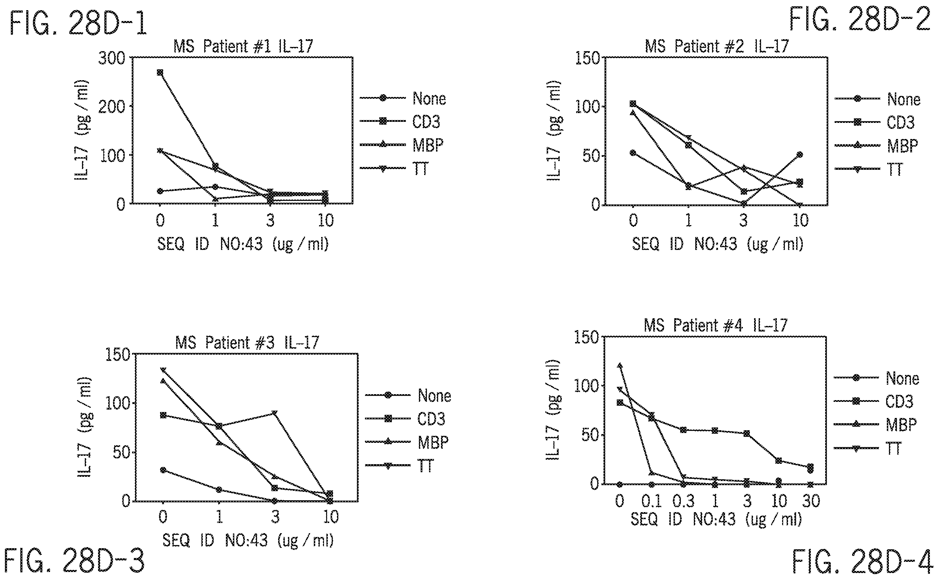

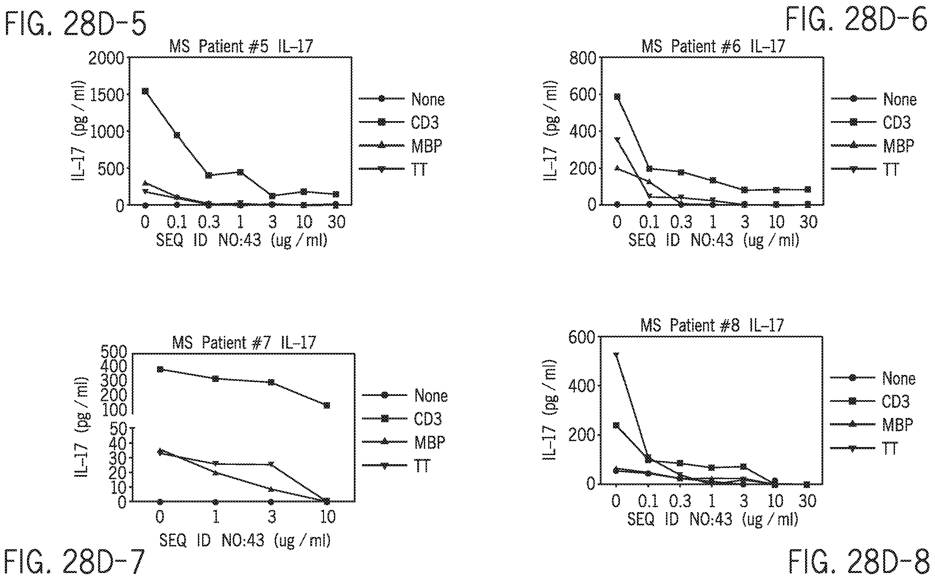

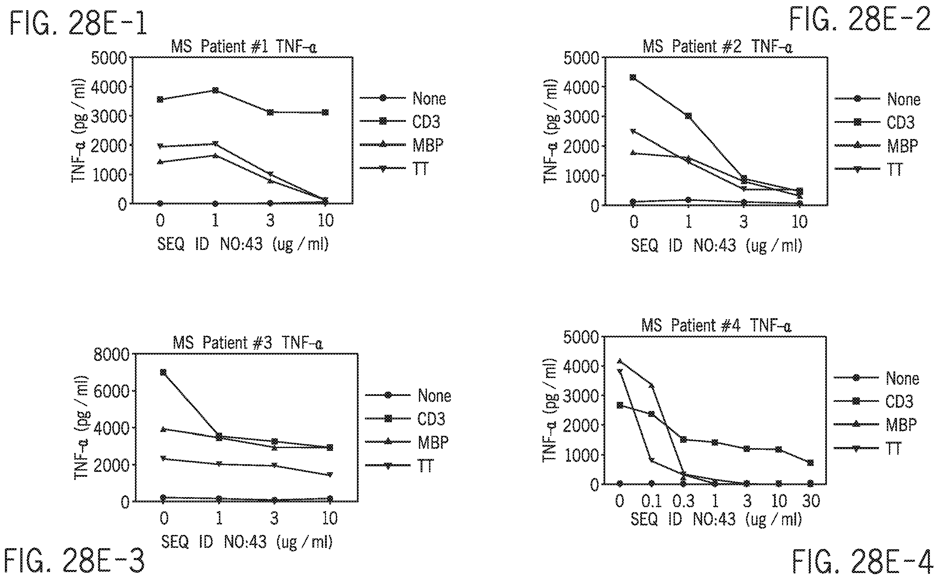

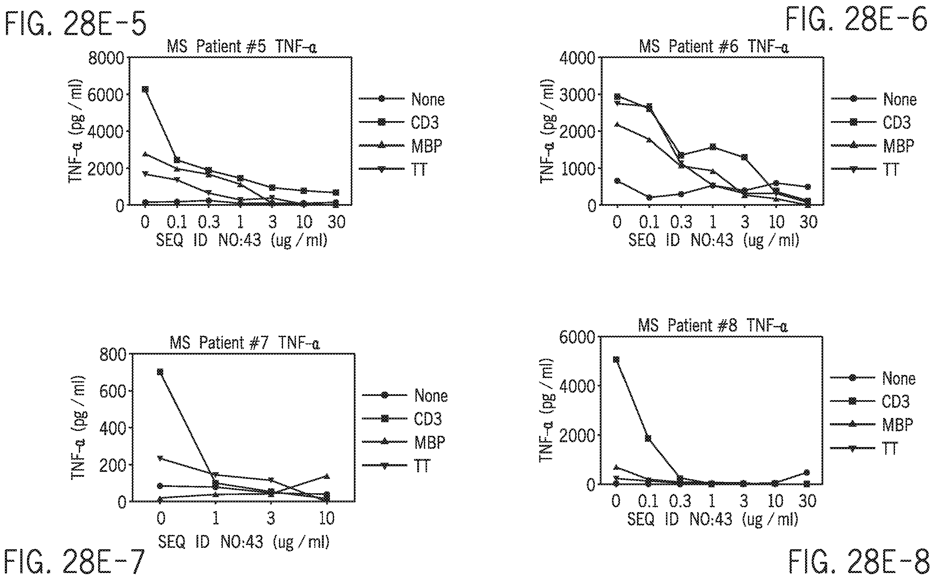

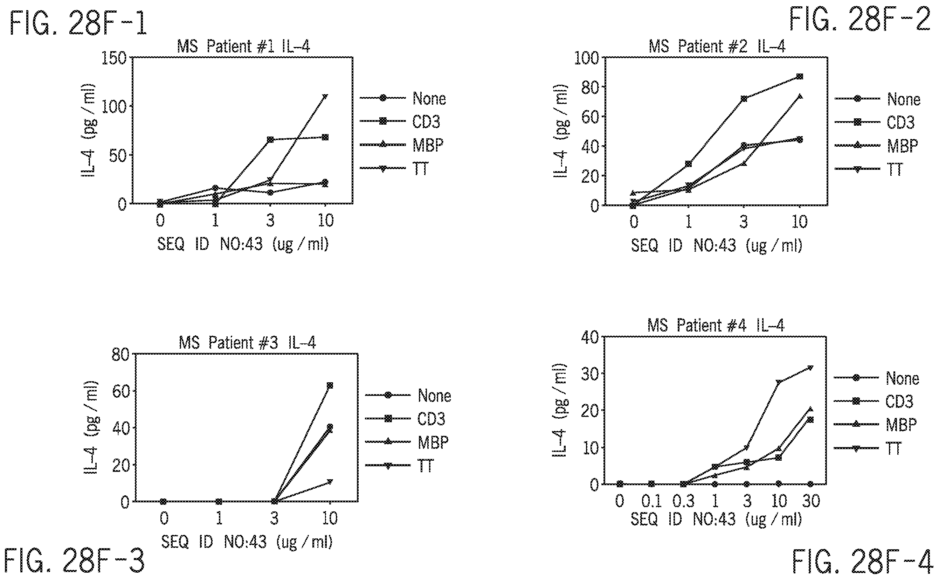

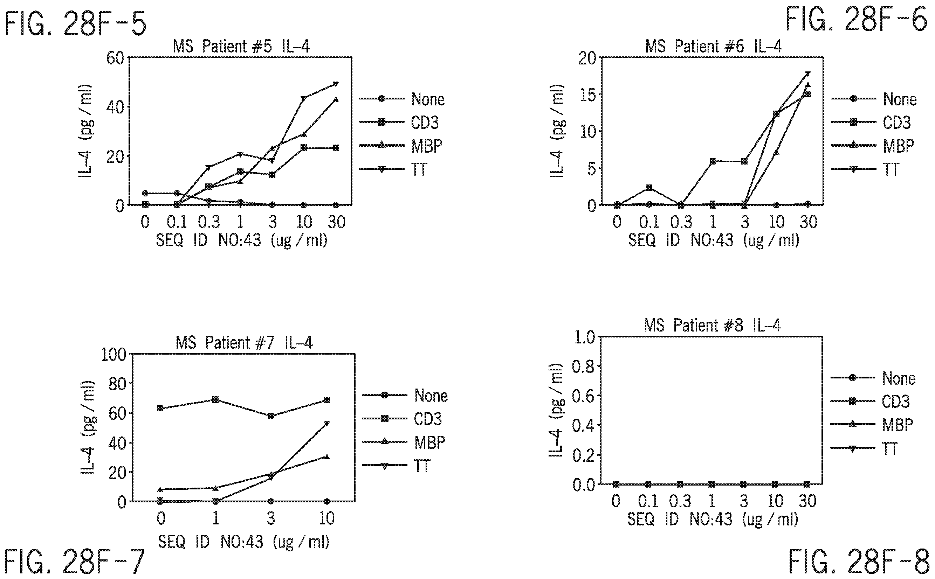

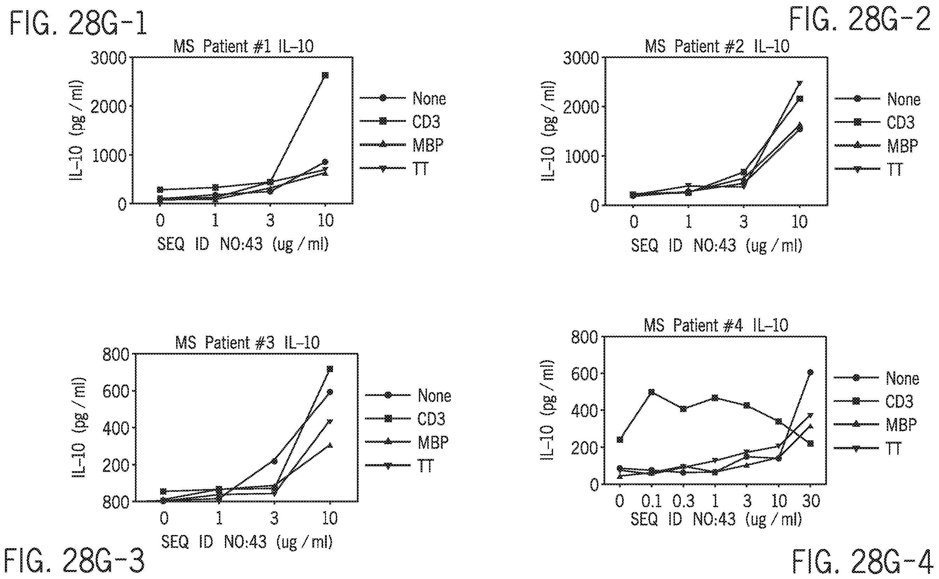

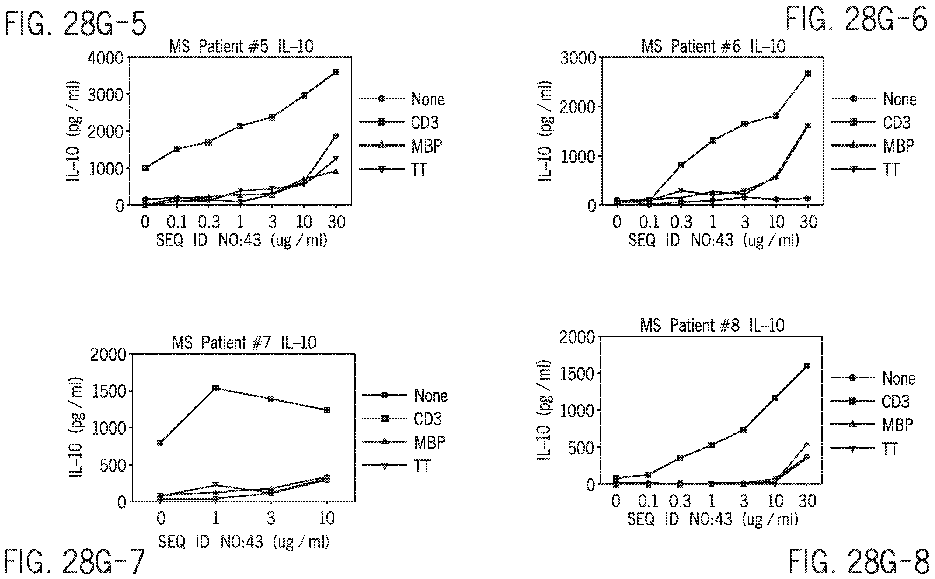

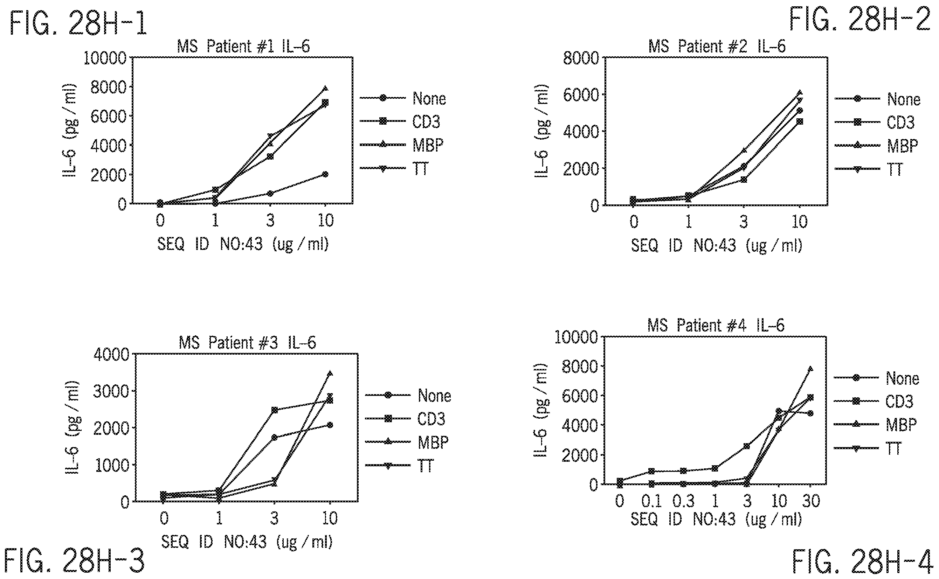

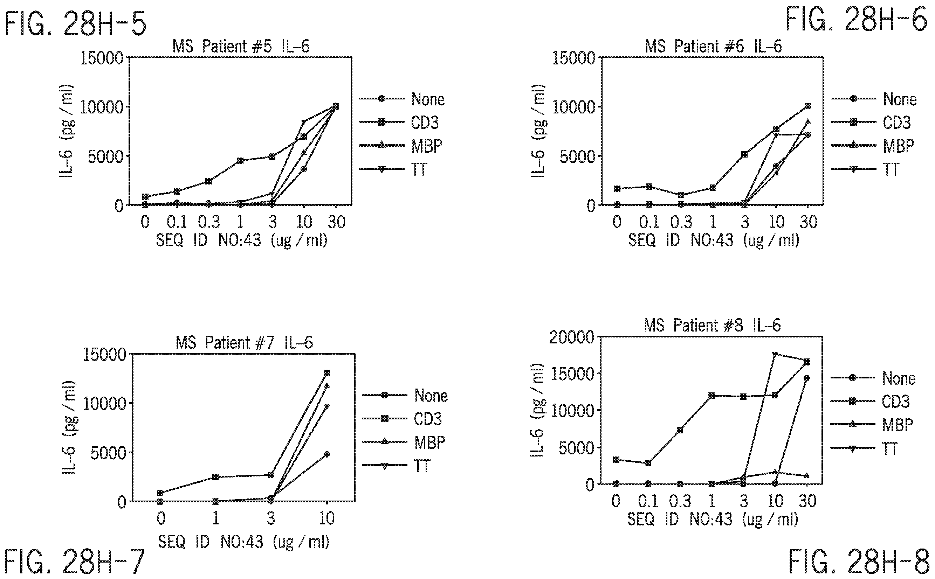

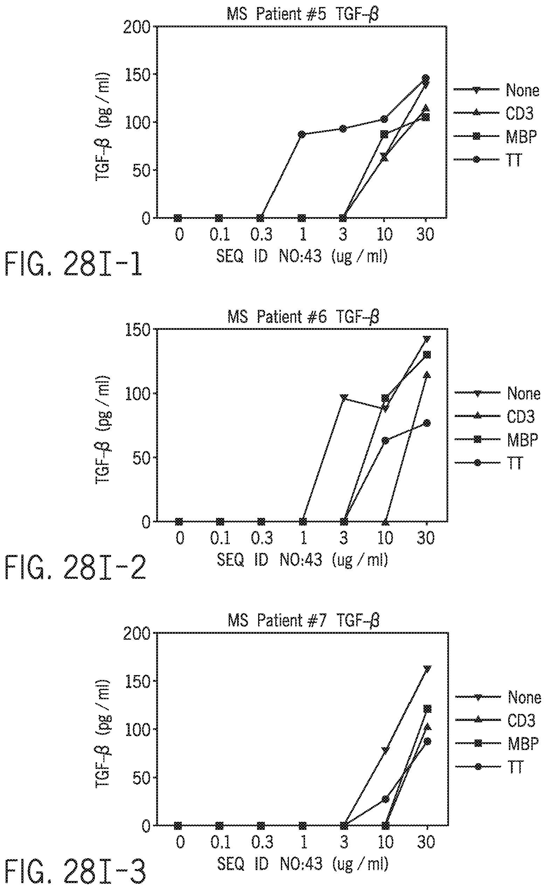

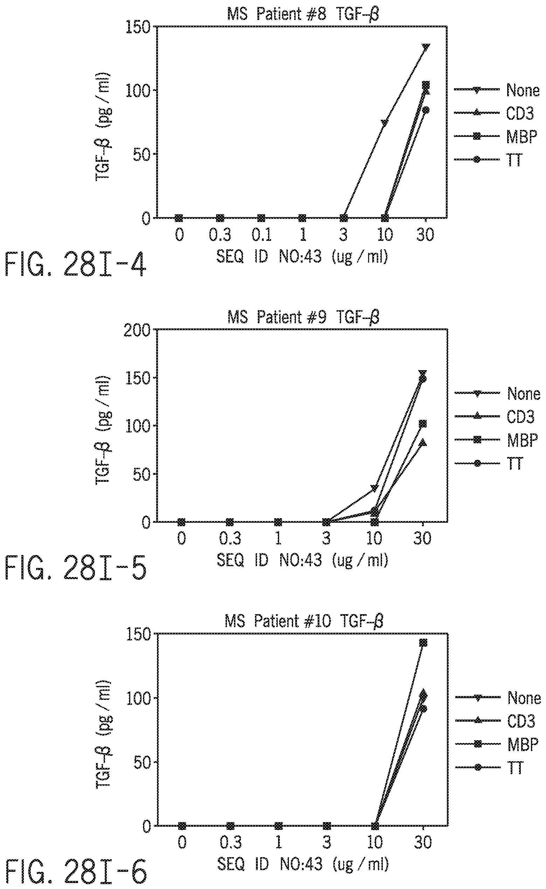

C10RF32 ECD-Fc (SEQ ID NO: 43) treatment of MS patients' PBMCs that were activated ex-vivo with MBP₈₅₋₉₆ resulted in inhibition of cells proliferation, as well as inhibition of IFNg, IL-17, TNFa and increase in IL-4, IL-10, TGFβ and IL-6 (Figure 28). Similar effects were observed upon activation of the MS patients' PBMCs with anti CD3 or TT₈₃₀₋₈₄₃. In addition, in the absence of activation, C10RF32 ECD-Fc (SEQ ID NO: 43) treatment resulted in elevation IL-10, IL-4, TGFβ and IL-6 in some of the donors while no effect was observed on proliferation, or on secresion of IFNg, IL-17, and TNFa. Increase in TGF-beta levels either alone or together with other cytokines in response to treatment with C10RF32 ECD-Fc might serve as a biomarker for patients that could benefit from treatment with C10RF32 ECD-Fc, and particularly point to higher potential to induce tolerance in those patients.

For example, optionally a C10RF32 polypeptide, such as C10RF32 ECD-Fc, is administered if TGF-beta is present at a sufficiently high level. Optionally, additionally or alternatively, the C10RF32 polypeptide is administered if one or more cytokines are present at a sufficiently high level. Optionally such treatment is initiated even without the presence of any overt symptoms of the disease.

### Summary:

These results are in agreement with previous observasions supporting immunomodulatory effects of C1ORF32 ECD-Fc manifested in downregulation of proinflammatory cytokines and upregulation of anti inflammatory cytokines. Such immunomodulatory effects have been previously described in blood cells from mice or from healthy human volunteers that were activated in vitro under Th driving conditions, as well as in lymph node cells and splenocytes from the EAE mouse model of multiple sclerosis, following treatment with C10RF32 ECD-Fc.

The increase of IL-6, may be part of immunomodulatory effect as this cytokine is known to be involved both in pro- and anti- inflammatory activities. For example, Treg17 cells can be induced in the presense of TGF-beta plus IL-6. Like conventional Treg, induction of regulatory Treg17 cells could play an important role in modulating and preventing certain autoimmune diseases.

## Claims

1. A C1ORF32 polypeptide for use in a method of treating an individual at risk of developing an autoimmune disease, comprising detecting a predisease process by a biomarker before onset of overt disease symptoms, wherein said autoimmune disease comprises rheumatoid arthritis, and wherein said biomarker includes one or more of a positive test for serum rheumatoid factor (RF) and antibodies to citrullinated protein antigens (ACPA); or alternatively being RF negative, but with high levels of serum ACPA (defined as being equal to 3 x upper limit of normal [ULN] for the assay); or a presence of joint pain caused by inflammatory tissue processes; and administering the C1ORF32 polypeptide to the individual.

2. The C1ORF32 polypeptide for use in a method of claim 1, wherein said biomarker is analyzed to determine a risk of early-onset disease, severe disease course or both.

3. The C1ORF32 polypeptide for use in a method of claim 2, wherein said biomarker includes one or more of a genomic, proteomic, metabolomic, lipidomic, glycomic, secretomic or serologic biomarker; or a family history, metagenome or microbiome analysis; or a combination thereof.

4. The C1ORF32 polypeptide for use in a method of claim 3, wherein said serologic biomarker comprises one or more of autoantibodies, antibodies to disease specific antigens, high blood glucose levels, high levels of self-reactive T cells, defects in regulatory T cells (Tregs), and/or chronic immune system involved inflammation.

5. The C1ORF32 polypeptide for use in a method of claim 1, wherein treatment prevents or at least slows development of rheumatoid arthritis symptoms, including to one or more of inflammation, fatigue, joint pain, joint tenderness, joint swelling, joint redness, joint warmth, joint stiffness, loss of joint range of motion, affecting more than one joint (polyarthritis), limping or joint deformity, or a combination thereof.

6. The C1ORF32 polypeptide for use in a method of claim 1, wherein the C1ORF32 polypeptide comprises the extracellular domain of C10RF32, or fragment or variant thereof, or a polypeptide comprising the extracellular domain of any one of SEQ ID NOs: 1-5, or a fragment or variant or homolog thereof.

7. The C1ORF32 polypeptide for use in a method of claim 6, wherein the C1ORF32 polypeptide is selected from the group consisting of polypeptide comprising a sequence of amino acid residues having at least 95% sequence identity with amino acid sequence depicted in any one of SEQ ID NOs:6-41.

8. The C1ORF32 polypeptide for use in a method of claim 7, where the C1ORF32 polypeptide is fused to a heterologous sequence to form a fusion protein, directly or indirectly via a linker peptide, a polypeptide sequence or a chemical linker.

9. The C1ORF32 polypeptide for use in a method of claim 8, wherein the heterologous sequence comprises at least a portion of an immunoglobulin constant domain.

10. The C1ORF32 polypeptide for use in a method of claim 9 wherein the fusion protein comprises an immunoglobulin heavy chain constant region corresponding to an antibody isotype selected from the group consisting of an IgG1, IgG2, IgG3, IgG4, IgM, IgE, IgA and IgD.

11. The C1ORF32 polypeptide for use in a method of claim 10, wherein the immunoglobulin constant domain comprises the hinge, CH2 and CH3 regions of a human IgG immunoglobulin, selected from the group consisting of Cy1, Cy2, Cy3 and Cy4 chain; or hybridization of hinge, CH1, CH2 and CH3 regions from different Fc isotypes.

12. The C1ORF32 polypeptide for use in a method of claim 11, wherein said different Fc isotypes comprise IgD and IgG4.

13. The C1ORF32 polypeptide for use in a method of claim 8, wherein the fusion protein further comprises a domain that mediates dimerization or multimerization of the fusion protein to form homodimers, heterodimers, homomultimers, or heteromultimers.

14. The C1ORF32 polypeptide for use in a method of claim 13, wherein the domain that mediates dimerization or multimerization is selected from the group consisting of one or more cysteines that are capable of forming an intermolecular disulfide bond with a cysteine on the partner fusion protein, a hinge region of IgG, a coiled-coil domain, an acid patch, a zinc finger domain, a calcium hand domain,a CHI region, a CL region, a leucine zipper domain, an SH2 (src homology 2) domain, an SH3 (src Homology 3) domain, a PTB (phosphotyrosine binding) domain, a WW domain, a PDZ domain, a 14-3-3 domain, a WD40 domain, an EH domain, a Lim domain, an isoleucine zipper domain, and a dimerization domain of a receptor dimer pair.

15. The C1ORF32 polypeptide for use in a method of claim 8, wherein the fusion protein comprises the polypeptide of any one of SEQ ID NOs: 43, 64, 66-88.

16. The C1ORF32 polypeptide for use in a method of claim 8, wherein the fusion protein is a dimeric protein comprising said first and said second fusion proteins, wherein the first and the second fusion proteins are bound to one another by covalent or noncovalent bonds to form a dimer.

17. The C1ORF32 polypeptide for use in a method of claim 16, wherein the fusion proteins are bound together by disulfide bonds.

18. The C1ORF32 polypeptide for use in a method of claim 1, wherein the polypeptide or fusion protein is administered in the form of a pharmaceutical composition, and a pharmaceutically acceptable diluent or carrier, adapted for treatment of immune related disorder.

19. The C1ORF32 polypeptide for use in a method of claim 1, wherein rheumatoid arthritis comprises one or more of rheumatoid arthritis, gout and pseudo-gout, juvenile idiopathic arthritis, juvenile rheumatoid arthritis, Still's disease, ankylosing spondylitis, rheumatoid vasculitis and conditions related to rheumatoid arthritis.

20. The C1ORF32 polypeptide for use in a method of claim 19, wherein said conditions relating to rheumatoid arthritis include osteoarthritis, sarcoidosis, Henoch-Schonlein purpura, psoriatic arthritis, reactive arthritis, spondyloarthropathy, septic arthritis, haemochromatosis, hepatitis, vasculitis, Wegener's granulomatosis, Lyme disease, familial mediterranean fever, hyperimmunoglobulinemia D with recurrent fever, TNF receptor associated periodic syndrome, and enteropathic arthritis associated with inflammatory bowel disease.

## Patentansprüche

1. C1ORF32-Polypeptid zur Verwendung in einem Verfahren zur Behandlung eines Individuums mit einem Risiko zum Entwickeln einer Autoimmunerkrankung, umfassend Nachweisen eines Vorerkrankungsprozesses durch einen Biomarker vor dem Einsetzen von offenkundigen Erkrankungssymptomen, wobei die Autoimmunerkrankung rheumatoide Arthritis umfasst, und wobei der Biomarker eines oder mehr von einem positiven Test auf Serum-Rheumafaktor (RF) und Antikörper gegen citrullinierte Proteinantigene (ACPA); oder alternativ RF-negativ, aber mit hohen Levels von Serum-ACPA (definiert als 3 x obere Grenze von normalem [ULN] für den Test); oder einer Gegenwart von Gelenkschmerz verursacht durch entzündliche Gewebeprozesse, einschließt; und Verabreichen des C1ORF32-Polypeptids an das Individuum.

2. C1ORF32-Polypeptid zur Verwendung in einem Verfahren nach Anspruch 1, wobei der Biomarker zur Bestimmung eines Risikos für eine früh einsetzende Erkrankung, einen schweren Erkrankungsverlauf oder beides analysiert wird.

3. C1ORF32-Polypeptid zur Verwendung in einem Verfahren nach Anspruch 2, wobei der Biomarker eines oder mehr von einem Genom-, Proteom-, Metabolom-, Lipidom-, Glycom-, Sekretom- oder serologischen Biomarker; oder einer familiären Vorgeschichte, Metagenom- oder Mikrobiomanalyse; oder einer Kombination davon einschließt.

4. C1ORF32-Polypeptid zur Verwendung in einem Verfahren nach Anspruch 3, wobei der serologische Biomarker eines oder mehr von Autoantikörpern, Antikörpern gegen erkrankungsspezifische Antigene, hohen Blutzuckerspiegeln, großen Mengen an selbstreaktiven T-Zellen, Defekten bei regulatorischen T-Zellen (Tregs) und/oder chronischer Entzündung, bei welcher das Immunsystem einbezogen ist, umfasst.

5. C1ORF32-Polypeptid zur Verwendung in einem Verfahren nach Anspruch 1, wobei Behandlung Entwicklung von rheumatoider Arthritis-Symptomen, einschließend eines oder mehr von Entzündung, Müdigkeit, Gelenkschmerz, Gelenkdolenz, Gelenkschwellung, Gelenkrötung, Gelenkwärme, Gelenksteifheit, Einschränkung von Gelenkbewegungsbereich, Betroffensein von mehr als einem Gelenk (Polyarthritis), Hinken oder Gelenkdeformierung oder einer Kombination davon, verhindert oder zumindest verlangsamt.

6. C10RF32-Polypeptid zur Verwendung in einem Verfahren nach Anspruch 1, wobei das C1ORF32-Polypeptid die extrazelluläre Domäne von C1ORF32 oder ein Fragment oder eine Variante davon, oder ein Polypeptid, umfassend die extrazelluläre Domäne von einer der SEQ ID Nrn.: 1-5, oder ein Fragment oder eine Variante oder ein Homolog davon umfasst.

7. C1ORF32-Polypeptid zur Verwendung in einem Verfahren nach Anspruch 6, wobei das C1ORF32-Polypeptid aus der Gruppe ausgewählt ist, welche aus Polypeptiden besteht, die eine Sequenz von Aminosäureresten mit mindestens 95 % Sequenzidentität mit einer Aminosäuresequenz, welche in einer der SEQ ID Nrn.: 6-41 gezeigt ist, umfassen.

8. C1ORF32-Polypeptid zur Verwendung in einem Verfahren nach Anspruch 7, wobei das C1ORF32-Polypeptid an eine heterologe Sequenz direkt oder indirekt über ein Linkerpeptid, eine Polypeptidsequenz oder einen chemischen Linker fusioniert ist, um ein Fusionsprotein zu bilden.

9. C1ORF32-Polypeptid zur Verwendung in einem Verfahren nach Anspruch 8, wobei die heterologe Sequenz mindestens einen Anteil von einer konstanten Immunglobulindomäne umfasst.

10. C1ORF32-Polypeptid zur Verwendung in einem Verfahren nach Anspruch 9, wobei das Fusionsprotein eine konstante Region einer schweren Kette eines Immunglobulins, die einem Antikörperisotyp entspricht, welcher aus der Gruppe ausgewählt ist, die aus IgG1, IgG2, IgG3, IgG4, IgM, IgE, IgA und IgD besteht, umfasst.

11. C10RF32-Polypeptid zur Verwendung in einem Verfahren nach Anspruch 10, wobei die konstante Immunglobulindomäne die Hinge-, CH2- und CH3-Regionen von einem humanen IgG-Immunglobulin, ausgewählt aus der Gruppe, welche aus Cγ1-, Cγ2-, Cγ3- und Cγ4-Kette besteht; oder Hybridisierung von Hinge-, CH1-, CH2- und CH3-Regionen von unterschiedlichen Fc-Isotypen umfasst.

12. C10RF32-Polypeptid zur Verwendung in einem Verfahren nach Anspruch 11, wobei die unterschiedlichen Fc-Isotypen IgD und IgG4 umfassen.

13. C1ORF32-Polypeptid zur Verwendung in einem Verfahren nach Anspruch 8, wobei das Fusionsprotein ferner eine Domäne, welche Dimerisierung oder Multimerisierung des Fusionsproteins vermittelt, umfasst, um Homodimere, Heterodimere, Homomultimere oder Heteromultimere zu bilden.

14. C1ORF32-Polypeptid zur Verwendung in einem Verfahren nach Anspruch 13, wobei die Domäne, welche Dimerisierung oder Multimerisierung vermittelt, aus der Gruppe ausgewählt ist, die besteht aus einem oder mehr Cysteinen, welche in der Lage sind zum Bilden einer intermolekularen Disulfid-Bindung mit einem Cystein an dem Partnerfusionsprotein, einer Hinge-Region von IgG, einer Doppelwendel-Domäne, einem Säurepatch, einer Zinkfinger-Domäne, einer Calciumhand-Domäne, einer CHI-Region, einer CL-Region, einer Leucin-Zipperdomäne, einer SH2 (Src-homology 2)-Domäne, einer SH3 (Src-homology 3)-Domäne, einer PTB (Phosphotyrosinbindenden)-Domäne, einer WW-Domäne, einer PDZ-Domäne, einer 14-3-3-Domäne, einer WD40-Domäne, einer EH-Domäne, einer Lim-Domäne, einer Isoleucin-Zipperdomäne und einer Dimerisierungsdomäne von einem Rezeptordimerpaar.

15. C1ORF32-Polypeptid zur Verwendung in einem Verfahren nach Anspruch 8, wobei das Fusionsprotein das Polypeptid von einer der SEQ ID Nrn.: 43, 64, 66-88 umfasst.

16. C1ORF32-Polypeptid zur Verwendung in einem Verfahren nach Anspruch 8, wobei das Fusionsprotein ein dimeres Protein ist, umfassend das erste und das zweite Fusionsprotein, wobei das erste und das zweite Fusionsprotein durch kovalente oder nicht-kovalente Bindungen aneinander gebunden sind, um ein Dimer zu bilden.

17. C1ORF32-Polypeptid zur Verwendung in einem Verfahren nach Anspruch 16, wobei die Fusionsproteine durch Disulfid-Bindungen verbunden sind.

18. C1ORF32-Polypeptid zur Verwendung in einem Verfahren nach Anspruch 1, wobei das Polypeptid oder Fusionsprotein verabreicht wird in der Form einer pharmazeutischen Zusammensetzung, und ein pharmazeutisch verträgliche(s/r) Verdünnungsmittel oder Träger, angepasst zur Behandlung einer immunologischen Störung.

19. C1ORF32-Polypeptid zur Verwendung in einem Verfahren nach Anspruch 1, wobei rheumatoide Arthritis eines oder mehr von rheumatoider Arthritis, Gicht und Pseudogicht, juveniler idiopathischer Arthritis, juveniler rheumatoider Arthritis, Morbus Still, Morbus Bechterew, rheumatoider Vaskulitis und Zuständen, welche mit rheumatoider Arthritis in Zusammenhang stehen, umfasst.

20. C1ORF32-Polypeptid zur Verwendung in einem Verfahren nach Anspruch 19, wobei die Zustände, welche mit rheumatoider Arthritis in Zusammenhang stehen, Osteoarthritis, Sarkoidose, Purpura Schönlein-Henoch, Arthritis psoriatica, reaktive Arthritis, Spondylarthropathie, Arthritis purulenta, Hämochromatose, Hepatitis, Vaskulitis, Wegener-Granulomatose, Lyme-Krankheit, familiäres Mittelmeerfiber, Hyperimmunglobulinämie D mit wiederkehrendem Fieber, TNF-Rezeptor-assoziiertes periodisches Syndrom und Arthritis enteropathica in Zusammenhang mit entzündlicher Darmerkrankung einschließen.

## Revendications

1. Polypeptide C1ORF32 destiné à être utilisé dans un procédé de traitement d'un individu présentant un risque de développer une maladie auto-immune comprenant la détection d'un processus prépathologique par un biomarqueur avant l'apparition de symptômes apparents de la maladie, dans lequel ladite maladie auto-immune comprend l'arthrite rhumatoïde, et dans lequel ledit biomarqueur comprend un ou plusieurs d'un test positif du facteur rhumatoïde sérique (RF) et des anticorps des antigènes protéines citrullinées (ACPA) ; ou, à titre de variante, négatif pour le RF mais avec des niveaux élevés de sérum ACPA (définis comme étant égaux à 3 x la limite supérieure normale [LSN] pour l'analyse) ; ou la présence d'une douleur articulaire provoquée par des processus d'inflammation des tissus ; et l'administration du polypeptide C1ORF32 à l'individu.

2. Polypeptide C1ORF32 destiné à être utilisé dans un procédé selon la revendication 1, dans lequel on analyse ledit biomarqueur pour déterminer un risque d'apparition précoce de la maladie, d'évolution grave de la maladie, ou les deux.

3. Polypeptide C1ORF32 destiné à être utilisé dans un procédé selon la revendication 2, dans lequel ledit biomarqueur comprend un ou plusieurs biomarqueurs de type génomique, protéomique, métabolomique, lipidomique, glycomique, sécrétomique ou sérologique ; ou une analyse des antécédents familiaux, du métagénome ou du microbiome; ou une combinaison de ceux-ci.

4. Polypeptide C1ORF32 destiné à être utilisé dans un procédé selon la revendication 3, dans lequel ledit biomarqueur sérologique comprend un ou plusieurs des autoanticorps, des anticorps d'antigènes spécifiques d'une maladie, des niveaux élevés de glycémie, des niveaux élevés de cellules T auto-réactives, des défauts des cellules T régulatrices (Tregs) et/ou une inflammation chronique liée au système immunitaire.

5. Polypeptide C1ORF32 destiné à être utilisé dans un procédé selon la revendication 1, dans lequel le traitement prévient ou tout au moins ralentit le développement des symptômes de l'arthrite rhumatoïde, y compris un ou plusieurs symptômes parmi l'inflammation, la fatigue, les douleurs articulaires, la sensibilité articulaire, le gonflement articulaire, les rougeurs articulaires, la chaleur articulaire, les raideurs articulaires, la perte d'amplitude de mouvement, affectant plus d'une articulation (polyarthrite), la claudication ou la difformité des articulations, ou une combinaison de ceux-ci.

6. Polypeptide C1ORF32 destiné à être utilisé dans un procédé selon la revendication 1, dans lequel le polypeptide C1ORF32 comprend le domaine extracellulaire de C1ORF32, ou un fragment ou un variant de celui-ci, ou un polypeptide comprenant le domaine extracellulaire de l'une quelconque de SEQ ID N°1 à 5 ou un fragment ou un variant ou un homologue de ceux-ci.

7. Polypeptide C1ORF32 destiné à être utilisé dans un procédé selon la revendication 6, dans lequel le polypeptide C1ORF32 est choisi dans le groupe constitué des polypeptides comprenant une séquence de résidus d'acides aminés présentant une identité de séquence d'au moins 95 % avec la séquence d'acides aminés décrite dans l'un quelconque de SEQ ID N°6 à 41.

8. Polypeptide C1ORF32 destiné à être utilisé dans un procédé selon la revendication 7, dans lequel le polypeptide C1ORF32 est fusionné à une séquence hétérologue pour former une protéine de fusion, directement ou indirectement par l'intermédiaire d'un peptide de liaison, d'une séquence de polypeptides ou d'un lieur chimique.

9. Polypeptide C1ORF32 destiné à être utilisé dans un procédé selon la revendication 8, dans lequel la séquence hétérologue comprend au moins une partie d'un domaine constant d'immunoglobuline.

10. Polypeptide C1ORF32 destiné à être utilisé dans un procédé selon la revendication 9, dans lequel la protéine de fusion comprend une région constante de chaîne lourde d'immunoglobuline correspondant à un isotype d'anticorps choisi dans le groupe comprenant IgG1, IgG2, IgG3, IgG4, IgM, IgE, IgA et IgD.

11. Polypeptide C1ORF32 destiné à être utilisé dans un procédé selon la revendication 10, dans lequel le domaine constant d'immunoglobuline comprend les régions charnière, CH2 et CH3 d'une immunoglobuline humaine IgG, choisie dans le groupe consistant en les chaînes Cγ1, Cγ2, Cγ3 et Cγ4; ou l'hybridation des régions charnière, CH1, CH2 et CH3 de différents isotypes de Fc.

12. Polypeptide C1ORF32 destiné à être utilisé dans un procédé selon la revendication 11, dans lequel les différents isotypes de Fc comprennent IgD et IgG4.

13. Polypeptide C1ORF32 destiné à être utilisé dans un procédé selon la revendication 8, dans lequel la protéine de fusion comprend en outre un domaine induisant la dimérisation ou la multimérisation de la protéine de fusion pour former des homodimères, des hétérodimères, des homomultimères ou des hétéromultimères.

14. Polypeptide C1ORF32 destiné à être utilisé dans un procédé selon la revendication 13, dans lequel le domaine qui induit la dimérisation ou la multimérisation est choisi dans le groupe constitué d'une ou plusieurs cystéines capables de former une liaison intermoléculaire disulfure avec une cystéine sur la protéine de fusion partenaire, une région charnière de IgG, un domaine de superhélice, un patch d'acides, un domaine à doigt de zinc, un domaine en main lié au calcium, une région CHI, une région CL, un domaine glissière à leucine, un domaine SH2 (homologie src 2), un domaine SH3 (homologie src 3), un domaine PTB (liaison à des phosphotyrosines), un domaine WW, un domaine PDZ, un domaine 14-3-3, un domaine WD40, un domaine EH, un domaine Lim, un domaine glissière à isoleucine et un domaine de dimérisation d'une paire dimériques de récepteurs.

15. Polypeptide C1ORF32 destiné à être utilisé dans un procédé selon la revendication 8, dans lequel la protéine de fusion comprend le polypeptide de l'une quelconque de SEQ ID N° 43, 64, 66 à 68.

16. Polypeptide C1ORF32 destiné à être utilisé dans un procédé selon la revendication 8, dans lequel la protéine de fusion est une protéine dimère comprenant ladite première et ladite seconde protéines de fusion, dans lequel la première et la seconde protéines de fusion sont liées l'une à l'autre par des liaisons covalentes ou non-covalentes pour former un dimère.

17. Polypeptide C1ORF32 destiné à être utilisé dans un procédé selon la revendication 16, dans lequel les protéines de fusion sont liées par des liaisons disulfures.

18. Polypeptide C1ORF32 destiné à être utilisé dans un procédé selon la revendication 1, dans lequel le polypeptide ou la protéine de fusion est administré sous forme de composition pharmaceutique et d'un diluant ou excipient pharmaceutiquement acceptable adapté au traitement de troubles de l'immunité.

19. Polypeptide C1ORF32 destiné à être utilisé dans un procédé selon la revendication 1, dans lequel l'arthrite rhumatoïde comprend une ou plusieurs pathologies parmi l'arthrite rhumatoïde, la goutte et la pseudo-goutte, l'arthrite idiopathique juvénile, l'arthrite rhumatoïde juvénile, la maladie de Still, la spondylite ankylosante, la vascularite rhumatoïde et les pathologies liées à l'arthrite rhumatoïde.

20. Polypeptide C1ORF32 destiné à être utilisé dans un procédé selon la revendication 19, dans lequel lesdites pathologies liées à l'arthrite rhumatoïde comprennent l'ostéoarthrite, la sarcoïdose, le purpura de Henoch-Schönlein, l'arthrite psoriasique, l'arthrite réactionnelle, la spondyloarthropathie, l'arthrite septique, l'hémochromatose, l'hépatite, la vascularite, la granulomatose de Wegener, la maladie de Lyme, la fièvre méditerranéenne familiale, le syndrome de fièvre récurrente avec hyperimmunoglobulinémie D, le syndrome périodique associé au récepteur du facteur de nécrose tumorale (TNF) et l'arthrite entéropathique associée à la maladie inflammatoire de l'intestin.


### SEQUENCE LISTING

<110> Compugen Ltd
<120> POLYPEPTIDES AND USES THEREOF AS A DRUG FOR TREATMENT OF AUTOIMMUNE
   DISORDERS
<130>
<160> 88
<210> 1
   <211> 639
   <212> PRT
   <213> Homo Sapiens
<400> 1
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Asp Ser
   145                 150                 155                 160
   Val Glu Leu Leu Val Leu Gly Arg Thr Gly Leu Leu Ala Asp Leu Leu
                   165                 170                 175
   Pro Ser Phe Ala Val Glu Ile Met Pro Glu Trp Val Phe Val Gly Leu
   180 185 190
   Val Leu Leu Gly Val Phe Leu Phe Phe Val Leu Val Gly Ile Cys Trp
   195 200 205
   Cys Gln Cys Cys Pro His Ser Cys Cys Cys Tyr Val Arg Cys Pro Cys
   210 215 220
   Cys Pro Asp Ser Cys Cys Cys Pro Gln Ala Leu Tyr Glu Ala Gly Lys
   225 230 235 240
   Ala Ala Lys Ala Gly Tyr Pro Pro Ser Val Ser Gly Val Pro Gly Pro
   245 250 255
   Tyr Ser Ile Pro Ser Val Pro Leu Gly Gly Ala Pro Ser Ser Gly Met
   260 265 270
   Leu Met Asp Lys Pro His Pro Pro Pro Leu Ala Pro Ser Asp Ser Thr
   275 280 285
   Gly Gly Ser His Ser Val Arg Lys Gly Tyr Arg Ile Gln Ala Asp Lys
   290 295 300
   Glu Arg Asp Ser Met Lys Val Leu Tyr Tyr Val Glu Lys Glu Leu Ala
   305 310 315 320
   Gln Phe Asp Pro Ala Arg Arg Met Arg Gly Arg Tyr Asn Asn Thr Ile
   325 330 335
   Ser Glu Leu Ser Ser Leu His Glu Glu Asp Ser Asn Phe Arg Gln Ser
   340 345 350
   Phe His Gln Met Arg Ser Lys Gln Phe Pro Val Ser Gly Asp Leu Glu
   355 360 365
   Ser Asn Pro Asp Tyr Trp Ser Gly Val Met Gly Gly Ser Ser Gly Ala
   370 375 380
   Ser Arg Gly Pro Ser Ala Met Glu Tyr Asn Lys Glu Asp Arg Glu Ser
   385 390 395 400
   Phe Arg His Ser Gln Pro Arg Ser Lys Ser Glu Met Leu Ser Arg Lys
   405 410 415
   Asn Phe Ala Thr Gly Val Pro Ala Val Ser Met Asp Glu Leu Ala Ala
   420 425 430
   Phe Ala Asp Ser Tyr Gly Gln Arg Pro Arg Arg Ala Asp Gly Asn Ser
   435 440 445
   His Glu Ala Arg Gly Gly Ser Arg Phe Glu Arg Ser Glu Ser Arg Ala
   450 455 460
   His Ser Gly Phe Tyr Gln Asp Asp Ser Leu Glu Glu Tyr Tyr Gly Gln
   465 470 475 480
   Arg Ser Arg Ser Arg Glu Pro Leu Thr Asp Ala Asp Arg Gly Trp Ala
   485 490 495
   Phe Ser Pro Ala Arg Arg Arg Pro Ala Glu Asp Ala His Leu Pro Arg
   500 505 510
   Leu Val Ser Arg Thr Pro Gly Thr Ala Pro Lys Tyr Asp His Ser Tyr
   515 520 525
   Leu Gly Ser Ala Arg Glu Arg Gln Ala Arg Pro Glu Gly Ala Ser Arg
   530 535 540
   Gly Gly Ser Leu Glu Thr Pro Ser Lys Arg Ser Ala Gln Leu Gly Pro
   545 550 555 560
   Arg Ser Ala Ser Tyr Tyr Ala Trp Ser Pro Pro Gly Thr Tyr Lys Ala
   565 570 575
   Gly Ser Ser Gln Asp Asp Gln Glu Asp Ala Ser Asp Asp Ala Leu Pro
   580 585 590
   Pro Tyr Ser Glu Leu Glu Leu Thr Arg Gly Pro Ser Tyr Arg Gly Arg
   595 600 605
   Asp Leu Pro Tyr His Ser Asn Ser Glu Lys Lys Arg Lys Lys Glu Pro
   610 615 620
   Ala Lys Lys Thr Asn Asp Phe Pro Thr Arg Met Ser Leu Val Val
   625 630 635
<210> 2
   <211> 254
   <212> PRT
   <213> Homo Sapiens
<400> 2
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Asp Ser
   145                 150                 155                 160
   Val Glu Leu Leu Val Leu Gly Arg Thr Gly Leu Leu Ala Asp Leu Leu
                   165                 170                 175
   Pro Ser Phe Ala Val Glu Ile Met Pro Glu Trp Val Phe Val Gly Leu
               180                 185                 190
   Val Leu Leu Gly Val Phe Leu Phe Phe Val Leu Val Gly Ile Cys Trp
           195                 200                 205
   Cys Gln Cys Cys Pro His Ser Cys Cys Cys Tyr Val Arg Cys Pro Cys
       210                 215                 220
   Cys Pro Asp Ser Cys Cys Cys Pro Gln Ala Cys Glu Tyr Ser Asp Arg
   225                 230                 235                 240
   Trp Gly Asp Arg Ala Ile Glu Arg Asn Val Tyr Leu Ser Thr
                   245                 250
<210> 3
   <211> 254
   <212> PRT
   <213> Homo Sapiens
<400> 3
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Gly Ser
   145                 150                 155                 160
   Leu Gly Leu Leu Val Leu Gly Arg Thr Gly Leu Leu Ala Asp Leu Leu
                   165                 170                 175
   Pro Ser Phe Ala Val Glu Ile Met Pro Glu Trp Val Phe Val Gly Leu
               180                 185                 190
   Val Leu Leu Gly Val Phe Leu Phe Phe Val Leu Val Gly Ile Cys Trp
           195                 200                 205
   Cys Gln Cys Cys Pro His Ser Cys Cys Cys Tyr Val Arg Cys Pro Cys
       210                 215                 220
   Cys Pro Asp Ser Cys Trp Cys Pro Gln Ala Cys Glu Tyr Ser Asp Arg
   225                 230                 235                 240
   Trp Gly Asp Arg Ala Ile Glu Arg Asn Val Tyr Leu Ser Thr
                   245                 250
<210> 4
   <211> 235
   <212> PRT
   <213> Homo Sapiens
<400> 4
   Met Asp Arg Val Leu Leu Arg Trp Ile Ser Leu Phe Trp Leu Thr Ala
   1               5                   10                  15
   Met Val Glu Gly Leu Gln Val Thr Val Pro Asp Lys Lys Lys Val Ala
               20                  25                  30
   Met Leu Phe Gln Pro Thr Val Leu Arg Cys His Phe Ser Thr Ser Ser
           35                  40                  45
   His Gln Pro Ala Val Val Gln Trp Lys Phe Lys Ser Tyr Cys Gln Asp
       50                  55                  60
   Arg Met Gly Glu Ser Leu Gly Met Ser Ser Thr Arg Ala Gln Ser Leu
   65                  70                  75                  80
   Ser Lys Arg Asn Leu Glu Trp Asp Pro Tyr Leu Asp Cys Leu Asp Ser
                   85                  90                  95
   Arg Arg Thr Val Arg Val Val Ala Ser Lys Gln Gly Ser Thr Val Thr
               100                 105                 110
   Leu Gly Asp Phe Tyr Arg Gly Arg Glu Ile Thr Ile Val His Asp Ala
           115                 120                 125
   Asp Leu Gln Ile Gly Lys Leu Met Trp Gly Asp Ser Gly Leu Tyr Tyr
       130                 135                 140
   Cys Ile Ile Thr Thr Pro Asp Asp Leu Glu Gly Lys Asn Glu Asp Ser
   145                 150                 155                 160
   Val Glu Leu Leu Val Leu Glu Trp Val Phe Val Gly Leu Val Leu Leu
                   165                 170                 175
   Gly Val Phe Leu Phe Phe Val Leu Val Gly Ile Cys Trp Cys Gln Cys
               180                 185                 190
   Cys Pro His Ser Cys Cys Cys Tyr Val Arg Cys Pro Cys Cys Pro Asp
   195 200 205
   Ser Cys Cys Cys Pro Gln Ala Cys Glu Tyr Ser Asp Arg Trp Gly Asp
   210 215 220
   Arg Ala Ile Glu Arg Asn Val Tyr Leu Ser Thr
   225 230 235
<210> 5
   <211> 235
   <212> PRT
   <213> Homo Sapiens
<400> 5
   Met Asp Arg Val Leu Leu Arg Trp Ile Ser Leu Phe Trp Leu Thr Ala
   1 5 10 15
   Met Val Glu Gly Leu Gln Val Thr Val Pro Asp Lys Lys Lys Val Ala
   20 25 30
   Met Leu Phe Gln Pro Thr Val Leu Arg Cys His Phe Ser Thr Ser Ser
   35 40 45
   His Gln Pro Ala Val Val Gln Trp Lys Phe Lys Ser Tyr Cys Gln Asp
   50 55 60
   Arg Met Gly Glu Ser Leu Gly Met Ser Ser Thr Arg Ala Gln Ser Leu
   65 70 75 80
   Ser Lys Arg Asn Leu Glu Trp Asp Pro Tyr Leu Asp Cys Leu Asp Ser
   85 90 95
   Arg Arg Thr Val Arg Val Val Ala Ser Lys Gln Gly Ser Thr Val Thr
   100 105 110
   Leu Gly Asp Phe Tyr Arg Gly Arg Glu Ile Thr Ile Val His Asp Ala
   115 120 125
   Asp Leu Gln Ile Gly Lys Leu Met Trp Gly Asp Ser Gly Leu Tyr Tyr
   130 135 140
   Cys Ile Ile Thr Thr Pro Asp Asp Leu Glu Gly Lys Asn Glu Gly Ser
   145 150 155 160
   Leu Gly Leu Leu Val Leu Glu Trp Val Phe Val Gly Leu Val Leu Leu
                   165                 170                 175
   Gly Val Phe Leu Phe Phe Val Leu Val Gly Ile Cys Trp Cys Gln Cys
               180                 185                 190
   Cys Pro His Ser Cys Cys Cys Tyr Val Arg Cys Pro Cys Cys Pro Asp
           195                 200                 205
   Ser Cys Trp Cys Pro Gln Ala Cys Glu Tyr Ser Asp Arg Trp Gly Asp
       210                 215                 220
   Arg Ala Ile Glu Arg Asn Val Tyr Leu Ser Thr
   225                 230                 235
<210> 6
   <211> 144
   <212> PRT
   <213> Homo Sapiens
<400> 6
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
<210> 7
   <211> 145
   <212> PRT
   <213> Homo Sapiens
<400> 7
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val
   145
<210> 8
   <211> 146
   <212> PRT
   <213> Homo Sapiens
<400> 8
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu
   145
<210> 9
   <211> 147
   <212> PRT
   <213> Homo Sapiens
<400> 9
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly
   145
<210> 10
   <211> 148
   <212> PRT
   <213> Homo Sapiens
<400> 10
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg
   145
<210> 11
   <211> 149
   <212> PRT
   <213> Homo Sapiens
<400> 11
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr
   145
<210> 12
   <211> 150
   <212> PRT
   <213> Homo Sapiens
<400> 12
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly
   145 150
<210> 13
   <211> 151
   <212> PRT
   <213> Homo Sapiens
<400> 13
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu
   145                 150
<210> 14
   <211> 152
   <212> PRT
   <213> Homo Sapiens
<400> 14
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu
   145 150
<210> 15
   <211> 153
   <212> PRT
   <213> Homo Sapiens
<400> 15
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala
   145 150
<210> 16
   <211> 154
   <212> PRT
   <213> Homo Sapiens
<400> 16
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp
   145                 150
<210> 17
   <211> 155
   <212> PRT
   <213> Homo Sapiens
<400> 17
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu
   145                 150                 155
<210> 18
   <211> 156
   <212> PRT
   <213> Homo Sapiens
<400> 18
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu
   145 150 155
<210> 19
   <211> 157
   <212> PRT
   <213> Homo Sapiens
<400> 19
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro
   145 150 155
<210> 20
   <211> 158
   <212> PRT
   <213> Homo Sapiens
<400> 20
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser
   145                 150                 155
<210> 21
   <211> 159
   <212> PRT
   <213> Homo Sapiens
<400> 21
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro Ser Phe
   145 150 155
<210> 22
   <211> 160
   <212> PRT
   <213> Homo Sapiens
<400> 22
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro Ser Phe Ala
   145 150 155 160
<210> 23
   <211> 161
   <212> PRT
   <213> Homo Sapiens
<400> 23
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro Ser Phe Ala
   145 150 155 160
   Val
<210> 24
   <211> 162
   <212> PRT
   <213> Homo Sapiens
<400> 24
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu
<210> 25
   <211> 163
   <212> PRT
   <213> Homo Sapiens
<400> 25
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro Ser Phe Ala
   145 150 155 160
   Val Glu Ile
<210> 26
   <211> 164
   <212> PRT
   <213> Homo Sapiens
<400> 26
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro Ser Phe Ala
   145 150 155 160
   Val Glu Ile Met
<210> 27
   <211> 165
   <212> PRT
   <213> Homo Sapiens
<400> 27
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro Ser Phe Ala
   145 150 155 160
   Val Glu Ile Met Pro
   165
<210> 28
   <211> 166
   <212> PRT
   <213> Homo Sapiens
<400> 28
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro Ser Phe Ala
   145                 150                 155                 160
   Val Glu Ile Met Pro Glu
                   165
<210> 29
   <211> 167
   <212> PRT
   <213> Homo Sapiens
<400> 29
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Pro Glu Trp
                   165
<210> 30
   <211> 168
   <212> PRT
   <213> Homo Sapiens
<400> 30
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Pro Glu Trp Val
                   165
<210> 31
   <211> 169
   <212> PRT
   <213> Homo Sapiens
<400> 31
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro Ser Phe Ala
   145 150 155 160
   Val Glu Ile Met Pro Glu Trp Val Phe
   165
<210> 32
   <211> 147
   <212> PRT
   <213> Homo Sapiens
<400> 32
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu
   145
<210> 33
   <211> 148
   <212> PRT
   <213> Homo Sapiens
<400> 33
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu Trp
   145
<210> 34
   <211> 149
   <212> PRT
   <213> Homo Sapiens
<400> 34
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu Trp Val
   145
<210> 35
   <211> 150
   <212> PRT
   <213> Homo Sapiens
<210> 36
   <211> 151
   <212> PRT
   <213> Homo Sapiens
<400> 36
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu Trp Val Phe Val
   145                 150
<210> 37
   <211> 152
   <212> PRT
   <213> Homo Sapiens
<400> 37
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu Trp Val Phe Val Gly
   145                 150
<210> 38
   <211> 104
   <212> PRT
   <213> Homo Sapiens
<400> 38
   Cys His Phe Ser Thr Ser Ser His Gln Pro Ala Val Val Gln Trp Lys
   1 5 10 15
   Phe Lys Ser Tyr Cys Gln Asp Arg Met Gly Glu Ser Leu Gly Met Ser
   20 25 30
   Ser Thr Arg Ala Gln Ser Leu Ser Lys Arg Asn Leu Glu Trp Asp Pro
   35 40 45
   Tyr Leu Asp Cys Leu Asp Ser Arg Arg Thr Val Arg Val Val Ala Ser
   50 55 60
   Lys Gln Gly Ser Thr Val Thr Leu Gly Asp Phe Tyr Arg Gly Arg Glu
   65 70 75 80
   Ile Thr Ile Val His Asp Ala Asp Leu Gln Ile Gly Lys Leu Met Trp
   85 90 95
   Gly Asp Ser Gly Leu Tyr Tyr Cys
   100
<210> 39
   <211> 164
   <212> PRT
   <213> Homo Sapiens
<400> 39
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro Ser Gly Gly
   145 150 155 160
   Val Glu Ile Met
<210> 40
   <211> 164
   <212> PRT
   <213> Homo Sapiens
<400> 40
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro Ser Gly Ala
   145 150 155 160
   Val Glu Ile Met
<210> 41
   <211> 164
   <212> PRT
   <213> Homo Sapiens
<400> 41
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
               115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Ala Ala
   145                 150                 155                 160
   Val Glu Ile Met
<210> 42
   <211> 408
   <212> PRT
   <213> Artificial Sequence
<220>
   <223> Fusion protein: human C1ORF32-ECD fused to mouse IgG2a Fc
<400> 42
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Gly Ser Glu Asn Leu Tyr Phe Gln Gly Ser Gly Glu
                   165                 170                 175
   Pro Arg Gly Pro Thr Ile Lys Pro Cys Pro Pro Cys Lys Cys Pro Ala
               180                 185                 190
   Pro Asn Leu Leu Gly Gly Pro Ser Val Phe Ile Phe Pro Pro Lys Ile
           195                 200                 205
   Lys Asp Val Leu Met Ile Ser Leu Ser Pro Ile Val Thr Cys Val Val
       210                 215                 220
   Val Asp Val Ser Glu Asp Asp Pro Asp Val Gln Ile Ser Trp Phe Val
   225                 230                 235                 240
   Asn Asn Val Glu Val His Thr Ala Gln Thr Gln Thr His Arg Glu Asp
                   245                 250                 255
   Tyr Asn Ser Thr Leu Arg Val Val Ser Ala Leu Pro Ile Gln His Gln
               260                 265                 270
   Asp Trp Met Ser Gly Lys Glu Phe Lys Cys Lys Val Asn Asn Lys Asp
           275                 280                 285
   Leu Pro Ala Pro Ile Glu Arg Thr Ile Ser Lys Pro Lys Gly Ser Val
       290                 295                 300
   Arg Ala Pro Gln Val Tyr Val Leu Pro Pro Pro Glu Glu Glu Met Thr
   305                 310                 315                 320
   Lys Lys Gln Val Thr Leu Thr Cys Met Val Thr Asp Phe Met Pro Glu
                   325                 330                 335
   Asp Ile Tyr Val Glu Trp Thr Asn Asn Gly Lys Thr Glu Leu Asn Tyr
               340                 345                 350
   Lys Asn Thr Glu Pro Val Leu Asp Ser Asp Gly Ser Tyr Phe Met Tyr
           355                 360                 365
   Ser Lys Leu Arg Val Glu Lys Lys Asn Trp Val Glu Arg Asn Ser Tyr
       370                 375                 380
   Ser Cys Ser Val Val His Glu Gly Leu His Asn His His Thr Thr Lys
   385                 390                 395                 400
   Ser Phe Ser Arg Thr Pro Gly Lys
   405
<210> 43
   <211> 396
   <212> PRT
   <213> Artificial Sequence
<220>
   <223> Fusion protein: human C1ORF32-ECD fused to human IgG1 Fc
<400> 43
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Arg Thr Gly Leu Leu Ala Asp Leu Leu Pro Ser Phe Ala
   145 150 155 160
   Val Glu Ile Met Glu Pro Lys Ser Ser Asp Lys Thr His Thr Cys Pro
   165 170 175
   Pro Cys Pro Ala Pro Glu Leu Leu Gly Gly Pro Ser Val Phe Leu Phe
   180 185 190
   Pro Pro Lys Pro Lys Asp Thr Leu Met Ile Ser Arg Thr Pro Glu Val
   195 200 205
   Thr Cys Val Val Val Asp Val Ser His Glu Asp Pro Glu Val Lys Phe
   210 215 220
   Asn Trp Tyr Val Asp Gly Val Glu Val His Asn Ala Lys Thr Lys Pro
   225 230 235 240
   Arg Glu Glu Gln Tyr Asn Ser Thr Tyr Arg Val Val Ser Val Leu Thr
   245 250 255
   Val Leu His Gln Asp Trp Leu Asn Gly Lys Glu Tyr Lys Cys Lys Val
   260 265 270
   Ser Asn Lys Ala Leu Pro Ala Pro Ile Glu Lys Thr Ile Ser Lys Ala
   275 280 285
   Lys Gly Gln Pro Arg Glu Pro Gln Val Tyr Thr Leu Pro Pro Ser Arg
   290 295 300
   Asp Glu Leu Thr Lys Asn Gln Val Ser Leu Thr Cys Leu Val Lys Gly
   305 310 315 320
   Phe Tyr Pro Ser Asp Ile Ala Val Glu Trp Glu Ser Asn Gly Gln Pro
   325 330 335
   Glu Asn Asn Tyr Lys Thr Thr Pro Pro Val Leu Asp Ser Asp Gly Ser
   340 345 350
   Phe Phe Leu Tyr Ser Lys Leu Thr Val Asp Lys Ser Arg Trp Gln Gln
   355 360 365
   Gly Asn Val Phe Ser Cys Ser Val Met His Glu Ala Leu His Asn His
   370 375 380
   Tyr Thr Gln Lys Ser Leu Ser Leu Ser Pro Gly Lys
   385 390 395
<210> 44
   <211> 233
   <212> PRT
   <213> Artificial Sequence
<220>
   <223> Mouse IgG2a Fc
<400> 44
   Glu Pro Arg Gly Pro Thr Ile Lys Pro Cys Pro Pro Cys Lys Cys Pro
   1               5                   10                  15
   Ala Pro Asn Leu Leu Gly Gly Pro Ser Val Phe Ile Phe Pro Pro Lys
               20                  25                  30
   Ile Lys Asp Val Leu Met Ile Ser Leu Ser Pro Ile Val Thr Cys Val
           35                  40                  45
   Val Val Asp Val Ser Glu Asp Asp Pro Asp Val Gln Ile Ser Trp Phe
       50                  55                  60
   Val Asn Asn Val Glu Val His Thr Ala Gln Thr Gln Thr His Arg Glu
   65                  70                  75                  80
   Asp Tyr Asn Ser Thr Leu Arg Val Val Ser Ala Leu Pro Ile Gln His
                   85                  90                  95
   Gln Asp Trp Met Ser Gly Lys Glu Phe Lys Cys Lys Val Asn Asn Lys
               100                 105                 110
   Asp Leu Pro Ala Pro Ile Glu Arg Thr Ile Ser Lys Pro Lys Gly Ser
           115                 120                 125
   Val Arg Ala Pro Gln Val Tyr Val Leu Pro Pro Pro Glu Glu Glu Met
       130                 135                 140
   Thr Lys Lys Gln Val Thr Leu Thr Cys Met Val Thr Asp Phe Met Pro
   145                 150                 155                 160
   Glu Asp Ile Tyr Val Glu Trp Thr Asn Asn Gly Lys Thr Glu Leu Asn
                   165                 170                 175
   Tyr Lys Asn Thr Glu Pro Val Leu Asp Ser Asp Gly Ser Tyr Phe Met
               180                 185                 190
   Tyr Ser Lys Leu Arg Val Glu Lys Lys Asn Trp Val Glu Arg Asn Ser
           195                 200                 205
   Tyr Ser Cys Ser Val Val His Glu Gly Leu His Asn His His Thr Thr
       210                 215                 220
   Lys Ser Phe Ser Arg Thr Pro Gly Lys
   225                 230
<210> 45
   <211> 232
   <212> PRT
   <213> Homo Sapiens
<400> 45
   Glu Pro Lys Ser Cys Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225 230
<210> 46
   <211> 232
   <212> PRT
   <213> Artificial Sequence
<220>
   <223> Human IgG1 Fc C220S
<400> 46
   Glu Pro Lys Ser Ser Asp Lys Thr His Thr Cys Pro Pro Cys Pro Ala
   1 5 10 15
   Pro Glu Leu Leu Gly Gly Pro Ser Val Phe Leu Phe Pro Pro Lys Pro
   20 25 30
   Lys Asp Thr Leu Met Ile Ser Arg Thr Pro Glu Val Thr Cys Val Val
   35 40 45
   Val Asp Val Ser His Glu Asp Pro Glu Val Lys Phe Asn Trp Tyr Val
   50 55 60
   Asp Gly Val Glu Val His Asn Ala Lys Thr Lys Pro Arg Glu Glu Gln
   65 70 75 80
   Tyr Asn Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
   100 105 110
   Leu Pro Ala Pro Ile Glu Lys Thr Ile Ser Lys Ala Lys Gly Gln Pro
   115 120 125
   Arg Glu Pro Gln Val Tyr Thr Leu Pro Pro Ser Arg Asp Glu Leu Thr
   130 135 140
   Lys Asn Gln Val Ser Leu Thr Cys Leu Val Lys Gly Phe Tyr Pro Ser
   145 150 155 160
   Asp Ile Ala Val Glu Trp Glu Ser Asn Gly Gln Pro Glu Asn Asn Tyr
   165 170 175
   Lys Thr Thr Pro Pro Val Leu Asp Ser Asp Gly Ser Phe Phe Leu Tyr
   180 185 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 47
   <211> 227
   <212> PRT
   <213> Artificial Sequence
<220>
   <223> Human IgG1 Fc without hinge
<400> 47
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Asp Glu Leu Thr Lys Asn Gln Val Ser
       130                 135                 140
   Leu Thr Cys Leu Val Lys Gly Phe Tyr Pro Ser Asp Ile Ala Val Glu
   145 150 155 160
   Trp Glu Ser Asn Gly Gln Pro Glu Asn Asn Tyr Lys Thr Thr Pro Pro
   165 170 175
   Val Leu Asp Ser Asp Gly Ser Phe Phe Leu Tyr Ser Lys Leu Thr Val
   180 185 190
   Asp Lys Ser Arg Trp Gln Gln Gly Asn Val Phe Ser Cys Ser Val Met
   195 200 205
   His Glu Ala Leu His Asn His Tyr Thr Gln Lys Ser Leu Ser Leu Ser
   210 215 220
   Pro Gly Lys
   225
<210> 48
   <211> 2
   <212> PRT
   <213> Artificial Sequence
<220>
   <223> Synthetic polypeptide linker
<400> 48
   Gly Ser
   1
<210> 49
   <211> 4
   <212> PRT
   <213> Artificial Sequence
<220>
   <223> Synthetic polypeptide linker
<400> 49
   Gly Ser Gly Ser
   1
<210> 50
   <211> 2
   <212> PRT
   <213> Artificial Sequence
<220>
   <223> Synthetic polypeptide linker
<400> 50
   Ala Ser
   1
<210> 51
   <211> 4
   <212> PRT
   <213> Artificial Sequence
<220>
   <223> Synthetic polypeptide linker
<400> 51
   Gly Gly Gly Ser
   1
<210> 52
   <211> 5
   <212> PRT
   <213> Artificial Sequence
<220>
   <223> Synthetic polypeptide linker
<400> 52
   Gly Gly Gly Gly Ser
   1 5
<210> 53
   <211> 10
   <212> PRT
   <213> Artificial Sequence
<220>
   <223> Synthetic polypeptide linker
<400> 53
   Gly Gly Gly Gly Ser Gly Gly Gly Gly Ser
   1 5 10
<210> 54
   <211> 15
   <212> PRT
   <213> Artificial Sequence
<220>
   <223> Synthetic polypeptide linker
<400> 54
   Gly Gly Gly Gly Ser Gly Gly Gly Gly Ser Gly Gly Gly Gly Ser
   1 5 10 15
<210> 55
   <211> 20
   <212> PRT
   <213> Artificial Sequence
<220>
   <223> Synthetic polypeptide linker
<400> 55
   Gly Gly Gly Gly Ser Gly Gly Gly Gly Ser Gly Gly Gly Gly Ser Gly
   1               5                   10                  15
   Gly Gly Gly Ser
               20
<210> 56
   <211> 11
   <212> PRT
   <213> Artificial Sequence
<220>
   <223> Synthetic polypeptide linker
<400> 56
   Gly Ser Glu Asn Leu Tyr Phe Gln Gly Ser Gly
   1               5                   10
<210> 57
   <211> 12
   <212> PRT
   <213> Artificial Sequence
<220>
   <223> Synthetic polypeptide linker
<400> 57
   Ala Gly Ala Ala Ala Lys Gly Ala Ala Ala Lys Ala
   1               5                   10
<210> 58
   <211> 17
   <212> PRT
   <213> Artificial Sequence
<220>
   <223> Synthetic polypeptide linker
<400> 58
   Ala Gly Ala Ala Ala Lys Gly Ala Ala Ala Lys Gly Ala Ala Ala Lys
   1               5                   10                  15
   Ala
<210> 59
   <211> 22
   <212> PRT
   <213> Artificial Sequence
<220>
   <223> Synthetic polypeptide linker
<400> 59
   Ala Gly Ala Ala Ala Lys Gly Ala Ala Ala Lys Gly Ala Ala Ala Lys
   1               5                   10                  15
   Gly Ala Ala Ala Lys Ala
               20
<210> 60
   <211> 27
   <212> PRT
   <213> Artificial Sequence
<220>
   <223> Synthetic polypeptide linker
<400> 60
   Ala Gly Ala Ala Ala Lys Gly Ala Ala Ala Lys Gly Ala Ala Ala Lys
   1               5                   10                  15
   Gly Ala Ala Ala Lys Gly Ala Ala Ala Lys Ala
               20                  25
<210> 61
   <211> 7
   <212> PRT
   <213> Artificial Sequence
<220>
   <223> Synthetic polypeptide linker
<400> 61
   Ala Gly Ala Ala Ala Lys Ala
   1               5
<210> 62
   <211> 408
   <212> PRT
   <213> Artificial Sequence
<220>
   <223> Fusion protein: human C1ORF32-ECD fused to mouse IgG2a Fc
   N297A with GS-TEV-GS linker
<400> 62
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Gly Ser Glu Asn Leu Tyr Phe Gln Gly Ser Gly Glu
                   165                 170                 175
   Pro Arg Gly Pro Thr Ile Lys Pro Cys Pro Pro Cys Lys Cys Pro Ala
               180                 185                 190
   Pro Asn Leu Leu Gly Gly Pro Ser Val Phe Ile Phe Pro Pro Lys Ile
           195                 200                 205
   Lys Asp Val Leu Met Ile Ser Leu Ser Pro Ile Val Thr Cys Val Val
       210                 215                 220
   Val Asp Val Ser Glu Asp Asp Pro Asp Val Gln Ile Ser Trp Phe Val
   225                 230                 235                 240
   Asn Asn Val Glu Val His Thr Ala Gln Thr Gln Thr His Arg Glu Asp
                   245                 250                 255
   Tyr Ala Ser Thr Leu Arg Val Val Ser Ala Leu Pro Ile Gln His Gln
               260                 265                 270
   Asp Trp Met Ser Gly Lys Glu Phe Lys Cys Lys Val Asn Asn Lys Asp
           275                 280                 285
   Leu Pro Ala Pro Ile Glu Arg Thr Ile Ser Lys Pro Lys Gly Ser Val
       290                 295                 300
   Arg Ala Pro Gln Val Tyr Val Leu Pro Pro Pro Glu Glu Glu Met Thr
   305                 310                 315                 320
   Lys Lys Gln Val Thr Leu Thr Cys Met Val Thr Asp Phe Met Pro Glu
                   325                 330                 335
   Asp Ile Tyr Val Glu Trp Thr Asn Asn Gly Lys Thr Glu Leu Asn Tyr
               340                 345                 350
   Lys Asn Thr Glu Pro Val Leu Asp Ser Asp Gly Ser Tyr Phe Met Tyr
           355                 360                 365
   Ser Lys Leu Arg Val Glu Lys Lys Asn Trp Val Glu Arg Asn Ser Tyr
       370                 375                 380
   Ser Cys Ser Val Val His Glu Gly Leu His Asn His His Thr Thr Lys
   385                 390                 395                 400
   Ser Phe Ser Arg Thr Pro Gly Lys
                   405
<210> 63
   <211> 233
   <212> PRT
   <213> Artificial Sequence
<220>
   <223> mouse IgG2a Fc N297A
<400> 63
   Glu Pro Arg Gly Pro Thr Ile Lys Pro Cys Pro Pro Cys Lys Cys Pro
   1               5                   10                  15
   Ala Pro Asn Leu Leu Gly Gly Pro Ser Val Phe Ile Phe Pro Pro Lys
               20                  25                  30
   Ile Lys Asp Val Leu Met Ile Ser Leu Ser Pro Ile Val Thr Cys Val
           35                  40                  45
   Val Val Asp Val Ser Glu Asp Asp Pro Asp Val Gln Ile Ser Trp Phe
       50                  55                  60
   Val Asn Asn Val Glu Val His Thr Ala Gln Thr Gln Thr His Arg Glu
   65                  70                  75                  80
   Asp Tyr Ala Ser Thr Leu Arg Val Val Ser Ala Leu Pro Ile Gln His
                   85                  90                  95
   Gln Asp Trp Met Ser Gly Lys Glu Phe Lys Cys Lys Val Asn Asn Lys
               100                 105                 110
   Asp Leu Pro Ala Pro Ile Glu Arg Thr Ile Ser Lys Pro Lys Gly Ser
           115                 120                 125
   Val Arg Ala Pro Gln Val Tyr Val Leu Pro Pro Pro Glu Glu Glu Met
       130                 135                 140
   Thr Lys Lys Gln Val Thr Leu Thr Cys Met Val Thr Asp Phe Met Pro
   145                 150                 155                 160
   Glu Asp Ile Tyr Val Glu Trp Thr Asn Asn Gly Lys Thr Glu Leu Asn
                   165                 170                 175
   Tyr Lys Asn Thr Glu Pro Val Leu Asp Ser Asp Gly Ser Tyr Phe Met
               180                 185                 190
   Tyr Ser Lys Leu Arg Val Glu Lys Lys Asn Trp Val Glu Arg Asn Ser
           195                 200                 205
   Tyr Ser Cys Ser Val Val His Glu Gly Leu His Asn His His Thr Thr
       210                 215                 220
   Lys Ser Phe Ser Arg Thr Pro Gly Lys
   225                 230
<210> 64
   <211> 396
   <212> PRT
   <213> Artificial Sequence
<220>
   <223> Fusion protein: human C1ORF32-ECD fused to human IgG1 Fc
   C220S N297A with GS-TEV-GS linker
<400> 64
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Glu Pro Lys Ser Ser Asp Lys Thr His Thr Cys Pro
                   165                 170                 175
   Pro Cys Pro Ala Pro Glu Leu Leu Gly Gly Pro Ser Val Phe Leu Phe
               180                 185                 190
   Pro Pro Lys Pro Lys Asp Thr Leu Met Ile Ser Arg Thr Pro Glu Val
           195                 200                 205
   Thr Cys Val Val Val Asp Val Ser His Glu Asp Pro Glu Val Lys Phe
       210                 215                 220
   Asn Trp Tyr Val Asp Gly Val Glu Val His Asn Ala Lys Thr Lys Pro
   225                 230                 235                 240
   Arg Glu Glu Gln Tyr Ala Ser Thr Tyr Arg Val Val Ser Val Leu Thr
                   245                 250                 255
   Val Leu His Gln Asp Trp Leu Asn Gly Lys Glu Tyr Lys Cys Lys Val
               260                 265                 270
   Ser Asn Lys Ala Leu Pro Ala Pro Ile Glu Lys Thr Ile Ser Lys Ala
           275                 280                 285
   Lys Gly Gln Pro Arg Glu Pro Gln Val Tyr Thr Leu Pro Pro Ser Arg
       290                 295                 300
   Asp Glu Leu Thr Lys Asn Gln Val Ser Leu Thr Cys Leu Val Lys Gly
   305                 310                 315                 320
   Phe Tyr Pro Ser Asp Ile Ala Val Glu Trp Glu Ser Asn Gly Gln Pro
                   325                 330                 335
   Glu Asn Asn Tyr Lys Thr Thr Pro Pro Val Leu Asp Ser Asp Gly Ser
               340                 345                 350
   Phe Phe Leu Tyr Ser Lys Leu Thr Val Asp Lys Ser Arg Trp Gln Gln
           355                 360                 365
   Gly Asn Val Phe Ser Cys Ser Val Met His Glu Ala Leu His Asn His
       370                 375                 380
   Tyr Thr Gln Lys Ser Leu Ser Leu Ser Pro Gly Lys
   385                 390                 395
<210> 65
   <211> 232
   <212> PRT
   <213> Artificial Sequence
<220>
   <223> human IgG1 Fc C220S N297A
<400> 65
   Glu Pro Lys Ser Ser Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
   20 25 30
   Lys Asp Thr Leu Met Ile Ser Arg Thr Pro Glu Val Thr Cys Val Val
   35 40 45
   Val Asp Val Ser His Glu Asp Pro Glu Val Lys Phe Asn Trp Tyr Val
   50 55 60
   Asp Gly Val Glu Val His Asn Ala Lys Thr Lys Pro Arg Glu Glu Gln
   65 70 75 80
   Tyr Ala Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
   100 105 110
   Leu Pro Ala Pro Ile Glu Lys Thr Ile Ser Lys Ala Lys Gly Gln Pro
   115 120 125
   Arg Glu Pro Gln Val Tyr Thr Leu Pro Pro Ser Arg Asp Glu Leu Thr
   130 135 140
   Lys Asn Gln Val Ser Leu Thr Cys Leu Val Lys Gly Phe Tyr Pro Ser
   145 150 155 160
   Asp Ile Ala Val Glu Trp Glu Ser Asn Gly Gln Pro Glu Asn Asn Tyr
   165 170 175
   Lys Thr Thr Pro Pro Val Leu Asp Ser Asp Gly Ser Phe Phe Leu Tyr
   180 185 190
   Ser Lys Leu Thr Val Asp Lys Ser Arg Trp Gln Gln Gly Asn Val Phe
   195 200 205
   Ser Cys Ser Val Met His Glu Ala Leu His Asn His Tyr Thr Gln Lys
   210 215 220
   Ser Leu Ser Leu Ser Pro Gly Lys
   225 230
<210> 66
   <211> 401
   <212> PRT
   <213> Artificial Sequence
<220>
   <223> Fusion protein: human C1ORF32-ECD fused to human IgG1 Fc
   with G4Sx1 linker, with C220S mutation
<400> 66
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Gly Gly Gly Gly Ser Glu Pro Lys Ser Ser Asp Lys
                   165                 170                 175
   Thr His Thr Cys Pro Pro Cys Pro Ala Pro Glu Leu Leu Gly Gly Pro
               180                 185                 190
   Ser Val Phe Leu Phe Pro Pro Lys Pro Lys Asp Thr Leu Met Ile Ser
           195                 200                 205
   Arg Thr Pro Glu Val Thr Cys Val Val Val Asp Val Ser His Glu Asp
       210                 215                 220
   Pro Glu Val Lys Phe Asn Trp Tyr Val Asp Gly Val Glu Val His Asn
   225 230 235 240
   Ala Lys Thr Lys Pro Arg Glu Glu Gln Tyr Asn Ser Thr Tyr Arg Val
   245 250 255
   Val Ser Val Leu Thr Val Leu His Gln Asp Trp Leu Asn Gly Lys Glu
   260 265 270
   Tyr Lys Cys Lys Val Ser Asn Lys Ala Leu Pro Ala Pro Ile Glu Lys
   275 280 285
   Thr Ile Ser Lys Ala Lys Gly Gln Pro Arg Glu Pro Gln Val Tyr Thr
   290 295 300
   Leu Pro Pro Ser Arg Asp Glu Leu Thr Lys Asn Gln Val Ser Leu Thr
   305 310 315 320
   Cys Leu Val Lys Gly Phe Tyr Pro Ser Asp Ile Ala Val Glu Trp Glu
   325 330 335
   Ser Asn Gly Gln Pro Glu Asn Asn Tyr Lys Thr Thr Pro Pro Val Leu
   340 345 350
   Asp Ser Asp Gly Ser Phe Phe Leu Tyr Ser Lys Leu Thr Val Asp Lys
   355 360 365
   Ser Arg Trp Gln Gln Gly Asn Val Phe Ser Cys Ser Val Met His Glu
   370 375 380
   Ala Leu His Asn His Tyr Thr Gln Lys Ser Leu Ser Leu Ser Pro Gly
   385 390 395 400
   Lys
<210> 67
   <211> 406
   <212> PRT
   <213> Artificial Sequence
<220>
   <223> Fusion protein: human C1ORF32-ECD fused to human IgG1 Fc
   with G4Sx2 linker, with C220S mutation
<400> 67
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Gly Gly Gly Gly Ser Gly Gly Gly Gly Ser Glu Pro
                   165                 170                 175
   Lys Ser Ser Asp Lys Thr His Thr Cys Pro Pro Cys Pro Ala Pro Glu
               180                 185                 190
   Leu Leu Gly Gly Pro Ser Val Phe Leu Phe Pro Pro Lys Pro Lys Asp
           195                 200                 205
   Thr Leu Met Ile Ser Arg Thr Pro Glu Val Thr Cys Val Val Val Asp
       210                 215                 220
   Val Ser His Glu Asp Pro Glu Val Lys Phe Asn Trp Tyr Val Asp Gly
   225                 230                 235                 240
   Val Glu Val His Asn Ala Lys Thr Lys Pro Arg Glu Glu Gln Tyr Asn
                   245                 250                 255
   Ser Thr Tyr Arg Val Val Ser Val Leu Thr Val Leu His Gln Asp Trp
               260                 265                 270
   Leu Asn Gly Lys Glu Tyr Lys Cys Lys Val Ser Asn Lys Ala Leu Pro
   275 280 285
   Ala Pro Ile Glu Lys Thr Ile Ser Lys Ala Lys Gly Gln Pro Arg Glu
   290 295 300
   Pro Gln Val Tyr Thr Leu Pro Pro Ser Arg Asp Glu Leu Thr Lys Asn
   305 310 315 320
   Gln Val Ser Leu Thr Cys Leu Val Lys Gly Phe Tyr Pro Ser Asp Ile
   325 330 335
   Ala Val Glu Trp Glu Ser Asn Gly Gln Pro Glu Asn Asn Tyr Lys Thr
   340 345 350
   Thr Pro Pro Val Leu Asp Ser Asp Gly Ser Phe Phe Leu Tyr Ser Lys
   355 360 365
   Leu Thr Val Asp Lys Ser Arg Trp Gln Gln Gly Asn Val Phe Ser Cys
   370 375 380
   Ser Val Met His Glu Ala Leu His Asn His Tyr Thr Gln Lys Ser Leu
   385 390 395 400
   Ser Leu Ser Pro Gly Lys
   405
<210> 68
   <211> 416
   <212> PRT
   <213> Artificial Sequence
<220>
   <223> Fusion protein: human C1ORF32-ECD fused to human IgG1 Fc
   with G4Sx4 linker, with C220S mutation
<400> 68
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Gly Gly Gly Gly Ser Gly Gly Gly Gly Ser Gly Gly
                   165                 170                 175
   Gly Gly Ser Gly Gly Gly Gly Ser Glu Pro Lys Ser Ser Asp Lys Thr
               180                 185                 190
   His Thr Cys Pro Pro Cys Pro Ala Pro Glu Leu Leu Gly Gly Pro Ser
           195                 200                 205
   Val Phe Leu Phe Pro Pro Lys Pro Lys Asp Thr Leu Met Ile Ser Arg
       210                 215                 220
   Thr Pro Glu Val Thr Cys Val Val Val Asp Val Ser His Glu Asp Pro
   225                 230                 235                 240
   Glu Val Lys Phe Asn Trp Tyr Val Asp Gly Val Glu Val His Asn Ala
                   245                 250                 255
   Lys Thr Lys Pro Arg Glu Glu Gln Tyr Asn Ser Thr Tyr Arg Val Val
               260                 265                 270
   Ser Val Leu Thr Val Leu His Gln Asp Trp Leu Asn Gly Lys Glu Tyr
           275                 280                 285
   Lys Cys Lys Val Ser Asn Lys Ala Leu Pro Ala Pro Ile Glu Lys Thr
       290                 295                 300
   Ile Ser Lys Ala Lys Gly Gln Pro Arg Glu Pro Gln Val Tyr Thr Leu
   305                 310                 315                 320
   Pro Pro Ser Arg Asp Glu Leu Thr Lys Asn Gln Val Ser Leu Thr Cys
                   325                 330                 335
   Leu Val Lys Gly Phe Tyr Pro Ser Asp Ile Ala Val Glu Trp Glu Ser
               340                 345                 350
   Asn Gly Gln Pro Glu Asn Asn Tyr Lys Thr Thr Pro Pro Val Leu Asp
           355                 360                 365
   Ser Asp Gly Ser Phe Phe Leu Tyr Ser Lys Leu Thr Val Asp Lys Ser
       370                 375                 380
   Arg Trp Gln Gln Gly Asn Val Phe Ser Cys Ser Val Met His Glu Ala
   385                 390                 395                 400
   Leu His Asn His Tyr Thr Gln Lys Ser Leu Ser Leu Ser Pro Gly Lys
                   405                 410                 415
<210> 69
   <211> 396
   <212> PRT
   <213> Artificial Sequence
<220>
   <223> Fusion protein: human C1ORF32-ECD fused to human IgG1 Fc
   no linker, with C220S and N297A mutations
<400> 69
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Glu Pro Lys Ser Ser Asp Lys Thr His Thr Cys Pro
                   165                 170                 175
   Pro Cys Pro Ala Pro Glu Leu Leu Gly Gly Pro Ser Val Phe Leu Phe
               180                 185                 190
   Pro Pro Lys Pro Lys Asp Thr Leu Met Ile Ser Arg Thr Pro Glu Val
           195                 200                 205
   Thr Cys Val Val Val Asp Val Ser His Glu Asp Pro Glu Val Lys Phe
       210                 215                 220
   Asn Trp Tyr Val Asp Gly Val Glu Val His Asn Ala Lys Thr Lys Pro
   225                 230                 235                 240
   Arg Glu Glu Gln Tyr Ala Ser Thr Tyr Arg Val Val Ser Val Leu Thr
                   245                 250                 255
   Val Leu His Gln Asp Trp Leu Asn Gly Lys Glu Tyr Lys Cys Lys Val
               260                 265                 270
   Ser Asn Lys Ala Leu Pro Ala Pro Ile Glu Lys Thr Ile Ser Lys Ala
           275                 280                 285
   Lys Gly Gln Pro Arg Glu Pro Gln Val Tyr Thr Leu Pro Pro Ser Arg
       290                 295                 300
   Asp Glu Leu Thr Lys Asn Gln Val Ser Leu Thr Cys Leu Val Lys Gly
   305                 310                 315                 320
   Phe Tyr Pro Ser Asp Ile Ala Val Glu Trp Glu Ser Asn Gly Gln Pro
                   325                 330                 335
   Glu Asn Asn Tyr Lys Thr Thr Pro Pro Val Leu Asp Ser Asp Gly Ser
               340                 345                 350
   Phe Phe Leu Tyr Ser Lys Leu Thr Val Asp Lys Ser Arg Trp Gln Gln
           355                 360                 365
   Gly Asn Val Phe Ser Cys Ser Val Met His Glu Ala Leu His Asn His
       370                 375                 380
   Tyr Thr Gln Lys Ser Leu Ser Leu Ser Pro Gly Lys
   385                 390                 395
<210> 70
   <211> 401
   <212> PRT
   <213> Artificial Sequence
<220>
   <223> Fusion protein: human C1ORF32-ECD fused to human IgG1 Fc
   with G4Sx1 linker, with C220S and N297A mutations
<400> 70
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Gly Gly Gly Gly Ser Glu Pro Lys Ser Ser Asp Lys
   165 170 175
   Thr His Thr Cys Pro Pro Cys Pro Ala Pro Glu Leu Leu Gly Gly Pro
   180 185 190
   Ser Val Phe Leu Phe Pro Pro Lys Pro Lys Asp Thr Leu Met Ile Ser
   195 200 205
   Arg Thr Pro Glu Val Thr Cys Val Val Val Asp Val Ser His Glu Asp
   210 215 220
   Pro Glu Val Lys Phe Asn Trp Tyr Val Asp Gly Val Glu Val His Asn
   225 230 235 240
   Ala Lys Thr Lys Pro Arg Glu Glu Gln Tyr Ala Ser Thr Tyr Arg Val
   245 250 255
   Val Ser Val Leu Thr Val Leu His Gln Asp Trp Leu Asn Gly Lys Glu
   260 265 270
   Tyr Lys Cys Lys Val Ser Asn Lys Ala Leu Pro Ala Pro Ile Glu Lys
   275 280 285
   Thr Ile Ser Lys Ala Lys Gly Gln Pro Arg Glu Pro Gln Val Tyr Thr
   290 295 300
   Leu Pro Pro Ser Arg Asp Glu Leu Thr Lys Asn Gln Val Ser Leu Thr
   305 310 315 320
   Cys Leu Val Lys Gly Phe Tyr Pro Ser Asp Ile Ala Val Glu Trp Glu
   325 330 335
   Ser Asn Gly Gln Pro Glu Asn Asn Tyr Lys Thr Thr Pro Pro Val Leu
   340 345 350
   Asp Ser Asp Gly Ser Phe Phe Leu Tyr Ser Lys Leu Thr Val Asp Lys
   355 360 365
   Ser Arg Trp Gln Gln Gly Asn Val Phe Ser Cys Ser Val Met His Glu
   370 375 380
   Ala Leu His Asn His Tyr Thr Gln Lys Ser Leu Ser Leu Ser Pro Gly
   385 390 395 400
   Lys
<210> 71
   <211> 406
   <212> PRT
   <213> Artificial Sequence
<220>
   <223> Fusion protein: human C1ORF32-ECD fused to human IgG1 Fc
   with G4Sx2 linker, with C220S and N297A mutations
<400> 71
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Gly Gly Gly Gly Ser Gly Gly Gly Gly Ser Glu Pro
                   165                 170                 175
   Lys Ser Ser Asp Lys Thr His Thr Cys Pro Pro Cys Pro Ala Pro Glu
               180                 185                 190
   Leu Leu Gly Gly Pro Ser Val Phe Leu Phe Pro Pro Lys Pro Lys Asp
           195                 200                 205
   Thr Leu Met Ile Ser Arg Thr Pro Glu Val Thr Cys Val Val Val Asp
       210                 215                 220
   Val Ser His Glu Asp Pro Glu Val Lys Phe Asn Trp Tyr Val Asp Gly
   225                 230                 235                 240
   Val Glu Val His Asn Ala Lys Thr Lys Pro Arg Glu Glu Gln Tyr Ala
                   245                 250                 255
   Ser Thr Tyr Arg Val Val Ser Val Leu Thr Val Leu His Gln Asp Trp
               260                 265                 270
   Leu Asn Gly Lys Glu Tyr Lys Cys Lys Val Ser Asn Lys Ala Leu Pro
           275                 280                 285
   Ala Pro Ile Glu Lys Thr Ile Ser Lys Ala Lys Gly Gln Pro Arg Glu
       290                 295                 300
   Pro Gln Val Tyr Thr Leu Pro Pro Ser Arg Asp Glu Leu Thr Lys Asn
   305                 310                 315                 320
   Gln Val Ser Leu Thr Cys Leu Val Lys Gly Phe Tyr Pro Ser Asp Ile
                   325                 330                 335
   Ala Val Glu Trp Glu Ser Asn Gly Gln Pro Glu Asn Asn Tyr Lys Thr
               340                 345                 350
   Thr Pro Pro Val Leu Asp Ser Asp Gly Ser Phe Phe Leu Tyr Ser Lys
           355                 360                 365
   Leu Thr Val Asp Lys Ser Arg Trp Gln Gln Gly Asn Val Phe Ser Cys
       370                 375                 380
   Ser Val Met His Glu Ala Leu His Asn His Tyr Thr Gln Lys Ser Leu
   385                 390                 395                 400
   Ser Leu Ser Pro Gly Lys
                   405
<210> 72
   <211> 416
   <212> PRT
   <213> Artificial Sequence
<220>
   <223> Fusion protein: human C1ORF32-ECD fused to human IgG1 Fc
   with G4Sx4 linker, with C220S and N297A mutations
<400> 72
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Arg Thr Gly Leu Leu Ala Asp Leu Leu Pro Ser Phe Ala
   145                 150                 155                 160
   Val Glu Ile Met Gly Gly Gly Gly Ser Gly Gly Gly Gly Ser Gly Gly
                   165                 170                 175
   Gly Gly Ser Gly Gly Gly Gly Ser Glu Pro Lys Ser Ser Asp Lys Thr
               180                 185                 190
   His Thr Cys Pro Pro Cys Pro Ala Pro Glu Leu Leu Gly Gly Pro Ser
           195                 200                 205
   Val Phe Leu Phe Pro Pro Lys Pro Lys Asp Thr Leu Met Ile Ser Arg
       210                 215                 220
   Thr Pro Glu Val Thr Cys Val Val Val Asp Val Ser His Glu Asp Pro
   225                 230                 235                 240
   Glu Val Lys Phe Asn Trp Tyr Val Asp Gly Val Glu Val His Asn Ala
                   245                 250                 255
   Lys Thr Lys Pro Arg Glu Glu Gln Tyr Ala Ser Thr Tyr Arg Val Val
               260                 265                 270
   Ser Val Leu Thr Val Leu His Gln Asp Trp Leu Asn Gly Lys Glu Tyr
           275                 280                 285
   Lys Cys Lys Val Ser Asn Lys Ala Leu Pro Ala Pro Ile Glu Lys Thr
       290                 295                 300
   Ile Ser Lys Ala Lys Gly Gln Pro Arg Glu Pro Gln Val Tyr Thr Leu
   305                 310                 315                 320
   Pro Pro Ser Arg Asp Glu Leu Thr Lys Asn Gln Val Ser Leu Thr Cys
                   325                 330                 335
   Leu Val Lys Gly Phe Tyr Pro Ser Asp Ile Ala Val Glu Trp Glu Ser
               340                 345                 350
   Asn Gly Gln Pro Glu Asn Asn Tyr Lys Thr Thr Pro Pro Val Leu Asp
           355                 360                 365
   Ser Asp Gly Ser Phe Phe Leu Tyr Ser Lys Leu Thr Val Asp Lys Ser
       370                 375                 380
   Arg Trp Gln Gln Gly Asn Val Phe Ser Cys Ser Val Met His Glu Ala
   385                 390                 395                 400
   Leu His Asn His Tyr Thr Gln Lys Ser Leu Ser Leu Ser Pro Gly Lys
                   405                 410                 415
<210> 73
   <211> 379
   <212> PRT
   <213> Artificial Sequence
<220>
   <223> Fusion protein: human C1ORF32-ECD fused to human IgG1 Fc
   no linker, with C220S mutation
<400> 73
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Glu Pro Lys Ser Ser Asp Lys Thr His Thr Cys Pro Pro
   145 150 155 160
   Cys Pro Ala Pro Glu Leu Leu Gly Gly Pro Ser Val Phe Leu Phe Pro
   165 170 175
   Pro Lys Pro Lys Asp Thr Leu Met Ile Ser Arg Thr Pro Glu Val Thr
   180 185 190
   Cys Val Val Val Asp Val Ser His Glu Asp Pro Glu Val Lys Phe Asn
   195 200 205
   Trp Tyr Val Asp Gly Val Glu Val His Asn Ala Lys Thr Lys Pro Arg
   210 215 220
   Glu Glu Gln Tyr Asn Ser Thr Tyr Arg Val Val Ser Val Leu Thr Val
   225 230 235 240
   Leu His Gln Asp Trp Leu Asn Gly Lys Glu Tyr Lys Cys Lys Val Ser
   245 250 255
   Asn Lys Ala Leu Pro Ala Pro Ile Glu Lys Thr Ile Ser Lys Ala Lys
   260 265 270
   Gly Gln Pro Arg Glu Pro Gln Val Tyr Thr Leu Pro Pro Ser Arg Asp
   275 280 285
   Glu Leu Thr Lys Asn Gln Val Ser Leu Thr Cys Leu Val Lys Gly Phe
   290 295 300
   Tyr Pro Ser Asp Ile Ala Val Glu Trp Glu Ser Asn Gly Gln Pro Glu
   305 310 315 320
   Asn Asn Tyr Lys Thr Thr Pro Pro Val Leu Asp Ser Asp Gly Ser Phe
   325 330 335
   Phe Leu Tyr Ser Lys Leu Thr Val Asp Lys Ser Arg Trp Gln Gln Gly
   340 345 350
   Asn Val Phe Ser Cys Ser Val Met His Glu Ala Leu His Asn His Tyr
   355 360 365
   Thr Gln Lys Ser Leu Ser Leu Ser Pro Gly Lys
   370 375
<210> 74
   <211> 384
   <212> PRT
   <213> Artificial Sequence
<220>
   <223> Fusion protein: human C1ORF32-ECD fused to human IgG1 Fc
   with G4Sx1 linker, with C220S mutation
<400> 74
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Gly Gly Gly Gly Ser Glu Pro Lys Ser Ser Asp Lys Thr
   145                 150                 155                 160
   His Thr Cys Pro Pro Cys Pro Ala Pro Glu Leu Leu Gly Gly Pro Ser
                   165                 170                 175
   Val Phe Leu Phe Pro Pro Lys Pro Lys Asp Thr Leu Met Ile Ser Arg
               180                 185                 190
   Thr Pro Glu Val Thr Cys Val Val Val Asp Val Ser His Glu Asp Pro
           195                 200                 205
   Glu Val Lys Phe Asn Trp Tyr Val Asp Gly Val Glu Val His Asn Ala
       210                 215                 220
   Lys Thr Lys Pro Arg Glu Glu Gln Tyr Asn Ser Thr Tyr Arg Val Val
   225                 230                 235                 240
   Ser Val Leu Thr Val Leu His Gln Asp Trp Leu Asn Gly Lys Glu Tyr
                   245                 250                 255
   Lys Cys Lys Val Ser Asn Lys Ala Leu Pro Ala Pro Ile Glu Lys Thr
               260                 265                 270
   Ile Ser Lys Ala Lys Gly Gln Pro Arg Glu Pro Gln Val Tyr Thr Leu
           275                 280                 285
   Pro Pro Ser Arg Asp Glu Leu Thr Lys Asn Gln Val Ser Leu Thr Cys
       290                 295                 300
   Leu Val Lys Gly Phe Tyr Pro Ser Asp Ile Ala Val Glu Trp Glu Ser
   305                 310                 315                 320
   Asn Gly Gln Pro Glu Asn Asn Tyr Lys Thr Thr Pro Pro Val Leu Asp
                   325                 330                 335
   Ser Asp Gly Ser Phe Phe Leu Tyr Ser Lys Leu Thr Val Asp Lys Ser
               340                 345                 350
   Arg Trp Gln Gln Gly Asn Val Phe Ser Cys Ser Val Met His Glu Ala
   355 360 365
   Leu His Asn His Tyr Thr Gln Lys Ser Leu Ser Leu Ser Pro Gly Lys
   370 375 380
<210> 75
   <211> 389
   <212> PRT
   <213> Artificial Sequence
<220>
   <223> Fusion protein: human C1ORF32-ECD fused to human IgG1 Fc
   with G4Sx2 linker, with C220S mutation
<400> 75
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Gly Gly Gly Gly Ser Gly Gly Gly Gly Ser Glu Pro Lys
   145 150 155 160
   Ser Ser Asp Lys Thr His Thr Cys Pro Pro Cys Pro Ala Pro Glu Leu
   165 170 175
   Leu Gly Gly Pro Ser Val Phe Leu Phe Pro Pro Lys Pro Lys Asp Thr
   180 185 190
   Leu Met Ile Ser Arg Thr Pro Glu Val Thr Cys Val Val Val Asp Val
   195 200 205
   Ser His Glu Asp Pro Glu Val Lys Phe Asn Trp Tyr Val Asp Gly Val
   210 215 220
   Glu Val His Asn Ala Lys Thr Lys Pro Arg Glu Glu Gln Tyr Asn Ser
   225 230 235 240
   Thr Tyr Arg Val Val Ser Val Leu Thr Val Leu His Gln Asp Trp Leu
   245 250 255
   Asn Gly Lys Glu Tyr Lys Cys Lys Val Ser Asn Lys Ala Leu Pro Ala
   260 265 270
   Pro Ile Glu Lys Thr Ile Ser Lys Ala Lys Gly Gln Pro Arg Glu Pro
   275 280 285
   Gln Val Tyr Thr Leu Pro Pro Ser Arg Asp Glu Leu Thr Lys Asn Gln
   290 295 300
   Val Ser Leu Thr Cys Leu Val Lys Gly Phe Tyr Pro Ser Asp Ile Ala
   305 310 315 320
   Val Glu Trp Glu Ser Asn Gly Gln Pro Glu Asn Asn Tyr Lys Thr Thr
   325 330 335
   Pro Pro Val Leu Asp Ser Asp Gly Ser Phe Phe Leu Tyr Ser Lys Leu
   340 345 350
   Thr Val Asp Lys Ser Arg Trp Gln Gln Gly Asn Val Phe Ser Cys Ser
   355 360 365
   Val Met His Glu Ala Leu His Asn His Tyr Thr Gln Lys Ser Leu Ser
   370 375 380
   Leu Ser Pro Gly Lys
   385
<210> 76
   <211> 399
   <212> PRT
   <213> Artificial Sequence
<220>
   <223> Fusion protein: human C1ORF32-ECD fused to human IgG1 Fc
   with G4Sx4 linker, with C220S mutation
<400> 76
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Gly Gly Gly Gly Ser Gly Gly Gly Gly Ser Gly Gly Gly
   145                 150                 155                 160
   Gly Ser Gly Gly Gly Gly Ser Glu Pro Lys Ser Ser Asp Lys Thr His
                   165                 170                 175
   Thr Cys Pro Pro Cys Pro Ala Pro Glu Leu Leu Gly Gly Pro Ser Val
               180                 185                 190
   Phe Leu Phe Pro Pro Lys Pro Lys Asp Thr Leu Met Ile Ser Arg Thr
           195                 200                 205
   Pro Glu Val Thr Cys Val Val Val Asp Val Ser His Glu Asp Pro Glu
       210                 215                 220
   Val Lys Phe Asn Trp Tyr Val Asp Gly Val Glu Val His Asn Ala Lys
   225 230 235 240
   Thr Lys Pro Arg Glu Glu Gln Tyr Asn Ser Thr Tyr Arg Val Val Ser
   245 250 255
   Val Leu Thr Val Leu His Gln Asp Trp Leu Asn Gly Lys Glu Tyr Lys
   260 265 270
   Cys Lys Val Ser Asn Lys Ala Leu Pro Ala Pro Ile Glu Lys Thr Ile
   275 280 285
   Ser Lys Ala Lys Gly Gln Pro Arg Glu Pro Gln Val Tyr Thr Leu Pro
   290 295 300
   Pro Ser Arg Asp Glu Leu Thr Lys Asn Gln Val Ser Leu Thr Cys Leu
   305 310 315 320
   Val Lys Gly Phe Tyr Pro Ser Asp Ile Ala Val Glu Trp Glu Ser Asn
   325 330 335
   Gly Gln Pro Glu Asn Asn Tyr Lys Thr Thr Pro Pro Val Leu Asp Ser
   340 345 350
   Asp Gly Ser Phe Phe Leu Tyr Ser Lys Leu Thr Val Asp Lys Ser Arg
   355 360 365
   Trp Gln Gln Gly Asn Val Phe Ser Cys Ser Val Met His Glu Ala Leu
   370 375 380
   His Asn His Tyr Thr Gln Lys Ser Leu Ser Leu Ser Pro Gly Lys
   385 390 395
<210> 77
   <211> 379
   <212> PRT
   <213> Artificial Sequence
<220>
   <223> Fusion protein: human C1ORF32-ECD fused to human IgG1 Fc
   no linker, with C220S and N297A mutations
<400> 77
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Glu Pro Lys Ser Ser Asp Lys Thr His Thr Cys Pro Pro
   145 150 155 160
   Cys Pro Ala Pro Glu Leu Leu Gly Gly Pro Ser Val Phe Leu Phe Pro
   165 170 175
   Pro Lys Pro Lys Asp Thr Leu Met Ile Ser Arg Thr Pro Glu Val Thr
   180 185 190
   Cys Val Val Val Asp Val Ser His Glu Asp Pro Glu Val Lys Phe Asn
   195 200 205
   Trp Tyr Val Asp Gly Val Glu Val His Asn Ala Lys Thr Lys Pro Arg
   210 215 220
   Glu Glu Gln Tyr Ala Ser Thr Tyr Arg Val Val Ser Val Leu Thr Val
   225 230 235 240
   Leu His Gln Asp Trp Leu Asn Gly Lys Glu Tyr Lys Cys Lys Val Ser
   245 250 255
   Asn Lys Ala Leu Pro Ala Pro Ile Glu Lys Thr Ile Ser Lys Ala Lys
   260 265 270
   Gly Gln Pro Arg Glu Pro Gln Val Tyr Thr Leu Pro Pro Ser Arg Asp
           275                 280                 285
   Glu Leu Thr Lys Asn Gln Val Ser Leu Thr Cys Leu Val Lys Gly Phe
       290                 295                 300
   Tyr Pro Ser Asp Ile Ala Val Glu Trp Glu Ser Asn Gly Gln Pro Glu
   305                 310                 315                 320
   Asn Asn Tyr Lys Thr Thr Pro Pro Val Leu Asp Ser Asp Gly Ser Phe
                   325                 330                 335
   Phe Leu Tyr Ser Lys Leu Thr Val Asp Lys Ser Arg Trp Gln Gln Gly
               340                 345                 350
   Asn Val Phe Ser Cys Ser Val Met His Glu Ala Leu His Asn His Tyr
           355                 360                 365
   Thr Gln Lys Ser Leu Ser Leu Ser Pro Gly Lys
       370                 375
<210> 78
   <211> 384
   <212> PRT
   <213> Artificial Sequence
<220>
   <223> Fusion protein: human C1ORF32-ECD fused to human IgG1 Fc
   with G4Sx1 linker, with C220S and N297A mutations
<400> 78
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Gly Gly Gly Gly Ser Glu Pro Lys Ser Ser Asp Lys Thr
   145                 150                 155                 160
   His Thr Cys Pro Pro Cys Pro Ala Pro Glu Leu Leu Gly Gly Pro Ser
                   165                 170                 175
   Val Phe Leu Phe Pro Pro Lys Pro Lys Asp Thr Leu Met Ile Ser Arg
               180                 185                 190
   Thr Pro Glu Val Thr Cys Val Val Val Asp Val Ser His Glu Asp Pro
           195                 200                 205
   Glu Val Lys Phe Asn Trp Tyr Val Asp Gly Val Glu Val His Asn Ala
       210                 215                 220
   Lys Thr Lys Pro Arg Glu Glu Gln Tyr Ala Ser Thr Tyr Arg Val Val
   225                 230                 235                 240
   Ser Val Leu Thr Val Leu His Gln Asp Trp Leu Asn Gly Lys Glu Tyr
                   245                 250                 255
   Lys Cys Lys Val Ser Asn Lys Ala Leu Pro Ala Pro Ile Glu Lys Thr
               260                 265                 270
   Ile Ser Lys Ala Lys Gly Gln Pro Arg Glu Pro Gln Val Tyr Thr Leu
           275                 280                 285
   Pro Pro Ser Arg Asp Glu Leu Thr Lys Asn Gln Val Ser Leu Thr Cys
       290                 295                 300
   Leu Val Lys Gly Phe Tyr Pro Ser Asp Ile Ala Val Glu Trp Glu Ser
   305                 310                 315                 320
   Asn Gly Gln Pro Glu Asn Asn Tyr Lys Thr Thr Pro Pro Val Leu Asp
                   325                 330                 335
   Ser Asp Gly Ser Phe Phe Leu Tyr Ser Lys Leu Thr Val Asp Lys Ser
               340                 345                 350
   Arg Trp Gln Gln Gly Asn Val Phe Ser Cys Ser Val Met His Glu Ala
   355 360 365
   Leu His Asn His Tyr Thr Gln Lys Ser Leu Ser Leu Ser Pro Gly Lys
   370 375 380
<210> 79
   <211> 389
   <212> PRT
   <213> Artificial Sequence
<220>
   <223> Fusion protein: human C1ORF32-ECD fused to human IgG1 Fc
   with G4Sx2 linker, with C220S and N297A mutations
<400> 79
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Gly Gly Gly Gly Gly Ser Gly Gly Gly Gly Ser Glu Pro Lys
   145 150 155 160
   Ser Ser Asp Lys Thr His Thr Cys Pro Pro Cys Pro Ala Pro Glu Leu
   165 170 175
   Leu Gly Gly Pro Ser Val Phe Leu Phe Pro Pro Lys Pro Lys Asp Thr
               180                 185                 190
   Leu Met Ile Ser Arg Thr Pro Glu Val Thr Cys Val Val Val Asp Val
           195                 200                 205
   Ser His Glu Asp Pro Glu Val Lys Phe Asn Trp Tyr Val Asp Gly Val
       210                 215                 220
   Glu Val His Asn Ala Lys Thr Lys Pro Arg Glu Glu Gln Tyr Ala Ser
   225                 230                 235                 240
   Thr Tyr Arg Val Val Ser Val Leu Thr Val Leu His Gln Asp Trp Leu
                   245                 250                 255
   Asn Gly Lys Glu Tyr Lys Cys Lys Val Ser Asn Lys Ala Leu Pro Ala
               260                 265                 270
   Pro Ile Glu Lys Thr Ile Ser Lys Ala Lys Gly Gln Pro Arg Glu Pro
           275                 280                 285
   Gln Val Tyr Thr Leu Pro Pro Ser Arg Asp Glu Leu Thr Lys Asn Gln
       290                 295                 300
   Val Ser Leu Thr Cys Leu Val Lys Gly Phe Tyr Pro Ser Asp Ile Ala
   305                 310                 315                 320
   Val Glu Trp Glu Ser Asn Gly Gln Pro Glu Asn Asn Tyr Lys Thr Thr
                   325                 330                 335
   Pro Pro Val Leu Asp Ser Asp Gly Ser Phe Phe Leu Tyr Ser Lys Leu
               340                 345                 350
   Thr Val Asp Lys Ser Arg Trp Gln Gln Gly Asn Val Phe Ser Cys Ser
           355                 360                 365
   Val Met His Glu Ala Leu His Asn His Tyr Thr Gln Lys Ser Leu Ser
       370                 375                 380
   Leu Ser Pro Gly Lys
   385
<210> 80
   <211> 399
   <212> PRT
   <213> Artificial Sequence
<220>
   <223> Fusion protein: human C1ORF32-ECD fused to human IgG1 Fc
   with G4Sx4 linker, with C220S and N297A mutations
<400> 80
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Gly Gly Gly Gly Gly Ser Gly Gly Gly Gly Ser Gly Gly Gly
   145                 150                 155                 160
   Gly Ser Gly Gly Gly Gly Ser Glu Pro Lys Ser Ser Asp Lys Thr His
                   165                 170                 175
   Thr Cys Pro Pro Cys Pro Ala Pro Glu Leu Leu Gly Gly Pro Ser Val
               180                 185                 190
   Phe Leu Phe Pro Pro Lys Pro Lys Asp Thr Leu Met Ile Ser Arg Thr
           195                 200                 205
   Pro Glu Val Thr Cys Val Val Val Asp Val Ser His Glu Asp Pro Glu
       210                 215                 220
   Val Lys Phe Asn Trp Tyr Val Asp Gly Val Glu Val His Asn Ala Lys
   225 230 235 240
   Thr Lys Pro Arg Glu Glu Gln Tyr Ala Ser Thr Tyr Arg Val Val Ser
   245 250 255
   Val Leu Thr Val Leu His Gln Asp Trp Leu Asn Gly Lys Glu Tyr Lys
   260 265 270
   Cys Lys Val Ser Asn Lys Ala Leu Pro Ala Pro Ile Glu Lys Thr Ile
   275 280 285
   Ser Lys Ala Lys Gly Gln Pro Arg Glu Pro Gln Val Tyr Thr Leu Pro
   290 295 300
   Pro Ser Arg Asp Glu Leu Thr Lys Asn Gln Val Ser Leu Thr Cys Leu
   305 310 315 320
   Val Lys Gly Phe Tyr Pro Ser Asp Ile Ala Val Glu Trp Glu Ser Asn
   325 330 335
   Gly Gln Pro Glu Asn Asn Tyr Lys Thr Thr Pro Pro Val Leu Asp Ser
   340 345 350
   Asp Gly Ser Phe Phe Leu Tyr Ser Lys Leu Thr Val Asp Lys Ser Arg
   355 360 365
   Trp Gln Gln Gly Asn Val Phe Ser Cys Ser Val Met His Glu Ala Leu
   370 375 380
   His Asn His Tyr Thr Gln Lys Ser Leu Ser Leu Ser Pro Gly Lys
   385 390 395
<210> 81
   <211> 379
   <212> PRT
   <213> Artificial Sequence
<220>
   <223> Fusion protein: human C1ORF32-ECD fused to human IgG1 Fc
   no linker, with C220S mutation
<400> 81
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu Glu Pro Lys Ser Ser Asp Lys Thr His Thr Cys Pro Pro
   145                 150                 155                 160
   Cys Pro Ala Pro Glu Leu Leu Gly Gly Pro Ser Val Phe Leu Phe Pro
                   165                 170                 175
   Pro Lys Pro Lys Asp Thr Leu Met Ile Ser Arg Thr Pro Glu Val Thr
               180                 185                 190
   Cys Val Val Val Asp Val Ser His Glu Asp Pro Glu Val Lys Phe Asn
           195                 200                 205
   Trp Tyr Val Asp Gly Val Glu Val His Asn Ala Lys Thr Lys Pro Arg
       210                 215                 220
   Glu Glu Gln Tyr Asn Ser Thr Tyr Arg Val Val Ser Val Leu Thr Val
   225                 230                 235                 240
   Leu His Gln Asp Trp Leu Asn Gly Lys Glu Tyr Lys Cys Lys Val Ser
                   245                 250                 255
   Asn Lys Ala Leu Pro Ala Pro Ile Glu Lys Thr Ile Ser Lys Ala Lys
               260                 265                 270
   Gly Gln Pro Arg Glu Pro Gln Val Tyr Thr Leu Pro Pro Ser Arg Asp
   275 280 285
   Glu Leu Thr Lys Asn Gln Val Ser Leu Thr Cys Leu Val Lys Gly Phe
   290 295 300
   Tyr Pro Ser Asp Ile Ala Val Glu Trp Glu Ser Asn Gly Gln Pro Glu
   305 310 315 320
   Asn Asn Tyr Lys Thr Thr Pro Pro Val Leu Asp Ser Asp Gly Ser Phe
   325 330 335
   Phe Leu Tyr Ser Lys Leu Thr Val Asp Lys Ser Arg Trp Gln Gln Gly
   340 345 350
   Asn Val Phe Ser Cys Ser Val Met His Glu Ala Leu His Asn His Tyr
   355 360 365
   Thr Gln Lys Ser Leu Ser Leu Ser Pro Gly Lys
   370 375
<210> 82
   <211> 384
   <212> PRT
   <213> Artificial Sequence
<220>
   <223> Fusion protein: human C1ORF32-ECD fused to human IgG1 Fc
   with G4Sx1 linker, with C220S mutation
<400> 82
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu Gly Gly Gly Gly Ser Glu Pro Lys Ser Ser Asp Lys Thr
   145                 150                 155                 160
   His Thr Cys Pro Pro Cys Pro Ala Pro Glu Leu Leu Gly Gly Pro Ser
                   165                 170                 175
   Val Phe Leu Phe Pro Pro Lys Pro Lys Asp Thr Leu Met Ile Ser Arg
               180                 185                 190
   Thr Pro Glu Val Thr Cys Val Val Val Asp Val Ser His Glu Asp Pro
           195                 200                 205
   Glu Val Lys Phe Asn Trp Tyr Val Asp Gly Val Glu Val His Asn Ala
       210                 215                 220
   Lys Thr Lys Pro Arg Glu Glu Gln Tyr Asn Ser Thr Tyr Arg Val Val
   225                 230                 235                 240
   Ser Val Leu Thr Val Leu His Gln Asp Trp Leu Asn Gly Lys Glu Tyr
                   245                 250                 255
   Lys Cys Lys Val Ser Asn Lys Ala Leu Pro Ala Pro Ile Glu Lys Thr
               260                 265                 270
   Ile Ser Lys Ala Lys Gly Gln Pro Arg Glu Pro Gln Val Tyr Thr Leu
           275                 280                 285
   Pro Pro Ser Arg Asp Glu Leu Thr Lys Asn Gln Val Ser Leu Thr Cys
       290                 295                 300
   Leu Val Lys Gly Phe Tyr Pro Ser Asp Ile Ala Val Glu Trp Glu Ser
   305                 310                 315                 320
   Asn Gly Gln Pro Glu Asn Asn Tyr Lys Thr Thr Pro Pro Val Leu Asp
                   325                 330                 335
   Ser Asp Gly Ser Phe Phe Leu Tyr Ser Lys Leu Thr Val Asp Lys Ser
               340                 345                 350
   Arg Trp Gln Gln Gly Asn Val Phe Ser Cys Ser Val Met His Glu Ala
   355 360 365
   Leu His Asn His Tyr Thr Gln Lys Ser Leu Ser Leu Ser Pro Gly Lys
   370 375 380
<210> 83
   <211> 389
   <212> PRT
   <213> Artificial Sequence
<220>
   <223> Fusion protein: human C1ORF32-ECD fused to human IgG1 Fc
   with G4Sx2 linker, with C220S mutation
<400> 83
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Glu Gly Gly Gly Gly Ser Gly Gly Gly Gly Ser Glu Pro Lys
   145 150 155 160
   Ser Ser Asp Lys Thr His Thr Cys Pro Pro Cys Pro Ala Pro Glu Leu
   165 170 175
   Leu Gly Gly Pro Ser Val Phe Leu Phe Pro Pro Lys Pro Lys Asp Thr
               180                 185                 190
   Leu Met Ile Ser Arg Thr Pro Glu Val Thr Cys Val Val Val Asp Val
           195                 200                 205
   Ser His Glu Asp Pro Glu Val Lys Phe Asn Trp Tyr Val Asp Gly Val
       210                 215                 220
   Glu Val His Asn Ala Lys Thr Lys Pro Arg Glu Glu Gln Tyr Asn Ser
   225                 230                 235                 240
   Thr Tyr Arg Val Val Ser Val Leu Thr Val Leu His Gln Asp Trp Leu
                   245                 250                 255
   Asn Gly Lys Glu Tyr Lys Cys Lys Val Ser Asn Lys Ala Leu Pro Ala
               260                 265                 270
   Pro Ile Glu Lys Thr Ile Ser Lys Ala Lys Gly Gln Pro Arg Glu Pro
           275                 280                 285
   Gln Val Tyr Thr Leu Pro Pro Ser Arg Asp Glu Leu Thr Lys Asn Gln
       290                 295                 300
   Val Ser Leu Thr Cys Leu Val Lys Gly Phe Tyr Pro Ser Asp Ile Ala
   305                 310                 315                 320
   Val Glu Trp Glu Ser Asn Gly Gln Pro Glu Asn Asn Tyr Lys Thr Thr
                   325                 330                 335
   Pro Pro Val Leu Asp Ser Asp Gly Ser Phe Phe Leu Tyr Ser Lys Leu
               340                 345                 350
   Thr Val Asp Lys Ser Arg Trp Gln Gln Gly Asn Val Phe Ser Cys Ser
           355                 360                 365
   Val Met His Glu Ala Leu His Asn His Tyr Thr Gln Lys Ser Leu Ser
       370                 375                 380
   Leu Ser Pro Gly Lys
   385
<210> 84
   <211> 399
   <212> PRT
   <213> Artificial Sequence
<220>
   <223> Fusion protein: human C1ORF32-ECD fused to human IgG1 Fc
   with G4Sx4 linker, with C220S mutation
<400> 84
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu Gly Gly Gly Gly Ser Gly Gly Gly Gly Ser Gly Gly Gly
   145                 150                 155                 160
   Gly Ser Gly Gly Gly Gly Ser Glu Pro Lys Ser Ser Asp Lys Thr His
                   165                 170                 175
   Thr Cys Pro Pro Cys Pro Ala Pro Glu Leu Leu Gly Gly Pro Ser Val
               180                 185                 190
   Phe Leu Phe Pro Pro Lys Pro Lys Asp Thr Leu Met Ile Ser Arg Thr
           195                 200                 205
   Pro Glu Val Thr Cys Val Val Val Asp Val Ser His Glu Asp Pro Glu
       210                 215                 220
   Val Lys Phe Asn Trp Tyr Val Asp Gly Val Glu Val His Asn Ala Lys
   225 230 235 240
   Thr Lys Pro Arg Glu Glu Gln Tyr Asn Ser Thr Tyr Arg Val Val Ser
   245 250 255
   Val Leu Thr Val Leu His Gln Asp Trp Leu Asn Gly Lys Glu Tyr Lys
   260 265 270
   Cys Lys Val Ser Asn Lys Ala Leu Pro Ala Pro Ile Glu Lys Thr Ile
   275 280 285
   Ser Lys Ala Lys Gly Gln Pro Arg Glu Pro Gln Val Tyr Thr Leu Pro
   290 295 300
   Pro Ser Arg Asp Glu Leu Thr Lys Asn Gln Val Ser Leu Thr Cys Leu
   305 310 315 320
   Val Lys Gly Phe Tyr Pro Ser Asp Ile Ala Val Glu Trp Glu Ser Asn
   325 330 335
   Gly Gln Pro Glu Asn Asn Tyr Lys Thr Thr Pro Pro Val Leu Asp Ser
   340 345 350
   Asp Gly Ser Phe Phe Leu Tyr Ser Lys Leu Thr Val Asp Lys Ser Arg
   355 360 365
   Trp Gln Gln Gly Asn Val Phe Ser Cys Ser Val Met His Glu Ala Leu
   370 375 380
   His Asn His Tyr Thr Gln Lys Ser Leu Ser Leu Ser Pro Gly Lys
   385 390 395
<210> 85
   <211> 379
   <212> PRT
   <213> Artificial Sequence
<220>
   <223> Fusion protein: human C1ORF32-ECD fused to human IgG1 Fc
   no linker, with C220S and N297A mutations
<400> 85
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu Glu Pro Lys Ser Ser Asp Lys Thr His Thr Cys Pro Pro
   145                 150                 155                 160
   Cys Pro Ala Pro Glu Leu Leu Gly Gly Pro Ser Val Phe Leu Phe Pro
                   165                 170                 175
   Pro Lys Pro Lys Asp Thr Leu Met Ile Ser Arg Thr Pro Glu Val Thr
               180                 185                 190
   Cys Val Val Val Asp Val Ser His Glu Asp Pro Glu Val Lys Phe Asn
           195                 200                 205
   Trp Tyr Val Asp Gly Val Glu Val His Asn Ala Lys Thr Lys Pro Arg
       210                 215                 220
   Glu Glu Gln Tyr Ala Ser Thr Tyr Arg Val Val Ser Val Leu Thr Val
   225                 230                 235                 240
   Leu His Gln Asp Trp Leu Asn Gly Lys Glu Tyr Lys Cys Lys Val Ser
                   245                 250                 255
   Asn Lys Ala Leu Pro Ala Pro Ile Glu Lys Thr Ile Ser Lys Ala Lys
               260                 265                 270
   Gly Gln Pro Arg Glu Pro Gln Val Tyr Thr Leu Pro Pro Ser Arg Asp
           275                 280                 285
   Glu Leu Thr Lys Asn Gln Val Ser Leu Thr Cys Leu Val Lys Gly Phe
   290 295 300
   Tyr Pro Ser Asp Ile Ala Val Glu Trp Glu Ser Asn Gly Gln Pro Glu
   305 310 315 320
   Asn Asn Tyr Lys Thr Thr Pro Pro Val Leu Asp Ser Asp Gly Ser Phe
   325 330 335
   Phe Leu Tyr Ser Lys Leu Thr Val Asp Lys Ser Arg Trp Gln Gln Gly
   340 345 350
   Asn Val Phe Ser Cys Ser Val Met His Glu Ala Leu His Asn His Tyr
   355 360 365
   Thr Gln Lys Ser Leu Ser Leu Ser Pro Gly Lys
   370 375
<210> 86
   <211> 384
   <212> PRT
   <213> Artificial Sequence
<220>
   <223> Fusion protein: human C1ORF32-ECD fused to human IgG1 Fc
   with G4Sx1 linker, with C220S and N297A mutations
<400> 86
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu Gly Gly Gly Gly Ser Glu Pro Lys Ser Ser Asp Lys Thr
   145                 150                 155                 160
   His Thr Cys Pro Pro Cys Pro Ala Pro Glu Leu Leu Gly Gly Pro Ser
                   165                 170                 175
   Val Phe Leu Phe Pro Pro Lys Pro Lys Asp Thr Leu Met Ile Ser Arg
               180                 185                 190
   Thr Pro Glu Val Thr Cys Val Val Val Asp Val Ser His Glu Asp Pro
           195                 200                 205
   Glu Val Lys Phe Asn Trp Tyr Val Asp Gly Val Glu Val His Asn Ala
       210                 215                 220
   Lys Thr Lys Pro Arg Glu Glu Gln Tyr Ala Ser Thr Tyr Arg Val Val
   225                 230                 235                 240
   Ser Val Leu Thr Val Leu His Gln Asp Trp Leu Asn Gly Lys Glu Tyr
                   245                 250                 255
   Lys Cys Lys Val Ser Asn Lys Ala Leu Pro Ala Pro Ile Glu Lys Thr
               260                 265                 270
   Ile Ser Lys Ala Lys Gly Gln Pro Arg Glu Pro Gln Val Tyr Thr Leu
           275                 280                 285
   Pro Pro Ser Arg Asp Glu Leu Thr Lys Asn Gln Val Ser Leu Thr Cys
       290                 295                 300
   Leu Val Lys Gly Phe Tyr Pro Ser Asp Ile Ala Val Glu Trp Glu Ser
   305                 310                 315                 320
   Asn Gly Gln Pro Glu Asn Asn Tyr Lys Thr Thr Pro Pro Val Leu Asp
                   325                 330                 335
   Ser Asp Gly Ser Phe Phe Leu Tyr Ser Lys Leu Thr Val Asp Lys Ser
               340                 345                 350
   Arg Trp Gln Gln Gly Asn Val Phe Ser Cys Ser Val Met His Glu Ala
   355 360 365
   Leu His Asn His Tyr Thr Gln Lys Ser Leu Ser Leu Ser Pro Gly Lys
   370 375 380
<210> 87
   <211> 389
   <212> PRT
   <213> Artificial Sequence
<220>
   <223> Fusion protein: human C1ORF32-ECD fused to human IgG1 Fc
   with G4Sx2 linker, with C220S and N297A mutations
<400> 87
   Leu Gln Val Thr Val Pro Asp Lys Lys Lys Val Ala Met Leu Phe Gln
   1 5 10 15
   Pro Thr Val Leu Arg Cys His Phe Ser Thr Ser Ser His Gln Pro Ala
   20 25 30
   Val Val Gln Trp Lys Phe Lys Ser Tyr Cys Gln Asp Arg Met Gly Glu
   35 40 45
   Ser Leu Gly Met Ser Ser Thr Arg Ala Gln Ser Leu Ser Lys Arg Asn
   50 55 60
   Leu Glu Trp Asp Pro Tyr Leu Asp Cys Leu Asp Ser Arg Arg Thr Val
   65 70 75 80
   Arg Val Val Ala Ser Lys Gln Gly Ser Thr Val Thr Leu Gly Asp Phe
   85 90 95
   Tyr Arg Gly Arg Glu Ile Thr Ile Val His Asp Ala Asp Leu Gln Ile
   100 105 110
   Gly Lys Leu Met Trp Gly Asp Ser Gly Leu Tyr Tyr Cys Ile Ile Thr
   115 120 125
   Thr Pro Asp Asp Leu Glu Gly Lys Asn Glu Asp Ser Val Glu Leu Leu
   130 135 140
   Val Leu Glu Gly Gly Gly Gly Ser Gly Gly Gly Gly Ser Glu Pro Lys
   145 150 155 160
   Ser Ser Asp Lys Thr His Thr Cys Pro Pro Cys Pro Ala Pro Glu Leu
   165 170 175
   Leu Gly Gly Pro Ser Val Phe Leu Phe Pro Pro Lys Pro Lys Asp Thr
               180                 185                 190
   Leu Met Ile Ser Arg Thr Pro Glu Val Thr Cys Val Val Val Asp Val
           195                 200                 205
   Ser His Glu Asp Pro Glu Val Lys Phe Asn Trp Tyr Val Asp Gly Val
       210                 215                 220
   Glu Val His Asn Ala Lys Thr Lys Pro Arg Glu Glu Gln Tyr Ala Ser
   225                 230                 235                 240
   Thr Tyr Arg Val Val Ser Val Leu Thr Val Leu His Gln Asp Trp Leu
                   245                 250                 255
   Asn Gly Lys Glu Tyr Lys Cys Lys Val Ser Asn Lys Ala Leu Pro Ala
               260                 265                 270
   Pro Ile Glu Lys Thr Ile Ser Lys Ala Lys Gly Gln Pro Arg Glu Pro
           275                 280                 285
   Gln Val Tyr Thr Leu Pro Pro Ser Arg Asp Glu Leu Thr Lys Asn Gln
       290                 295                 300
   Val Ser Leu Thr Cys Leu Val Lys Gly Phe Tyr Pro Ser Asp Ile Ala
   305                 310                 315                 320
   Val Glu Trp Glu Ser Asn Gly Gln Pro Glu Asn Asn Tyr Lys Thr Thr
                   325                 330                 335
   Pro Pro Val Leu Asp Ser Asp Gly Ser Phe Phe Leu Tyr Ser Lys Leu
               340                 345                 350
   Thr Val Asp Lys Ser Arg Trp Gln Gln Gly Asn Val Phe Ser Cys Ser
           355                 360                 365
   Val Met His Glu Ala Leu His Asn His Tyr Thr Gln Lys Ser Leu Ser
       370                 375                 380
   Leu Ser Pro Gly Lys
   385
<210> 88
   <211> 399
   <212> PRT
   <213> Artificial Sequence
<220>
   <223> Fusion protein: human C1ORF32-ECD fused to human IgGl Fc
   with G4Sx4 linker, with C220S and N297A mutations
<400> 88
   Leu Gln Val Thr Val Pro Asp Lys Lys Lys Val Ala Met Leu Phe Gln
   1               5                   10                  15
   Pro Thr Val Leu Arg Cys His Phe Ser Thr Ser Ser His Gln Pro Ala
               20                  25                  30
   Val Val Gln Trp Lys Phe Lys Ser Tyr Cys Gln Asp Arg Met Gly Glu
           35                  40                  45
   Ser Leu Gly Met Ser Ser Thr Arg Ala Gln Ser Leu Ser Lys Arg Asn
       50                  55                  60
   Leu Glu Trp Asp Pro Tyr Leu Asp Cys Leu Asp Ser Arg Arg Thr Val
   65                  70                  75                  80
   Arg Val Val Ala Ser Lys Gln Gly Ser Thr Val Thr Leu Gly Asp Phe
                   85                  90                  95
   Tyr Arg Gly Arg Glu Ile Thr Ile Val His Asp Ala Asp Leu Gln Ile
               100                 105                 110
   Gly Lys Leu Met Trp Gly Asp Ser Gly Leu Tyr Tyr Cys Ile Ile Thr
           115                 120                 125
   Thr Pro Asp Asp Leu Glu Gly Lys Asn Glu Asp Ser Val Glu Leu Leu
       130                 135                 140
   Val Leu Glu Gly Gly Gly Gly Ser Gly Gly Gly Gly Ser Gly Gly Gly
   145                 150                 155                 160
   Gly Ser Gly Gly Gly Gly Ser Glu Pro Lys Ser Ser Asp Lys Thr His
                   165                 170                 175
   Thr Cys Pro Pro Cys Pro Ala Pro Glu Leu Leu Gly Gly Pro Ser Val
               180                 185                 190
   Phe Leu Phe Pro Pro Lys Pro Lys Asp Thr Leu Met Ile Ser Arg Thr
           195                 200                 205
   Pro Glu Val Thr Cys Val Val Val Asp Val Ser His Glu Asp Pro Glu
       210                 215                 220
   Val Lys Phe Asn Trp Tyr Val Asp Gly Val Glu Val His Asn Ala Lys
   225                 230                 235                 240
   Thr Lys Pro Arg Glu Glu Gln Tyr Ala Ser Thr Tyr Arg Val Val Ser
                   245                 250                 255
   Val Leu Thr Val Leu His Gln Asp Trp Leu Asn Gly Lys Glu Tyr Lys
               260                 265                 270
   Cys Lys Val Ser Asn Lys Ala Leu Pro Ala Pro Ile Glu Lys Thr Ile
           275                 280                 285
   Ser Lys Ala Lys Gly Gln Pro Arg Glu Pro Gln Val Tyr Thr Leu Pro
       290                 295                 300
   Pro Ser Arg Asp Glu Leu Thr Lys Asn Gln Val Ser Leu Thr Cys Leu
   305                 310                 315                 320
   Val Lys Gly Phe Tyr Pro Ser Asp Ile Ala Val Glu Trp Glu Ser Asn
                   325                 330                 335
   Gly Gln Pro Glu Asn Asn Tyr Lys Thr Thr Pro Pro Val Leu Asp Ser
               340                 345                 350
   Asp Gly Ser Phe Phe Leu Tyr Ser Lys Leu Thr Val Asp Lys Ser Arg
           355                 360                 365
   Trp Gln Gln Gly Asn Val Phe Ser Cys Ser Val Met His Glu Ala Leu
       370                 375                 380
   His Asn His Tyr Thr Gln Lys Ser Leu Ser Leu Ser Pro Gly Lys
   385                 390                 395